# EUROPEAN PATENT SPECIFICATION

(11) **EP 2 934 423 B1**
(45) Date of publication and mention of the grant of the patent: **24.10.2018**
(21) Application number: 13828963.2
(22) Date of filing: 20.12.2013
(51) Int. Cl.: A61J 7/00, A61M 5/14, F16M 11/00

(54) **SYSTEM, METHOD, AND APPARATUS FOR CLAMPING**
SYSTEM, VERFAHREN UND VORRICHTUNG ZUR KLEMMUNG
SYSTÈME, PROCÉDÉ ET APPAREIL POUR SERRAGE

(30) Priority: 21.12.2012 US 201213723242; 21.12.2012 WO PCT/US2012/071490; 21.12.2012 US 201213724568; 21.12.2012 US 201213725790; 21.12.2012 US 201213723239; 21.12.2012 WO PCT/US2012/071112; 21.12.2012 WO PCT/US2012/071142; 21.12.2012 US 201213723251; 21.12.2012 US 201213723253; 21.12.2012 US 201213723244; 21.12.2012 WO PCT/US2012/071131; 21.12.2012 US 201213723235; 21.12.2012 US 201213723238; 15.03.2013 US 201313833712; 08.07.2013 US 201361843574 P
(43) Date of publication of application: 28.10.2015
(60) Divisional application: 17152512.4
(73) Proprietor: DEKA Products Limited Partnership, Manchester, NH 03101 (US)
(72) Inventor: KAMEN, Dean, Bedford, NH 03110 (US); JANWAY, Jeffrey, M., Hooksett, NH 03106 (US); FRIEDRICH, Thomas, A., Loudon, NH 03307 (US); GRAY, Larry, B., Merrimack, NH 03054 (US); SABIN, Erik N., Manchester, NH 03104 (US); KERWIN, John, M., Manchester, NH 03104 (US); FICHERA, Stephen, L., Salem, NH 03079 (US); LANIGAN, Richard, J., Concord, NH 03301 (US)
(74) Representative: Chapman, Patrick
(86) International application number: PCT/US2013/077270
(87) International publication number: WO 2014/100744

(56) References cited:
- EP-A2- 0 966 944
- WO-A1-01/36027
- US-A1- 2007 190 826
- US-A1- 2008 116 157

## Description

### BACKGROUND

### Field of Disclosure

The present disclosure relates generally to releasably attaching an object to another object (e.g., clamping a medical device onto a pole). More particularly, the present disclosure relates to a system, method, and apparatus for mounting an object onto a pole or other support structure.

### Description of Related Art

Patient care generally involves a number of medical devices and systems that are used to monitor and treat a patient. The specific medical devices required vary with each patient and may change during the course of treatment. Medical devices often require monitoring by health care providers and so need to be easily accessible. They are often expensive, so redundancy is rarely possible, and a given device will often need to be moved to a different patient after a treatment is completed. Given their expense, medical devices need to be firmly and safely attached to a location to prevent either their damage or an interruption to patient care should they come unattached.

Medical devices are typically attached to a vertical pole located near the bedside of their assigned patient. This arrangement facilitates: the attached equipment to be customized according to patient's treatment, convenient monitoring by health care providers, minimizing the length of tubing or other connections between the patient and the device, and moving the pole and the attached equipment to follow movement of the patient. A typical attachment involves a brace fixed to the medical device and a threaded screw that can be tightened to squeeze a section of the support pole positioned between the brace and the screw. Typically, turning the screw clockwise advances the screw into the interior of the brace and attaches the medical device to the pole; counterclockwise rotation retracts the screw and allows the device to be removed. Once the advancing screw contacts the support pole, it exerts a predominantly compression-based force into the pole which holds the medical device in position against the downward pull of gravity. The user manually adjusts the clamp to poles of different diameter by varying the number of screw rotations and rotational direction of screw rotations thus controlling how far into the brace interior the screw is extended. Such positioning and adjustment faces a number of constraints, for example, it can be time consuming, there is risk of cross-threading, there is risk of human error (i.e. not tightening enough) etc.

US 2008/116157 A1 describes an apparatus for safely storing and retrieving power cords and electronic wiring associated with a mobile intravenous pole serving patients in intensive care settings wherein a plurality of devices are required. The apparatus enables the patient to be mobile without causing power cords and wiring to become tangled and dragging on patient room or hospital floors.

US 2007/190826 A1 describes a console with a support device having two vertical support tubes and an assembly device. The assembly device is used to mount at least one tray to the support device. The tray has an upper wall and a lower wall. There is at least one storage space between the walls, and the storage space is provided to accommodate electric cable, preferably along with a power plug, or another line. In this manner, interfering sections of cable or lines are avoided in the work area.

EP 0 966 944 A2 describes a mobile care cart which mates with an architectural headwall or pivoting power column that allows a hospital to create a general care patient room that can be upgraded quickly and efficiently to a critical care room upon demand without remodeling the room. The apparatus allows a general care flexible headwall or pivoting power column with services that can be upgraded or downgraded easily. In addition, the care cart supports critical care devices such as ventilators or pumps at the bedside. The care cart can be coupled to the patient bed for manually transporting the patient. During transport, the mobile care cart provides uninterrupted power for critical care devices.

WO 01/36027 A1 describes an interface device that secures an instrument to a docking station having a casing, the casing having at least one signal port and a mounting rail mounted within a recessed portion of the casing. The signal ports may be power or data communications ports. A back panel forms part of an instrument housing for housing the instrument, a first portion protrudes rearward from the back panel and a first recess is carried by the protruding portion. The first recess is dimensioned to receive the mounting rail. A rail cam is rotatably mounted within the protruding portion and is aligned with the first recess to receive and retain the mounting rail and at least one first-portion signal port carried by the first portion. The first portion is dimensioned to fit within the recessed portion of the casing such that the rail cam is positioned to receive the mounting rail and the at least one first-portion signal port is aligned, in a complementary fashion, with the at least one casing signal port. A pole clamp assembly may be positioned near the first and second portions. A pivot member is moveable between a retracted position and an extended position and a post having an axis. The post is mounted to the pivot member for axial movement and mounted thereto such that when the pivot member is retracted the axis of the post is substantially parallel with the back panel, and when the pivot member is extended the axis of the post is substantially perpendicular to the back panel.

### SUMMARY

### Clamp Mechanisms

The invention is defined in the attached independent claim to which reference should now be made. Further, optional features may be found in the sub-claims appended thereto.

In accordance with an embodiment of the present disclosure a clamp comprises a housing. The clamp may also include at least one pawl. The at least one pawl may be pivotally coupled to a pivot point. The clamp may also include a lift bar. The lift bar may be operatively coupled to the at least one pawl. The lift bar may be configured to control the at least one pawl. The clamp may also include at least one bias member operatively coupled to the housing. The at least one bias member may be configured to bias the at least one pawl toward a first position. The clamp may additionally include an actuator operatively coupled to the lift bar. The actuator may be configured move the lift bar to thereby move the at least one pawl to a second position.

In some embodiments, the said housing may include a means of coupling the clamp to a load. In some embodiments, the clamp may be configured to couple to a medical device. In some embodiments, the medical device may be an infusion pump. In some embodiments, the medical device may be a peristaltic infusion pump.

In some embodiments, the clamp may be configured such that a downward pull of gravity on the clamp causes the at least one pawl to amplify the clamping force exerted on a clamped object.

In some embodiments, the housing further comprises at least one track. In some embodiments, the housing has at least one handle.

In some embodiments, at least one of the at least one pawl further comprises a gripping surface configured to engage a clamped object. The gripping surface may be made of a material which will firmly grip, but not deform, a clamped object.

In some embodiments, at least one of the at least one bias member may be a coil spring. At least one of the at least one bias member may be a gas spring. At least one of the at least one bias member may be a torsion spring. At least one of the at least one bias member may made of a springy, compressible material. At least one of the at least one bias member may be a constant force spring.

In some embodiments, said housing includes a back plate with at least one handle coupled thereto.

The clamp may further comprise at least one track, wherein the at least one track is inclined and offset from the housing.

In still other embodiments, the clamp may further comprise at least one pawl assembly. The at least one pawl assembly may include a pawl of the at least one pawl, and the pawl may be pivotally coupled to the pawl assembly.

In some embodiments the at least one pawl assembly may further comprise a sliding wedge and the pawl may be pivotally coupled to the sliding wedge. The sliding wedge comprises an engagement surface configured for movement along the at least one track. In some embodiments the at least one pawl assembly may be slidingly coupled to the lift bar. The lift bar may be configured such that all of the at least pawl move in unison with each other.

In some embodiments, the housing may comprise a vertical groove configured for engaging with an engagement surface of the lift bar to thereby guide the movement of the lift bar.

In some embodiments, the at least one pawl may be configured to engage with a girth a variety of different clamped objects.

In some embodiments, the actuator may comprise a pull handle. The pull handle may be configured for being operated by a user so as to overcome the at least one bias member and move the at least one pawl from the first position to the second position.

In some embodiments, the housing may include at least one catch. The at least one catch may be configured to engage the actuator and hold it in one of the first and second positions.
In some embodiments, the clamp the housing may comprise a first and a second inclined track offset from a back plate. The at least one pawl may comprise a first pawl pivotally coupled to a first sliding wedge. The first sliding wedge may be configured to ride along the first track. A second pawl may be pivotally coupled to a second sliding wedge. The second sliding wedge may be configured to ride along the second track. The lift bar may be configured to slidingly couple to the first and second sliding wedges such that the lift bar thereby ensures the first and second pawls move in unison with one another. The at least one bias member may be configured to bias the lift bar to the first position. A handle may be coupled to the lift bar and configured for being operated by a user so as to overcome the at least one bias member to thereby move the first and second pawls to the second position. Additionally, a catch, may be configured to engage a notch in said handle and when engaged holds the handle in one of the first and second positions.

In some embodiments, the housing may comprises at least one vertical track.
In some embodiments at least one pair of pawls may be pivotally coupled to the housing. The at least one pair of pawls may be coupled together by the lift bar. The lift bar may ensure that the at least one pair of pawls move in unison.

In some embodiments, the said lift bar may comprise an engagement surface for movement along said track in said housing.

In some embodiments, the actuator may be a pivotal actuator handle. The pivotal actuator handle may be configured to be pulled by the user in order to move the clamp between the first position and the second position.

In some embodiments, the housing of the clamp may comprise at least one vertical track. The at least one pawl may comprise first and second pawls each pivotally coupled to the housing. The lift bar may be coupled to the first and second pawls. The lift bar may be configured to ensure the first and second pawls pivot in unison with each another. The at least one bias member may configured to bias the lift bar towards the first position. The actuator handle may be configured for being operated by a user so as to overcome the at least one bias member to move the lift bar towards the second position.

In some embodiments, the housing may comprise at least one track located on an interior surface of the housing along at least one wall of at least one hollow cavity in the housing. The at least one track may be vertical.

In some embodiments, the housing may further comprise at least one fixed gripping surface. The said housing may comprise a back plate to which the at least one fixed gripping surface is coupled. The at least one fixed gripping surface may formed of a material which will firmly grip, but not deform a clamped object.

In some embodiments, the at least one pawl may comprise only a single pawl. Opposite said single pawl may be a fixed gripping surface. The first pawl and opposite fixed gripping surface may be configured to automatically mimic the girth of a clamped object.

In some embodiments, the lift bar may comprise an engagement surface for movement along the at least one vertical track. The lift bar may couple to a single pawl. Movement of the lift bar may cause the single pawl to pivot about the single pawl's pivot point.

In some embodiments the said actuator may be a depressible trigger.

In some embodiments, the housing of the clamp may comprise at least one hollow cavity with at least one vertical track running along at least a part of an interior wall of the hollow cavity. The clamp may comprise at least one fixed gripping surface. The at least one pawl may comprise a single pawl pivotally coupled to the housing. The lift bar may comprise an engagement surface for engaging the at least one vertical track on at least a part of the interior wall of the housing. The lift bar may couple to the single pawl thereby causing it to pivot about its pivot point as the lift bar move along the at least one vertical track. The at least one bias member may be configured to bias the lift bar to the first position. The actuator may be configured for being operated by a user so as to overcome the at least one bias member thereby move the lift bar to the second position.

In accordance with an embodiment of the present disclosure, a method of making a clamp may comprise providing a housing such that the housing comprises at least one track. The method may also comprise providing at least one pawl configured for engaging a clamped object such that the at least one pawl is pivotally coupled to a pivot point. The method may also comprise providing a lift bar such that the lift bar may be coupled to the at least one pawl and such that the lift bar may be capable of controlling the movement of the at least one pawl. The method may also comprise providing at least one bias member such that the at least one bias member may be configured to bias the at least one pawl to a first position. The method may also comprise providing an actuator such that the actuator may be configured for being operated by a user so as to overcome the at least one bias member to move the at least one pawl to a second position.

In some embodiments, providing the said clamp comprises providing the said clamp for use with medical devices and accessories.

In some embodiments, providing said housing comprises providing a means of coupling to a load.

In some embodiments, providing the means of coupling to the load comprises providing the means of coupling to a load which is one of a medical device and a medical accessory.

In some embodiments, providing one of the medical device and medical accessory may comprise providing an infusion pump.

In some embodiments, providing the infusion pump may comprise providing a peristaltic infusion pump.

In some embodiments, providing said housing may comprise providing at least one handle on the housing.

In some embodiments, providing said at least one pawls may further comprise providing a gripping surface to engage a clamped object on at least a part of a surface of the at least one pawl. Providing said gripping surface may comprise providing said gripping surface being of a material which will firmly grip, but not deform the clamped object.

In some embodiments, providing the said at least one bias member may comprise providing at least one coil spring. Providing the said at least one bias member may comprise providing at least one gas spring. Providing the said at least one bias member may comprise providing at least one torsion spring. Providing the said at least one bias member may comprise providing at least one springy, compressible material.

In some embodiments, providing the housing may comprise providing a back plate with at least one handle.

In some embodiments, providing the at least one track may comprise providing the at least one track such that the at least one track is inclined and offset from the housing.

In some embodiments, providing at least one pawl may comprise providing the at least one pawl such that the at least one pawl is pivotally coupled on a pawl assembly.

In some embodiments, providing the clamp may comprise providing the at least one pawl such that the at least one pawl is pivotally coupled to a sliding wedge. Providing the sliding wedge may comprise providing the sliding wedge with an engagement surface for movement along the at least one track.

In some embodiments, providing the pawl assembly may comprise providing the pawl assembly such that the pawl assembly may be slidably coupled to the lift bar. Providing the lift bar may comprise providing the lift bar such that the lift bar is capable of moving the pawl assembly.

In some embodiments, providing the housing may comprise providing a vertical groove on the housing which engages an engagement surface on the lift bar thereby guiding the movement of the lift bar.

In some embodiments, providing the clamp may comprise providing the clamp such that the clamp is capable of automatically mimicking the girth of a variety of different clamped objects.

In some embodiments, providing the actuator may comprise providing a pull handle. Providing the pull handle may comprise providing the pull handle such that the pull handle is capable of being operated by a user so as to overcome the bias members and move the clamp from a first position to a second position.

In some embodiments, providing the housing may comprise providing at least one catch.

In some embodiments, providing the at least one bias member may comprise providing a constant force spring.

In some embodiments, providing the at least one catch may comprise providing the at least one catch such that the at least one catch is able to engage the actuator and hold the actuator in one of the first position and the second position.

In some embodiments, providing the clamp may comprise providing the housing, such that the housing comprises two inclined track offset from a back plate. Providing a first pawl assembly such that a pawl is pivotally coupled to a sliding wedge. Providing the sliding wedge may comprise providing the sliding wedge such that the sliding wedge may be able to ride along one of the inclined tracks. Providing a second pawl assembly opposite and symmetrical to the first pawl assembly such a second pawl is pivotally coupled to a second sliding wedge, and such that the second sliding wedge may able to ride along the other of the inclined tracks. Providing the lift bar such that a crosspiece of the lift bar couples to the two pawl assemblies and such that the lift bar ensures the pawl assemblies move in unison with one another. Providing the at least one bias member such that the at least one bias member biases the said clamp to a first position. Providing a handle, said handle capable of being operated by a user so as to overcome the at least one bias member and move the clamp to a second position. Providing a catch such that said catch may be capable of engaging a notch in said handle and when engaged holds clamp in either the first or second position. Providing the clamp such that the downward pull of gravity on the clamp causes the sliding wedges to move toward each other.
In some embodiments, providing the at least one track may comprise providing at least one vertical track.

In some embodiments, providing at least one pawl may comprise providing at least one pair of pawls pivotally coupled to the housing.

In some embodiments, providing the at least one pair of pawls may comprise providing the at least one pair of pawls such that the at least one pair of pawls are coupled together by the lift bar and wherein the lift bar ensures that the at least one pair of pawls move in unison.

In some embodiments, providing the lift bar may comprise providing the lift bar with an engagement surface for movement along the at least one track in the housing.

In some embodiments, providing the actuator may comprise providing a pivotal actuator handle.

In some embodiments, providing the pivotal actuator handle may comprise supporting the pivotal actuator handle such that the pivotal actuator handle may be pulled by the user toward at least one handle on the housing in order to move the clamp from the first position to the second position.

In some embodiments, providing the clamp may comprise providing the housing, such that the housing may comprise at least one pair of vertical tracks. Providing at least one pair of pawls pivotally coupled to the housing. Providing the lift bar such that the said lift bar couples to the at least one pair of pawls and wherein the lift bar ensures the at least one pair of pawls pivot in unison with one another. Providing the at least one bias member such that the at least one bias member biases the said clamp to the first position. Providing the actuator handle, said actuator handle capable of being operated by a user so as to overcome the at least one bias member and move the clamp to the second position. And providing the clamp such that the downward pull of gravity on the clamp causes the pawls of the at least one pair of pawls to pivot toward each other.

In some embodiments, providing the at least one track may comprise locating the at least one track on the interior of the housing along at least one wall of at least one hollow cavity.

In some embodiments, providing the at least one track may comprise providing the at least one track such that the at least one track is vertical.

In some embodiments, providing the housing may further comprise providing at least one fixed gripping surface on the housing.

In some embodiments, providing the housing may comprise providing a back plate to which the at least one fixed gripping surface is coupled.

In some embodiments, providing the at least one fixed gripping surface may comprise providing the at least one fixed gripping surface such that the at least one fixed gripping surface is of a material which will firmly grip, but not deform a clamped object.

In some embodiments, providing the at least one pawl may comprise providing only a single pawl.
In some embodiments, providing the single pawl may comprise providing a fixed gripping surface opposite the single pawl.

In some embodiments, providing the single pawl and opposite fixed gripping surface may comprise providing the single pawl and the opposite fixed gripping surface such that the single pawl and the opposite fixed gripping surface are capable of automatically mimicking the girth of a clamped object.

In some embodiments, providing the lift bar may comprise providing an engagement surface on the lift bar for movement along the at least one track.

In some embodiments, providing the lift bar may comprise providing the lift bar such that the lift bar couples to a single pawl and wherein movement of the lift bar causes the single pawl to pivot about the pivot point.

In some embodiments, providing the actuator may comprise providing a depressible trigger.

In some embodiments, providing the clamp may comprise providing the housing such that said housing may comprise at least one hollow cavity with at least one vertical track running along at least a part of the hollow cavity. Providing at least one fixed gripping surface. Providing the at least one pawl wherein providing the at least one pawl comprises providing a single pawl pivotally coupled to the housing. Providing the lift bar such that the said lift bar has an engagement surface for engaging the at least one vertical track, and such that the lift bar couples to the single pawl, causing it to pivot about the pivot point as the lift bar moves along the said track. Providing the least one bias member such that the at least one bias member biases the said clamp to the first position. Providing the actuator, such that said actuator is capable of being operated by a user so as to overcome the at least one bias member and move the clamp to the second position. And providing the clamp such that the downward pull of gravity on the clamp causes the single pawl to rotate toward the at least one fixed gripping surface.

In accordance with another embodiment of the disclosure, a clamp may comprise a guide plate having a first end, a second end, and a plurality of surfaces, first gripper mounted on one of the plurality of surfaces, and a second gripper slidingly coupled to one of the plurality of surfaces, said second gripper located between said first gripper and said second end. The clamp may also comprise an actuator, said actuator rotatably attached to said guide plate, the actuator configured and positioned on said guide plate such that rotation of said actuator moves said second gripper towards said first gripper. The clamp may also comprise at least one bias member configured to bias the second gripper to a first position.

In some embodiments, the at least one bias member may be a compression spring.

In some embodiments, said second gripper is mounted to a slider sled, said slider sled being in sliding connection with said guide plate and configured to allow said second gripper to move between the first position and a second position.

In some embodiments, the clamp may further comprise at least one spring support mounted to said slider sled. Said at least one spring support may comprise at least one portion with a diameter less than a diameter of said at least one compression spring. Said portion of said at least one spring support may be positioned to fit inside the diameter of said at least one compression spring.

In some embodiments, the at least one spring support may further comprise an expanded end, wherein said expanded end is an end nearest to said first gripper, and wherein said end has a diameter greater than the diameter of said at least one compression spring.

In some embodiments, the clamp may further comprise a pressure plate, said pressure plate slidingly coupled to both said slider sled and to said guide plate, and may further comprise a projection, said projection located adjacent to said actuator and positioned such that rotation of said actuator moves said projection towards said first gripper.

In some embodiments, the clamp may further comprise at least one bias member housing attached to said pressure plate. Said at least one bias member housing may be hollow and may comprise a sealed end. Said at least one bias member housing may comprise a diameter greater than the diameter of said at least one bias member.

In some embodiments, the clamp may further comprise a bias member located on said guide plate and oriented such that movement of said second gripper towards said first gripper stores mechanical energy in said bias member.

In some embodiments, the guide plate may further comprise a bias member support, said bias member support coupled to said guide plate and sized to support said bias member.

In some embodiments, at least one of said second gripper or said first gripper may be comprised of a material which will firmly grip, but not deform a clamped object.

In some embodiments, at least a part of at least one of the first gripper or second gripper may be comprised of polyurethane.

In some embodiments, at least one of said second gripper or said first gripper may be at least partially covered by a removable surface.

In some embodiments, at least one of said second gripper or said first gripper may comprise at least one approximately semi-circular or contoured face.

In some embodiments, one of the plurality of surfaces of said guide plate may comprise a support wall, said support wall supporting said first gripper. In some embodiments, the support structure may further comprise one or more buttresses, said buttresses extending from said support wall to said guide plate.

In some embodiments, said actuator may comprise a handle.

In some embodiments said actuator may comprise a cam with at least one flat segment.

In accordance with another embodiment of the present disclosure, a clamp may comprise a guide plate having a first end, a second end, and a plurality of surfaces, a first gripper coupled to one of the plurality of surfaces, a second plate slidingly coupled to one of the plurality of surfaces of the guide plate, a second gripper coupled to the second plate, and at least one bias member, said bias member coupled to both said guide plate and said second plate.

In some embodiments, the guide plate may further comprise a member adapted as a palm support. Said member may be U-shaped.

In some embodiments, the second plate may further comprise a rack. Said second plate may further comprise a second member, said second member adapted as a handle. Said handle may be U-shaped.

In some embodiments, at least one of said second gripper or said first gripper may be comprised of a material which will firmly grip, but not deform a clamped object.

In some embodiments at least one of said second gripper or said first gripper may be at least partially covered by a removable surface.

In some embodiments at least one of said second gripper or said first gripper may comprise at least one approximately semi-circular or contoured face.

In some embodiments, one of said plurality of surfaces of said guide plate may comprise a support wall, said support wall supporting said first gripper.

In some embodiments, the clamp may further comprise one or more buttresses, said buttresses extending from said support wall to said guide plate.

In some embodiments, said second plate may comprise a support wall, said support wall supporting said second gripper.

In some embodiments, the second plate may further comprise one or more buttresses, said buttresses extending from said second plate support wall to said second plate.

In some embodiments, the clamp may further comprise a pinion gear in operative engagement with said rack of said second plate.

In some embodiments, said second plate comprises an aperture through which the pinion gear project. In some embodiments, at least one edge of said aperture may comprise the teeth of said rack.

In some embodiments, the clamp may further comprise a gear shaft, said gear shaft coupled to said guide plate. Said pinion gear may rotate about the axis of said gear shaft.

In some embodiments, the clamp may further comprise a ratcheter.

In some embodiments, said ratcheter may comprise a ratcheting lever, said ratcheting lever may comprise, a ratcheting lever input structure, a ratcheting lever output structure and, a ratcheting lever hub rotatable about the axis of the gear shaft and to which the ratcheting lever input structure and output structure are coupled.

In some embodiments the input structure of the ratcheting lever may comprise a ratcheting lever handle.

In some embodiments, the output structure of the ratcheting lever may comprise one or more members. The members of the output structure may support at least one pawl.

In some embodiments, actuation of the ratcheting lever may cause the pawl to operatively engage the pinion gear through an orifice in the ratcheting lever hub.

In some embodiments, actuation of the ratcheting lever may cause the second gripper to displace from the first position toward a second position.

In some embodiments, the clamp may further comprise an over-center linkage wherein the over-center linkage is in an over-center position when the second gripper is in one of the first position and second position.

In some embodiments, the clamp may be for use with medical devices.

In some embodiments, the at least one bias member may be an extension spring.

In some embodiments, the untensioned length of said extension spring may be slightly less than the distance between an extension spring coupling point on the guide plate and an extension spring coupling point on the second plate.

In accordance with another embodiment of the present disclosure a clamp may comprise a housing having a first end, a second end, and a plurality of surfaces. The clamp may comprise a first gripper base coupled to one of said plurality of surfaces. The clamp may comprise a second gripper base slidable about one of the said plurality of surfaces, said second gripper base located between said first gripper base and said second end. The clamp may also comprise at least one bias member, an actuator , said actuator rotatably coupled to said housing, and at least one gear.

In some embodiments, the at least one of the at least one gear may be an eccentric cam gear.

In some embodiments, the first gripper may be coupled to the first gripper base and a second gripper may be coupled to the second gripper base.

In some embodiments, at least one of said mobile gripper or said fixed gripper may be comprised of a material which will firmly grip, but not deform a clamped object.

In some embodiments, at least one of said first gripper or said second gripper may be at least partially covered by a removable surface.

In some embodiments, at least one of said first gripper or said second gripper may comprise at least one approximately semi-circular or contoured face.

In some embodiments, said actuator may be a handle. The handle may be roughly L-shaped comprising a horizontal arm and a vertical arm. Said vertical arm may comprise a latch housing sized to accommodate an actuator handle latch.

In some embodiments, the latch housing comprises at least one bias member, said bias member positioned to bias said actuator handle latch to a first position.

In some embodiments, the said actuator handle latch may catch on a structure of the housing when in the first position disallowing any rotation of the actuator.

In some embodiments, the clamp may further comprise a slider sled.

In some embodiments, said slider sled may comprise at least one guide recess sized to fit a guide projection on said second gripper base.

In some embodiments, the clamp may further comprise a slider sled, said slider sled may comprise a means for a slidably coupling to said second gripper base.

In some embodiments, the clamp may further comprise at least one bias member support coupled to at least one face of said slider sled.

In some embodiments, the at least one of the at least one bias member may be a coil spring.

In some embodiments, said bias member support may comprise a projection sized to fit within a coil diameter of a compression spring.

In some embodiments, the bias member support may further comprise an end, said end may be attached to said bias member support and may have a diameter greater than said coil diameter of said compression spring.

In some embodiments, at least one of the at least one gear may be eccentrically and rotatably coupled to a gear shaft.

In some embodiments, a gear shaft may rotate when the actuator is actuated.

In some embodiments, the clamp may further comprise at least one additional cam gear, said additional cam gear may be positioned to be rotated by said gear on said gear shaft.

In some embodiments, said additional cam gear may be eccentrically and rotatably attached to said second gripper.

In some embodiments, an additional cam gear may eccentrically and rotatably attached to said slider sled.

In some embodiments, said additional cam gear may be rotatably connected to said gear by a linkage.

In some embodiments, said linkage may be a roughly claw-shaped body, said linkage may be configured to restrict the arc through which the gear and additional cam gear are capable of rotating.

In some embodiments, the clamp may further comprise a latch, said latch may be an operatively displaceable body secured to said first gripper base.

In some embodiments, said latch may comprise at least one surface that defines a catch.

In some embodiments, the latch may catch at least one portion of the actuator, disallowing further actuation of the actuator.

In some embodiments, the clamp may further comprise a latch, said latch may be an operatively displaceable body secured to said first gripper base. Said latch may comprise at least one surface defining a catch, said catch capable of engaging a portion of the horizontal arm of the handle and thereby disallowing further actuation of said handle.

In some embodiments, said latch may comprise a trough flanked by at least one sloped surface.

In some embodiments, the latch may further comprise at least one bias member configured to bias the latch to a first position.

In some embodiments, the latch may assume a second position during at least a part of actuation of the actuator.

In some embodiments, the latch may be in the first position after full actuation of the actuator and operatively engage the actuator to prevent further actuation of the actuator.

In accordance with another embodiment of the present disclosure a clip may comprise a torsion latch, said torsion latch comprising a beam having a front, a back, and a bottom. The clip may further comprise at least one spring holder, said spring holder comprising a pair of approximately circular projections attached to said bottom of said torsion latch. The clip may further comprise at least one torsion spring, said torsion spring sized to fit between said pair of approximately circular projections. The clip may further comprise at least one latch hook. The at least one latch hook may comprise a notch. The torsion latch may further be configured to pivot between a first position and a second position.

In some embodiments, the clip may be configured to attach a medical device to a support structure.

In some embodiments, the clip may further comprise a latch wedge, said latch wedge may be a triangular prism projecting from at least a portion of said front of said torsion latch.

In some embodiments, the latch may further comprise at least two latch hooks.

In accordance with another embodiment of the present disclosure a clamp may comprise a housing, first and second gripper jaws, both of said gripper jaws at least partially contained within said housing, a first bracket comprising part of said first gripper jaw, and a second bracket comprising part of said second gripper jaw, a first gripping surface coupled to at least one surface of the first bracket, a second gripping surface coupled to at least one surface of the second bracket, at least one gear, said gear operatively coupled to said first gripper jaw and said second gripper jaw, and at least one bias member attached to said housing and to at least one of the first and second gripper jaws.

In some embodiments, the at least one bias member may comprise two bias members, one of said bias members extending from said first gripper jaw to said housing, the other of said bias members extending from said second gripper jaw to said housing.

In some embodiments, the bias members may be extension springs.

In some embodiments, the first and second gripper jaws may comprise at least one toothed surface.

In some embodiments, said at least one gear is a pinion gear may operatively engage with at least one of said toothed surfaces of said first or said second gripper jaw.

In some embodiments, the clamp may further comprise a handle, said handle pivotally attached to said first gripper jaw. Said handle may be moveable between a first and a second position.

In some embodiments, the clamp may further comprise at least one linkage, said linkage may extend from said handle to said first gripper jaw.

In some embodiments, at least one of the at least one linkages may be an over-center linkage.

In some embodiments, at least one of the at least one linkages may operatively couple the handle to a cam, such that when said handle is moved to said second position, said cam pushes said first gripper jaw and said second gripper jaw closer together.

In some embodiments, the over-center linkage may be in an over-center position when the handle is in the second position.

In accordance with another embodiment of the present disclosure a clamp may comprise a base for attaching an object, said base having a centerline. The clamp may also comprise a pair of grippers, said pair of grippers oriented obliquely to said centerline of said base.

In some embodiments, the object may be a medical device.

In accordance with another embodiment of the present disclosure a clamp may comprise a housing, first gripper and second gripper, at least one of the first and second grippers being moveable, and and actuator. The actuator may be configured to actuate the moveable gripper of the first and second grippers between a first position and a second position. The clamp may further comprise at least one linkage. The at least one linkage may operatively couple the actuator to the mobile gripper. The clamp may further comprise at least one bias member configured and positioned so as to supply a clamping force when the moveable gripper of the first and second grippers is in one of the first and second positions.

In some embodiments, said at least one linkage may be an over-center linkage. The over-center linkage may be in an over-center orientation when the mobile gripper is in one of the first position and second position.

In some embodiments, said first gripper and second gripper may be oriented obliquely to a centerline of said clamp.

In some embodiments, said moveable gripper may be slidingly coupled to a driven member.

In some embodiments, said driven member may be slidingly coupled to the housing.

In some embodiments, at least two of the bias members may be compression springs, said compression springs may be positioned such that when compressed the compression springs are configured to exert a clamping force on a clamped object.

In some embodiments, at least one bias member may be a constant force spring, said at least one constant force spring may be positioned such that when unwound a clamping force is exerted against a clamped object.

In some embodiments, the actuator may be a handle.

In some embodiments, at least one of the at least one bias members may be an extension spring said extension spring attached to said handle at a first end and to said housing at a second end.

In some embodiments, said at least one extension spring may be an over-center spring and may be in an over-center orientation when the moveable gripper is in one of first position and second position.

In some embodiments the clamp may further comprise a latch, said latch may be pivotally coupled to said actuator and comprising a latch projection.

In some embodiments, said latch may be pivotable between a first position and a second position. Said latch may comprise a latch body with a plurality of faces at least one of which may further comprise at least one ergonomic feature.

In some embodiments, the latch may be biased to the first position by at least one torsion spring.

In some embodiments the clamp may further comprise a latch catch, said latch catch may be a part of one of the first gripper jaw and second gripper jaw.

In some embodiments, said latch catch may be configured to retain said latch projection when said actuator has actuated the moveable gripper to one of the first position and second position.

In some embodiments, pivoting the latch from the first position to the second position may release the latch projection from said latch catch.

In some embodiments, the clamp may be for use with medical devices and medical accessories.

In some embodiments, the housing may include a means of coupling the clamp to a load. The load may be a medical device. In some embodiments, the medical device may be a peristaltic infusion pump or syringe pump infusion pump.

In some embodiments, at least at part of at least one of the grippers may comprise a gripping surface being of a material which may firmly grip, but not deform a clamped object.

In some embodiments, the said gripping surface may be removable and/or replaceable.

In some embodiments, the said gripping surface may comprise a semi-circular or contoured face.

In one embodiment of the present disclosure, a clamp includes a housing, a fixed gripper, a driven member, a moveable gripper and an actuator. The housing includes first and second tracks. The fixed gripper is coupled to the housing. The driven member is configured to slide within the first and second tracks of the housing. The moveable gripper is operatively coupled to the driven member. The actuator is configured to move the driven member towards a first position to thereby move the moveable gripper towards the fixed gripper. The actuator is further configured to move the driven member towards a second position to thereby move the moveable gripper away from the fixed gripper. The actuator may be a handle pivotally coupled to the housing. The clamp may further include first and second linkages. The first linkage may be coupled a first side of the handle and a first side of the driven member, and the second linkage may be coupled to a second side of the handle and to a second side of the driven member.

The clamp may further comprise a gripper sled slidably coupled to the driven member. A bias member may be configured to bias the gripper sled within the driven member towards the fixed gripper.

The driven member may include a stop member configured to prevent movement of the gripper sled relative to the driven member beyond a predetermined location of the driven member. The moveable gripper may be coupled to the gripper sled.

The bias member may be a constant force spring, a compression spring, or other compressible or expandable spring.

The clamp may be configured to allow the gripper sled to stop when abutting against an object while allowing the driven member to continue to move as the actuator is further actuated.

The gripper sled may be rigidly coupled to the moveable gripper, and the clamp may further include a bias member configured to bias the gripper sled within the driven member towards the fixed gripper.

In yet another embodiment, a clamp includes a housing, a fixed gripping means, and a moveable gripping means. The fixed gripping means is for rigidly being coupled to the housing. The moveable gripping means is for gripping the clamp onto an object.

### Rack Apparatus and Rack System

In the present disclosure, a rack may include a support member that has a first end portion and a second end portion that is opposite to the first end portion. The rack may also include at least one mount. The at least one mount may be coupled to the support member and may be disposed on the support member between the first end portion and the second end portion of the support member. In addition, a clamp may be coupled to the support member, and the clamp may be configured to have a clamped position and an unclamped position.

In an exemplary embodiment, the support member may be a cylindrically shaped object, such as a pole. In certain embodiments, the at least one mount may be approximately perpendicular to the support member. The at least one mount may also be elongated in a first direction, wherein the first direction is approximately perpendicular to the support member. The at least one mount may also include a substantially planar surface. Similarly, each of the at least one mount may be a plate. Additionally, the at least one mount may be pivotally connected to the support member. The at least one mount may also be configured to rotate about a longitudinal axis of the support member. Furthermore, the at least one mount may be hingably coupled to the support member. In certain embodiments, the hinge may be configured to have an axis of rotation in a transverse plane of the support member. In other embodiments, the hinge may be configured to have an axis of rotation in a longitudinal plane of the support member. In addition, the at least one mount may be removably coupled to the support member. Alternatively, the at least one mount may be fixedly coupled to the support member. The at least one mount may also include a flange that extends upwardly from a second end of the at least one mount, wherein the second end of the at least one mount is opposite to a first end of the at least one mount.

In a preferred embodiment, the at least one mount may be configured to receive a medical device. The medical device may be attachable to any one of the at least one mount. Likewise, the medical device may be detachable from any one of the at least one mount.

The rack may further comprise a base member that may be coupled to the support member. The base member may be positioned in spaced relation to the support member and may be configured to provide a moment of force that is sufficient to counteract a moment of force about the clamp of the rack. In a preferred embodiment, the base member may be configured to abut a support structure at a resting point and thereby position the support member at a distance away from the support structure. The base member may include a notch at the resting point where the base member abuts the support structure, and the notch may have a radius of curvature. Alternatively, the base member may include a clamp that is configured to clamp onto a support structure. In a preferred embodiment, the base member may be operatively coupled to the second end portion of the support member. In embodiments where the base member is coupled to the second end portion of the support member, two or more wheels may be coupled to the base member. In certain embodiments the at least two wheels may be removably coupled to the base member. In other embodiments, a wheel assembly may couple at least two wheels to the base member. The wheel assembly may likewise be removably coupled to the base member. Furthermore, the base member may itself be configured to receive a medical device.

The clamp of the rack may include a fixed gripper and a mobile gripper. In a preferred embodiment, the clamp may be operatively coupled to the first end portion of the support member. To couple with a support structure, the mobile gripper may move in a first direction towards the fixed gripper. To decouple from the support structure, the mobile gripper may move in a second direction away from the fixed gripper. The fixed gripper and the mobile gripper may be shaped to couple with a range of different support structures. Thus, the clamp may be configured to removably couple with a support structure when the clamp is in clamped position.

To enable the at least one mount, the support member, and the clamp to be carried as a group, the rack may further comprise a handle that may be coupled to the first end portion of the support member and that may be disposed above the at least one mount. The handle may approximate the shape of a "U" and may be configured to extend in an approximately perpendicular direction to the support member.

Each of the at least one mount may also include a respective connector. The connector may be configured to interface with a medical device, such as a monitoring client. In a preferred embodiment, the respective connector of the at least mount may be configured to receive power. The power may be supplied by a power system that is configured to supply power to the respective connector of the at least one mount. The power system may be configured to receive balanced alternating-current power and to supply direct-current power to the respective connector of the at least one mount. Similarly, the power system may be configured to receive unbalanced alternating-current power and to supply direct-current power to the respective connector of the at least one mount. The power system may include a power-supply system that is operatively coupled to the support member, and each of the at least one mount may include a respective power-transmission system that is configured to provide power to the respective connector of the at least one mount. The base member may operatively include elements of the aforementioned power system.

In certain embodiments, the respective connector of the at least one mount may be configured to carry signals. To carry signals between respective connectors, each of the at least one mount may include a respective support-plate bus that is connected to the respective connector therein, and the respective support-plate bus may interface with a central bus that is operatively coupled to the support member.

Substantially rigid materials such as aluminum alloys, stainless steel alloys, steel alloys, and engineering polymers may be used to construct the rack and components like the at least one mount, the support member, the base member, and the clamp. In addition, at least a portion of the support member, the at least one mount, the base member, and the clamp may include an antibacterial, an antimicrobial, or an antiviral coating.

A rack system may include the rack described above. The rack system may further comprise at least one device that may be adapted to be received by any one of the at least one mount of the rack. The device may further include a clamp mechanism that is configured to operatively and removably couple with the support member of the rack. In addition, the device may include a connector that may be configured to electrically communicate with the respective connector of any one of the at least one mount. The clamp mechanism of the at least one device may comprise any one of the clamp mechanisms described above. In a preferred embodiment of the rack system, the device may be a medical device.

In some embodiments of a rack apparatus, the rack apparatus may comprise at least one support pole having a first end portion and a second end portion, at least one of a clamp assembly, hanger, or handle attached to the first end portion of the support pole, at least one mount connector, at least one alignment feature operatively coupled to the support pole; and a base member attached to the second end portion of the support pole.

The at least one mount connector may configured to operatively engage with a medical device connector. At least one of the at least one alignment feature may be configured to align the medical device connector with the at least one of the at least one mount connector. At least one of the at least one alignment feature may be included on a collar on the support pole.

The rack apparatus may further comprise a power system. The power system may be configured to provide power to at least one medical device via the at least one mount connector. The rack apparatus further may comprise a communication system configured to allow an attached medical device to communicate with at least one other attached medical device via the at least one mount connector. The communication system may be configured use at least one of a CANbus protocol and USB protocol.

In some embodiments, the base member may comprise, a power connector, a power supply, a main power cable electrically connecting the power connector and the power supply, and at least one transmission cable connecting the power supply and the at least one mount connector.

The support pole may be configured to accept a clamp. In such embodiments, the clamp may comprise
a housing including first and second tracks, a fixed gripper coupled to the housing, a driven member configured to slide within the first and second tracks of the housing, a moveable gripper operatively coupled to the driven member, and an actuator configured to move the driven member towards a first position to thereby move the moveable gripper towards the fixed gripper, the actuator further configured to move the driven member towards a second position to thereby move the moveable gripper away from the fixed gripper.

The clamp may be configured to couple a medical device to the support pole of the rack apparatus. A medical device connector may be disposed on the clamp and may be configured to operatively engage with at least one of the at least one mount connector to receive at least one of a network connection and power for the medical device.

The rack apparatus may be configured to couple to an IV pole. The at least one support pole may be an IV pole. The base member further may comprise at least one wheel.

In some embodiments, the rack apparatus may be part of a system comprising the rack apparatus. In such embodiments, the rack apparatus may comprise at least one support pole having a first end portion and a second end portion, at least one of a clamp assembly, hanger, or handle attached to the first end portion of the support pole, at least one mount connector, at least one alignment feature operatively coupled to the at least one support pole; and a base member attached to the second end portion of the support pole. The system may also comprise a clamp configured to clamp to the at least one support pole. The clamp may comprise a housing including first and second tracks, a fixed gripper coupled to the housing, a driven member configured to slide within the first and second tracks of the housing, a moveable gripper operatively coupled to the driven member; and an actuator configured to move the driven member towards a first position to thereby move the moveable gripper towards the fixed gripper, the actuator further configured to move the driven member towards a second position to thereby move the moveable gripper away from the fixed gripper.

In some embodiments the at least one of the at least one mount connector may be configured to operatively engage with a medical device connector. At least one of the at least one alignment feature may be configured to align the medical device connector with the at least one of the at least one mount connector. The medical device connector may be disposed on one of the fixed gripper or movable gripper of the clamp. The medical device connector may be configured to operatively engage with the at least one of the at least one mount connector to receive at least one of a network connection and power for a medical device. The medical device may be a monitoring client comprising a tablet computer. The medical device may be an infusion pump. The medical device may be a PCA. The medical device may be a physiological monitor.

### Protective Mechanisms

In some embodiments of the present disclosure, the connectors may be disposed on protective mechanisms that may be coupled to the rack. In one specific embodiment, a protective mechanism includes: a guide member; a connector that is coupled to the guide member; an actuation member having first and second end portions, wherein the first end portion of the actuation member is pivotally coupled to the guide member; and a cover member that may be pivotally coupled to the guide member. The cover member may be configured to interact with the actuation member so as to pivot to thereby uncover the connector when the actuation member pivots in a first direction and to pivot to cover the connector when the actuation member pivots in a second, opposite direction. The protective mechanism may also include a backstop member that is disposed on a guide member face. The backstop member may also have a backstop member face that is approximately perpendicular to the guide member face. The connector may be disposed on the backstop member face.

In a specific embodiment, the cover member of the protective mechanism may be adapted to uncover the connector when the actuation member pivots from a first position to a second position. The cover member may be adapted to cover the connector when the actuation member pivots in a second, opposite direction from the second position to the first position. The backstop member face may define a recess or an aperture that is configured to receive at least a portion of the cover member when the cover member is completely uncovered and the actuation member is in the second position.

In yet another embodiment of the present disclosure, the protective mechanism may further comprise a compliant gasket that may be coupled to the backstop member and configured to encompass the connector. The cover member may include a perimeter rib that may be adapted to seal against the compliant gasket when the actuation member is in the first position and the connector is covered by the cover member. In some embodiments, the compliant gasket may include a first portion, a second portion, and a transitional portion between the first and second portions of the compliant gasket; the perimeter rib may be adapted to seal against at least the first portion of the compliant gasket when the actuation member is in a first position and the connector is covered, and the perimeter rib may be adapted to seal against at least the second portion of the compliant gasket when the actuation member is in the second position and the connector is uncovered.

To receive a device, the protective mechanism may further comprise first and second rail projections. Each rail projection may have a web portion and a wider head portion such that the web portion couples the head portion to the guide member face. The first and second rail projections may be disposed on the guide member face such that they are approximately parallel and in spaced relation to one another. Between the first and second rail projections, the guide member may define an aperture that extends from a first guide member face (the aforementioned guide member face) to a second guide member face, or the guide member may define a recess that is disposed on the guide member face. Both the recess and the aperture may be adapted to receive a portion of the actuation member.

The actuation member of the protective mechanism may include a sloped face that defines a sloped portion of the actuation member. In some embodiments, due to the sloped face, the sloped portion of the actuation member may increase in cross-sectional area from the first end portion of the actuation member to a point where the sloped face ends between the first and second end portions of the actuation member. The sloped face may slope such that the sloped face protrudes from the plane of the guide member face when the actuation member is in a first position and when the actuation member is in a second position. The sloped face may lie substantially in the plane of the face guide member face. The protective mechanism may further comprise at least one actuation spring that may bias the actuation member such that the actuation member may automatically return to the first position under the force of the actuation spring. Thus, the at least one actuation spring may have a first end that is coupled to the actuation member and a second end that is coupled to the guide member.

The protective mechanism may also include a latch member that may be pivotally coupled to the guide member at a pivot point between first and second end portions of the latch member. Additionally, the latch member may define an aperture that is capable of receiving at least a portion of the actuation member, and the latch member may include a latch projection that may be disposed on the first end portion of the latch member and protrude from the face of the guide member when the latch member is in a latched position. Like the actuation member, the latch member may be adapted to automatically return to the latched position under the force of one or more latch springs. Thus, each of the one or more latch springs may have a first end that is coupled to the backstop member and a second end that is coupled to the latch member at a point between the pivot point and the second end portion of the latch member. To arrest pivotal movement of the latch member under the force of the at least one latch spring, the protective mechanism may further comprise at least one arrester projection that may be coupled to the guide member and disposed in spaced relation to the pivot point of the latch member such that the at least one arrester projection may arrest pivotal movement of the latch member when the latch member pivots to the latched position.

In an embodiment of the present disclosure, the cover member is a protective member that includes a cover portion. The protective member may have a first end portion that is coupled to one of the guide member and the backstop member, and the protective member may have a second end portion that includes the cover portion. To protect the connector, the protective member may be adapted to engage with the actuation member such that pivotal movement of the actuation member in a first direction from the first position to the second position may cause the protective member to pivot from a protective position to a non-protective position, and thereby uncover the connector. Likewise, pivotal movement of the actuation member in a second, opposite direction from the second position to the first position may cause the protective member to pivot from the non-protective position to the protective position to thereby cover the connector.

The actuation member of the first embodiment may include first and second channeled projections disposed on the second end portion of the actuation member. The first and second channeled projection may be spaced apart such that a portion of the protective member may be received between them. To enable the actuation member to actuate the protective member, the protective member may include first and second actuation projections that are adapted to respectively engage the first and second channeled projections of the actuation member. Thus, the first and second channeled projections may respectively include a first channel and a second channel, wherein each channel is shaped and sized such that pivotal movement of the actuation member form the first position to the second position may cause the protective member to pivot from the protective position to the non-protective position.

In some embodiments of the present disclosure, the protective mechanism may include a latch member. Furthermore, the first embodiment of the protective mechanism may include a latch member that defines a latch aperture between the first end portion of the latch member and the pivot point of the latch member. The latch aperture may be configured such that the actuation member may pass through the latch aperture when the actuation member is in the first position, and the latch aperture may receive at least the cover portion of the protective member when actuation member is in the second position and the protective member is in the non-protective position.

The first embodiment of the protective mechanism may further comprise a compliant gasket that may be coupled to the backstop member and encompass the connector. To seal the connector within the compliant gasket, the cover portion of the protective member may include a perimeter rib that is adapted to seal against the compliant gasket when the protective member is in the protective position.

A second embodiment of the protective mechanism may differ from the first embodiment of the protective mechanism. The second embodiment of the protective mechanism may include at least one first link-member and at least one second link-member. Each of the at least one first link-member may be configured to have a respective first end portion and a respective second portion such that the respective first end portion may be pivotally coupled to the second end portion of the actuation member. Likewise, each of the at least one second link-member may have a respective first end portion and respective second portion. The respective first end portion of the at least one second link-member may be pivotally coupled to each of the backstop member at a first point and the respective second end portion of a respective at least one first link-member at a second point. The first point and the second point may be disposed in spaced relation such that pivotal and substantially translational movement of the second end portion of the actuation member may be transmitted through the at least one first link-member and thus cause the at least one second link-member to pivot about the first point.

The cover member of the second embodiment of the protective mechanism may be pivotally coupled to the respective second end portion of the at least one second link-member such that the cover member may pivot to a non-protective position, and thereby uncover the connector, when the actuation member pivots in the first direction and to pivot to a protective position, and thereby cover the connector, when the actuation member pivots in the second direction. To enable the at least one second link-member to couple with the cover member, the second embodiment of the protective mechanism may further comprise an at least one pass-thru aperture defined by the backstop member, and each of the at least one second link-member may be disposed within a respective at least one pass-thru aperture.

In yet another embodiment of the present disclosure, the protective mechanism may include a compliant gasket. The compliant gasket may be coupled to the backstop member and have a first portion, a second portion, and a transition portion between the first portion and the second portion of the compliant gasket. The first portion of the compliant gasket may be configured to encompass the connector. The transition portion of the compliant gasket may be configured to encompass each of the at least one pass-thru aperture. And the second portion of the compliant gasket may be configured to mirror the first portion of the compliant gasket.

To seal against the compliant gasket, the cover member may include a perimeter ridge. The perimeter ridge may be adapted to compress the first portion of the compliant gasket and a portion of the transition portion such that the perimeter ridge encompasses the connector and each of the at least one pass-thru apertures when the cover member is in the protective position. When the cover member is in the non-protective position, the perimeter ridge may be adapted to seal against the compliant gasket such that the perimeter ridge compresses the second portion of the compliant gasket and a portion of the transition portion such that the perimeter ridge encompasses each of the at least one pass-thru aperture.

The protective mechanism may further include a backstop member recess or a backstop member aperture defined by the backstop member face. The backstop member recess and the backstop member aperture may be configured to receive at least a portion of the cover member when the cover member is in the non-protective position.

The protective mechanism may be one embodiment of a component of a system for receiving a device. The system for receiving a device may comprise at least one protective mechanism and at least one receivable device. The at least one protective mechanism may include a guide member having a guide member face, a connector that is coupled to the guide member, an actuation member, a cover member, and at least one rigid member disposed on the guide member. The actuation member may be configured to have a first end portion and a second end portion, wherein the first end portion may be pivotally coupled to the guide member, and thus, the actuation member may pivot in a first direction from a first position to a second position and in a second direction from the second position to the first position. The cover member may be pivotally coupled to the guide member and adapted to interact with the actuation member so as to pivot to uncover the connector when that actuation member pivots in the first direction and to pivot to cover the connector when the actuation member pivots in the second, opposite direction. In any embodiment described herein, the protective mechanism may be adapted to be a protective mechanism of the system for receiving a device.

The at least one receivable device of the system for receiving a device may comprise a connector and an at least one channel. The connector may be disposed on the receivable device such that it is capable of interfacing with the connector of the at least one protective mechanism. The at least one channel may be disposed on the receivable device and configured to receive the at least one rigid member of a respective at least one protective mechanism. The at least one receivable device may further comprise a respective latch recess defined by a respective face of the at least one receivable device. The respective latch recess may be adapted to engage with a latch member projection of the at least one protective mechanism such that the at least one receivable device is securely received by the at least one protective mechanism.

In one example embodiment, a clamp apparatus is depicted. The clamp apparatus may comprise a body, a first handle and a second handle. The first handle and the second handle may be operatively coupled to the body. The clamp apparatus also includes a first movable gripper and a second movable gripper. The first movable gripper and the second movable gripper are coupled to the first handle and the second handle, respectively. In one example embodiment, the body is positioned intermediately between the handles and the grippers. The first handle and the second handle are fixedly coupled to the first movable gripper and the second movable gripper, respectively, thereby controlling the movement of the first movable gripper and the second movable gripper. The clamp apparatus also includes a first gear set and a second gear set that are rotatably coupled to the body, and operatively coupled to the first handle and the second handle, respectively, as well as the first movable gripper and the second movable gripper, respectively. The first gear set and the second gear set are configured to operatively engage one another. In one example embodiment, the first gear set may include an upper first gear, and a lower first gear that is fixedly coupled to the upper first gear, such that the upper first gear and the lower first gear move together in unison. Similarly, the second gear set may include an upper second gear, and a lower second gear that is fixedly coupled to the upper second gear, such that the upper second gear and the lower second gear move together in unison. The upper first gear and the lower first gear may be configured to operatively engage the upper second gear and the lower second gear, respectively.

The clamp apparatus also includes at least one bias member operatively engaged with the first handle and the second handle. The at least one bias member is configured to bias the first handle and the second handle toward a first position. The first movable gripper and the second movable gripper are engaged with one another, defining a clamped position, when the first handle and the second handle are in the first position. The first handle and the second handle are configured to thereby move, under actuation, to a second position, whereby the first movable gripper and the second movable gripper are disengaged from one another, defining an unclamped position.

In some embodiments, the clamp apparatus further comprises a gripping surface on the first movable gripper and the second movable gripper, configured to engage a clamped object. In some embodiments, the grippers are configured to clamp onto a pole. In one example embodiment, the clamp apparatus is for use with medical devices and medical accessories. In one example embodiment, the clamp apparatus is configured to couple a medical device to a support pole. The pole may be an IV pole. The medical device may be a monitor comprising a tablet computer. In one example embodiment, the clamp apparatus is configured to couple an infusion pump to a support pole. The infusion pump may be a peristaltic infusion pump. In one example embodiment, the clamp apparatus is capable of automatically mimicking the girth of a variety of different clamped objects.

In one example embodiment, at least part of at least one of the first movable gripper and the second movable gripper may be comprised of a material which will firmly grip, but not deform a clamped object. In some embodiments, at least a part of at least one of the first movable gripper and the second movable gripper may be comprised of polyurethane. In some embodiments, at least part of at least one of the grippers may be comprised of rubber, or may be coated in a rubbery, gripping material. In some embodiments, at least one of the first movable gripper and the second movable gripper may be at least partially covered by a removable surface. In some embodiments, at least one of the first movable gripper and the second movable gripper may comprise at least one approximately arcuate, semi-circular, or contoured face at least on the gripping surface.

In one example embodiment, at least a part of at least one of the first movable gripper and the second movable gripper has fingers. In one example embodiment, the first movable gripper and the second movable gripper both have fingers. The fingers of the first movable gripper and the second movable gripper may be interdigitated when the grippers are engaged with one another, corresponding to the handles being in the first position. The fingers of each gripper may be partially interdigitated due to partial engagement of the grippers with one another, corresponding to the handles being in an intermediate position between the first and second positions.

In some embodiments, the at least one bias member is a spring. Further, the at least one bias member may be a flat spring or a leaf spring. In one example embodiment, the at least one bias member may be at least one array of multiple bias members. Further, the at least one bias member may be an array of multiple flat springs arranged in a layered configuration. In one example embodiment, the at least one bias member may be made of a flexible, compressible material. In some embodiments, the at least one bias member may comprise a first bias member and a second bias member. In one embodiment, the first bias member may be a first bias member array, including multiple individual bias members, and the second bias member may be a second bias member array, also including multiple individual bias members. In one example embodiment, the first and/or second bias members may include a single bias member. Additionally, the first and second bias members or the individual bias members and may be springs, or, in one example embodiment, may be torsion springs.

In one example embodiment, the first handle and the second handle of the clamp apparatus may be paddles. In one example embodiment, the handles may be concave shaped away from or towards the body, the handles being actuatable. The handles may be configured to be pulled by a user from a first side, or pushed by the user from a second side, in order to move the grippers from the first position to the second position. In some embodiments, the first handle and the second handle may further comprise a palm support. The member adapted as a palm support may be U-shaped. In one example embodiment, the first handle and a second handle may provide a pair of pull handles. The handles are configured for operation by a user so as to overcome the bias member array and actuate the first movable gripper and the second movable gripper from the first position to the second position.

In one example embodiment, the clamp apparatus comprises a third gear set and a fourth gear set, the gears operatively coupled to the first handle and the second handle, respectively, and rotatably coupled to the body. In one example embodiment, the third and fourth gear sets may share an axis of rotation with the first gear set and the second gear set, respectively. The third gear set and the fourth gear set may be operatively coupled to the first movable gripper and the second movable gripper, respectively. The third and fourth gears may be configured to operatively engage a locking mechanism in association with the handles. The locking mechanism comprises a first hook, a first catch, a second hook, and a second catch. In one example embodiment, the third and fourth gears may be operatively engaged with one another.

In one example embodiment, the handles and gears may be configured to permit slight initial rotational movement of the handles in advance of subsequent rotational movement of the grippers, when moving the first and second handles from the first position to the second position. Similarly, the handles and gears may be configured to permit slight additional rotational movement of the handles following the stoppage of the rotational movement of the grippers, when moving the first and second handles from the second position back to the first position. The initial slight rotational movement of the handles may perform an unlocking function, freeing the grippers to move.

In accordance with an embodiment of the present disclosure, a clamp comprises a body, the body having a first end and a second end. The clamp may also include a lever, the lever operatively coupled to the first end of the body. The clamp may also include a movable gripper. The movable gripper may be coupled to an intermediate portion of the body, between the first end and second end. The clamp may also include at least two fixed grippers. The fixed grippers may be positioned at the second end of the body. The fixed grippers may be configured to approximately oppose the movable gripper such as to secure a pole from opposing sides. The clamp may also include a connector member. The connector member may have a first end operatively coupled to the lever and a second end operatively coupled to the movable gripper.

In some embodiments, the movable gripper may be rotatable about a coupling point of the intermediate portion of the body. The movable gripper may also be approximately wedge-shaped, having a narrow end and a wide end. The narrow end of the movable gripper may be coupled to the body, and the movable gripper may be rotatable about the narrow end. The wide end of the movable gripper may have a ridged surface. Further, the ridged surface may extend along the wide end of the wedge-shaped movable gripper. The wide end of the movable gripper may have a semi-circular or contoured face opposing the at least two fixed grippers. The wide end of the movable gripper may also be configured to complement the shape of a pole.

In some embodiments, the grippers further comprise gripping surfaces configured to engage a clamped object. The gripping surfaces may be made of a material which will firmly grip, but not deform, a clamped object. In some embodiments, the grippers are configured to close onto a pole. In some embodiments, the grippers may be rubber. The grippers may be coated in a rubbery, gripping material.

In some embodiments, providing the body may comprise providing a back plate to which the at least two fixed grippers are coupled.

In some embodiments, providing the movable gripper and opposite fixed grippers may comprise providing the movable gripper and the opposite fixed grippers such that the movable gripper and the opposite fixed grippers are capable of automatically mimicking the girth of a clamped object.

In some embodiments, the connector member may be configured to rotate the movable gripper upon actuation of the connector member.

In some embodiments, the connector member includes a bias member. The bias member may be a spring, and in some embodiments the spring may be a compression spring. The biased member may be a compressible or expandable spring. In some embodiments, the connector member includes a piston. The piston may be a spring-biased piston. The bias member may be oriented such that movement of the connector member towards the movable gripper stores mechanical energy in the bias member.

In some embodiments, the connector member may be rotatably connected to the lever at the first end of the connector member. The connector member may be coupled to a lever joint, the lever joint positioned at an end of the lever. The connector member may also be rotatably connected to the movable gripper at the second end of the connector member. The connector member may be configured to, under actuation of the lever, extend towards the movable gripper, thereby rotating the movable gripper towards the fixed grippers.

In some embodiments, the clamp may further comprise a bias member support coupled to the connector member. The bias member support may have a portion with a diameter less than a diameter of the bias member. The portion of the bias member support may be positioned to fit inside the diameter of the bias member.

In some embodiments, the clamp may further comprise a bias member housing coupled to the connector member. The bias member housing may be hollow and may have a sealed end. The bias member housing may have a diameter greater than the diameter of the bias member. In some embodiments, the lever of the clamp may be a handle. The lever may be configured to, under actuation, rotate towards the body of the clamp. The lever may include a lever joint at an end of the lever. The lever joint may be a cam, such that when the lever is moved to the first position, the cam pushes the connector member, thereby pushing the movable gripper closer to the fixed grippers. The lever is configured to move the connector member towards a first position and thereby move the movable gripper towards the fixed grippers. The lever is further configured to move the connector member towards a second position and thereby move the movable gripper away from the fixed grippers. The clamp may be configured to allow the moveable gripper to stop when abutting against an object while allowing the connector member to continue to move as the lever is further actuated.

In some embodiments, the lever may include a slideable ring coaxially aligned with and surrounding the top end of the lever, the top end being nearest a lever joint. The slideable ring may be configured to free the lever from a locked position. The slideable ring may be configured to slide out of a notch in the lever joint, thereby unlocking the lever and freeing the lever to rotate. The slideable ring may include a ring bias member, the ring bias member configured to bias the slideable ring to a notched position. The ring bias member may be a compression or expansion spring.

In still other embodiments, the clamp may further comprise a locking assembly, the locking assembly configured to interact with the movable gripper. The locking assembly may include a pawl, and the pawl may include a pawl bias member. The bias member may be a spring, and in some embodiments the bias member may be a torsion spring. The pawl may be rotatably coupled to the locking assembly, the pawl configured to rotate into contact with an upper ridged surface of the movable gripper, locking the gripper in place.

In some embodiments the locking assembly may further comprise a slideable member, and the pawl may be in contact with the slideable member. The slideable member may have a first end in contact with the lever joint. The slideable member may contact an outer surface of the lever joint, the outer surface having a depressed portion. The locking assembly may be configured to move the slideable member into the depressed portion of the lever joint, allowing the pawl to rotate into contact with the movable gripper and thereby locking the movable gripper in place.

In some embodiments, the clamp may be for use with medical devices and medical accessories.

In some embodiments, the body may include a means of coupling the clamp to a load. The load may be a medical device. In some embodiments, the medical device may be a peristaltic infusion pump or syringe pump infusion pump.

In some embodiments, the clamp may be configured to couple a medical device to a support pole. The pole may be an IV pole. The medical device may be a monitor comprising a tablet computer.

### BRIEF DESCRIPTION OF THE DRAWINGS

These and other aspects will become more apparent from the following detailed description of the various embodiments of the present disclosure with reference to the drawings wherein:
FIGS. 1A-1E show several views of a clamp in accordance with an embodiment of the present disclosure;
FIGS. 2A-2E show several views of a clamp in accordance with an embodiment of the present disclosure;
FIGS. 3A-3E show several views of a clamp in accordance with an embodiment of the present disclosure;
FIGS. 4A-4D show several views of a clamp in accordance with an embodiment of the present disclosure;
FIGS. 5A-5D show several views of a clamp in accordance with an embodiment of the present disclosure;
FIGS. 6A-6G show several views of a clamp in accordance with an embodiment of the present disclosure;
FIGS. 7A-7D show several views of a clamp in accordance with an embodiment of the present disclosure;
FIGS. 8A-8D show several views of a clamp in accordance with an embodiment of the present disclosure;
FIGS. 8E-8F show an alternate embodiment of the clamp shown in FIGS. 8A-8D in accordance with an embodiment of the present disclosure;
FIG. 8G shows an alternate embodiment of a moveable gripper assembly with a housing in accordance with an embodiment of the present disclosure;
FIG. 9A is a perspective view of an exemplary embodiment of a rack apparatus in accordance with an embodiment of the present disclosure;
FIG. 9B is a perspective view of an exemplary embodiment of a device mount, like those depicted in FIG. 9a, wherein the device mount includes a support plate that is adapted to receive a medical device in accordance with an embodiment of the present disclosure;
FIG. 9C is a perspective view of an exemplary embodiment of a joint member that is adapted to couple with the embodiment of a device mount that is depicted in FIG. 9b in accordance with an embodiment of the present disclosure;
FIG. 9D is a perspective view of an exemplary embodiment of a base member that includes a power system that is configured to transmit power to at least one device mount like the embodiment depicted in FIG. 9b in accordance with an embodiment of the present disclosure;
FIG. 9E depicts a perspective view of another exemplary embodiment of a rack apparatus in accordance with an embodiment of the present disclosure;
FIG. 9F is a perspective view of yet another exemplary embodiment of a rack apparatus in accordance with an embodiment of the present disclosure;
FIG. 9G-H are perspective views of an example collar of a rack apparatus in accordance with an embodiment of the present disclosure;
FIG. 9I is a perspective view of an example rack apparatus including a number of mount connectors in accordance with an embodiment of the present disclosure;
FIG. 9J is a perspective view of an example rack apparatus in accordance with an embodiment of the present disclosure;
FIG. 9K is an enlarged view of a portion of the support pole of the example rack apparatus depicted in FIG. 9j in accordance with an embodiment of the present disclosure;
FIG. 10A is a perspective view of an exemplary embodiment of a rack system, wherein the embodiment of a rack depicted in FIG. 9A includes a support pole adapted to couple with the clamp of a medical device in accordance with an embodiment of the present disclosure;
FIG. 10B is a close-up, perspective view of the exemplary embodiment of a rack system depicted in FIG. 10A, wherein the rack embodiment includes a mount connector that may couple to a device connector when the medical device couples with the support pole in accordance with an embodiment of the present disclosure; and
FIG. 10C is another alternate perspective view of the exemplary embodiment of a rack system depicted in FIG. 10A, wherein an embodiment of a medical device includes an embodiment of a device connector that may couple to a mount connector, like the embodiment depicted in FIG. 10B, when the medical device couples with the support pole in accordance with an embodiment of the present disclosure.
FIG. 10D is a perspective view of an exemplary embodiment of a rack system including the embodiment of a rack depicted in FIG. 9e in accordance with an embodiment of the present disclosure;
FIG. 10E is a close-up perspective view of an example infusion pump with a flange in place in the guide trough of a support plate of the example rack embodiment depicted in FIG. 9e in accordance with an embodiment of the present disclosure;
FIG. 10F is a perspective view of an exemplary embodiment of a rack system, wherein the embodiment of a rack depicted in FIG. 9f includes a support pole adapted to couple with the clamp of a medical device in accordance with an embodiment of the present disclosure;
FIG. 10G depicts a perspective view of a support pole of a rack system, wherein the support pole includes a number of mount connectors on a mount connector strip in accordance with an embodiment of the present disclosure;
FIG. 10H depicts a perspective view of a number of mount connectors on a mount connector strip in accordance with an embodiment of the present disclosure;
FIG. 10I depicts a view of a part of an example support pole of an example rack system, wherein the support pole includes a mount connector on a mount connector strip in accordance with an embodiment of the present disclosure;
FIG. 10J shows a side view of an example mount connector coupled to a mount connector strip in accordance with an embodiment of the present disclosure;
FIG. 10K shows a perspective view of an example gripper of a clamp apparatus, wherein the example gripper includes a device connector in accordance with an embodiment of the present disclosure;
FIG. 10L is a close-up perspective view of an exemplary embodiment of a rack system, wherein the embodiment of a rack depicted in FIG. 9F includes a support pole adapted to couple with the clamp of a medical device in accordance with an embodiment of the present disclosure;
FIG. 10M is a perspective view of an exemplary embodiment of a rack system, wherein the embodiment of a rack depicted in FIG. 9J includes a support pole adapted to couple with the clamp of a medical device in accordance with an embodiment of the present disclosure; and
FIG. 10N depicts a close-up perspective view of an exemplary embodiment of a rack system, wherein the embodiment of a rack depicted in FIG. 9J includes a support pole adapted to couple with the clamp of a medical device in accordance with an embodiment of the present disclosure.
FIG. 11A depicts an embodiment of a pivotable cover mechanism in accordance with an embodiment of the present disclosure;
FIG. 11B depicts an actuation spring of an example embodiment of the pivotable-cover mechanism depicted in FIG. 11A acting on the sloped portion of an actuation member, wherein the actuation spring is disposed in an actuation spring pocket in accordance with an embodiment of the present disclosure;
FIG. 11C depicts an example embodiment of the pivotable-cover mechanism wherein a mount connector is covered by a protective member in accordance with an embodiment of the present disclosure;
FIG. 11D depicts an embodiment of a pivotable cover mechanism wherein the protective member is in a non-protective position in accordance with an embodiment of the present disclosure;
FIGS. 11E-I depict several cross-sectional views of an embodiment of a pivotable cover mechanism in accordance with an embodiment of the present disclosure;
FIG. 11J depicts a view of an example embodiment of the pivotable cover mechanism, wherein the protective member is shown in a protective and a non-protective position and wherein the mount connector is of a type having multiple spring contacts in accordance with an embodiment of the present disclosure;
FIG. 11K depicts a cross-sectional view of part of the pivotable cover mechanism, wherein the mount connector is of a type having multiple spring contacts in accordance with an embodiment of the present disclosure;
FIG. 12A depicts a view of an embodiment of a clamshell mechanism wherein the cover member is in a non-protective position in accordance with an embodiment of the present disclosure;
FIG. 12B depicts a view of an embodiment of a clamshell mechanism wherein the cover member is in a protective position in accordance with an embodiment of the present disclosure;
FIG. 12C depicts a close-up perspective view of a part of an embodiment of a clamshell mechanism in accordance with an embodiment of the present disclosure;
FIG. 12D depicts a cross sectional view of a clamshell mechanism wherein the cover member includes a perimeter rib in accordance with an embodiment of the present disclosure;
FIGS. 12E-H depict a number of cross-sectional views of a clamshell mechanism in accordance with an embodiment of the present disclosure;
FIG. 12I-J depict a number of views an exemplary embodiment of a series of the clamshell mechanisms with compliant gasket systems in accordance with an embodiment of the present disclosure;
FIG. 13A depicts an embodiment of an example receivable device in accordance with an embodiment of the present disclosure;
FIG. 13B depicts an example embodiment of a receivable device that includes first and second channels and a latch recess in accordance with an embodiment of the present disclosure;
FIG. 13C depicts a cross-section view of the example pivotable cover mechanism of FIG. 11a wherein a latch member projection is in engagement with a latch recess of an example embodiment of a receivable device in accordance with an embodiment of the present disclosure;
FIG. 13D-G depict a number of cross-section views of an example receivable device and the example clamshell mechanism of FIG. 12a wherein the progression of FIGS. 13d-g demonstrates how receiving a receivable device may cause a clamshell mechanism to automatically reveal a mechanism connector in accordance with an embodiment of the present disclosure;
FIGS. 14A-14E show several views of a clamp in accordance with an embodiment of the present disclosure;
FIGS. 15A-15D show several views of a clamp in accordance with an embodiment of the present disclosure;
FIGS. 16A-16E show several views of a clamp in accordance with an embodiment of the present disclosure; and
FIGS. 17A-17D show several views of a clamp in accordance with an embodiment of the present disclosure.

### DETAILED DESCRIPTION

### CLAMP MECHANISMS

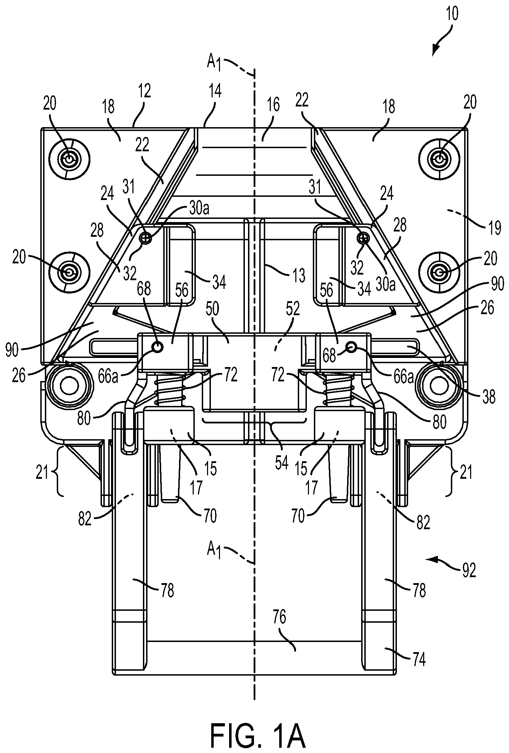

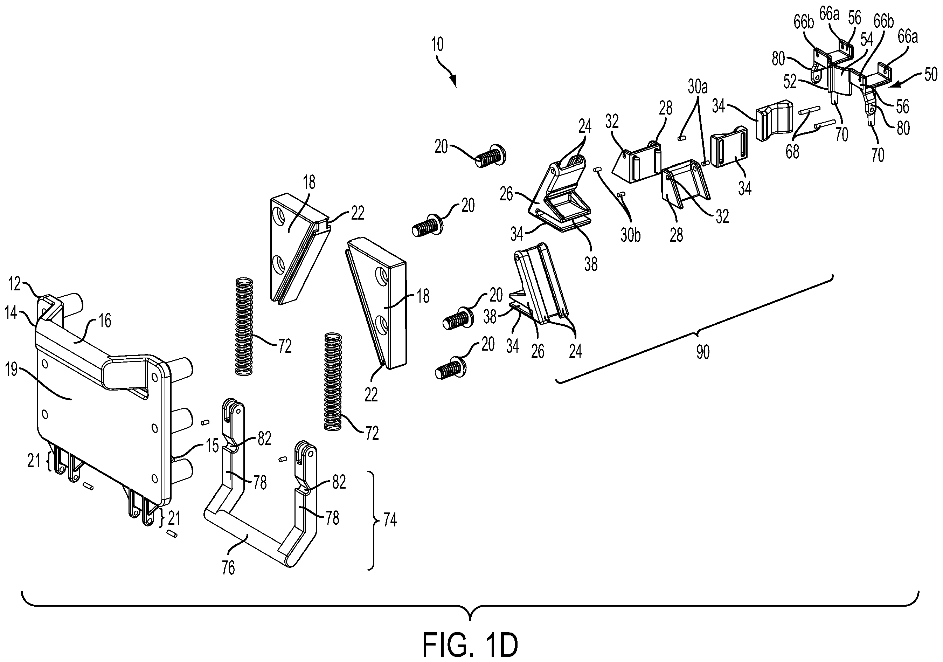

In one example embodiment, as shown in **FIGS. 1A-1E****,** a clamp apparatus **10** is depicted. The clamp apparatus **10** comprises a housing **12.** In the shown embodiment, the housing **12** has a back plate **14,** which is generally planar. On one portion of the back plate **14** is a raised grip **16** extending away from the housing **12.** The grip **16** affords the user ease of movement along a clamped object **100** generally extending along an axis **A1.** The grip **16** is also meant to aid in carrying. The grip **16** may be made of the same material as the rest of the housing **12,** may be made of a different material, or may be made of a combination thereof. Possible materials may include, but are not limited to, rubber, polymer, composite, metal, plastic, foam, etc. Additionally, the grip **16** may comprise ergonomic finger groves, nubs, a ribbed texture, a honeycombed texture, etc.

The rear of the back plate **14** may also feature any of a variety of mechanisms **19** (not shown) to attach a load to the clamp apparatus **10.** Such mechanisms **19** may include, but are not limited to, brackets, magnets, straps, suction cups, hooks, screws or bolts, a friction fit, etc. This load could be any number of things, especially a medical device (such as an infusion pump, or peristaltic infusion pump), I.V. bag, etc.

On the front portion of the back plate **14,** a groove **13** runs vertically down the centerline (shown as a line of short and long dashes) of the back plate **14.** The groove **13** is further described below. Two compression spring pockets **15** are coupled to the back plate 14 and are raised off the bottom of the front face of the back plate **14.** The compression spring pockets **15** may be generally cylindrical and hollow much like a cup. The compression spring pockets **15** will be elaborated upon later.

Extending off the bottom edge of the back plate **14** toward the bottom of the page are two twin catch structures **21** which are symmetrical around the centerline of the back plate **14.** The catch structures **21** are formed such that a first portion of the structure **21** is a member which extends toward the bottom of page in a manner substantially perpendicular to the bottom edge of the back plate **14.** A second portion of the structure **21** is a member extending toward the bottom of the page in the same manner as the first portion. The first and second portions are offset from each other so as to allow a crosspiece to form a bridge between the first and second portion of the catch structure **21.** The crosspiece of the catch structure **21** runs in a direction substantially parallel to the bottom edge of the back plate **14.** The catch structure **21** will be further elaborated upon later.

In the example embodiment, two blocks **18** are fixedly coupled to the front of the back plate **14** by any variety of means. This could include, but is not to be limited to, screws **20** (as shown), bolts, welds, etc. The back plate **14** and blocks **18** can also be formed as a continuous part during manufacture. The blocks **18** are offset by some distance from the back plate **14.**

The blocks **18** are generally right triangles with their hypotenuses facing **A1.** It should be appreciated, however, that the blocks **18** could take any shape so long as the interior face of the blocks **18** extends in a suitable direction. The blocks **18** also display symmetry around **A1.**

Along the inward facing sides of the blocks **18** there may be tracks **22.** The tracks **22** may engage corresponding protrusions **24** on a surface of a sliding wedge **26.** These components interact in such a way that the sliding wedges **26** are able to traverse the span of the tracks **22.** In the example embodiment, the sliding wedges **26** are approximately "L" shaped, but this should not be construed as limiting the sliding wedges **26** to only an "L" shape. It should also be noted that in place of the protrusions **24** on the sliding wedge **26,** any other type of suitable engagement surfaces, such as ball bearings or rollers, could be employed. In other embodiments, the track **22** may be raised off the blocks **18.** In such embodiments, the protrusions **24** would be replaced by another suitable engagement surface such as a recessed groove, rollers, ball bearings, etc. In yet some additional embodiments, a track **22** comprises the rack portion of a rack and pinion, be the track 22 in a raised or recessed configuration; in place of the protrusions **24,** on the sliding wedge **26,** one or more pinion gears would extend so as to engage the rack track **22,** in this specific embodiment.

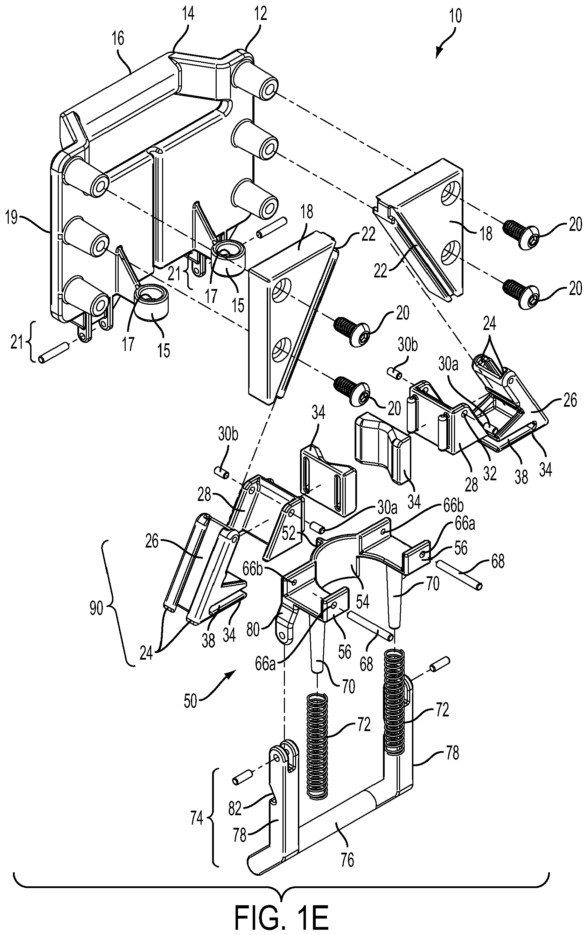

At the top of both the sliding wedges **26,** a pawl **28** may be pivotally coupled. In the embodiment shown in **FIGS. 1A-1E** this is accomplished by means of a pair of pins **30** (though a single pin, hinge, or other suitable arrangement could also be used) running through openings **32** which extend through both the sliding wedge **26** and the pawl **28.** One pin **30a** pivotally couples the pawl to the sliding wedge **26** through the front surfaces of the sliding wedge **26** and the pawl **28.** Likewise, the second **30b** of the pair of pins **30** (best shown in **FIG.** 1E) pivotally couples the pawl **28** to the sliding wedge **26** through the rear surfaces of the pawl **28** and the sliding wedge **26.** Bushings **31** may also be present in some embodiments to provide a bearing surface.

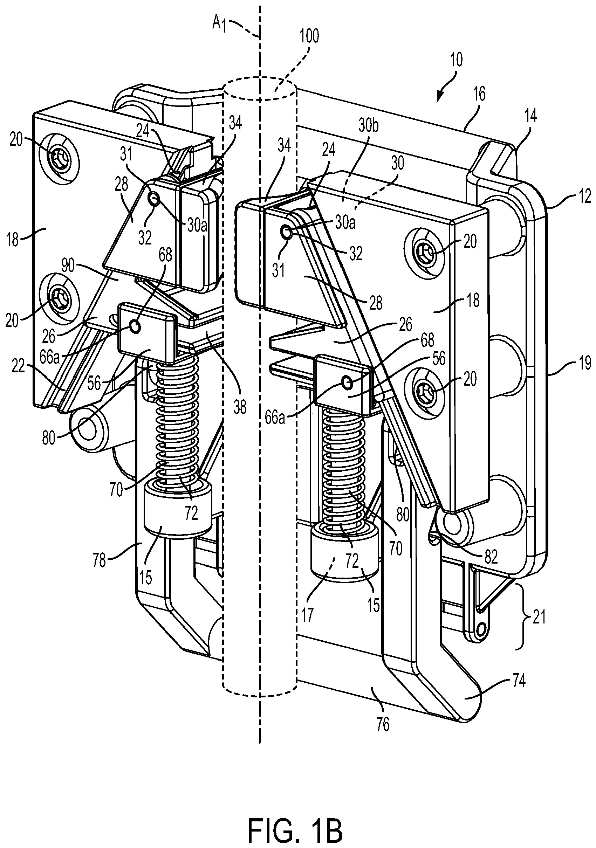

On at least a portion of the pawls **28** there may be a gripping surface **34** which engages the clamped object **100.** This gripping surface **34** consists of a material chosen for its gripping ability. The gripping surface **34** may be made of a high friction material, a compressible material, a material exhibiting both these qualities, or any other suitable material. The gripping surface **34** is made of a material which allows a firm grip without the deformation of a clamped object **100.** Additionally, the gripping surface **34** may be contoured (as is easily seen in **FIG. 1B**).

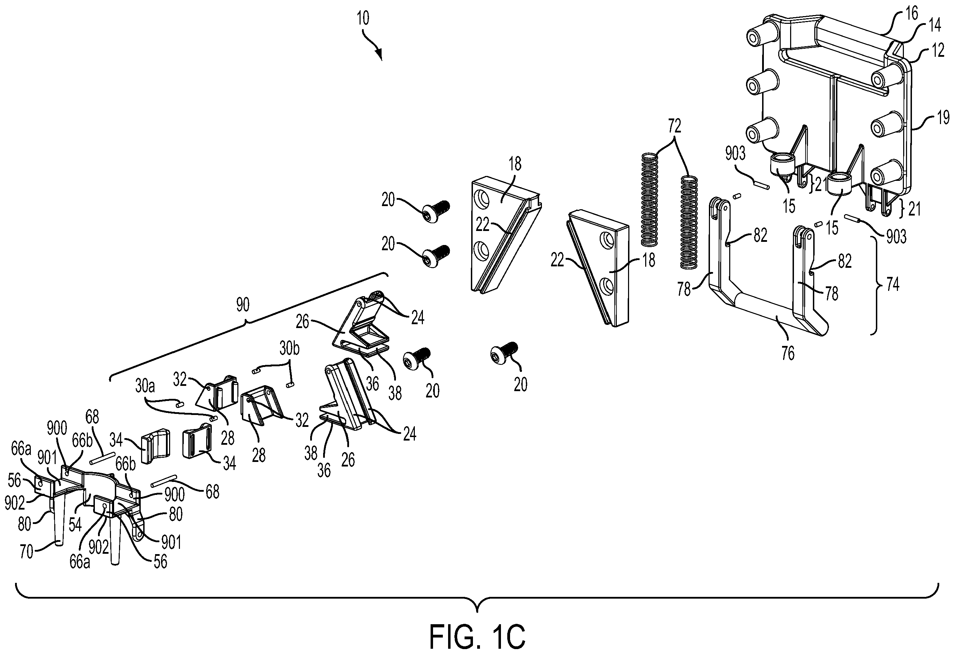

Best shown in the clamp apparatus **10** exploded views in **FIGS. 1C-1E****,** the bottom of the sliding wedge **26** may feature a flange **36.** The flange **36** extends inward, at an angle substantially perpendicular to the axial direction **A1,** from the portion of the sliding wedge **26** which engages the tracks **22.** A slot **38** is cut into the flange **36** and will be elaborated upon later.

Together, the sliding wedge **26,** the pawl **28,** and the gripping surface **34** comprise a sliding wedge-pawl assembly **90.** The sliding wedge-pawl assemblies **90** are capable of movement, together as a unit, up and down the track **22.** This allows the clamp apparatus **10** to adjust to and grip clamped objects **100** of a variety of different girths such that the distance between the gripping surfaces **34** of the sliding wedge-pawl assemblies **90** mimics the diameter of a clamped object **100.**

The clamp apparatus **10,** in this exemplary embodiment, also comprises a second assembly, the spring handle assembly **92.** At the top of the spring handle assembly **92** is a guided lift bar **50.** The rear portion of the guided lift bar **50** has a vertical ridge **52** which engages with the vertical groove **13** in the back plate **14.** This constricts the guided lift bar **50** to movement up and down in the axial direction **A1.**

In the embodiment shown in **FIGS. 1A-1E****,** the center span **54** of the guided lift bar **50** arcs/curves or bends toward the back plate **14.** This allows the guided lift bar **50** to better accommodate the clamped object **100.**

On each the right and left side of the center span **54,** a member **56** may be attached which fits around the flange **36** of the sliding wedge **26.** The member **56** is formed such that a first portion **900** of the member **56** extends off the center span **54** on a plane substantially parallel to the back plate **14.** Extending off the bottom of first portion **900** at an angle substantially perpendicular to the first portion is a second portion **901** of the member **56.** This second portion **901** is formed such that the edge of the second portion **901** distal to **A1** is straight and occupies the same vertical plane extended off the distal edge of the first portion **900.** The edge of the second portion **901** of the member **56** proximal to **A1** tapers toward the distal edge of the second portion **901.** This taper again helps to accommodate the clamped object **100.** The member **56** has a third portion **902** which is attached to the second portion **901** such that the bottom of the third portion **902** is coupled to the front edge of the second portion **901** at an angle that is substantially perpendicular. The third portion **902** extends on a plane parallel to the first portion **900.** The edge of the third portion **902** distal to **A1** is straight and occupies the same vertical plane extended off the distal edge of the first portion **900.** The proximal edge of the third portion **902** is flush with the proximal, tapered edge of the second portion **901** and extends upwards from it in a substantially perpendicular manner.

In the example embodiment in **FIGS**. **1A-1** **E,** the third portion **902** of the member **56** described above has a hole **66a** creating a passage through the third portion **902.** Likewise, the first portion 900 also has a hole **66b** creating a passage through the first portion 900. The centers of both holes **66a, 66b** extend along a common axis which is substantially perpendicular to the front face of each the first and third portions 900, 902 of the member **56.** The locations of the holes **66a** and **66b** are selected such that they are in line with the slots **38** in the sliding wedges **26** when the clamp apparatus **10** is assembled. Placing the holes **66a** and **66b** at this location allows the insertion of dowels **68** through each of the holes **66a** and **66b** and their corresponding slots **38,** thus coupling the sliding wedge-pawl assemblies **90** to the spring handle assembly **92.** Though the example embodiments employ the use of a dowel **68** to couple the two assemblies together, other means of coupling the assemblies, such as but not limited to, a bar, rollers, ball bearings, etc. could be implemented.

In the example embodiment, when both assemblies **90** and **92** are coupled together, the guided lift bar **50** functions as a crossbar which ensures that the right and left sliding wedge-pawl assemblies **90** move together in unison along the tracks **22.** This coupling also allows the spring handle assembly **92** to control whether the clamp apparatus **10** is in the open or closed position.

Coupled to the bottom of the second portion 901 of the members **56** a generally cylindrical shape **70** may be extended downward (in additional embodiments, other shapes may be used). As shown in the example embodiments in **FIGS. 1A-1E****,** the generally cylindrical shape **70** may taper slightly in diameter as it extends farther away from the bottom of the second portion 901 of the member **56** toward the bottom of the page. The generally cylindrical shape 70 may be solid or hollow. A coil spring **72** surrounds the generally cylindrical shape **70.** One end of the coil spring **72** abuts the bottom of the second portion 901 of the member **56** from which the generally cylindrical shape **70** extends. The other end of the coil spring **72** seats in the compression spring pocket **15** on the back plate **14** mentioned above. The bottom of the compression spring pocket **15** has a hole **17** through which the generally cylindrical shape **70** may pass as the clamp apparatus **10** is moved to/in the open position. Though the shown embodiments use a coil spring **72,** other embodiments could conceivably employ any other suitable bias member. A wide variety of suitable bias members may be employed. Examples of suitable bias members include, but are not limited to, a gas spring using a bladder, a piston type arrangement, a compression spring made of a compressible, springy material such as rubber, an extension spring, a constant force spring, etc.

In the example embodiment, the coil springs **72** bias the clamp apparatus **10** toward the closed position (as shown in **FIG. 1B**). That is, the coil springs 72 bias the wedges 26 to slide up the tracks 22 such that the pawls 28 approach each other towards the clamped object 100 (e.g., a pole). In the closed position, the sliding wedge-pawl assemblies **90** are sufficiently at the top of the tracks **22** to clamp the pawls 28 onto the clamped object 100 (via attached gripping surfaces 34). The guided lift bar **50** is also at a higher position in the vertical groove **13** in the back plate **14.** Also in this position, the coupling dowel **68,** in relation to **A1,** is located in a more distal end of the slot **38** in the flange **36** of the sliding wedge **26.**

If a clamped object **100** is present in the example embodiment, the coil springs **72** bias the clamping apparatus **10** to clamp down on the object **100.** Depending on the size of the clamped object **100,** the sliding wedge-pawl assemblies' **90** location on the track **22** will vary so that the distance between the sliding wedge-pawl assemblies **90** will mimic the diameter of the clamped object **100.** The larger the clamped object **100** the lower the sliding wedge-pawl assemblies **90** will be on the track **22.** Similarly and consequentially, the location of the guided lift bar **50** along the groove **13** will be lower with larger clamped objects **100.**

The clamping apparatus **10** in the example embodiment is designed in such a way as to utilize the force of gravity to increase the clamping force. As gravity pulls on the clamp, especially when a load is attached to the back plate **14,** a force is exerted on the sliding wedge-pawl assemblies **90.** This force causes the sliding wedge-pawl assemblies **90** to want to ride further up the tracks **22.** Since the clamped object **100** is in the way, the sliding wedge-pawl assemblies **90** cinch up on and exert more clamping force on the clamped object **100.** Additionally, because the pawls **28** are pivotally coupled to the sliding wedge **26,** the pull of gravity causes the point of contact on the pawls **28** to want to swing up and into the clamped object **100.** Since the clamped object **100** is in the way, the pawls **28** cinch up on and exert more clamping force on the clamped object **100.**

In order to move the clamping apparatus **10** to the open position, a pull handle **74** may be pulled down. In the example embodiment, the pull handle **74** comprises a grip **76** and one or more posts **78** extending from the grip **76.** The grip **76** may be made of the same material as the rest of the pull handle **74,** may be made of a different material, or may be made of a combination thereof. Possible materials may include, but are not limited to, rubber, polymer, composite, metal, plastic, foam, etc. Additionally, the handle **74** may comprise ergonomic finger groves, nubs, a ribbed texture, a honeycombed texture, etc.

The one or more posts **78** of the pull handle **74** extend up to a corresponding number of arms **80** on the guided lift bar **50.** The posts **78** are coupled to the arms **80** on the guided lift bar **50** through any of a variety of means. In the example embodiment, coupling is accomplished by means of a pin which runs through both the arm **80** and post **78.** In other embodiments, this coupling may be accomplished in any number of suitable ways including, but not limited to, welds, bolts, screws, etc. The pull handle **74** and guided lift bar **50** could also be made as a single continuous part during manufacture. In some embodiments, the posts **78** extend straight down to the grip **76.** In other embodiments, the posts **78** may be arcuated or have a bend out toward the rear of the page to allow greater ease in grasping the grip **76.** Additionally, in some embodiments, including the example embodiment, the posts **78** have a notch **82** which runs across the back of the posts **78** in a direction substantially parallel to the bottom edge of the back plate **14.**

As aforementioned, to move the clamping apparatus **10** from the closed position to the open position, a pull handle **74** may need to be pulled down. In the example embodiment, as the pull handle **74** is pulled down, the guided lift bar **50** is also pulled down the groove **13** in the back plate **14.** This causes the compression springs **72** to become compressed and causes the generally cylindrical shape **70** to extend through the hole **17** in the compression spring pockets **15.** Pulling down the pull handle **74** also causes the sliding wedge-pawl assemblies **90** to slide down the tracks **22.** Due to the slope of the tracks **22,** moving the clamping apparatus **10** to the open position also causes the location of the coupling dowel **68** within the slot **38** to change. When the clamp is in the fully open position, the coupling dowel **68** is at the most proximal end of the slot **38** in relation to **A1.**

In the example embodiment, to hold the clamping apparatus **10** in the fully open position against the restoring force of the compression springs **72,** the notch **82** in the pull handle **74** may be engaged with the catch structure **21** extending off the back plate **14.** When the clamping apparatus **10** is locked in the open position, the crosspiece 903 of the catch structure **21** is caught by the notch **82** of the pull handle **74** thereby disallowing the compression springs **72** to return the clamping apparatus **10** to the closed position. Other embodiments may employ other types of catch mechanisms in addition to the elbow type catch in the example embodiment. Other suitable catches may include, but are not limited to, a magnetic catch, a ball catch, a latch, a roller catch, etc.

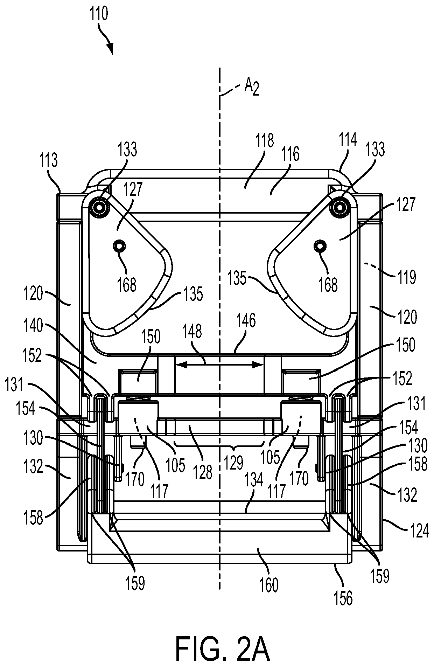

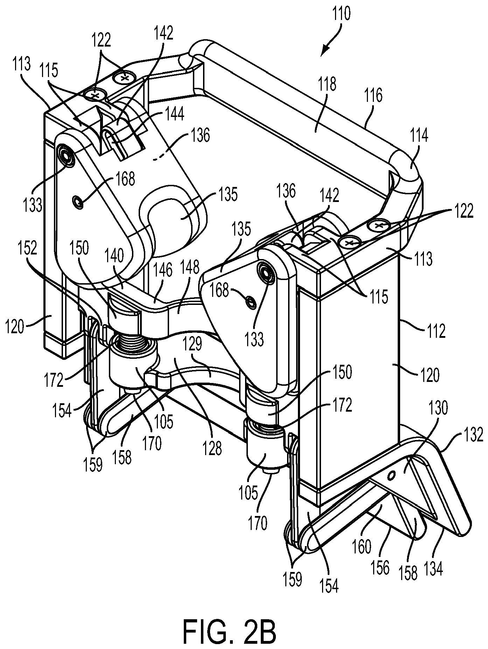

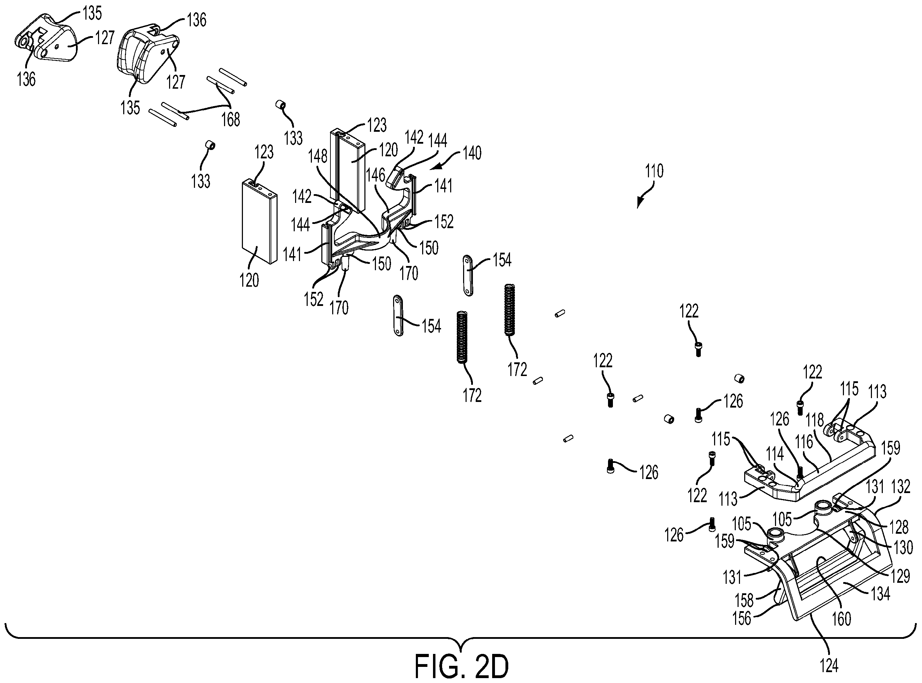

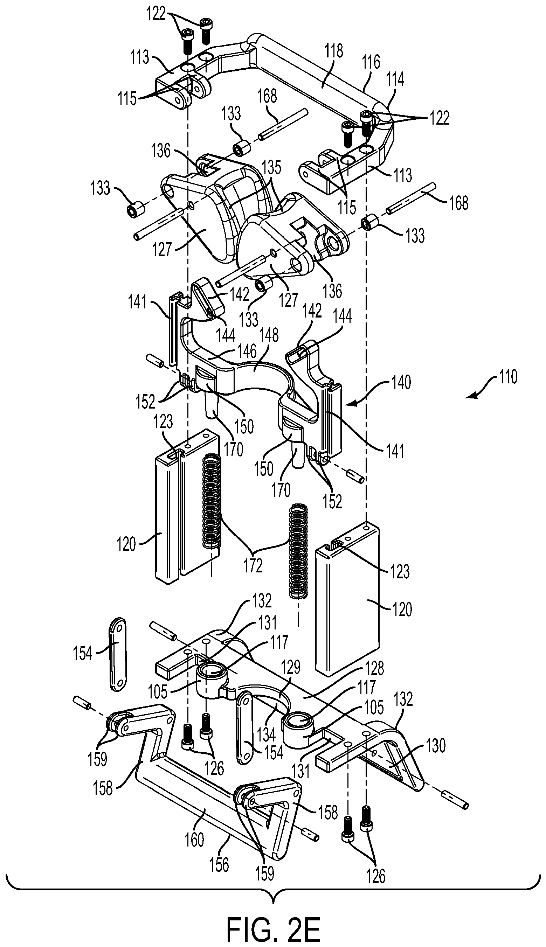

In another embodiment, as shown in **FIGS. 2A-2E****,** a clamp apparatus **110** is depicted. The clamp apparatus **110** comprises a housing **112.** The housing **112** resembles a frame. The housing **112** comprises an upper handle **114** at the top of the housing **112.** In the example embodiment, the upper handle **114** is essentially "U" shaped with the bottom, grip portion **116** of the "U" extended toward the back of the page (directions given in relation to the embodiment depicted in **FIG. 2A**). In other embodiments, the upper handle **114** need not take the shape of a "U", but rather any other desirable form. The grip portion **116** of the upper handle **114** may be cylindrical, planar, or take any other desired form. The grip portion **116** of the upper handle **114** may also have gentle ergonomic finger grooving, nubs, a ribbed texture, a honeycombed texture, etc. **118** (not shown) to increase ease of use. The grip portion **116** may be made of the same material as the rest of the upper handle **114,** may be made of a different material, or may be made of a combination thereof. Possible materials may include, but are not limited to, rubber, polymer, composite, metal, plastic, foam, etc.

In the example embodiment, the uprights **113** of the "U" extend from the grip portion **116** toward the front of the page. The uprights **113** of the "U" each comprise a set of brackets **115** which extend substantially perpendicularly from the faces of the uprights **113** most proximal to **A2** toward **A2.**

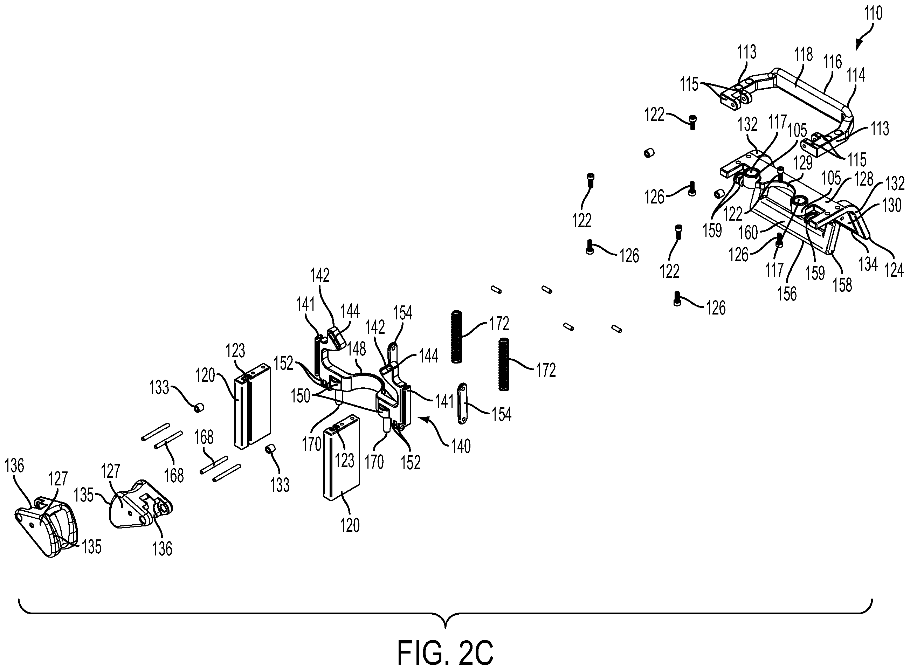

The housing **112** in the example embodiment also comprises one or more members **120** extending from the upper handle **114.** In the embodiment shown in **FIGS. 2A-2E****,** two substantially planar members **120** extend down in parallel fashion from the upper handle **114** at an angle that is generally perpendicular to the bottom surface of the upper handle **114.** The members **120** may be coupled to the upper handle **114** with screws **122** (as shown best in **FIGS. 2C-2E**), bolts, welds, or by any other manner. The upper handle **114** and one or more vertical members **120** may also be formed as a single part during manufacture. The members **120** may also comprise tracks **123** on the faces of the members **120** most proximal to **A2.** In the example embodiment, the tracks **123** run vertically up the face of each member **120** though this need not be true of every embodiment. Additionally, in the example embodiment, the tracks **123** are cut into the members **120.** In other embodiments, the tracks may be raised off the members **120.**

The housing **112** may also comprise a lower handle **124.** In the example embodiment, the lower handle **124** is coupled to the bottom edges of the members **120.** The lower handle **124** may be coupled to the members **120** in any of a variety of ways including screws **126,** bolts, welds, etc (as best shown in **FIGs. 2C-2E**). The lower handle **124** may also be formed with the members **120** as a single continuous part during manufacture. In other embodiments, the upper handle **116,** members **120,** and lower handle **124** are all formed as a continuous part in manufacture. Spanning the distance between the members **120,** the lower handle **124** may comprise a crosspiece **128.** The center span **129** of the crosspiece **128** may arc/curve or bend toward the back of the page to better accommodate a clamped object **100.** The crosspiece **128** also may comprise a pair of compression spring pockets **105.** The compression spring pockets **105** are generally cylindrical and are hollow much like a cup. In the example embodiment, the bottom of the compression spring pockets **105** have an opening **117.** A pair of brackets **130** extend off the bottom of the crosspiece **128** and will be elaborated upon later. The crosspiece **128** may have recessed portions **131** spanning the distance between the distal sides of the compression spring pockets **105** (in relation to **A2**) and the arms **132** of the lower handle **124** (elaborated upon in the following paragraph).

The lower handle **124** extends toward the back of the page in a manner similar to the upper handle **114.** The arms **132** of the lower handle **124** may be arcuated or have a bend which arcs/bends the lower handle **124** toward the bottom of the page. The arms **132** of the lower handle **124** are joined by a grip **134** at the part of the handle closest to the bottom of the page.

The grip **134** may be made of the same material as the rest of the lower handle **124,** may be made of a different material, or may be made of a combination thereof. Possible materials may include, but are not limited to, rubber, polymer, composite, metal, plastic, foam, etc. Additionally, the grip **134** may comprise ergonomic finger groves, nubs, a ribbed texture, a honeycombed texture, etc.

The housing **112** may also feature any of a variety of mechanisms **119** (not shown) to attach a load to the clamp apparatus **110.** Such mechanisms **119** may include, but are not limited to, brackets, magnets, straps, suction cups, hooks, screws or bolts, a friction fit, etc. This load could be any number of things, especially a medical device (such as an infusion pump, or peristaltic infusion pump), I.V. bag, etc.

The clamping apparatus **110** may further comprise a set of pawls **127** which are pivotally coupled to the brackets **115** of the upper handle **116.** The set of pawls **127** may be coupled to the brackets **115** of the upper handle by any of a variety of ways. Additionally, bushings **133** may be present to provide a bearing surface. The pawls **127** may have a trough **136** cut into them essentially along the center plane of the pawls **127** running parallel to the plane of the grip **116** shown in the example embodiment. The trough **136** will be elaborated upon later.

On at least a portion of the pawls **127** there may be a gripping surface **135** which engages the clamped object **100.** The gripping surface **135** may consist of a material chosen for its gripping ability. The gripping surface **135** could be made of a high friction material, a compressible material, a material exhibiting both those qualities, or any other suitable material. The gripping surface **135** is made of a material which allows a firm grip without the deformation of a clamped object **100.** Additionally, the gripping surface **135** may be contoured. Though the example embodiment includes a single set of pawls **127,** in other embodiments, further sets of pawls **127** may be added to the clamping apparatus **110** to afford the clamping apparatus **110** added stability.

In the example embodiment, the clamping apparatus **110** also comprises a lift bar guide **140.** The lift bar guide **140** comprises a set of protrusions **141** which engage with the tracks **123** in the members **120.** This enables the lift bar guide **140** to travel along the track **123** in the members **120.** In place of protrusions **141** some alternate embodiments employ a variety of different engagement surfaces. These surfaces include, but are not limited to, rollers, ball bearings, etc. In other embodiments, the track **123** may be raised off the members **120.** In such embodiments, the protrusions **141** would be replaced by another suitable engagement surface such as a recessed groove, rollers, ball bearings, etc. It would also be conceivable for some embodiments to use a track **123,** be it raised or recessed, comprising the rack portion of a rack and pinion. In place of the protrusions **141,** on the lift bar guide **140,** one or more pinion gears would extend so as to engage the rack track **123.**

The top portion of the lift bar guide **140** may comprise a set of wings **142** which project inward toward **A2.** The wings **142** are shaped such that they are able to fit within the trough **136** in the pawls **127.** The wings **142** have a slit **144** cut into them (best shown in **FIGS. 2C-2E**) similar to the slot **38** depicted in **FIGS. 1A-1E****.** A coupling dowel **168** couples the pawls **127** to the lift bar guide **140** through the slit **144** in the wings **142.** The lift bar guide **140** has a crossbar **146.** This enables the lift bar guide **140** to cause the pawls **127** to move in unison. The center span **148** of the crossbar **146** may be arced/bent toward the back of the page to better accommodate a clamped object **100.**

On each side of the arced center span **148,** recessed compression spring pockets **150** are recessed into bottom face the lift bar guide **140.** From the centers of the recessed compression spring pockets **150** a generally cylindrical shape **170** extends (though the shape need not be cylindrical in all embodiments) toward the bottom of the page. The generally cylindrical shape **170** may be solid or hollow. The generally cylindrical shape **170** may taper slightly in diameter as it extends farther away from the bottom face of the lift bar guide **140.** The diameter of the generally cylindrical shape **170** is such it occupies much of the center of the recessed compression spring pocket **150,** but leaves a ring surrounding the base of the generally cylindrical shape **170.** One end of a coil spring **172** is seated in the ring surrounding the generally cylindrical shape **170** in the recessed compression spring pocket **150.** The other end of the coil spring **172** abuts the bottom of the compression spring pocket **105** on the lower handle **124** mentioned above. The bottom of the compression spring pocket **105** has a hole **117** through which the generally cylindrical shape **170** may pass as the clamp apparatus **110** is moved to/in the open position. Though the shown embodiments use a coil spring **172,** other embodiments could conceivably employ any other suitable bias member configuration. A wide variety of suitable bias members could be employed. Examples of suitable bias members include, but are not limited to, a gas spring using a bladder, piston type arrangement, a compression spring made of a compressible, springy material such as rubber, an extension spring, constant force spring, spring steel, etc.

In the shown embodiment, more distal from **A2** than the recessed compression spring pockets **150,** a set of brackets **152** extends downward on each side of the bottom face of the lift bar guide **140.** In some embodiments, the placement of the recessed compression spring pockets **150** or other suitable bias structure and the brackets **152** may be switched. Coupled to the brackets **152** on the lift bar guide **140** there may be a link structure **154.** In the example embodiments, the link structure **154** is a generally oblong disc with rounded edges. In other embodiments, the link structure **154** may take other forms and shapes. Examples of link structures **154** in other possible embodiments may include, but are not limited to, prismatic joints, any of a variety or springs, etc. It would also be conceivable to forgo the brackets **152** while coupling a camming surface to the actuator lever handle **156** (introduced in the following paragraph) thus effectively making the lift bar guide **140** a cam follower.

In the example embodiment, the other end of the link structure **154** is coupled to an actuator lever handle **156.** The actuator lever handle **156** has a set of members **158.** One end of the members **158** may be fitted with brackets **159** which allows the members **158** to couple to the link structure **154** as is shown in the example embodiment. From their coupling point to the link structure **154,** the members **158** may extend to and are coupled to the brackets **130** projecting off the bottom face of the crosspiece **128** of the lower handle **124.** In some embodiments, a torsion spring may be employed where the members **158** of the actuator lever handle **156** couple to the crosspiece **128** brackets **130.** The torsion spring may be a substitute for, or used in conjunction with the coil spring **172** or other suitable bias structure. From their coupling point on the crosspiece **128** brackets **130,** the members **158** arc/curve or bend steeply downward. In the example embodiments the members **158** bend at nearly a right angle, though other suitable angles may be used. A gripping portion **160** spans the distance between lowest ends of the members **158.**

The gripping portion **160** may be made of the same material as the rest of the actuator lever handle **156,** may be made of a different material, or may be made of a combination thereof. Possible materials may include, but are not limited to, rubber, polymer, composite, metal, plastic, foam, etc. Additionally, the gripping portion **160** may comprise ergonomic finger grooves, nubs, a ribbed texture, a honeycombed texture, etc.

In the example embodiment, the coil springs **172** bias the clamping apparatus **110** toward the closed position. In the closed position the lift bar guide **140** is at its highest point of travel along the tracks **123** in the members **120.** The pawls **127** are rotated up and inward toward **A2.** Also in the closed position, the coupling dowel **168** is at the bottom of the slit **144** in the wings **142** of the lift bar guide **140.**

If a clamped object **100** is present in the example embodiment, the coil springs **172** bias the clamp apparatus **110** to clamp down on the object **100.** Depending on the size of the clamped object **100,** the lift bar guide's **140** location on the track **123** will vary. The larger the clamped object **100** the lower the lift bar guide **140** will be on the track **123.** Additionally, the pawls **127** will not be fully rotated up and inward toward **A2.** Instead the distance between the gripping surfaces **135** of the pawls **127** will mimic the diameter of the clamped object **100.** This also means that the location of the coupling dowel **168** will be somewhat closer to the top of the slit **144.**

The clamp apparatus **110** in the example embodiment is designed in such a way as to utilize the force of gravity to increase the clamping force. As gravity pulls on the clamp apparatus **110,** especially when a load is attached to the housing **112** the force causes the pawls **127** to want to rotate further in towards **A2.** Since the clamped object **100** is in the way, the pressure of the pawls **127** against the clamped object **100** increases and the clamping apparatus **110** grips the clamped object **100** more vigorously.

To open the clamp apparatus **110** in the example embodiment, a user's hand may reach around the lower handle **124** and grasp the actuator lever handle **156** with their fingers. The user may then pull the actuator lever handle **156** toward the lower handle **124** of the housing **112.** This causes the actuator lever handle **156** to pivot about its coupling to the brackets **130** on the cross piece **128** of the lower handle **124.** This in turn pulls down on the link structure **154** which couples the actuator lever handle **156** to the lift bar guide **140.** As the link structure **154** is pulled downward, the lift bar guide **140** travels down the tracks **123** in the members **120** of the housing **112.** As the lift bar guide **140** travels downward, the compression springs **172** are compressed and the generally cylindrical shape **170** extends through the hole **117** in the compression spring pockets **105** on the crosspiece **128** of the lower handle **124.** The downward travel of the lift bar guide **140** also causes the pawls **127** to rotate downward and away from **A2.** This is caused by the slit **144** in the wings **142** of the lift bar guide **140** sliding over the coupling dowel **168** until the coupling dowel **168** reaches the top of the slit **144.** When the coupling dowel **168** is in this position, the pawls **127** are fully open. The clamp apparatus **110** may then be placed on a clamped object **100.** Once the actuator lever handle **156** is released, the compression springs **172** will bias the clamp apparatus **110** to close and clamp down on the clamped object **100.**

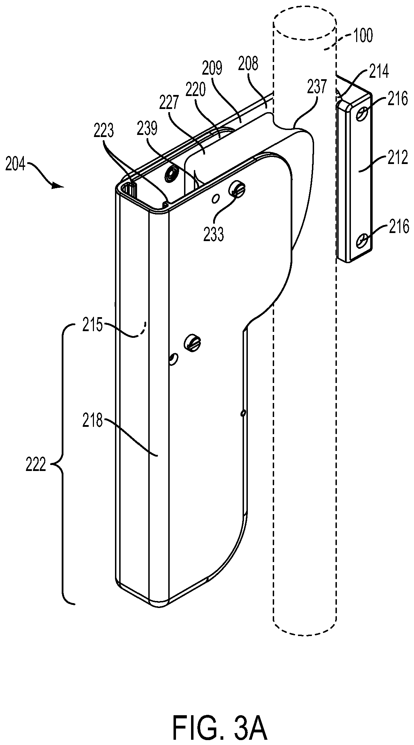

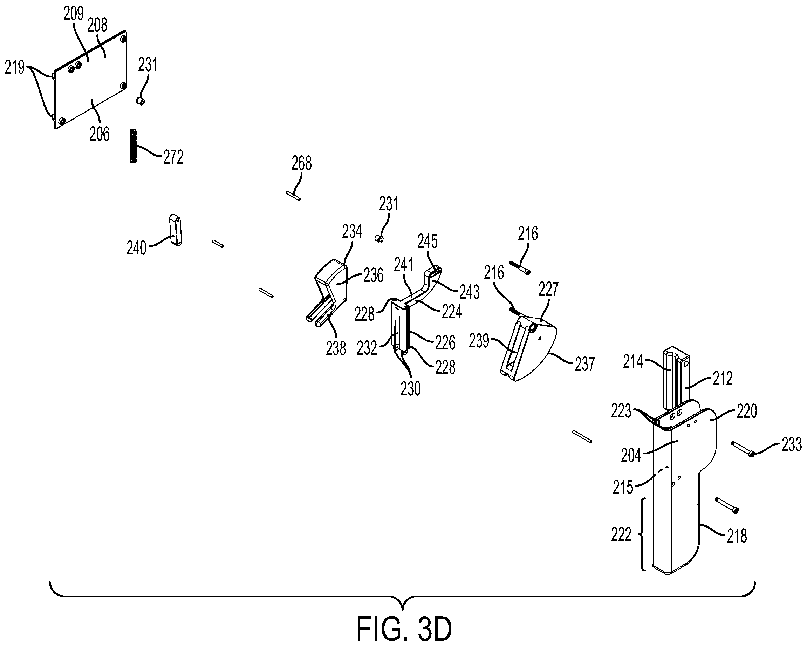

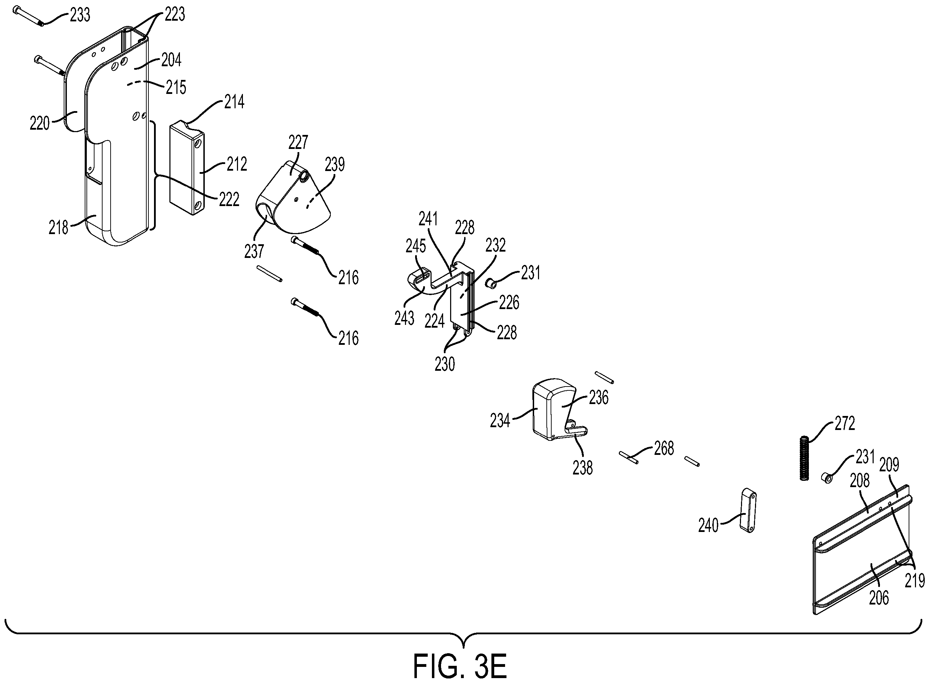

In another embodiment shown in **FIGS. 3A-3E****,** a clamp apparatus **202** is depicted. The clamp apparatus 202 comprises a housing **204.** The housing **204** comprises a number of portions. The first portion of the housing **204** may include a back plate **206.** The back plate **206** may be substantially planar as shown in **FIGS. 3A-3E****.**

The back plate **206** may also include a gripping handle **208** (not shown). The gripping portion **209** of the gripping handle **208** may be made of the same material as the rest of the handle **208,** may be made of a different material, or may be made of a combination thereof. Possible materials may include, but are not limited to, rubber, polymer, composite, metal, plastic, foam, etc. Additionally, the gripping portion **209** of the gripping handle **208** may comprise ergonomic finger groves, nubs, a ribbed texture, a honeycombed texture, etc.

Additionally, the back plate **206** may also feature any of a variety of mechanisms or mounts **219** which allow the user to attach a load to the clamp apparatus **202.** Such mechanisms **219** may include, but are not limited to, brackets, magnets, straps, suction cups, hooks, screws or bolts, a friction fit, etc. This load could be any number of things, especially a medical device (such as an infusion pump, or peristaltic infusion pump), I.V. bag, etc.

In the example embodiment shown in **FIGS. 3A-3E****,** on the right side of the front face of the back plate **206** a rectangular block **212** projects at an angle substantially perpendicular to the front face of the back plate **206.** The rectangular block **212** need not be rectangular in all embodiments. The rectangular block **212** is coupled to the back plate **206** in any of a variety of ways. The example embodiment employs screws **216,** but bolts, welds or any other suitable means could also be utilized. The back plate **206** and rectangular block **212** could also be formed as a continuous part during manufacture. The rectangular block **212** may be generally planar. The rectangular block **212** may also be arced/curved to better accommodate a clamped object **100.**

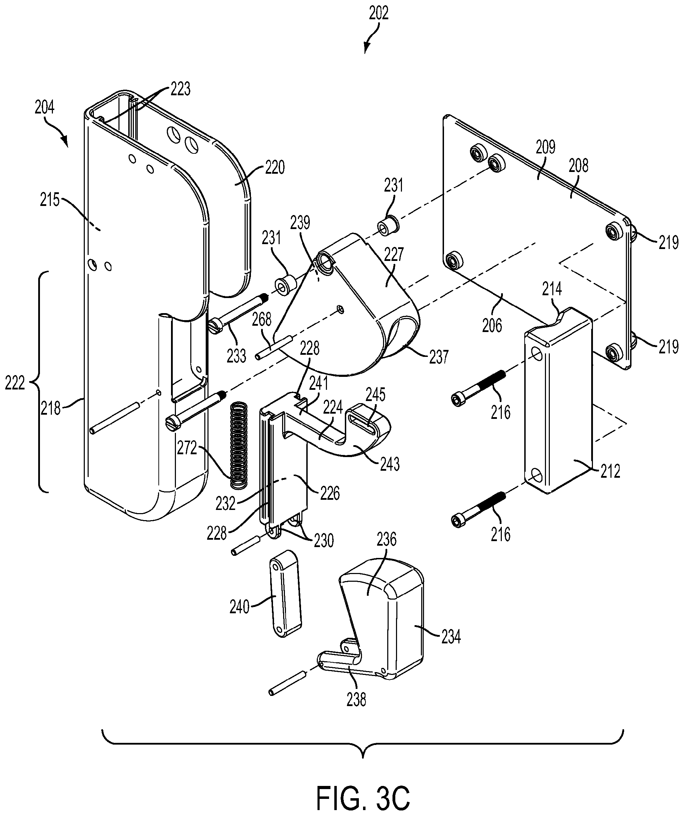

On at least a part of the inward facing side of the rectangular block **212,** a gripping surface **214** may be affixed. The gripping surface **214** can engage the clamped object **100.** This gripping surface **214** consists of a material chosen for its gripping ability. The gripping surface **214** could be made of a high friction material, a compressible material, a material exhibiting both of these qualities, or any other suitable material. The gripping surface **214** is made of a material which allows a firm grip without the deformation of a clamped object **100** Additionally, the gripping surface **214** may be contoured (as shown in **FIGS. 3C-3E**). In order to accommodate the contoured gripping surface **214** the inward face of the rectangular block **212** may also be contoured. Though the example embodiments only have one fixed gripping surface **214,** it would be conceivable to add additional fixed gripping surfaces to the clamping apparatus **202.**

The housing **204** may also comprise a second portion. The second portion of the housing may include a handle sleeve **218.** In the example embodiment, the handle sleeve **218** comprises a body which may be entirely hollow (as shown) or have one or more hollow cavities. In the example embodiment shown in **FIGS. 3A-3E****,** the top and a portion of the right side of the handle sleeve **218** are open to a hollow cavity. In alternate embodiments this need not always be the case. At the top of the handle sleeve **218** two rounded ears **220** project off the front and rear faces of the handle sleeve **218** toward the right of the page.

A portion of the handle sleeve **218** may have grip portion **222** to allow for greater ease of use. The gripping portion **222** may be made of the same material as the rest of the housing **204,** may be made of a different material, or may be made of a combination thereof. Possible materials may include, but are not limited to, rubber, polymer, composite, metal, plastic, foam, etc. Additionally, the gripping portion **222** may comprise ergonomic finger groves, nubs, a ribbed texture, a honeycombed texture, etc.

In the example embodiment, on at least one or both the interior of the front or/and rear faces of the handle sleeve **218** near the left face of the handle sleeve **218** are tracks **223** which extend at least some portion of the length of the handle sleeve **218.** In the embodiment in **FIGS. 3A-3E** the tracks **223** are raised and run vertical. Other embodiments may differ. For example, it would be conceivable to have a track **223** recessed into the sleeve handle. The track(s) **223** may also be cut into or raised out of the interior of the left face of the handle sleeve **218.** In some embodiments, the track **223** may be the rack of a rack and pinion arrangement.

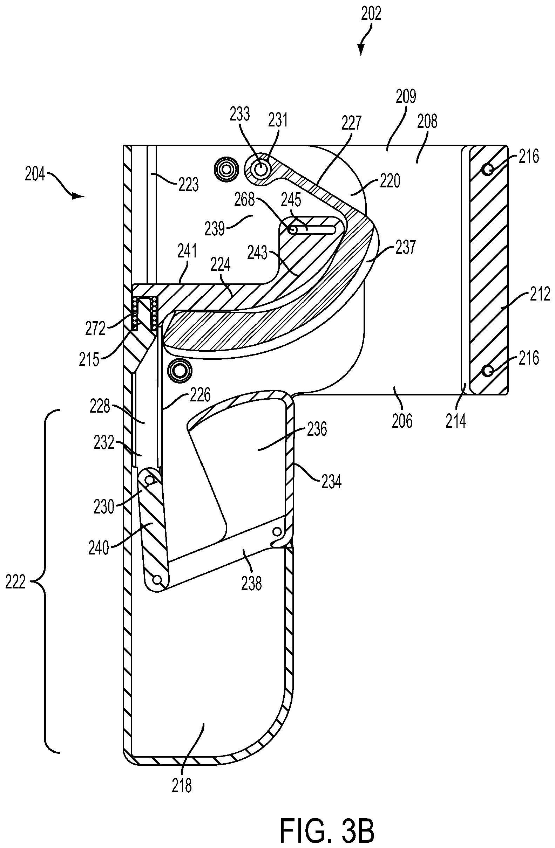

On the left face of the interior cavity, one or more compression spring pocket(s) **215** may be extended out into a hollow cavity as best shown in **FIG. 3B****.** The compression spring pocket(s) **215** may also be extended out from at least one or both the interior of the front or/and rear faces of the handle sleeve **218.** The compression spring pocket (s) **215** will be elaborated upon later.

At the top of the handle sleeve **218** a pawl **227** may be pivotally coupled. The pawl **227** may be pivotally coupled by any of a variety of means such as a screw **233** (as shown), pins, etc. Additionally, bushings **231** may be present to provide a bearing surface. The pawl **227** is able to swing about its pivot axis point within the cavity in the handle sleeve **218.** The pawl **227** is also able to swing about its pivot out towards the fixed gripping surface **214** on the interior face of the rectangular block **212.**

The surface of the pawl **227** facing the fixed gripping surface **214** on the interior face of the rectangular block **212** may be arced as best shown in **FIG. 3C****.** The surface of the pawl **227** facing the fixed gripping surface **214** on the interior face of the rectangular block **212** may further comprise a gripping surface **237.** The gripping surface **237** could be made of a high friction material, a compressible material, a material exhibiting both those qualities, or any other suitable material. The gripping surface **237** is made of a material which allows a firm grip without the deformation of a clamped object **100** Additionally, the gripping surface **237** may be contoured (as shown best in **FIGS. 3A-3E****).**

The pawl **227** may be additionally comprised of a trough **239** cut into the pawl **227** essentially along the center plane of the pawl **227** running parallel to the plane of the back plate **206.** The trough **239** is shaped such that it is able to accommodate the shape of a lift bar **241.** As best shown in **FIGS. 3C-3E****,** the lift bar **241** may comprise a first portion comprising a member **224** which projects into the trough **239** in the pawl **227.** The member **224** may be shaped such that at the right end of the member **224** there is a wing like projection **243.** Within the wing like projection **243,** there may be a slit **245.** It should be noted that the slit **144** in **FIGS. 2A-2E** is at an angle and the slit **245** in **FIGS. 3A-3E** is substantially horizontal. Alternate embodiments may employ slits oriented at any angle or may employ arced slits. A coupling dowel **268** runs through the slit **245** and into the pawl **227** coupling the lift bar **241** to the pawl **227.**

The lift bar **241** may also comprise a second portion in which a member **226** extends toward the bottom of the page at an angle that is substantially perpendicular to the member **224** of the first portion. The member **226** of the second portion has an engagement surface **228** which engages with the track **223** on the interior of the handle sleeve **218.** In the shown embodiment, the engagement surface **228** is depicted as a recessed groove. The engagement surface **228** may, however, be raised or take other forms including but not limited to, rollers, ball bearings, etc. In embodiments where the track **223** is the rack of a rack and pinion arrangement, one or more pinion gears capable of engaging the track **223** may be present on the member **226** of the second portion.

The member **226** of the second portion of the lift bar **241** may also have a bracket **230** extending off the bottom surface of the member **226.** The bracket **230** need not extend as shown at an angle substantially perpendicular to the bottom surface of the member **226.**

The member **226** of the second portion of the lift bar **241** may also be comprised of a groove or grooves **232** recessed into the face of the member **226** which abuts the interior surface of the handle sleeve **218** from which the compression spring pocket(s) **215** extend. The groove **232** is of a size and shape sufficient to fit around the compression spring pocket **215** which projects off the interior of the handle sleeve **218.** Additionally, the groove **232** does not run the entire length of the member **226** stopping at least some distance from the top of the member **226.** As shown, the diameter of the groove **232** may taper as it extends toward the top of the member **226.**

A coil spring **272** is placed in the groove **232** such that one end of the coil spring **272** abuts the bottom of the compression spring pocket **215.** The other end of the coil spring **272** abuts the top of the groove **232.** Though the shown embodiments use a coil spring **272,** other embodiments could conceivably employ any other suitable bias member. A wide variety of suitable bias members may be employed. Examples of suitable bias members include, but are not limited to, a gas spring (using a bladder arrangement, piston type arrangement, etc.), a compression spring made of a compressible, springy material such as rubber, an extension spring, constant force spring, and so on.

In the example embodiment, the coil spring **272** biases the clamp apparatus **202** toward the closed position **(****FIG. 3A****).** In the closed position, the coil spring **272** is not compressed. Additionally, the lift bar **241** is at its highest point of travel along the tracks **223** in the handle sleeve **218** of the housing **112.** Since the lift bar **241** is coupled to the pawl **227** via the coupling dowel **268,** this forces the pawl **227** to be pivoted up and in toward the fixed gripping surface **214.** In the closed position, the coupling dowel **268** abuts the right edge of the slit **245.**

If a clamped object **100** is present in the example embodiment, the coil spring **272** biases the clamp apparatus **202** to clamp down on the object **100.** Depending on the size of the clamped object **100,** the lift bar's **241** location on the track **223** will vary. The larger the clamped object **100** the lower the lift bar **241** will be on the track **223.** Additionally, the pawl **227** will not be fully rotated up and inward toward fixed gripping surface **214.** Instead the distance between the gripping surface **237** of the pawl **227** and the fixed gripping surface **214** will mimic the diameter of the clamped object **100.** This also means that the location of the coupling dowel **268** will be somewhat closer to the left of the slit **245.**

The clamp apparatus **202** in the example embodiment is designed in such a way as to utilize the force of gravity to increase the clamping force. As gravity pulls on the clamp apparatus **202,** especially when a load is attached to the housing **204** the force causes the pawl **227** to want to rotate further up and in towards the fixed gripping surface **214.** Since the clamped object **100** is in the way, the pressure of the pawl **227** against the clamped object **100** increases and the clamping apparatus **202** grips the clamped object **100** more vigorously. Furthermore, the clamped object **100** is pushed against the fixed gripping surface **214** with greater force again causing the clamping apparatus **202** to clamp more vigorously to the clamped object **100.**

This more vigorous clamping force is accomplished by ensuring that the pawl **227** is constructed and shaped in order to ensure the clamp apparatus **202** will be in static equilibrium with a clamped object **100** when the clamp apparatus **202** is clamped onto a clamped object **100.** This may require ensuring that the coefficient of friction of the pawl **227** is greater than the ratio of the vertical distance from the contact point of the pawl **227** on the clamped object **100** to the pivot point of the pawl **227** (said distance hereafter referred to as A) to the horizontal distance from the contact point on the pawl **227** to the pivot point of the pawl **227** (said distance hereafter referred to as B). The compliance and shape of the pawl **227** gripping surface **237** of the pawl **227** also is sufficiently configured.

As shown, the pawl **227** does not have a constant radius from the gripping surface **237** to the pivot point of the pawl **227.** If the radius is constant, and the pawl **227,** gripping surface **237,** or both are relatively compliant, A:B may become less than zero if the pawl **227,** gripping surface **237,** or both become compressed. If the radius of the pawl **227** constantly increases as best shown in **FIG. 3C****,** this cannot occur. The rate of increase in the radius of the pawl **227** may be chosen so that the ratio A:B does not become too large. This may be done to ensure that the coefficient of friction is not inordinately large.

In embodiments of the pawl **227** where the radius of the pawl **227** is constantly increasing and the pawl **227,** gripping surface **237,** or both are compliant, as the downward force of gravity acting on the clamp apparatus **202** increases the ratio A:B decreases. As a result, the normal forces present at the contact point of the pawl **227** on the clamped object **100** increase. The vertical reaction force increases as a result. This may create the more vigorous clamping force described above

To move the clamp apparatus **202** to the open position shown in the embodiment in **FIG. 3B****,** the user must actuate a trigger **234.** The trigger **234** has a button portion **236** which extends at least partially out of the right face of the handle sleeve **218** when the clamp apparatus **202** is in the closed position. Toward the lower right of the button portion **236,** the button portion **236** is pivotally coupled to the handle sleeve **218** by any of a variety of means. The button portion **236** may be hollow or solid. Projecting toward the left of the page of along the bottom plane of the button portion **236** of the trigger **234** may be one or more arms **238.** The one or more arms **238** may be capable of coupling to a linkage structure **240.** The linkage structure **240** also extends up to, and is coupled to, the bracket **230** which extends off the bottom surface of the lift bar **241.** As best shown in **FIG 3C-3E****,** the link structure **240** in the example embodiment is an oblong with rounded edges. In other embodiments, the link structure **240** may take other forms and shapes. Examples of link structures **240** in other possible embodiments may include, but are not limited to, prismatic joints, any of a variety or springs, etc. It would also be conceivable to forgo the brackets **230** while coupling a camming surface to the trigger **234** thus effectively making the lift bar **214** a cam follower.

In the example embodiment, when the trigger **234** is actuated, it acts as a lever pulling the linkage structure **240** and the lift bar **241** toward the bottom of the page. As the lift bar **241** is pulled down the track **223** on the handle sleeve **218** the coil spring **272** gets compressed. The slit **245** in the wing **243** of the lift bar **241** slides over the coupling dowel **268** until the coupling dowel **268** abuts the left most edge of the slit **245.** As a result, the pawl **227** rotates down and away from the fixed gripping surface **214** and into the open position. Releasing the trigger **234** causes the clamping apparatus **202** to return to the closed position as a result of the restoring force of the coil spring **272.** In alternate embodiments, a torsion spring may be employed where the button portion **236** of the trigger **234** is pivotally coupled to the handle sleeve **218.** The torsion spring may be a substitute for or used in conjunction with the coil spring **272** or other suitable bias member configuration.

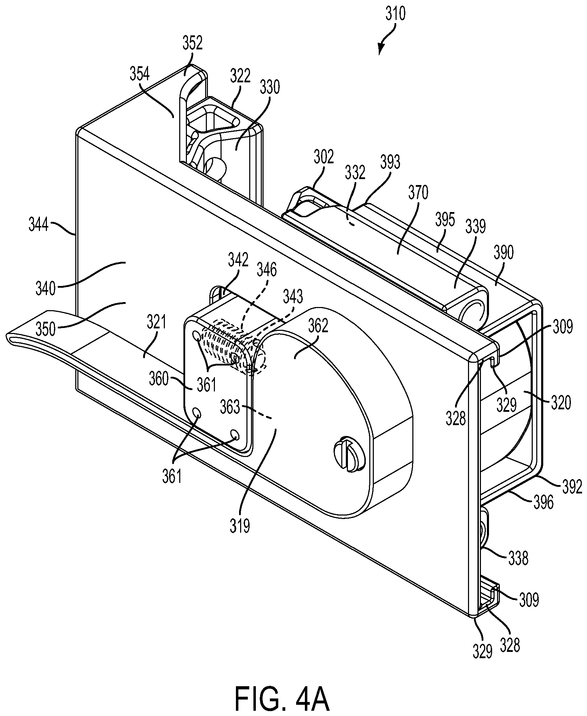

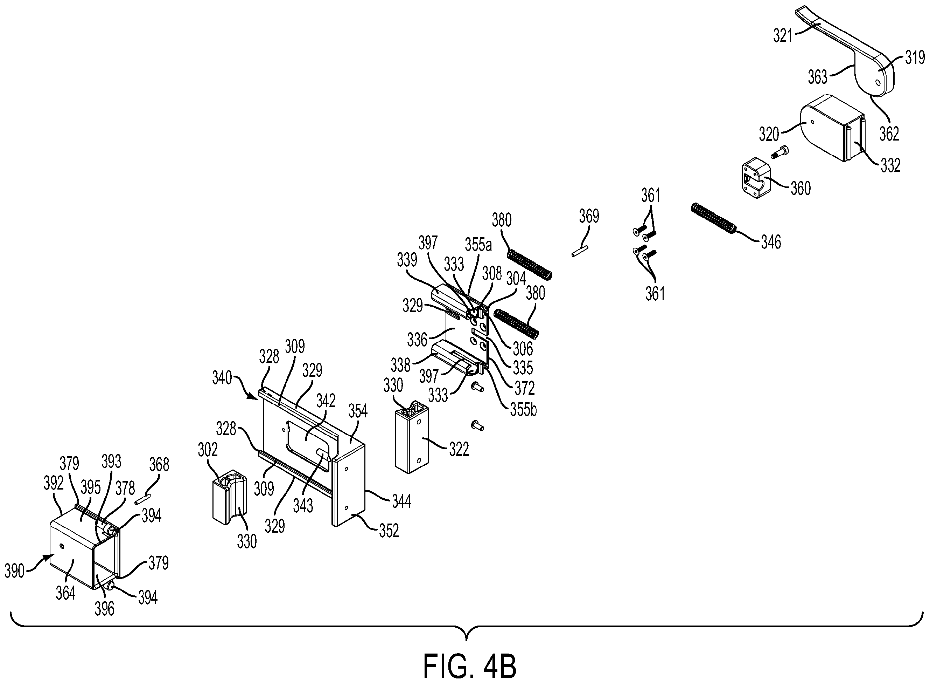

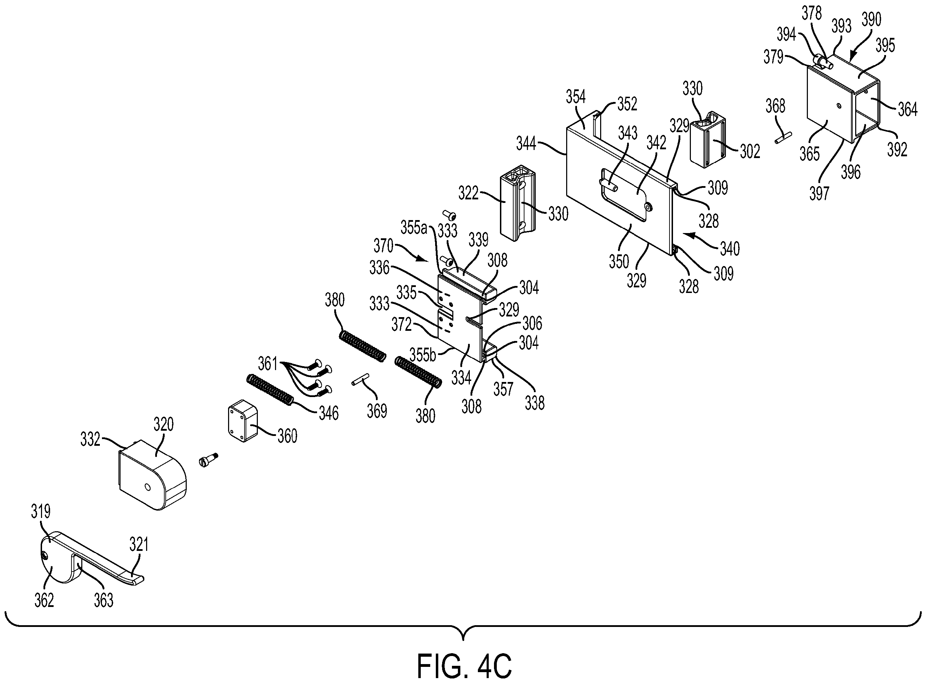

**Fig. 4A** shows a perspective view of a clamp apparatus **310** in the open position according to one embodiment of the present disclosure. A clamped object **100** may be squeezed between a fixed gripper **322** and a sliding gripper **302.** The fixed gripper **322** and sliding gripper **302** may consist of a material chosen for its gripping ability. The fixed gripper **322** and sliding gripper **302** may be made of a material which allows for a firm grip without the deformation of a clamped object **100.** The fixed gripper **322** and sliding gripper **302** may be made of a high friction material, a compressible material, a material exhibiting both these qualities, or any other suitable material. Suitable materials may include any suitable elastomeric or non-deformable substance, including but not limited to plastic, rubber, metal, foam, fabric, gel, etc. At least a portion of the fixed gripper **322** and sliding gripper **302** may comprise a roughly semi-circular depression or contour to accommodate a round clamped object **100** such as a pole.

In some embodiments, the fixed gripper **322** and sliding gripper **302** are formed from a relatively inelastic material, but have caps **330** (not shown) that fit substantially over the fixed gripper **322** and sliding gripper **302.** The cap **330** may be constructed from any suitable material, including but not limited to, elastic materials such as rubber, plastic, gel, foam, fabric, polyurethane, etc. The caps **330** may be replaceable and removably attached to the fixed gripper **322** and sliding gripper **302.**

The fixed gripper **322** may be firmly mounted to the fixed gripper mount end **344** of a guide plate **340.** In some embodiments, a gripper support wall **352** is attached to the fixed gripper mount end **344** of the guide plate **340** and provides additional support for the fixed gripper **322.** The gripper support wall **352** may optionally be supported by one or more buttresses **354** that span from at least a portion of the guide plate **340** to the gripper support wall **352.** In some embodiments, the buttresses **354** may be arched to maximize support.

At least one face of the guide plate **340** may also feature any of a variety of mechanisms **305** (not shown) to attach a load to the clamp apparatus **310.** Such mechanisms **305** may include, but are not limited to, brackets, magnets, straps, suction cups, hooks, screws or bolts, a friction fit, etc. This load could be any number of things, especially a medical device (such as an infusion pump, or peristaltic infusion pump), I.V. bag, etc.

The sliding gripper **302** is mounted to the sliding gripper mount end **332** of a sliding gripper base **320.** The position of the sliding gripper base **320** is adjustable to accommodate clamped objects **100** of various dimensions and girths. The sliding gripper base **320** will be elaborated upon later.

In an embodiment of the present disclosure shown in **FIG. 4A****,** the clamp apparatus **310** is depicted in the closed position (though a clamped object **100** is not present). To move the clamp apparatus **310** to the closed position, a user must rotate a handle assembly **319,** such that the hand grip **321** of the handle assembly **319** is pointed toward the left of the page as shown in **FIG. 4A****.** This action propels the sliding gripper **302** and all attached structures towards the fixed gripper **322.** If a clamped object **100** is present, the sliding gripper **302** will squeeze the clamped object **100** against the fixed gripper **322,** thus clamping the clamped object **100.**

The handle assembly **319** is rotatably attached to the front face **350** of the guide plate **340.** In the exemplary embodiment shown in **FIGS. 4A-4D****,** the handle assembly **319** is disposed on a plane approximately parallel to the plane of the front face **350** of the guide plate **340** regardless of whether the clamp apparatus **310** is in the open or closed position or in transit between an open and closed position. The handle assembly **319** is comprised of a number of portions. At least a one portion of the handle assembly **319** abuts a cam plate **360,** which is immovably attached to a pressure plate **370** (pressure plate **370** introduced in subsequent paragraphs). In the depicted exemplary embodiment in **FIGS. 4A-4D****,** the handle assembly **319** comprises a cam **362** positioned to contact the cam plate **360.** The rounded, contoured surface of the cam **362** grades into a planate section which spans the length of the hand grip **321.**

In some embodiments, hand grip **321** may be made of the same material as the rest of the handle assembly **319,** may be made of a different material, or may be made of a combination thereof. Possible materials may include, but are not limited to, rubber, polymer, composite, metal, plastic, foam, etc. The hand grip **321** may also comprise ergonomic finger groves, nubs, a ribbed texture, a honeycombed texture, etc. to facilitate ease of grasping.

Additionally, as shown in the example embodiment in **FIGS. 4A-4D** the cam **362** may include at least one flat segment **363.** Clockwise rotation of handle assembly **319** causes the cam **362** to rotate into the cam plate **360.** This displaces the cam plate **360** towards fixed gripper **322.** In the closed position, the cam **362** is fully rotated into the cam plate **360** and the flat segment **363** of the cam **362** abuts the right edge (relative to **FIG. 4A**) of the cam plate **360.** Additionally, in the fully closed position, the planate surface of the hand grip **321** may rest against the bottom edge of the cam plate **360.** The flat segment **363** of the cam **362** prevents the restoring force from a compressed return spring **346** (which spring loads the cam plate **360**) from pushing cam plate **360** back to the open position, and thus may effectively lock the clamp apparatus **310** in the closed position.

To open the clamp apparatus **310,** a user rotates the handle assembly **319** counter-clockwise. As the cam **362** releases pressure on the cam plate **360,** the compressed return spring **346** causes the cam plate **360** to automatically return back to the open position as the return spring **346** expands back to a relatively uncompressed state.

In the open position (not shown) the cam plate **360** comes to rest against the right edge (in reference to **FIG. 4A**) of an aperture **342** in the guide plate **340.** The aperture **342** is cut through the guide plate **340** at an angle which is substantially perpendicular to the front face **350** of the guide plate **340.** On the left vertical edge of the aperture **342** a return spring peg **343** may project into the aperture **342** in a direction substantially parallel to the plane of the front face **350** of the guide plate **340.** The return spring peg **343** is slightly smaller in diameter than the return spring **346.** The return spring **346,** may be seated around the return spring peg **343** (as shown in **FIG. 4A**). In the open position, the return spring **346** may be slightly compressed to prevent any "slop" and to keep the cam plate **360** against the right edge of the aperture **342.**

The cam plate **360** is immovably coupled to a pressure plate **370.** In the example embodiment shown in **FIGS. 4A-4E****,** the cam plate is coupled to the pressure plate **370** via screws **361.** In other embodiments, the cam plate **360** and pressure plate **370** may be coupled to each other in any number of ways, including, but not limited to welds, bolts, rivets, etc. In some embodiments, they may be formed as a continuous part during manufacture.

Since the cam plate **360** is attached to the pressure plate **370,** the pressure plate **370** also moves as the cam **362** of the handle assembly **319** displaces the cam plate **360.** When the return spring **346** expands as the clamp apparatus **310** is opened, the pressure plate **370** is also spring loaded to automatically return toward its open orientation. When the clamp apparatus **310** is fully opened, the pressure plate **370** may be approximately flush with the right edge of the guide plate **340** (in reference to **FIG. 4A**). In the example embodiment shown in **FIGS. 4A-4E****,** the pressure plate **370** may not extend out past the right edge of the guide plate **340** because the cam plate **360** to which it is immovably attached is restricted in movement by the right edge of the aperture **342** in the guide plate **340.**

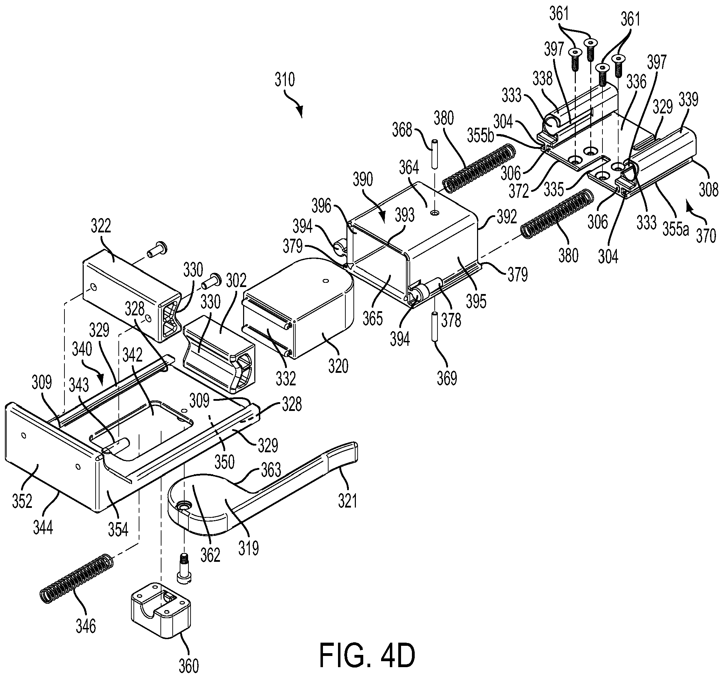

Extending perpendicularly from the center of the left edge **372** of the pressure plate **370** (in reference to **FIG. 4D**) into the pressure plate **370** is a return spring trough **335.** The return spring trough **335** allows the return spring **346** to fit comfortably into the clamp apparatus **310** when the clamp apparatus **310** is fully assembled and operated.

In the example embodiment shown in **FIGS. 4A-4D****,** the pressure plate **370** is slidingly coupled to the guide plate **340** by a tongue-in-groove type association. The top edge **355a** and bottom edge **355b** (relative to **FIGS. 4A-4C****)** of the pressure plate **370** function as the tongues. The top edge **355a** and bottom edge **355b** of the guide plate **370** ride along a track **328** which comprises a part of the guide plate **340** structure. In the embodiment depicted, the track **328** is a recessed groove which is cut out of flanges **329** extended off the top and bottom edges of the guide plate **340.** The flanges **329** project toward the back of the page (in relation to **FIG. 4A**) in a direction substantially perpendicular to the plane of the front face **350** of the guide plate **340.** As shown, the tracks **328** may be cut into the flanges **329** such that the tracks **328** run substantially parallel to the plane of the front face **350** of the guide plate **340.**

The clamp apparatus **310** in the illustrated embodiment in **FIGS. 4A-D** also comprises a gripper sled **390.** The gripper sled **390** may also be coupled to the clamp apparatus **310** by one or a number of tongue-in-groove associations. As shown, the gripper sled **390** may be slidably coupled to the pressure plate **370.** Additionally, at least one spring **380** may be disposed between the gripper sled **390** and pressure plate **370** to exert additional clamping force while the clamp apparatus **310** is in the closed position and a clamped object **100** is present.

In an example embodiment, the gripper sled **390** is a generally a hollow, mostly rectangular sleeve open on its right end **392** and left end **393** (relative to **FIG. 4A**). The sliding gripper base **320** may fit into, hollow interior of the sleeve-like gripper sled **390.** Other embodiments may close the left end **393** of the gripper sled **390** and attach the sliding gripper **302** to it such that the left end **393** of the gripper sled **390** performs the function of the sliding gripper base **320.**

In the exemplary embodiment shown in **FIGS. 4A-4D****,** the sliding gripper base **320** is immovably coupled inside the hollow interior of the gripper sled **390.** This may be accomplished in any number of ways. As shown, the sliding gripper base **320** may be coupled into the gripper sled by a first dowel **368** and a second dowel **369.** Other embodiments which employ dowels may use any suitable number of dowels. The first dowel **368** may be inserted through an orifice in the in the back face **364** of the gripper sled **390** into a corresponding orifice in the back face of the sliding gripper base **320** (directions refer to orientation of **FIG. 4A**). The second dowel **369** may be inserted through an orifice in the front face **365** of the gripper sled **390** into a corresponding orifice in the front face of the sliding gripper base **320.**

In the example embodiment shown in **FIGS. 4A-4D****,** the second dowel **369** is not flush with the front face **365** of the gripper sled **390.** Instead, at least a portion of the second dowel **369** projects past the front face **365** of the gripper sled **390.** At least a part of this portion of second dowel **369** rides along a slit **329** which is cut into the edge of the pressure plate **370** opposite the return spring trough **335.** As shown, the slit **329** may be cut into the said edge of the pressure plate **370** at an angle substantially perpendicular to said edge. The interaction of the slit **329** and second dowel **369** effectively restricts the movement of the gripper sled **390.** When the second dowel **369** abuts the left end of the slit **329,** the second dowel **369** and all attached components may travel no further toward the left of the page (in relation to **FIG. 4A**).

The gripper sled **390** may also comprise a set of ears **394.** As shown in the example embodiment in **FIGS.** 4A-D, one of the ears **394** may project off the top face **395** of the gripper sled **390** while the other projects off the bottom face **396** of the gripper sled **390.** In the embodiment illustrated in **FIGS. 4A-4D****,** each ear **394** comprises a post which supports a round cylinder whose elongate section runs in a direction parallel to the plane of the front face **365** of the gripper sled **390.** The ears **394** project off the top face **395** and bottom face **396** of the gripper sled **390** at an angle substantially perpendicular to the top face **395** and bottom face **396** of the gripper sled **390.** In alternate embodiments, the shape, thickness, construction, orientation, etc. of the ears **394** may differ. Additionally, some embodiments may comprise a compression spring peg **378** which projects off each ear **394.** The compression spring pegs **378** are similar to the return spring peg **343.**

In an embodiment of the present disclosure, the top and bottom edges of the front face **365** of the gripper sled **390** may comprise gripper sled tongues **379** which run at least partially along at least one of the top and bottom edges of the front face **365** of the gripper sled **390.** In the example embodiment shown in **FIGS. 4A-4D****,** the gripper sled tongues **379** project off the entire length of the top and bottom edges of the front face **365** of the gripper sled **390** and are extensions of the plane of the front face **365** of the gripper sled **390.**

Extending from the rear face **336** of the pressure plate **370** and oriented approximately parallel to the return spring trough **335** may be a top spring housing **339,** and a bottom spring housing **338.** In an exemplary embodiment shown in **FIGS. 4A-4D****,** the top spring housing **339** and bottom spring housing **338** both comprise a raised ridge **304** and a compression spring pocket **333.** The raised ridge **304** projects off the rear face **336** of the pressure plate **370** at an angle substantially perpendicular to rear face **336** of the pressure plate **370.** The raised ridges **304** run parallel to the top edge **355a** and bottom edge **355b** of the pressure plate **370.** As shown, the raised ridges **304** may span the entire length of the pressure plate **370.** The raised ridges **304** function as a post on which the compression spring pockets **333** of the top spring housing **339** and bottom spring housing **338** are coupled. As shown in the example embodiment in **FIGS. 4A-4D** the compression spring pockets **333** may be elongated along the entire length of the ridges **304.**

The compression spring pockets **333** overhang the ridges **304** forming "T" type shapes. The portions of the "T" type shapes facing the lateral center line of the pressure plate **370** form the grooves **306** of a tongue-in-groove arrangement in conjunction with the rear face of the pressure plate **370.** The gripper sled tongues **379** are slidably coupled into these grooves **306.**

The opposite portions of the "T" type shapes (those distal to the lateral centerline of the pressure plate **370)** also form the grooves **308** of another tongue-in-groove type arrangement in conjunction with the rear face of the pressure plate **370.** In the embodiment shown in **FIGS. 4A-4D****,** the distal grooves **308** slidably couple around tongues **309** formed by a part of the flanges **329** which are extended off the guide plate **340.**

The compression spring pockets **333** may be hollow so as to allow compression springs **380** to be seated inside the compression spring pockets **333.** In the embodiment shown in **FIGS. 4A-4D****,** the right end (relative to **FIG. 4A****)** of the compression spring pockets **333** is closed to provide a surface upon which the compression springs **380** may be compressed against. Additionally, the compression spring pockets **333** each feature a slot **397** (best shown in **FIG. 4D****)** which is cut out of the face of the compression spring pockets **333** most proximal to the lateral centerline of the pressure plate **370.**

When assembled, as detailed above, a compression spring **380** may be seated in each of the compression spring pockets **333.** One end of the compression springs **380** abuts the closed ends of the compression spring pockets **333.** The other ends of the compression springs **380** abut the right faces of the ears **394** which protrude off the top face **395** and bottom face **396** of the gripper sled **390.** The compression springs **380** fit around the compression spring pegs **378** which may extend from the ears **394** on the gripper sled **390.** This helps to keep the compression springs **380** firmly in place during operation and use of the clamp apparatus **310.** The compression springs **380** bias the gripper sled **390** and components immovably attached to it (notably sliding gripper **302** and sliding gripper base **320)** to the left of the page (relative to **FIG. 4A****)** until the second dowel **369** abuts the left end of the slit **329** and the components may move no further to the left of the page. This ensures that as the handle assembly **319** is actuated, the cam plate **360,** pressure plate **370,** gripper sled **390,** and attached components move together as a unit until the sliding gripper **302** encounters a clamped object **100.**

In the shown embodiment in **FIGS. 4A-4D****,** the diameter of the hollow portions of the compression spring pockets **333** is slightly larger than the diameter of the cylinder portion of the ears **394.** The slot **397** in the compression spring pockets **333** creates a path for the post portion of ears **394** to travel. When a force sufficient to overcome the bias force of the compression springs **380** is applied, the compression springs **380** begin to compress.

Such a force may be generated when a user rotates the handle assembly **319** and a clamped object **100** is present. As mentioned above, in the embodiment shown in **FIGS. 4A-4B****,** the cam plate **360,** pressure plate **370,** gripper sled **390** and attached components move together substantially as a unit until the sliding gripper **302** encounters a clamped object **100.** When the clamped object **100** comes into contact with the sliding gripper **302,** the sliding gripper **302** begins to push the clamped object **100** against the fixed gripper **322.** When the force which the clamped object **100** exerts back against the sliding gripper **302** becomes greater than the bias force of the compression springs **380,** the sliding gripper **302,** sliding gripper base **320,** gripper sled **390** and components immovably coupled to the gripper sled **390** stopping moving. The cam plate **360** and pressure plate **370** continue to move toward their closed orientation as the handle assembly **319** rotates to its closed orientation. This causes the compression springs **380** to begin to compress. As the compression springs **380** are compressed the ears **394** slide progressively further into the hollow portions of the compression spring pockets **333** and along the slots **397** of the compression spring pockets **333** until the clamp apparatus **310** reaches its fully closed orientation.

The force exerted by the compressed compression springs **380** on the clamped object **100** through the gripper sled **390** and sliding gripper **302** helps to create a more vigorous gripping force than could otherwise be achieved. Additionally, the restoring force of the compression springs **380** is complimentary to that provided by the return spring **346** when the clamp apparatus **310** is moved to the open position. The compression spring **380** restoring force causes the gripper sled **390** and immovably attached components to return back to their default orientation along slit **329** in the pressure plate **370.** The force exerted by the compressed compression springs **380** additionally facilitates opening of the clamp apparatus **310.**

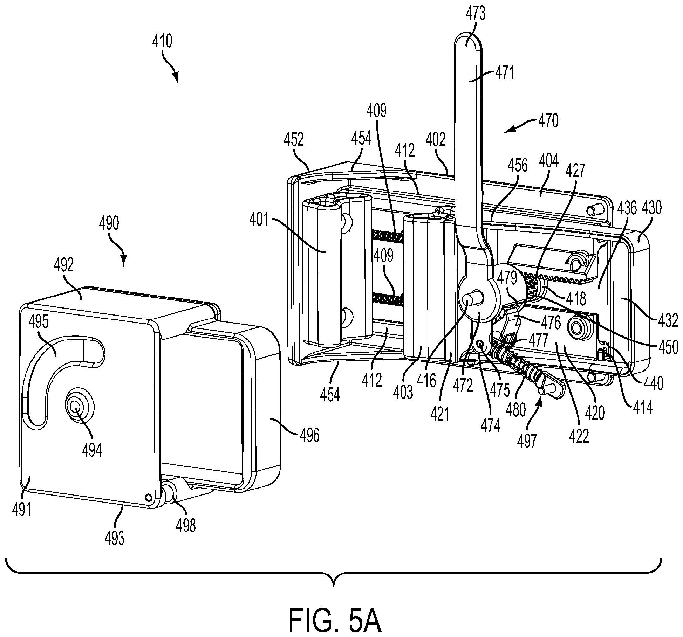

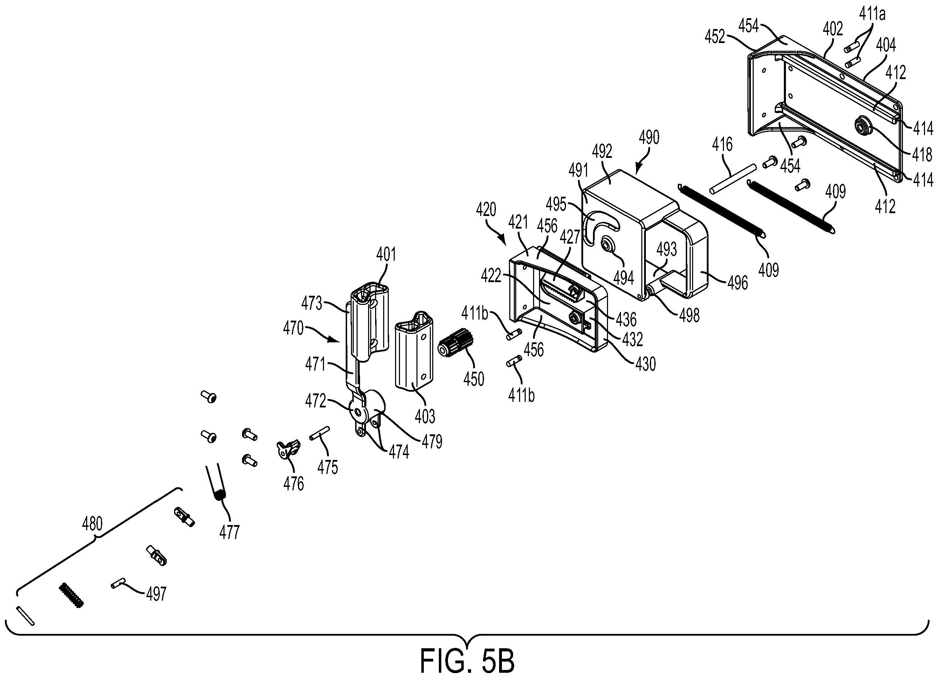

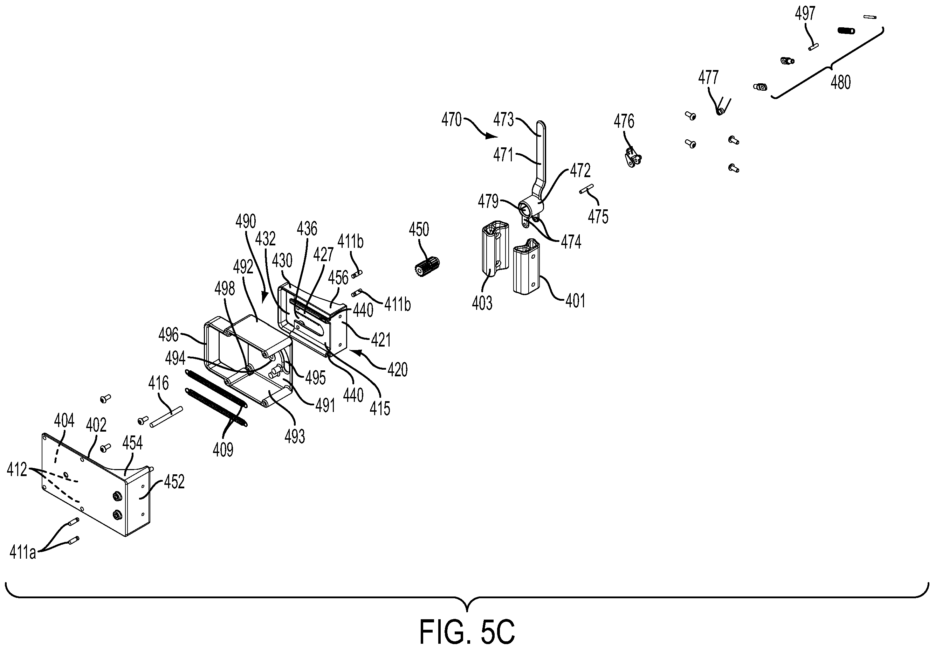

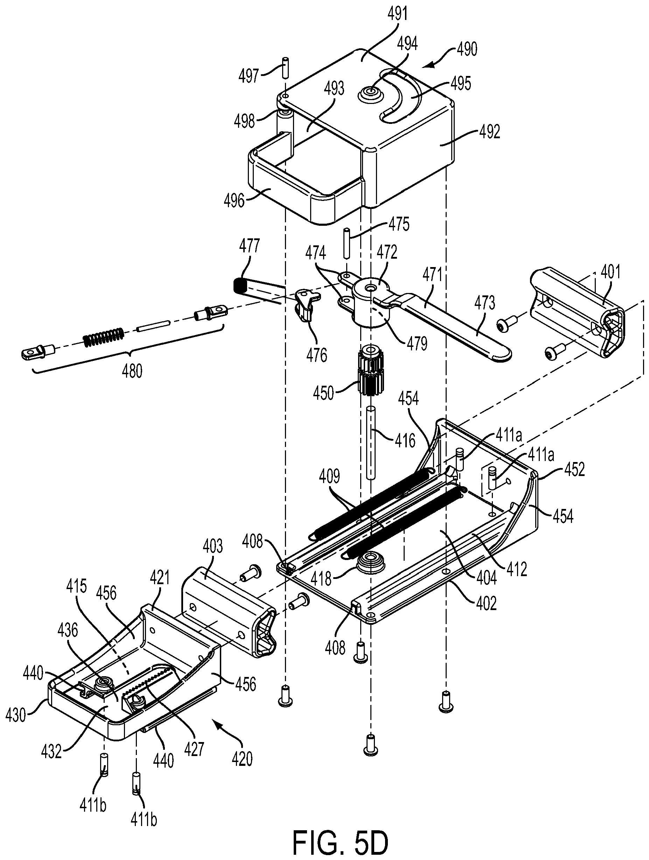

In an embodiment of the present disclosure shown in **FIGS. 5A-5D****,** the restoring force from a pair of tensioned springs **409** acts to clamp a clamped object **100** between a fixed gripper **401** and a sliding gripper **403.** The sliding gripper **403** can then be locked in place by a ratcheting pawl **476,** thus securing clamp apparatus **410** in the clamped position about a clamped object **100.**

In an exemplary embodiment, a fixed gripper **401** may be firmly attached to the front face **404** of an approximately rectangular back plate **402.** The gripping surface of the fixed gripper **401** is oriented perpendicularly to the front face **404** of the back plate **402.** In the embodiment shown in **FIGS. 5A-5D****,** a fixed gripper support wall **452** may be attached to the front face **404** of the back plate **402.** As shown, the fixed gripper support wall **452** may project from the left edge (in relation to **FIG. 5A****)** of the back plate **402** in a direction perpendicular to the front face **404** of the back plate **402.** Instead of attaching the fixed gripper **401** to front face **404** of the back plate **402,** the fixed gripper **401** may be fixedly coupled to the right face (in relation to **FIG. 5A****)** of the fixed gripper support wall **452.** This is desirable because the fixed gripper support wall **452** is able to provide additional support for the fixed gripper **401.** The fixed gripper support wall **452** may optionally be supported by one or more buttresses **454** that span from at least a portion of the back plate **402** to the fixed gripper support wall **452.** In some embodiments, the buttresses **454** may be arched to maximize support.

The fixed gripper **401** may consist of a material chosen for its gripping ability. The fixed gripper **401** may be made of a high friction material, a compressible material, a material exhibiting both these qualities, or any other suitable material. The fixed gripper **401** may be made of a material which allows a firm grip without the deformation of a clamped object **100.** Suitable materials may include any suitable elastomeric or non-deformable substance, including but not limited to plastic, rubber, metal, foam, fabric, gel, etc. At least a portion of the fixed gripper **401** may comprise a roughly semi-circular depression or contour to accommodate a round clamped object **100** such as a pole.

In some embodiments, the fixed gripper **401** is formed from a relatively inelastic material, but has a cap **458** (not shown) that fits substantially over the fixed gripper **401.** The cap **458** may be constructed from any suitably material, including but not limited to, elastic materials such as rubber, plastic, gel, foam, fabric, polyurethane, etc. The cap **458** may be replaceable and removably attached to the fixed gripper **401.**

In some embodiments, in addition to comprising the mounting site for the fixed gripper **401,** the support plate **402** also includes an attachment site **418** for a gear assembly and a track-way **412** for a rack plate **420.** The gear assembly attachment site **418,** track-way **412,** and rack plate **420** will be elaborated on in subsequent paragraphs.

In an example embodiment, the sliding gripper **403** is firmly attached to the front face **422** of a rack plate **420** such that the gripping surface of the sliding gripper **403** faces the gripping surface of the fixed gripper **401.** As shown in **FIGS. 5A-5D****,** the sliding gripper **403** is coupled to the front face **422** of the rack plate **420** near the edge of the rack plate **420** most proximal to the fixed gripper **401.** In some embodiments, the rack plate **420** may have the shape of a quadrilateral, specifically a rectangle. Some embodiments include a sliding gripper support base **421** which may be similar in varying degrees to the fixed gripper support wall **452.** The sliding gripper support base **421** may optionally have one or more buttresses **456** that span from at least a portion of the rack plate **420** to the sliding gripper support base **421.** In some embodiments, the buttresses **456** may be arched to maximize support.

The sliding gripper **403** may consist of a material chosen for its gripping ability. The sliding gripper **403** may be made of a high friction material, a compressible material, a material exhibiting both these qualities, or any other suitable material. The sliding gripper **403** may be made of a material which allows a firm grip without the deformation of a clamped object **100.** Suitable materials may include any suitable elastomeric or non-deformable substance, including but not limited to plastic, rubber, metal, foam, fabric, gel, etc. At least a portion of the sliding gripper **403** may comprise a roughly semi-circular depression or contour to accommodate a round clamped object **100** such as a pole.

In some embodiments, the sliding gripper **403** is formed from a relatively inelastic material, but has a cap **458** (not shown) that fits substantially over the sliding gripper **403.** The cap **458** may be constructed from any suitably material, including but not limited to, elastic materials such as rubber, plastic, gel, foam, fabric, polyurethane, etc. The cap **458** may be replaceable and removably attached to the fixed gripper **403.**

In the example embodiment shown in **FIGS. 5A-5D****,** the rack plate **420** is roughly rectangular. A handle **430** may project off the edge of the of the rack plate **420** most distal to the fixed gripper **401.** The handle **430** may be a part of a "U" shaped member. As shown, the bottom of the "U" shape and at least a portion of each upright of the "U" shape protrude from rack plate **420** forming a void **432.** The void **432** is defined by the edge of the rack plate **420** and the protruding sections of the "U" shaped handle **430.** A user's finger(s) may easily grip around the bottom of the "U" shape of the handle **430** via this void **432** when a user desires to manipulate the position of the rack plate **420.**

In the example embodiment shown in **FIGS. 5A-5D****,** at least a section of the uprights of the "U" shape of the handle **430** couple the handle **430** to the rack plate **420.** The uprights of the "U" shape of the handle **430** may project off the top and bottom spans (directions relative to orientation in **FIG. 5A****)** of the perimeter of the front face **422** of the rack plate **420** toward the front of the page at an angle substantially perpendicular to the front face **422** of the rack plate **420.** The rack plate **420** and handle **430** may be formed as a continuous part during manufacture. Additionally, the top sections of the uprights of the "U" shape of the handle **430** may comprise the buttresses **456** that span from at least a portion of the rack plate **420** to the sliding gripper support base **421.** In alternate embodiments, the handle **430** may be coupled to the rack plate **420** in any of a variety of ways and may take any suitable shape or size.

At least a portion of the handle **430** may be made of a material such as, but not limited to, rubber, polymer, composite, metal, plastic, foam, etc. Additionally, the handle **430** may comprise ergonomic finger groves, nubs, a ribbed texture, a honeycombed texture, etc.

The front face **404** of the back plate **402** may comprise at least one track-way **412** that runs substantially the full length of the width of the back plate **402.** In the embodiment shown in **FIGS. 5A-5B****,** twin track-ways **412** on the front face **404** of the back plate **402** run in parallel fashion from the edge of the back plate **402** on which the fixed gripper **401** is affixed to the opposite edge of the back plate **402.** The twin track-ways **412** run along planes parallel to the top and bottom edges (in reference to **FIG. 5A****)** of the back plate **402.** The track-ways **412** may support and guide the rack plate **420** as the clamp apparatus **410** is moved between its clamped and unclamped orientations.

In the exemplary embodiment shown in **FIGS. 5A-5D****,** each of the track-ways **412** comprise a groove **414** which is recessed into each track-way **412.** The groove **414** is recessed into the side of each track-way **412** which faces the other track-way **412.** This causes the track-ways **412** to have an "L" shape. The rear face **415** of the rack plate **420** comprises projections **440** which are dimensioned such that they may be received by the groove **414** in the track-ways **412** on the back plate **402.** This tongue-in-groove type arrangement slidingly and securely couples the back plate **402** and rack plate **420** together.

The clamp apparatus **410** is biased toward the closed position by at least one extension spring **409.** In the embodiment shown in **FIGS. 5A-5D****,** the clamp apparatus **410** comprises two extension springs **409.** One end of each extension spring **409** is hooked around an extension spring peg **411a.** Each extension spring peg **411a** projects toward the front of the page (relative to **FIG. 5A****)** from the back plate **402** at an angle perpendicular to the front face **404** of the back plate **402.** The other end of each extension spring **409** is hooked to another extension spring peg **411b.** Each extension spring peg **411b** projects toward the rear of the page (relative to **FIG. 5A****)** from the rear face **415** of the rack plate **420** and an angle substantially perpendicular to the rear face **415** of the rack plate **420.**

The extension spring pegs **411a** and **411b** may comprise a feature such as a notch to help ensure the extension springs **409** do not come off the extension spring pegs **411a** and **411b.** In some embodiments, the extension spring pegs **411a** and **411b** may be substituted for by a variety of different attachment means. In some embodiments, hooks, rings, eye bolts, U bolts, or any other arrangement obvious to one skilled in the art may be used. In other embodiments, the clamp apparatus **410** may not use extension springs **409** and instead use any other type of spring such as, but not limited to, a gas spring using a bladder, piston type arrangement, a compression spring, a compression spring made of a compressible, springy material such as rubber, an extension spring, a constant force spring, etc.

In an example embodiment, the non-tensioned length of the extension springs **409** is somewhat smaller than the distance between a set of extension spring pegs **411a** and **411b.** This is desirable because it ensures that the rack plate **420** and attached sliding gripper **403** are always biased against the fixed gripper **401** and that there is no "slop" in the clamp apparatus **410.** Pulling the rack plate **420** and attached sliding gripper **403** away from the fixed gripper **401** (i.e. toward the open position) thus may tension the extension springs **409,** and further spring load the clamp apparatus **410** toward the closed position. When the rack plate **420** is released, the clamp apparatus **410** will automatically default back toward its closed orientation due to the restoring force of the extension springs **409.**

In the exemplary embodiment depicted in **FIGS. 5A-5D****,** a user may open the clamp apparatus **410** by pulling the handle **430** as well as the attached rack plate **420** and sliding gripper **403** away from the fixed gripper **401.** While the clamp apparatus **410** is held in the open position, a clamped object **100** may be placed in the space between the fixed gripper **401** and the sliding gripper **403.** The clamp apparatus **410** may then be allowed to automatically return to the closed position by a user's release of the handle **430.**

Other embodiments, including the embodiment shown in **FIGS. 5A-5D****,** may comprise additional features which provide additional clamping force, make the clamp easier to operate, etc. In addition to the tongue-in-groove type arrangement mentioned above, an embodiment of the present disclosure comprises a lockable ratcheting rack and pinion type connection which may additionally be utilized to inform the movement of the rack plate **420.**

In some embodiments, a gear assembly attachment site **418** may comprise a projection jutting from the front face **404** of the back plate **402.** The gear assembly attachment site **418** is adapted to receive a gear shaft **416.** In an example embodiment, the gear shaft **416** is a rod or dowel made of metal, plastic, or other suitably durable material. The gear shaft **416** may allow a pinion gear **450** to freely rotate about the axis of the gear shaft **416.** In some embodiments, the gear assembly attachment site **418** may take the shape of a raised ring. In embodiments where the gear assembly attachment site **418** is shaped like a raised ring, the center, open section of the ring may have an internal diameter slightly, though not substantially larger than the diameter of gear shaft **416.** The gear shaft **416** may fit securely and non-rotatably within the internal diameter raised ring of the gear assembly attachment site **418.** A pinion gear **450** may be placed on the gear shaft **416.**

The rack plate **420** may comprise a slot that defines a pinion aperture **436** sized to allow the pinion gear **450** to protrude through the aperture **436** toward the front of the page (relative to **FIG. 5A**). As shown in the embodiment in **FIGS. 5A-5D****,** a rack **427** is positioned adjacent the aperture **436** such that the teeth of the rack **427** interdigitate with the teeth of the pinion gear **450.** Since the teeth of the rack **427** and teeth of the pinion gear **450** interdigitate, the pinion gear **450** rotates about the axis of the gear shaft **416** when the rack plate **420** is moved toward or away from the fixed gripper **401.**

The interaction of the teeth of the rack **427** and the teeth of the pinion gear **450** may be exploited via a ratcheting assembly **470** to ratchet the rack plate **420** and attached sliding gripper **403** against a clamped object **100.** This is desirable because it allows a user to generate more clamping force than the extension springs **409** alone are capable of generating. The ratcheting assembly **470** may also enable a user to lock the clamp apparatus **410** against a clamped object **100.**

As shown in the exemplary embodiment illustrated in **FIGS. 5A-5D****,** the ratcheting assembly **470** comprises a ratcheting lever **471.** The ratcheting lever **471** comprises a ratcheting lever hub **472.** The ratcheting lever hub **472** may be shaped like a cup which fits over the section of the pinion gear **450** protruding past the rack **427** of the rack plate **420.** The front face (relative to **FIG. 5A**) of the pinion gear **450** may abut the bottom of the cup formed by the ratcheting lever hub **472.** The ratcheting lever hub **472** comprises an orifice which may allow the ratcheting lever hub **472** to be slid onto the gear shaft **416.** In such embodiments, the gear shaft **416** becomes a fulcrum for the ratcheting lever **471.** The ratcheting lever hub **472** may also comprise an opening **479** in the wall of the ratcheting lever hub **472** cup which exposes a number of teeth of the pinion gear **450.**

The ratcheting lever **471** may further comprise a ratcheting lever handle **473.** In the example embodiment in **FIGS. 5A-5D****,** the ratcheting lever handle **473** acts as the input side of the ratcheting lever **471.** The ratcheting lever handle **473** may be grasped by a user and rotated about the axis of the gear shaft **416** to provide an input.

The ratcheting lever handle **473** may be made of the same material as the rest of the ratcheting lever **471,** may be made of a different material, or may be made of a combination thereof. Possible materials may include, but are not limited to, rubber, polymer, composite, metal, plastic, foam, etc. Additionally, the ratcheting lever handle **473** may comprise ergonomic finger groves, nubs, a ribbed texture, a honeycombed texture, etc.

The ratcheting lever **471** may further comprise at least two ratcheting lever posts **474** opposite the ratcheting lever handle **473** which function as the output side of the ratcheting lever **471.** The ratcheting lever posts **474** extend parallel to each other. One ratcheting lever post **474** is extended from the bottom section of the cup of the ratcheting lever hub **472.** The other ratcheting lever post **474** may be extended off the rim section of the cup of the ratcheting lever hub **472.** A ratcheting lever dowel **475** may span the distance between the ratcheting lever posts **474.** A ratcheting pawl **476** and torsion spring **477** may be positioned on the ratcheting lever dowel **475** between the two ratcheting lever posts **474.**

In the exemplary embodiment shown in **FIGS. 5A-5D** a user provides an input to the ratcheting lever lock **471** by rotating the ratcheting lever handle **473** substantially 90° counter-clockwise (relative to **FIG. 5A**) from the unlocked position to the locked position. In the unlocked position, the ratcheting lever handle **473** is oriented perpendicular to the top edge (relative to **FIG. 5A**) of the back plate **402** and the ratcheting pawl **476** is retracted away from the teeth of the pinion gear **450.**

As the ratcheting lever handle **473** is rotated to the locked position, the ratcheting pawl **476** rotates into and engages the teeth of the pinion gear **450** through the opening **479** in the ratcheting lever hub **472.** The torsion spring **477** applies a force against the ratcheting pawl **476** which keeps it in engagement with the teeth of the pinion gear **450.** As a user continues to rotate the ratcheting lever handle **473** the ratcheting pawl **476** catches a tooth of the pinion gear **450** and forces the pinion gear **450** to rotate with the ratcheting lever **471.** This rotation of the pinion gear **450** is transmitted to the rack **427** causing the rack **427** and the attached rack plate **420** and sliding gripper **403** to move toward the fixed gripper **401.** If a clamped object **100** is present, this movement squeezes the clamped object **100** against the fixed gripper **401** with more clamping force than the tensioned extension springs **409** alone can generate. The ratcheting pawl **476** additionally locks the clamp apparatus **410** into the ratcheted and closed position because the ratcheting pawl **476** obstructs any rotation of the pinion gear **450** in a direction which would result in movement of the rack **427,** rack plate **420** and attached sliding gripper **403** toward the open position.

In some embodiments, including the embodiment depicted in **FIGS. 5A-5D****,** the clamp apparatus **410** may comprise a cover **490.** In the embodiment shown in **FIGS. 5A-5D****,** the cover **490** has a front plate **491.** Extending perpendicularly off the top and bottom of the rear face (directions refer to orientation in **FIG. 5A****)** of the front plate **491** are a top plate **492** and a bottom plate **493.** The rear edges of the top plate **492** and the bottom plate **493,** which run parallel to the plane of the front plate **491,** may be immovably coupled to the cover **490** to the front face **404** of the back plate **403** via screws, or any other suitable fastening method. The right edge (relative to **FIG. 5A**) of the bottom plate **493** has a cutout **498.** A dowel **497** may run from the front plate **491** through the cutout **498.**

The front plate **491** of the cover **490** may comprise a second gear assembly attachment site **494.** The second gear assembly attachment site **494** may comprise an orifice which has a diameter slightly, though not substantially larger than the diameter of the gear shaft **416.** The gear shaft may fit securely and non-rotatably into the orifice of the second gear assembly attachment site **494.**

In some embodiments, the front plate **491** may comprise a ratcheting lever handle slot **495** through which the ratcheting lever arm **473** may extend. The ratcheting lever handle slot **495** may arc so as to allow uninhibited travel of the lever handle **473** from the unlocked position to the locked position.

In one embodiment, the cover **490** has a palm support **496.** The palm support **496** may be formed as a U-shaped member projecting from the cover **490** in a manner and direction similar to that of the handle **430** of the rack plate **420.** The palm support **496** is adapted for use as a carrying handle. The palm support **496** may also be utilized to aid in easy, one-handed opening of the clamp apparatus **410.** A user may place the palm support **496** in their palm and grasp the handle **430** by placing their finger(s) in the void **432.** By clenching their fist, a user may then transition the clamp apparatus **410** to the open position.

The palm support **496** may be made of the same material as the rest of the cover **490,** may be made of a different material, or may be made of a combination thereof. Possible materials may include, but are not limited to, rubber, polymer, composite, metal, plastic, foam, etc. Additionally, the palm support **496** may comprise ergonomic finger groves, nubs, a ribbed texture, a honeycombed texture, etc. to aid in carrying or grasping.

In some embodiments, the clamp apparatus **410** may comprise an over-center linkage **480** to help ensure the ratcheting lever lock **471** stays in a desired position. As shown in the embodiment in **FIGS. 5A-5D****,** the over-center linkage **480** is attached at one end to the dowel **497** running through the cutout **498** in the cover **490.** The other end of the over-center linkage **480** is attached to the ratcheting lever dowel **475** adjacent the ratcheting pawl **476** and torsion spring **477.** The over-center linkage **480** may bias the ratcheting lever lock **471** to stay in either the unlocked position or locked position. When the over-center linkage **480** is in the over center position the clamp apparatus **410** is kept in the locked position. Before the over-center linkage **480** reaches an over-center position, the clamp apparatus **410** is kept in the unlocked position.

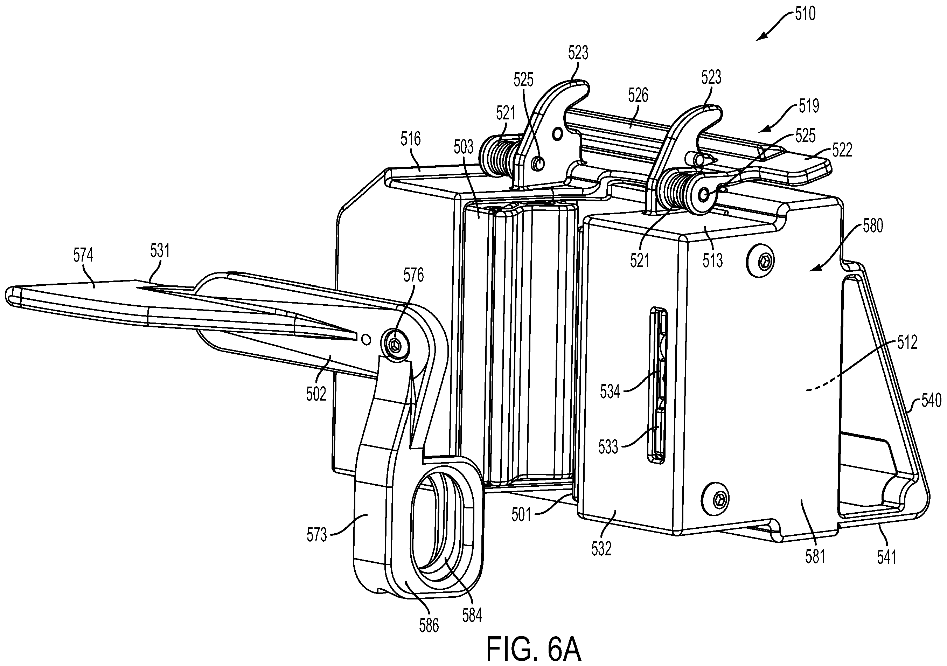

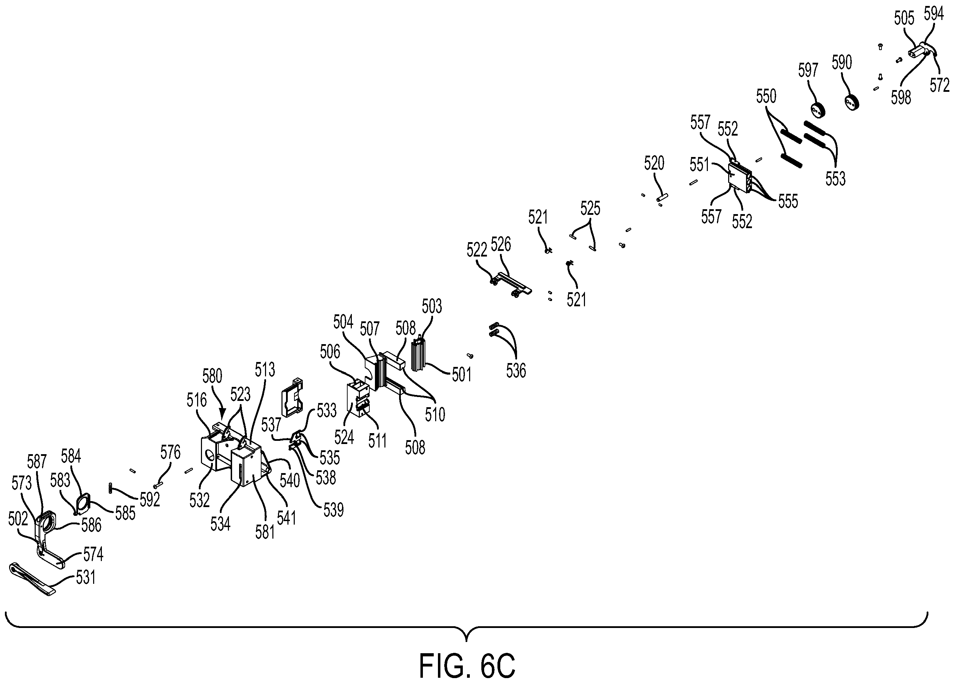

In another example embodiment of the present disclosure shown in **FIG. 6A-6G****,** a sliding gripper **503** is coupled to a sliding gripper base **504** and may be capable of movement towards a fixed gripper **501** mounted on a fixed gripper base **524.** As the sliding gripper **503** is displaced towards the fixed gripper **501,** a clamped object **100** placed between the fixed gripper **501** and sliding gripper **503** may be clamped between the fixed gripper **501** and sliding gripper **503.** As a clamped object **100** is clamped, at least one compression spring **550** compresses. The restoring force of the compressed compression spring **550** supplies additional clamping force as it pushes the sliding gripper **503** against the clamped object **100.** An actuator handle latch **584** locks the clamp apparatus **510** in the closed position, safely securing the clamp apparatus **510** and its attached load (for example, a medical device) to a clamped object **100.**

The fixed gripper **501** and sliding gripper **503** may be comprised of a material chosen for its gripping ability. The fixed gripper **501** and sliding gripper **503** may be made of high friction materials, compressible materials, materials exhibiting both these qualities, or any other suitable material. The fixed gripper **501** and sliding gripper **503** are made of materials which allow for a firm grip without the deformation of a clamped object **100.** Suitable materials may include any suitable elastomeric or non-deformable substance, including but not limited to plastic, rubber, metal, foam, fabric, gel, etc. At least a portion of the fixed gripper **501** and sliding gripper **503** may comprise roughly semi-circular depressions or contours to accommodate a round clamped object **100** such as a pole.

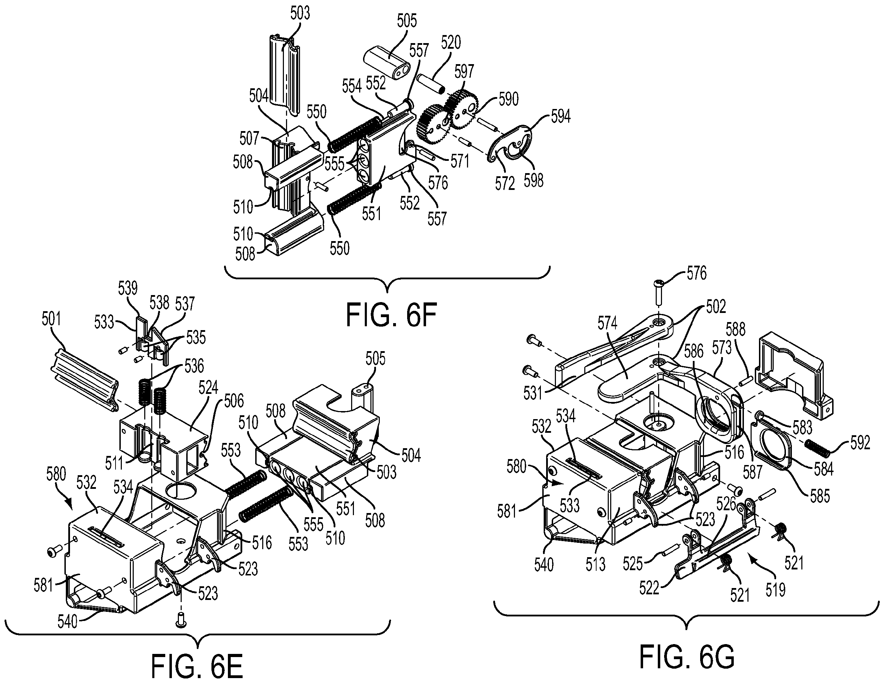

In the example embodiment shown in **FIGS. 6A-6G****,** the fixed gripper **501** is mounted to a fixed gripper base **524.** The fixed gripper base **524** comprises a fixed gripper attachment site **506.** The fixed gripper attachment site **506,** faces the sliding gripper **503.** As best shown in **FIG. 6E****,** the fixed gripper attachment site **506** may be a depression dimensioned to fit the contour of the fixed gripper **501.** In some embodiments, the fixed gripper attachment site **506** may frictionally retain the fixed gripper **501** by means of a friction fit. In alternate embodiments, the fixed gripper **501** may be coupled to the fixed gripper attachment site **506** by any of a variety of means including, but not limited to, screws, bolts, ultrasonic welds, magnets, adhesive, hook and loop tape, or any other suitable coupling means.

The fixed gripper base **524** may be a substantially rectangular block which fits into a cavity of the housing **580** of the clamp apparatus **510.** One side of the fixed gripper base **524** may be fixedly coupled to the right face **581** (relative to **FIG. 6A**) of the housing **580.** The fixed gripper base **524** may be coupled to the right face **581** of the housing **580** by any of a number of means, such as screws, bolts, ultrasonic welds, magnets, adhesive, or any other suitable coupling means. The fixed gripper base **524** may also comprise a strike plate spring bay **511.** The strike plate spring bay **511** will be elaborated upon later.

As best shown in **FIG. 6F****,** the sliding gripper base **504** may comprise both a sliding gripper attachment site **507** and a guide rail **508** to guide movement of the sliding gripper **503.** The sliding gripper attachment site **507** is located on the face of the sliding gripper base **504** which faces the left of the page (relative to **FIG. 6F**). As shown in **FIG. 6F****,** the sliding gripper attachment site **507** may be depression dimensioned to fit the contour of sliding gripper **503.** In some embodiments, including the embodiment in **FIGS. 6A-6G****,** gripper attachment site **507** may frictionally retain the sliding gripper **503** by means of a friction fit. In alternate embodiments, the sliding gripper **503** may be coupled to the sliding gripper attachment site **507** by screws, bolts, ultrasonic welds, magnets, adhesive, or any other suitable coupling means.

Offset from the sliding gripper attachment site **507** may be at least one guide rail **508.** In the example embodiment in **FIG. 6A-6G****,** there are two guide rails **508.** The guide rails **508** are offset from the sliding gripper attachment site **507** toward the front of the page (relative to **FIG. 6F**) and run perpendicular to the face of the sliding gripper base **504** on which the sliding gripper attachment site **507** is disposed. In some embodiments, a guide recess **510** may be defined along/into at least one surface of the guide rail(s) **508.** The at least one guide rail **508** and guide recess **510** will be elaborated upon later.

Some embodiments may also include a slider sled **551.** In some embodiments, the slider sled **551** is involved in four interrelated functions. First, the slider sled **551** provides a pre-defined track-way for the guide rails **508** of the sliding gripper base **504.** Second, the slider sled **551** may support at least one compression spring **550.** The compression spring(s) **550** may ensure that the slider sled **551,** sliding gripper base **504** and attached components move together as a unit until the sliding gripper **503** abuts a clamped object **100.** When the clamp apparatus **510** is locked in the closed position and the compression spring(s) **550** are compressed, the restoring force exerted by the compressed compression springs **550** provides additional clamping force against a clamped object **100.** Third, the slider sled **551** may comprise at least one return spring pocket **555.** A return spring **553** may be placed in each of the return spring pocket(s) **555.** The return springs **553** may bias the clamp apparatus **510** toward the open position and automatically return the slider sled **551** to the open position when the user actuates the clamp apparatus **510** into the open position. Fourth, the slider sled **551** may comprise a catch **571** which may act as a stop during user actuation of the clamp apparatus **510.**

In relation to the first function, the guide recess **510** is sized to fit a complimentary guide projection **554** located on at least one face of the slider sled **551.** In the embodiment shown in **FIGS. 6A-6G****,** the guide projections **554** run the length of the top face **558** and bottom face **556** of the slider sled **551.** The guide projections **554** may serve as a track-way to direct the slider gripper base **504** as it moves between an open and closed position. In one embodiment, the guide projection(s) **554** are raised ridges running the length of the top face **558** and bottom face **556** and fit into the guide recesses **510** on slider gripper base **504.** Alternatively, the guide projection **554** may be located on slider gripper base **504** or the guide rail(s) **508** of the slider gripper base **504** for movement along a guide groove **510** located on slider sled **551.** Other embodiments may use other guide configurations.

In some embodiments, the guide rail **508** may be hollow and the guide recess **510** may be a slot which is cut through the guide rail **508** and into the hollow portion of the guide rail **508.** The guide rail **508** may be open on one end and a compression spring **550** may be placed into the hollow portion of the guide rail **508** through this opening.

In relation to the second function, at least one of the guide projection(s) **554** on the slider sled **551** may feature a compression spring peg **552** on which one side of a compression spring **550** is seated. In one embodiment, the compression spring peg **552** is an essentially cylindrical structure with an end piece **575** that has a diameter greater than the diameter of its associated compression spring **550.** Movement of slider sled **551** relative to the sliding gripper base **504** compresses the compression spring **550** between the end piece **575** and the end wall of the hollow guide rail **508.** As the compression spring **550** is compressed, the compression spring peg **552** moves into the hollow of the guide rail(s) **508.** Such movement may occur when the clamp apparatus **510** is moved from the open position to the closed position and a clamped object **100** is present. Selection of a compression spring **550** of appropriate elasticity allows the restoring force generated during compression to be sufficient to return the sliding gripper **503** and sliding gripper base **504** to the open position, while at the same time not unduly opposing user actuation of the clamp apparatus **510.**

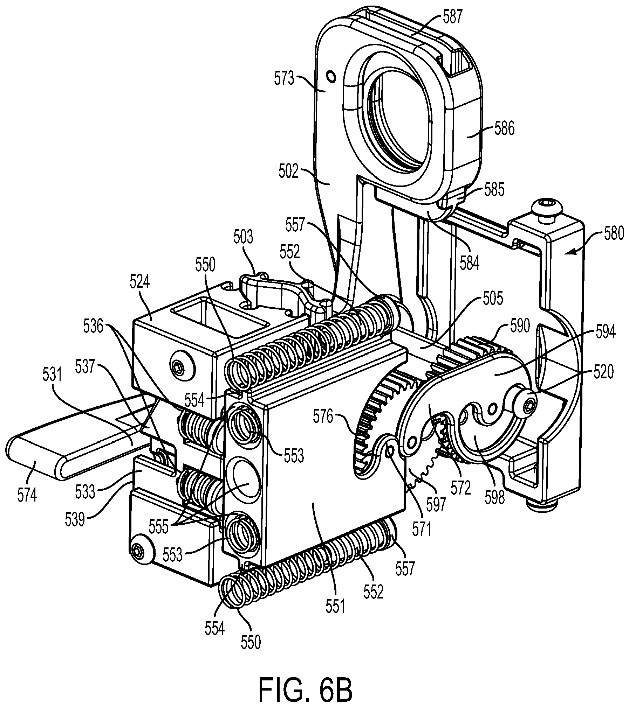

Relative to the third function, in some embodiments, the slider sled **551** may include at least one return spring **553** (best shown in **FIG. 6B**) which helps to bias the clamp apparatus **510** toward the open position. In the embodiment shown in **FIGS. 6A-6G****,** there are two return springs **553.** Each return spring **553** is seated in a return spring pocket **555** which has a diameter slightly larger than that of the return spring **553.** Each return spring pocket **555** is recessed into the left face (relative to **FIG. 6B**) of the slider sled **551.** One end of each return spring **553** abuts the bottom of its respective return spring pocket **555.** The opposite end of each return spring **533** abuts the inside of the right face **581** (relative to **FIG. 6A**) of the housing **580** of the clamp apparatus **510.** As the slider sled **551** is moved toward the right face **581** of the housing **580** when a user actuates the clamp apparatus **510** toward the closed position, the return springs **553** compress between the bottom of the return spring pockets **555** and the inside of the right face **581** of the housing **580.** When a user actuates the clamp apparatus **510** toward the open position, the restoring force exerted by the return springs 553 automatically returns the slider sled **551** to its open orientation.

In the embodiment illustrated in **FIGS. 6A-6G****,** there are three return spring pockets **555** yet only two return springs **553.** In some embodiments, including the illustrated embodiment, a user may add additional return springs **553** to the clamp apparatus **510** if such action is deemed desirable.

The fourth, catch function of the slider sled **551** requires a broader description of how a user may actuate the clamp apparatus **510.** As shown in **FIGS. 6A-6G****,** the clamp apparatus **510** may comprise an actuator handle **502.** User rotation of the actuator handle **502** may generate the force sufficient to actuate the clamp apparatus **510** toward the closed position. The actuator handle **502** is a roughly L-shaped structure comprised of a vertical arm **573** and a horizontal arm **574;** both arms merge at a substantially right angle. The actuator handle **502** comprises at least one means for a rotatably attaching the actuator handle **502** to the clamp apparatus **510.** In the example embodiment depicted in **FIGS. 6A-6G****,** the actuator handle **502** is coupled to a gear shaft **520** with a screw **576.** When the actuator handle **502** is rotated, the gear shaft **520** rotates about its axis.

At rest, the clamp apparatus **510** is biased to the open position. In the open position, the vertical arm **573** of the actuator handle **502** may point toward the bottom of the page as shown in **FIG. 6A****.** The horizontal arm **574** may project toward the left of the page in a manner perpendicular to the vertical arm **573** of the actuator handle **502** as shown in **FIG. 6A****.** To actuate the clamp apparatus **510** to the closed orientation, the actuator handle **502** must be rotated clockwise (in relation to **FIG. 6A**) substantially a full 180°.

In some embodiments, rotation of actuator handle **502** is converted to the linear motion propelling the sliding gripper **503** towards the fixed gripper **501.** Thus, rotation of the actuator handle **502** closes the clamp apparatus **510.** As mentioned above, rotation of the actuator handle **502** causes the rotation of a gear shaft **520.** In some embodiments, at least one cam gear **590** is driven by the rotation of the gear shaft **520.** Optionally, two or more cam gears **590** may be used to best accommodate the specific space and size needs of a particular embodiment of the clamp apparatus **510.**

In the embodiment shown in **FIGS. 6A-6G****,** the cam gear **590** is eccentrically attached to the gear shaft 520 at a distance "r" from the cam gear **590** center. In some embodiments an extension linkage **505** may project toward the center of the cam gear **590** from the gear shaft **520.** The extension linkage **505** may be coupled into the center of the cam gear **590** to help support rotation of the cam gear **590** as the actuator handle **502** is rotated. Over the approximately 180° of rotation of the actuator handle **502,** the cam gear **590** may displace a linear distance of approximately 2"r".

In the exemplary embodiment depicted in **FIGS. 6A-6G****,** linear movement of the cam gear **590** is multiplied and imparted to the sliding gripper **503** through a linkage cam gear **597.** The teeth of the linkage cam gear **597** and the teeth of the cam gear **590** interdigitate thus operatively coupling the cam gear **590** to the slider sled **551.** In some embodiments, the linkage cam gear **597** is eccentrically coupled to the slider sled **551** at distance "r" from the center of the linkage cam gear **597.** In the embodiment shown in **FIGS. 6A-6G** the linkage cam gear **597** is substantially a mirror image of the cam gear **590.** Additionally, the movement of the linkage cam gear **597** mirrors the movement of the cam gear **590.** Consequentially, a 180° rotation of the actuator handle **502** creates a linear displacement of 4"r" in the slider sled **551.** This causes the sliding gripper base **504** and sliding gripper **503** to displace toward the fixed gripper **501.** If a clamped object **100** is present, the slider sled **551** and sliding gripper base **504** move as a unit only until the sliding gripper **503** contacts the clamped object **100.** When the sliding gripper **503** contacts the clamped object **100.** The compression springs **550** begin to compress per the above description.

In embodiments where a smaller degree of linear displacement may be desirable, either the cam gear **590** or linkage cam gear **597** may not be eccentrically coupled into the clamp apparatus. This would halve the linear displace of slider sled **551.** Alternatively, the distance "r" could be increased or decreased to achieve a greater or lesser degree of displacement of the slider sled **551.**

The fourth, stop function of the slider sled **551** may prevent the actuator handle **502** from being rotated past the fully open orientation. As best shown in **FIG. 6B** the slider sled **551** features a catch **571.** The catch **571** may be a nub which projects into a claw shaped cutout **576** in the slider sled **551.** Other suitable shaped cutouts may alternatively be used. The catch **571** catches a claw shaped prong **572** which extends off a thin disc **594** which is coupled to the center of the cam gear **590.** The thin disc **594** may be coupled to the center of the linkage cam gear **597.** The thin disc **594** may feature a semi-circle track **598** which the gear shaft **520** may extend through. As the actuator handle **502** is rotated the thin disc **594** and attached prong **572** follow the eccentric motion of the cam gear **590.** The position of the gear shaft **520** along the semi-circle track **598** also changes. In the closed position, the gear shaft **520** may be located at the right end of the semi-circle track **598** (relative to **FIG. 6B**). Also in the closed position, the prong **572** may not intrude into the catch **571** cutout. After 90° of actuator handle **502** rotation toward the open position, the gear shaft **520** is located at the lowest point in the arc of the semi-circle track **598.** Consequently, the thin disc **594** and attached prong **572** are at the highest point in their travel and the prong **572** has entered the claw shaped cutout **576** above the nub catch **571.** In the fully open position, the gear shaft **520** may be located at the left end of the semi-circle track **598.** The prong **572** may fully protrude into the claw shaped cutout **576** and hook around the nub catch **571.** In this position, the actuator handle **502** may not be further rotated toward the open direction because the catch **571** blocks any further movement of the prong **572.** Additionally, further rotation of the actuator handle **502** is prohibited because the gear shaft **520** is at the end of the semi-circle track **598** and the thin disc **594** blocks any further travel.

In some embodiments, an actuator handle latch **584** functions to operatively prevent the actuator handle **502** from being rotated out of the locked position. The actuator handle latch **584** (best shown in **FIG. 6G**) may be a roughly rectangular, planar structure. There may be a hole through roughly the center of the actuator handle latch **584.** The hole may be large enough to comfortably accommodate a user's finger. Relative to **FIG. 6G****,** the top edge of the actuator handle latch **584** may comprise a latch compression spring peg **583** on which an actuator handle spring **592** may be seated. The bottom edge may comprise projections **585.**

In some embodiments, the vertical arm **573** of the actuator handle **502** comprises a latch housing **586.** As shown best in **FIG. 6G****,** the latch housing **586** extends perpendicularly from the vertical arm **573** and over the top face **513** of the clamp apparatus **510.** The latch housing **586** may comprise a channel **587** sized to fit the actuator handle latch **584,** latch compression spring peg **583** actuator handle spring **592** and the projections **585.** The channel **587** may be cut along the central plane of the latch housing **586** running perpendicular to the vertical arm **573.** The channel **587** guides movement of the actuator handle latch **584.** There may be a hole through roughly the center of the actuator latch housing **586** which is large enough to accommodate a user's finger.

The actuator handle latch **584** projects out of the actuator latch housing **586** and against the top face **513** of the housing **580.** A dowel **588** may run through the channel **587** above the actuator handle spring **592.** The dowel **588** is disposed such that the actuator handle spring **592** may bias the actuator handle latch **584** against the top face of the housing **580.**

In the path of the actuator handle latch **584** a ramp **516** is disposed. As the actuator handle **502** is rotated toward the closed position, the actuator handle latch **584** abuts the ramp **516.** As the actuator handle **502** continues to rotate toward the closed position, the actuator handle latch **584** rides up the ramp **516.** This causes the actuator handle latch **584** to be pushed up the channel **587** and into the actuator latch housing **586** which in turn compresses the actuator handle spring **592** between the dowel **588** and the latch compression spring peg **583.** When the actuator handle **502** is in the fully closed position, the actuator handle latch **584** clears the ramp **516** and the restoring force of the spring causes the actuator handle latch **584** to spring back against the top face **513** of the housing **580.** This locks the clamp apparatus **510** in the closed position as any movement toward the open position is prevented by the actuator handle latch **584** catching on the lip of the ramp **516.** To release the clamp apparatus **510** from the locked position, a user may insert a finger into the hole in the actuator handle latch **584** and latch housing **586** and pull the actuator handle latch **584** back inside the actuator latch housing **586.** This allows the actuator handle latch **584** to clear the lip of the ramp **516** thus allowing rotation of the actuator handle **502** toward the open position.

In some embodiments, the horizontal arm **574** of the actuator handle **502,** may also comprise a lock/latch feature **531.** This lock/latch feature **531** may be present in conjunction with or as a substitute for the actuator latch **584.** In embodiments where the horizontal arm **574** comprises a lock/latch feature **531,** the front face **532** of the clamp apparatus **510** housing **580** may comprise a slot **534** through which a spring loaded strike plate **533** protrudes. The strike plate **533** (best shown in FIG. **6E**) may be roughly planar. The bottom of the strike plate **533** (relative to **FIG. 6E**) may comprise at least one strike plate spring peg **535** on which a strike plate spring **536** is seated. In the embodiment depicted in **FIGS. 6A-6G****,** there are two strike plate spring pegs **535** and two accompanying strike plate springs **536.** The strike plate springs **536** fit inside the strike plate spring bay **511** recessed into the fixed gripper base **524.** In some embodiments, the top edge of the strike plate **533** (relative to **FIG. 6E**) may comprise a ramp portion **537,** a trough portion **538,** and a post portion **539.** The strike plate **533** protrudes from the slot **534.** The strike plate **533** may be pushed into the slot **534,** in the front face **532** of the housing **580** such that it does not protrude past the surface of the front face **532** of the housing **580.** In this position, the strike plate springs **536** are compressed between a portion of the strike plate spring bay **511** and the strike plate spring pegs **535.** This spring loads the strike plate **533** to automatically return to its protruding orientation.

As the actuator handle **502** is rotated to the closed position, the horizontal arm **574** of the actuator handle **502** contacts the ramp portion **537** of the strike plate **533.** As the horizontal arm **574** is further rotated, it moves to a more elevated section of the ramp portion **537.** Since the strike plate springs **536** are not strong enough to cause the horizontal arm **574** to deflect, the strike plate springs **536** compress and the strike plate **533** is pushed into the slot **534** to its non-protruding position. When the horizontal arm **574** passes the top of the ramp portion **537,** the restoring force of the strike plate springs **536,** causes the strike plate **533** to be pushed back toward its protruding position with the trough portion **538** abutting the horizontal arm **574.** This locks the clamp apparatus **510** in the closed position. In this locked position, the horizontal arm **574** cannot be further rotated toward the closed position because the post portion **539** of the strike plate **533** blocks such movement. Additionally, the horizontal arm may not progress toward the open position because it will abut and be restricted in movement by the lip of the ramp portion **537.** To unlock the clamp apparatus **510,** a user must depress the post portion of the strike plate **533** into the slot **534** and compress the strike plate springs **536.** This allows the horizontal arm **574** to clear the lip of the ramp **537** as a user rotates the actuator handle **502** toward the open position.

In some embodiments of the present disclosure, a quick release clip **519** may be used to secure a medical device or other object to the clamp apparatus **510.** The quick release clip **519** may comprise a torsion clip **522** and a latch hook **523.** In some embodiments of the present disclosure, at least one torsion spring **521** may be used to clip a load for the clamp apparatus **510** between the torsion clip **522** and the latch hook **523.** In the example embodiment shown in **FIGS. 6A-6G****,** two latch hooks **523** are firmly attached to the top face **513** of the housing **580.** The latch hooks **523** are offset from each other. The hook portions of the latch hooks **523** project toward the back of the page (relative to **FIG. 6A**). The torsion clip **522** is pivotally attached to the latch hook **523** by a fastening means **525,** which may for example be a pin, dowel, cotter pin, bolt, hex bolt, screw, or other means known to one skilled in the art. As shown in **FIGS. 6A-6G****,** the torsion clip **522** may be a relatively planar member which spans the distance between the two latch hooks **523.** In some embodiments, at least one surface of torsion clip **522** may comprise a catch **526.** The catch **526** may act as a stop for a receiving structure on a medical device or other object. The torsion spring(s) **521** may supplement the catch **526** by biasing the receiving structure into contact with the latch hooks **523.** The latch hooks **523** may also couple to a receiving structure on a medical device or other object. Rotation of the torsion clip **522** downwards spring loads each torsion spring **521** so that the torsion clip **522** will automatic pivot to the closed position when released. This is desirable because it causes the quick release clip **519** to automatically adjust to a load, such as medical device or other object, regardless of the size of the receiving structure.

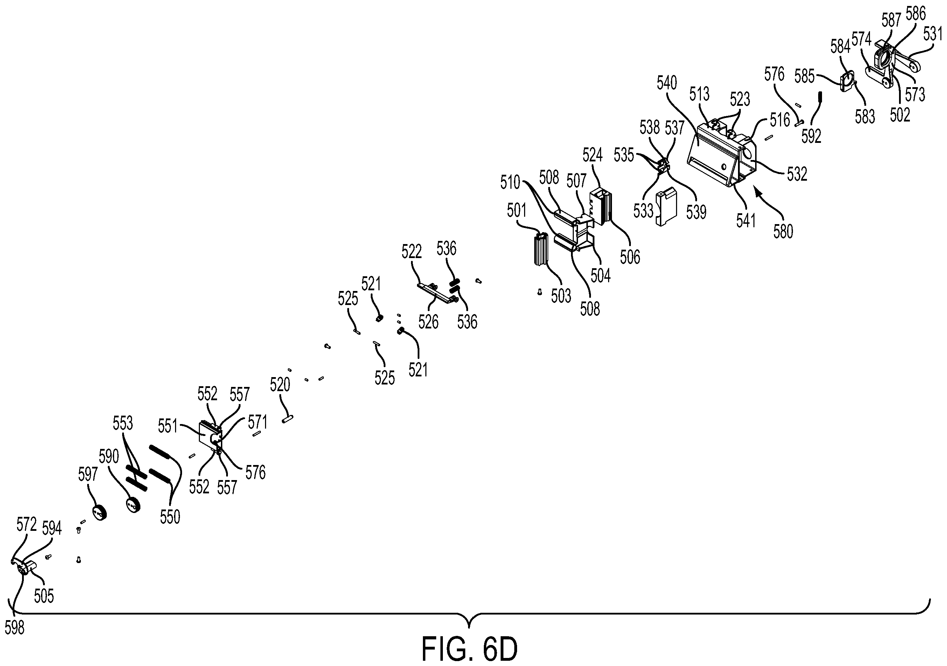

As best shown in **FIG. 6D****,** some embodiments may comprise a rest **540** for a medical device or other object which may be coupled to the clamp apparatus via the quick release clip **519.** As shown, the rest **540** may project at an angle from the top face **513** of the housing **580.** Extending perpendicularly from the bottom edge of the back face **512** of the housing **580** may be a rest support **541** for the rest **540.** The rest support **541** couples the back face **512** of the housing **580** to the rest **540.** Additionally, the rest **540** may have various features which help to hold the medical device or other object in place on the rest **540.**

The housing **580** or rest **540** may also feature any of a variety of mechanisms **515** (not shown) to attach a load to the clamp apparatus **510.** Such mechanisms **515** may include, but are not limited to, brackets, magnets, straps, suction cups, hooks, screws or bolts, a friction fit, etc. This load could be any number of things, especially a medical device (such as an infusion pump, or peristaltic infusion pump), I.V. bag, etc.

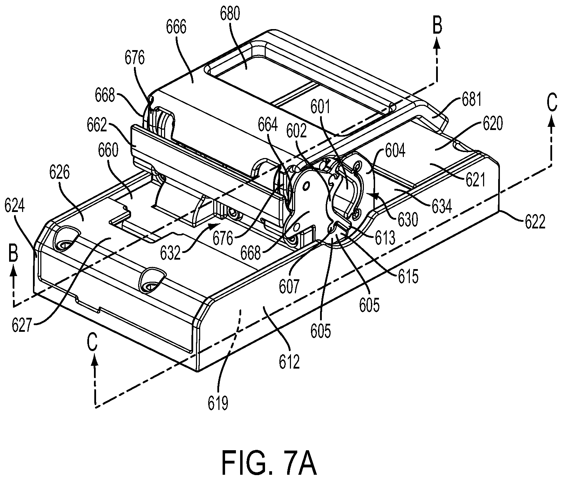

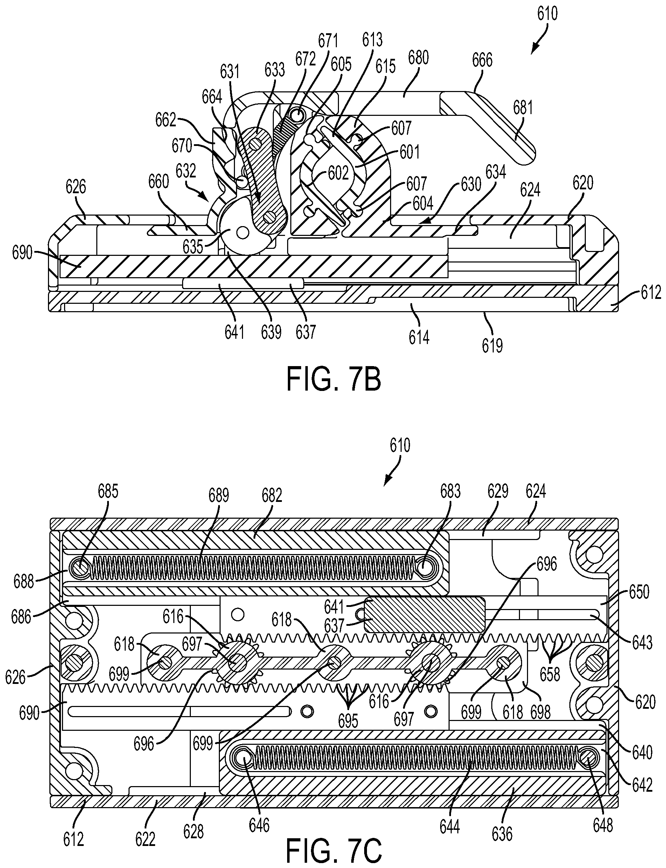

**FIGS. 7A-7D** show another embodiment of a clamp apparatus **610.** The clamp apparatus **610** comprises a first moving jaw **630** and a second moving jaw **632,** coupled to move in unison. A clamped object **100** may be clamped between the first moving jaw **630** and the second moving jaw **632** and clamped by the clamp apparatus **610.**

In some embodiments, the clamp apparatus **610** includes a housing **612.** As shown in **FIGS. 7A-7D****,** the housing **612** may be shaped like a rectangular tray. The bottom face **614** of the housing **612,** may be substantially planar. In some embodiments, the bottom face **614** of the housing **612** may have one or more gear attachment sites **616.** The bottom face may also have one or more raised posts **618.** The raised posts may comprise a hole sunk substantially into the center of the posts **618.** The hole may additionally be tapped to receive the thread of a screw. As shown in **FIG. 7A****,** the gear attachment sites **616** and the raised posts **618** may all be in line with each other. Also as shown, the gear attachment sites **616** and the raised posts **618,** may run along the center line of the bottom face **614** running parallel to the front wall **622** and back wall **624** of the housing **612.** The gear attachment sites **616** and raised posts **618** will be further elaborated upon later.

At least a portion of the housing **612** may also feature any of a variety of mechanisms **619** (not shown) to attach a load to the clamp apparatus **610.** Such mechanisms **619** may include, but are not limited to, brackets, magnets, straps, suction cups, hooks, screws or bolts, a friction fit, etc. This load could be any number of things, especially a medical device (such as an infusion pump, or peristaltic infusion pump), I.V. bag, etc.

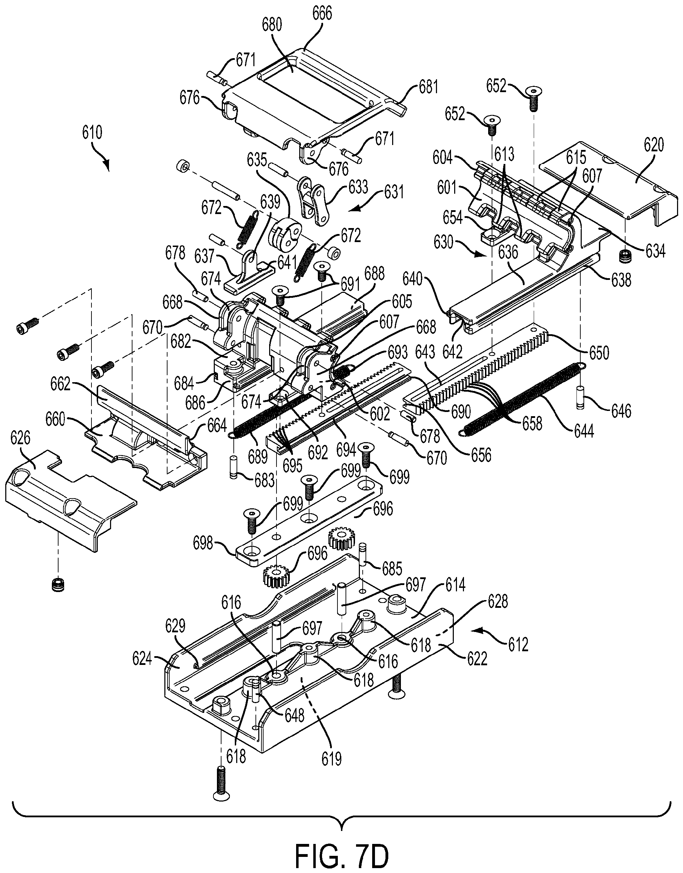

As mentioned above, the housing **612** may comprise a front wall **622** and a back wall **624.** Relative to **FIG. 7D****,** the front wall **622** projects toward the top of the page from the edge of the bottom face **614** which faces the front of the page. The front wall **622** projects substantially perpendicularly to the plane of the bottom face **614** of the housing **612.** The interior face of the front wall **622** may comprise a projecting track section **628** which runs parallel to the top and bottom edges of the front wall **622.** The back wall **624** projects toward the top of the page from the edge of the bottom face **614** of the housing **612** which faces the back of the page. The back wall **624** projects perpendicularly to the bottom face **614** of the housing **612.** The interior face of the back wall **624** may comprise a projecting track section **629** which runs parallel to the top and bottom edges of the back wall **624.**

In the embodiment shown in **FIGS. 7A-7D****,** the right side **620** and left side **626** of the housing **612** are detachable end caps. The right side **620** and left side **626** of the housing **612** may be coupled to the bottom face **614** of the housing **612** via screws, bolts, welds, or any other suitable means. In other embodiments, the right side **620** and left side **626** may be formed as a continuous part of the housing **612** during manufacture. The right side **620** of the housing **612** may have an overhanging flange **621** which overhangs a portion of the bottom face **614** of the housing **612.** Similarly, the left side **626** of the housing **612** may have an overhanging flange **627** which overhangs a portion of the bottom face **614** of the housing **612.**

In some embodiments, a first gripper **601** and a second gripper **602** are firmly attached to a first bracket **604** and a second bracket **606** respectively. The first bracket **604** and second bracket **606** respectively comprise a part of the first moving jaw **630** and second moving jaw **632.** In the example embodiment depicted in **FIG. 7A-7D****,** each of the first bracket **604** and second bracket **606** comprise friction fit features **607.** The friction fit features **607** allow the respective grippers **601** and **602** to be coupled to the first bracket **604** and second bracket **606.** In other embodiments, the grippers **601** and **602** may be coupled to the first bracket **604** and second bracket **606** by any number of coupling means including, but not limited to, screws, bolts, ultrasonic welds, magnets, adhesive, etc.

The first gripper **601** and second gripper **602** consists of a material chosen for its gripping ability. The first gripper **601** and second gripper **602** may be made of a high friction material, a compressible material, a material exhibiting both these qualities, or any other suitable material. The first gripper **601** and second gripper **602** are made of a material which allows a firm grip without the deformation of a clamped object **100.** Suitable materials may include any suitable elastomeric or non-deformable substance, including but not limited to plastic, rubber, metal, foam, fabric, gel, polyurethane, etc. At least a portion of the first gripper **601** and second gripper **602** may comprise roughly semi-circular depressions or contours to accommodate a round clamped object **100** such as a pole. The first gripper **601** and second gripper **602** may be replaceable.

In some embodiments, the first gripper **601** and second gripper **602** may comprise gripper teeth **613** which project from the top and bottom edges of the first gripper **601** and second gripper **602.** The gripper teeth **613** may be disposed about the first gripper **601** and second gripper **602** such that they may interdigitate with each other when the clamp apparatus **610** is in the closed position. The gripper teeth **613** allow the first gripper **601** and second gripper **602** to better encompass and hold a clamped object **100** when the clamp apparatus **610** in the closed position. The first bracket **604** and second bracket **606** may comprise bracket teeth **615** which support the gripper teeth **613 on** the first gripper **601** and second gripper **602.** The bracket teeth **615** may be disposed about the first bracket **604** and second bracket **606** such that they interdigitate with each other similarly to the gripper teeth **613.**

The first bracket **604** may have a flange **634** which extends perpendicularly off the face of the first bracket **604** opposite the face to which the first gripper **601** is attached. The flange **634** is shaped and disposed such that it may slide under the overhanging flange **621** of the right side **620** of the housing **612.** A polygonal block **636** may be fixedly coupled to the bottom face of the first bracket **604** (relative to **FIG. 7D**). In the example embodiment depicted in **FIGS. 7A-7D****,** the polygonal block **636** is specifically a long, rectangular block. The short, right and left ends of the long, rectangular block run parallel to the right edge of the flange 634 of the first bracket **604.** The long sides of the rectangular block in the example embodiment shown in **FIGS. 7A-7D****,** extend for roughly seventy-five percent of the length of the front wall **622** of the housing **612.** This may differ in alternate embodiments. The first bracket **604,** first gripper **601,** flange **634,** and polygonal block **636** collectively may comprise the first moving jaw **630.**

One side of the polygonal block **636** may abut the interior face of the front wall **622.** The side of the polygonal block **636** which abuts the interior face of the front wall **622** may include a recessed groove **638** which accepts the projecting track section **628** on the interior face of the front wall **622.** The projecting track section **628** operatively functions as a guide to inform the movement of the first moving jaw **630.**

The side of the polygonal block **636** opposite the recessed groove **638** may include a projecting jaw track section **640.** The projecting jaw track section **640** runs substantially parallel to the recessed groove **638.** The bottom of the polygonal block **636** may comprise an extension spring trough **642** which is sunk into the bottom face of the polygonal block **636.** The extension spring trough **642** also runs parallel to both the recessed groove **638** and projecting jaw track section **640.** The bottom of the polygonal block **636** may abut the bottom face **614** of the housing **612.**

A first extension spring **644** may be placed in the extension spring trough **642.** As shown in the embodiment in **FIGS. 7A-7D****,** the right end (relative to **FIG. 7D**) of the extension spring **644** may be coupled into the extension spring trough **642** by a first extension spring peg **646.** The left end of the extension spring **644** may be coupled to the bottom face **614** of the housing **612** by a second extension spring peg **648.** The first extension spring **644** biases the first moving jaw **630** toward the closed position. Moving the first moving jaw **630** from the closed position to the open position extends the first extension spring **644.** The restoring force from the first extension spring **644** will automatically cause the first moving jaw **630** to return to the closed position. When a clamped object **100** is present, the restoring force of the first extension spring **644** will cause the first moving jaw **630** to press the first gripper **601** into the clamped object **100,** automatically adjusting to the size or girth of the clamped object **100.**

In some embodiments, including the embodiment shown in **FIGS. 7A-7D****,** a first rack **650** may additionally be coupled to the bottom of the first moving jaw **630.** As shown, the first rack **650** is coupled to the first moving jaw **630** via two screws **652.** One screw **652** couples the first rack **650** to the first moving jaw **630** via a screw hole in the flange **634.** As shown, the first moving jaw **630** may further comprise a coupling ledge **654** which projects along the plane of the bottom of the first bracket **604.** The coupling ledge **654** projects toward the left of the page relative to **FIG. 7D****.** The second screw **652** couples the first rack **650** to the first moving jaw **630** through a screw hole in the coupling ledge **654.**

As shown in **FIGS. 7A-7D****,** the first rack **650** has a rack groove **656** recessed into the face of the first rack **650** which faces the back of the page relative to **FIG. 7D****.** The face opposite the rack groove **656** comprises a number of rack teeth **658.**

The second moving jaw **632** may be generally similar to the first moving jaw **630.** In the embodiment shown in **FIGS. 7A-7D****,** the second moving jaw **632** is similar to the first moving jaw **630** although it comprises some additional or different components. The second bracket **606** may comprise a second flange **660** which extends perpendicularly off the face of the second bracket **606** opposite the face to which the second gripper **603** is attached. As shown in **FIGS. 7A-7D****,** the second flange **660** may be detachable. In embodiments where the second flange **660** may be detachable, the second flange **660** may be coupled to the second bracket **606** via screws, bolts, magnets, adhesive, etc.

The second flange **660** may comprise a handle mechanism cover **662.** The handle mechanism cover **662** may be raised off the second flange **660** toward the top of the page. At least one section of the handle mechanism cover **662** may comprise an arcuated segment **664** which faces a pivoting handle **666.** The arcuated segment **664** allows the pivoting handle **666** to rotate. The handle mechanism cover **662** helps to keep foreign material and debris from getting inside the clamp apparatus **610.** The handle mechanism cover **662** does not abut the second bracket **606.** The handle mechanism cover **662** is offset from the second bracket **606** toward the left of the page relative to **FIG. 7D****.** The void created between the second bracket **606** and the handle mechanism cover **662** allows various linkages to couple the pivoting handle **666** to the inner workings of the clamp apparatus **610.**

The second bracket **606** may additionally comprise wings **668** which project off the front and back edges of the second bracket **606** toward the handle mechanism cover **662.** In the embodiment shown in **FIGS. 7A-7D****,** the wings **668** are not coupled to the handle mechanism cover **662.** A handle spring peg **670** extends through the bottom of each wing **668.** The handle spring pegs **670** protrude into the void between the second bracket **606** and the handle mechanism cover **662.** One end of a handle extension spring **672** may be placed around each handle spring peg **670.**

As shown in the embodiment depicted in **FIGS. 7A-7D** a slit **674** is recessed into the each wing **668** on a plane parallel to the front wall **622** and back wall **624** of the housing **612.** The slit **672** may effectively make the top portion of each wing **668** into a coupling bracket to which fins **676** projecting off the pivoting handle **666** may be inserted. A dowel **678** may run through each wing **668** into the slits **674** and through the fins **676** of the pivoting handle **666.** The dowels **678** pivotally couple the pivoting handle **666** to the wings **668** of the second bracket **606.** The dowels **678** act as the pivot axis for the pivoting handle **666.**

The fins **676** of the pivoting handle **666** may also comprise a hole through which a second set of handle spring pegs **671** may extend. The second set of handle spring pegs **671** may protrude into the void between the second bracket **606** and the handle mechanism cover **662.** The end of each handle extension spring **672** not connected to the first set of handle spring pegs **670** is connected to the second set of handle spring pegs **671.** The handle extension spring **672** thus acts as an over-center linkage and helps keep the pivoting handle **666** in the closed position if the pivoting handle **666** is in the closed position and helps keep the pivoting handle **666** in the open position if the pivoting handle **666** is in the open position.

In the example embodiments shown in **FIGS. 7A-7D****,** the pivoting handle **666** extends toward the right of the page. In some embodiments, including those displayed in **FIGS. 7A-7D****,** the pivoting handle **666** comprises an open section **680** through which a user may place their fingers. The open section **680** of the pivoting handle may be included to allow a user to grasp the pivoting handle **666** more easily. The pivoting handle may also comprise a bent or arced section **681.** Again, the bent or arced section of the pivoting handle **666** may make it easier for a user to grasp the pivoting handle **666.**

A portion of the bent or arced section **681** of the pivoting handle **666** may be made of the same material as the rest of the pivoting handle **666,** may be made of a different material, or may be made of a combination thereof. Possible materials may include, but are not limited to, rubber, polymer, composite, metal, plastic, foam, etc. Additionally, the bent or arced section **681** may comprise ergonomic finger groves, nubs, a ribbed texture, a honeycombed texture, etc. to afford a user greater ease of use.

The second moving jaw **632** may additionally comprise a second polygonal block **682.** The second polygonal block **682** may be fixedly coupled to the bottom face of the second bracket **606** (relative to **FIG. 7D**). In the example embodiment depicted in **FIGS. 7A-7D****,** the second polygonal block **682** is specifically a long, rectangular block. The short, right and left ends of the long, rectangular block run perpendicular to the planes of the front wall **622** and back wall **624** of the housing **612.** The long sides of the rectangular block in the example embodiment shown in **FIGS. 7A-7D**, extend for roughly seventy-five percent of the length of the back wall **624** of the housing **612.** This may differ in alternate embodiments.

One side of the second polygonal block **682** may abut the interior face of the back wall **624.** The side of the second polygonal block **682** which abuts the interior face of the back wall **624** may include a recessed groove **684** which accepts the projecting track section **629** on the interior face of the back wall **624.** The projecting track section **629** operatively functions as a guide to inform the movement of the second moving jaw **632.**

The side of the second polygonal block **682** opposite the recessed groove **684** may include a projecting second jaw track section **686.** The projecting second jaw track section **686** runs substantially parallel to the recessed groove **684.** The bottom of the second polygonal block **682** may comprise a second extension spring trough **688** which is sunk into the bottom face of the second polygonal block **682.** The extension spring trough **688** also runs parallel to both the recessed groove **684** and projecting second jaw track section **686.** The bottom of the second polygonal block **682** may abut the bottom face **614** of the housing **612.**

A second extension spring **689** may be placed in the extension spring trough **688.** As shown in the embodiment in **FIGS. 7A-7D****,** the left end (relative to **FIG. 7D**) of the second extension spring **689** may be coupled into the extension spring trough **688** by a third extension spring peg **683.** The right end of the extension spring **689** may be coupled to the bottom face **614** of the housing **612** by a fourth extension spring peg **685.** The second extension spring **689** biases the second moving jaw **632** toward the closed position. Moving the second moving jaw **632** from the closed position to the open position extends the second extension spring **689.** The restoring force from the second extension spring **689** will automatically cause the second moving jaw **632** to return to the closed position. When a clamped object **100** is present, the restoring force of the second extension spring **689** will cause the second moving jaw **632** to press the second gripper **603** into the clamped object **100,** automatically adjusting to the size or girth of the clamped object **100.**

In some embodiments, including the embodiment shown in **FIGS. 7A-7D****,** a second rack **690** may additionally be coupled to the bottom of the second moving jaw **632.** As shown, the second rack **690** is coupled to the second moving jaw **632** via two screws **691.** One screw **691** couples the second rack **690** to the second moving jaw **632** via a screw hole in a ledge **692** which projects under the second flange **660.** As shown, the second moving jaw **630** may further comprise an additional ledge **693** which projects along the plane of the bottom of the second bracket **606.** The additional ledge **693** projects toward the right of the page relative to **FIG. 7D****.** The second screw **691** couples the second rack **690** to the second moving jaw **632** through a screw hole in the additional ledge **693.**

As shown in **FIGS. 7A-7D****,** the second rack **690** has a second rack groove **694** recessed into the face of the second rack **690** which faces the front of the page relative to **FIG. 7D****.** The face opposite the second rack groove **694** comprises a number of second rack teeth **695.**

When the clamp apparatus **610** is assembled, the second rack groove **694** fits around and is guided by the projecting jaw track section **640** coupled to the first moving jaw **630.** Similarly the rack groove 656 fits around and is guided by the projecting second jaw track section **686.** The first rack teeth **658** and the second rack teeth **695** face each other. The first rack **650** and second rack **690** run substantially parallel to each other. The first rack teeth **568** and second rack teeth **695** mesh with teeth on opposite sides of at least one pinion gear **696.** The at least one pinion gear **696** may be placed on a gear shaft **697** which runs into the at least one gear attachment site **616** described earlier in the specification. In the embodiment depicted in **FIGS. 7A-7D****,** two pinion gears **696** are present. Each pinion gear **696** is placed on its own gear shaft **697** which in turn runs into its own gear attachment site **616** located on the bottom face **614** of the housing **612.** To ensure the pinion gears **696** do not stray off their associated gear shafts **697,** the pinion gears **696** may be sandwiched against the back face **614** of the housing **612** by a bar-like plate **698.** The bar-like plate **698** is coupled to the raised posts **618** which project off the back face **614** of the housing **612** via screws **699.**

Since both the first rack **650** and the second rack **690** mesh with the same pinion gear(s) **696** on opposite sides of said pinion gear(s) **696,** any movement of either the first moving jaw **630** or the second moving jaw **632** necessitates movement of the other moving jaw in the opposite direction. If one moving jaw is pulled to the open position, the other moving jaw must then also move to the open position. If one moving jaw retracts toward the closed position, the other moving jaw must then also retract toward the closed position.

The clamp apparatus **610** additionally comprises a tightening/locking mechanism **631.** The tightening/locking mechanism **631** may comprise a number of components. In the embodiment depicted in **FIGS. 7A-****7D,** the tightening/locking mechanism **631** comprises a linkage **633,** a cam **635,** and a cincher **637.** The cincher **637** may comprise a post **639** and a flat plate **641.** The tightening/locking mechanism **631** may be disposed in the void between the handle mechanism cover **662** and the gripper bracket **605.** The linkage 633 is pivotally coupled on one end to the pivoting handle **666.** The linkage **633** may be pivotally coupled to the pivoting handle **666** by any means known to one skilled in the art. The other end of the linkage 633 is pivotally coupled to an end of the cam 635. The other end of the cam **635** may comprise a slot which accepts the post **639** of the cincher **637.** The cam **635** may be pivotally coupled to the post **639** of the cincher **637** by any means known to one skilled in the art. In the example embodiment, the post **639 of** the cincher **637** projects perpendicularly from the flat plate **641** of the cincher **637.**

The flat plate **641** of the cincher **637** is disposed under the rack **650** of the first moving gripper **630** when the clamp apparatus **610** is fully assembled. The post **639** of the cincher **637** projects up through a channel **643** which is cut out of the rack **650.** The channel **643** may not run the entire length of the rack **650.**

In the embodiment shown in **FIGS. 7A-7D****,** as the pivoting handle **666** is pivoted from the open position to the closed position, the linkage **633** also moves. Movement of the linkage **633** causes the cam **635** to rotate. Rotation of the cam **635** causes the cincher **637** to experience linear displacement along the channel **643** of the rack **650.** Since the channel **643** does not run the entire length of the rack **650,** the post **639** of the cincher **637** abuts the end of the channel **643** and begins to cause linear displacement of the rack **650.** Linear displacement of the rack **650** causes both the first moving jaw **630** and second moving jaw **632** to move, cinch down on, and clamp harder on a clamped object **100.** In the embodiment shown in **FIGS. 7A-7D****,** the linkage **633** is also an over-center linkage. When the pivoting handle **666** moves all the way to the closed position, the linkage **633** assumes an over-center position. When the linkage **633** assumes this over-center position, the clamp apparatus **610** is effectively locked.

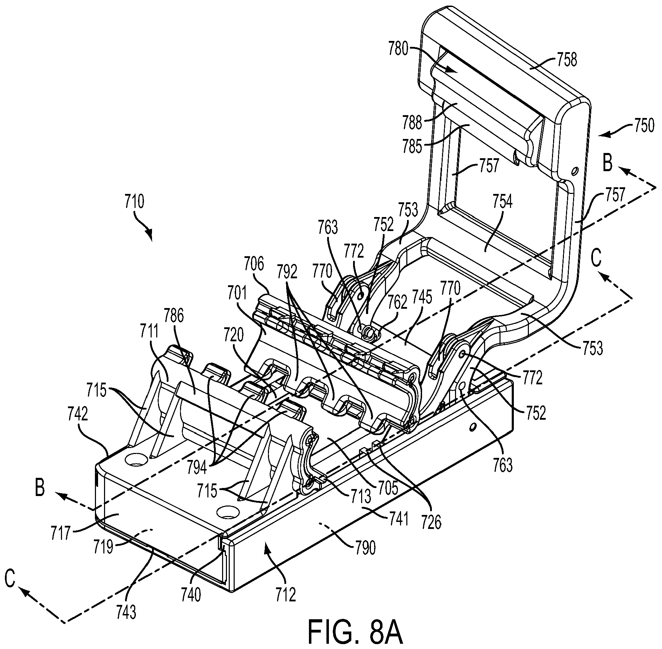

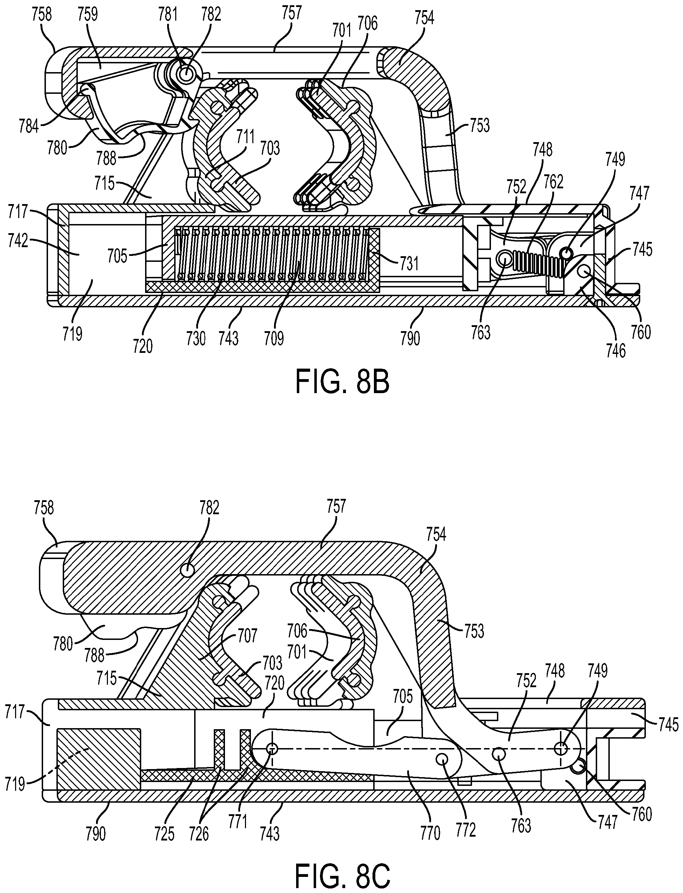

**FIGS. 8A-8D** show another example embodiment of a clamp apparatus **710.** In the clamp apparatus **710** shown in **FIGS. 8A-8D****,** a user rotates a toggle handle **750** to provide the force needed to propel a movable gripper assembly 704 towards a fixed gripper assembly **703** via at least one linkage **770** which may be an over-center linkage.

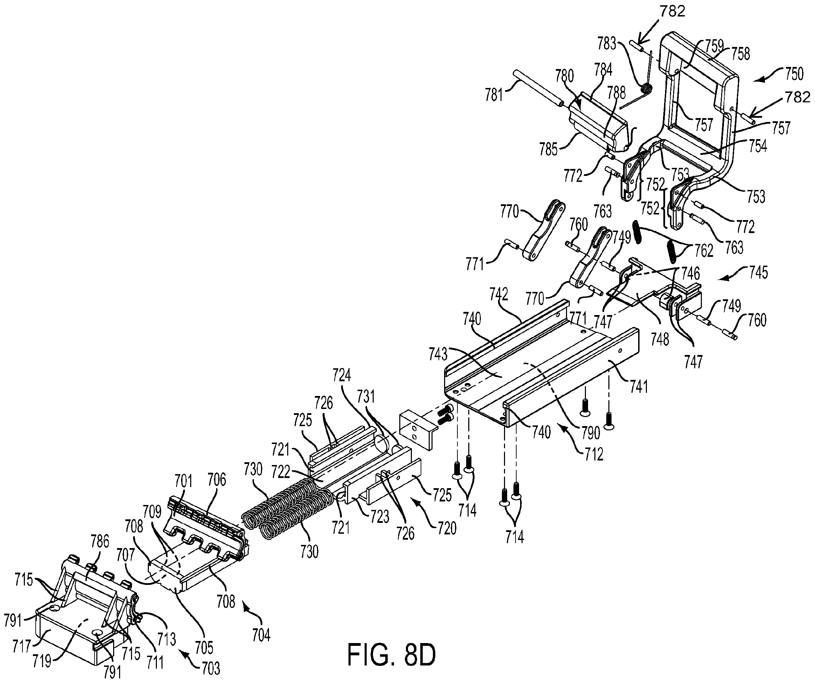

In some embodiments, such as the embodiment shown in **FIGS. 8A-8D****,** the fixed gripper assembly **703** comprises a fixed gripper cradle **711,** a fixed gripper **713,** and a fixed gripper base **717.** The fixed gripper cradle **711** extends off the top face of the fixed gripper base **717.** More specifically, the fixed gripper cradle **711** extends from the right edge (relative to **FIG. 8D**) of the fixed gripper base **717** at an angle roughly perpendicular to the top face of the fixed gripper base **717** and is fixedly coupled to the fixed gripper base **717.**

A fixed gripper **713** is coupled to the face of the fixed gripper cradle **711** which faces the movable gripper assembly **704.** The fixed gripper **713** may be coupled to the fixed gripper cradle **711** by any of a variety of coupling means including, but not limited to, screws, bolts, magnets, adhesive, ultrasonic welds, snap fit, friction fit. In some embodiments the fixed gripper **713** may be overmolded onto the fixed gripper cradle **711.**

The fixed gripper base **717** may be a roughly rectangular block as shown in **FIGS. 8A-8D****.** The fixed gripper base **717** may comprise a cavity **719** which is dimensioned to fit and surround the gripper sled **705** when the clamp apparatus **710** is in the closed orientation. The fixed gripper base **717** may also comprise at least one buttress **715** which helps to support the fixed gripper cradle **711.** The fixed gripper base **717** may comprise one or a number of threaded holes **791.** In the embodiment depicted in **FIGS. 8A-8D****,** four screws **714** run through the housing **712** of the clamp apparatus **710** and into corresponding threaded holes **791** in bottom of the fixed gripper base **717.** The four screws **714** couple the fixed gripper base **717** to the housing **712.** In alternate embodiments, different coupling methods may be employed including, bolts, welds, magnets, adhesive, and any other coupling method known to one skilled in the art. The fixed gripper base **717** may alternatively be a continuous part of the housing **712.**

In some embodiments, including the embodiment shown in **FIGS. 8A-8D****,** the movable gripper assembly **704** comprises a movable gripper cradle **706,** movable gripper **701,** and a gripper sled **705.** As shown in **FIGS. 8A-****8D,** the movable gripper cradle **706** extends off the top face of a gripper sled **705.** More specifically, the movable gripper cradle **706** extends from the right edge (relative to **FIG. 8D**) of the gripper sled **705** at an angle roughly perpendicular to the top face of the gripper sled **705** and is fixedly coupled to the gripper sled **705.** This may differ in alternate embodiments.

A movable gripper **701** is coupled to the face of the movable gripper cradle **706** which faces the fixed gripper assembly **703.** The movable gripper **701** may be coupled to the movable gripper cradle **706** by any of a variety of coupling means including, but not limited to, screws, bolts, magnets, adhesive, ultrasonic welds, snap fit, friction fit.

The movable gripper **701** and fixed gripper **713** may consist of a material chosen for its gripping ability. The movable gripper **701** and fixed gripper **713** may be made of a high friction material, a compressible material, a material exhibiting both these qualities, or any other suitable material. The movable gripper **701** and fixed gripper **713** are made of a material which allows for a firm grip without the deformation of a clamped object **100.** Suitable materials may include any suitable elastomeric or non-deformable substance, including but not limited to plastic, rubber, metal, foam, fabric, gel, etc. At least a portion of the movable gripper **701** and fixed gripper **713** may comprise roughly semi-circular depressions or contours to accommodate a round clamped object **100** such as a pole. The movable gripper **701** and fixed gripper **713** may be replaceable.

In some embodiments, the movable gripper **701** and fixed gripper **713** may comprise gripper teeth **792** (As shown in **FIG. 8A**) which project from the top and bottom edges of the movable gripper **701** and fixed gripper **713.** The gripper teeth **792** may be disposed about the movable gripper **701** and fixed gripper **713** such that they may interdigitate with each other when the clamp apparatus **710** is in the closed position. The gripper teeth **792** allow the movable gripper **701** and fixed gripper **713** to hold an increased range of clamped object **100** when the clamp apparatus **710** is in the closed position. By disposing the gripper teeth **794** such that they may interdigitate, the movable gripper **701** may move further toward the closed position. The movable gripper cradle **706** and the fixed gripper cradle **711** may comprise cradle teeth **794** which support the gripper teeth **792** on the movable gripper **701** and fixed gripper **713.** The cradle teeth **794** may be disposed about the movable gripper cradle **706** and the fixed gripper cradle **711** such that they interdigitate with each other similarly to the gripper teeth **792.**

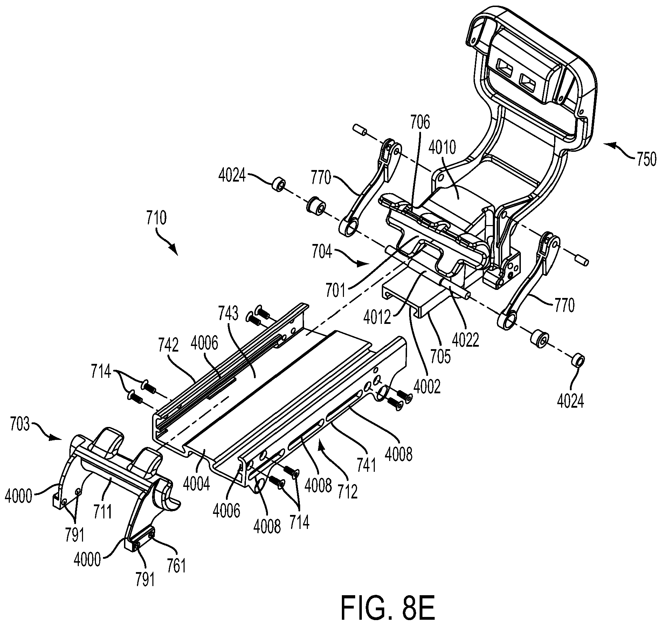

As illustrated in the example embodiment in **FIGS. 8A-8D****,** the gripper sled **705** may be roughly rectangular. The gripper sled **705** may be substantially hollow and open to the hollow on one end. In **FIGS. 8A-8D****,** the gripper sled **705** is hollow, except for a dividing wall **707** (relative to **FIG. 8D**) which extends from the interior bottom face of the hollow to the interior top face of the hollow. The dividing wall **707** divides the hollow portion of the gripper sled **705** into two spring bays **709** which are roughly equally dimensioned. The gripper sled **705** in **FIGS. 8A-****8D** is open to the hollow on its right end (relative to **FIG. 8D**). In the embodiment shown in **FIGS. 8A-8D** a spring **730** is seated in each of the spring bays **709.** The spring **730** is a compression spring **730.** In a preferred embodiment, the clamp apparatus **710** may be adapted to fit at least one constant force spring **4012** instead of or in addition to the compression spring **730.** Constant force springs **4012** may be used in other embodiments such as but not limited to those detailed above. Using a constant force spring **4012** is preferable because it may make the clamp apparatus **710** easier to operate, especially when it is being used to clamp a large/thick object. It may also allow the clamp apparatus **710** to be made more compactly. An alternative embodiment comprising a constant force spring **4012** is shown in **FIGS. 8E-8F****.**

The gripper sled **705** may also comprise sled projecting tracks **708** on its front and back faces (relative to orientation in **FIG. 8D**). The sled projecting tracks **708** fit into guide grooves **721** on a driven member **720.** In the example embodiment shown in **FIGS. 8A-8D****,** the driven member **720** is roughly "U" shaped. The bottom face **722** of the driven member **720** comprises the bottom span of the "U" shape. Projecting perpendicularly from front and back edges (relative to **FIG. 8D**) of the bottom face **722** of the driven member **720** toward the top of the page are a front upright wall **723** and a back upright wall **724.** The front upright wall **723** and back upright wall **724** comprise the upright spans of the "U" shape. The guide grooves **721** run along the surfaces of the front upright wall **723** and back upright wall **724** which face each other.

In some embodiments, the driven member **720** may comprise at least one appendage **725** which extends from either the front upright wall **723** or back upright wall **724.** In the exemplary embodiment illustrated in **FIGS. 8A-****8D,** the driven member **720,** includes two appendages **725.** One appendage **725** extends from the face of the front upright wall **723** opposite the face on which the guide groove **721** of the front upright wall **723** is disposed. The other appendage extends from the face of the back upright wall **724** opposite the face on which the guide groove **721** of the back upright wall **724** is disposed.

The appendages **725** are roughly "L" shaped. One portion of each appendage **725** projects from its corresponding front upright wall **723** or back upright wall **724** at an angle substantially perpendicular to the front upright wall **723** and back upright wall **724.** This portion of each appendage **725** comprises the horizontal span of the "L" shape. The vertical span of the "L" shape is formed by a second portion of the appendage **725** which projects toward the top of the page from the distal end of the first portion of the appendage **725** at an angle substantially perpendicular to the first portion of the appendage **725.** As shown in **FIGS. 8A-8D** the one or more appendages may be buttressed by at least one support piece **726.** In some embodiments, including the embodiment shown in **FIGS. 8A-8D****,** the one or more appendages may not span the entire length of the front upright wall **723** and back upright wall **724** of the driven member **720.** In the shown embodiment, the appendages stop short of the left edge (relative to **FIG. 8D**) of the driven member **720.**

The appendages **725** or a portion of the appendages **725** may fit into and slide along a grooved track **740** on front wall **741** and back wall **742** the housing **712.** The bottom of the driven member **720** may ride along the bottom face **743** of the housing **712.**

When the clamp apparatus **710** is assembled, the gripper sled **705** fits in the driven member **720** between the front upright wall **723** and back upright wall **724.** When the clamp apparatus **710** is not clamped around a clamped object **100** the gripper sled **705** fits in the driven member **720** such that the right and left faces (relative to **FIG. 8D**) of the gripper sled **705** are flush with the right and left edges of the driven member **720.** One end of each compression spring **730** abuts the interior left face (relative to **FIG. 8D**) of the hollow portion of the gripper sled **705.** The other end of each compression spring **730** abuts a compression spring disc **731** which projects toward the top of the page from the right edge (relative to **FIG. 8D**) of the driven member **720.** The compression springs **730** bias the gripper sled **705** to the unclamped position where the gripper sled **705** is flush with the right and left edges (relative to **FIG. 8D**) of the driven member **720.**

When the clamp apparatus **710** is actuated from the open position to a clamped position the driven member **720** moves toward the fixed gripper assembly **703** and the appendages **725** of the driven member **720** slide along the grooved tracks **740** on the housing **712.** In turn, this displaces the movable gripper assembly **704** toward the fixed gripper **703** assembly. Until the movable gripper **701** contacts a clamped object **100,** the driven member **720** and movable gripper assembly **703** move as a unit. When the movable gripper **701** comes into contact with a clamped object **100,** the movable gripper assembly **704** can make no further progress toward the fixed gripper assembly **703** because the clamped object **100** is in the way. The driven member **720** continues to move toward the fixed gripper assembly **703** compressing the compression springs **730** between the interior left wall (relative to **FIG. 8D****)** of the hollow portion of the gripper sled **705** and the compression spring discs **731.** The restoring force of the compression springs **730** causes the movable gripper assembly **704** to exert a more vigorous clamping force on the clamped object **100.**

When the clamp apparatus **710** is moved from a clamped position toward an open position, the restoring force of the compression springs **730** may automatically spring the clamp apparatus **710** back to the unclamped and open position.

The clamp apparatus **710** may be moved from the open position to the closed position by user actuation of a toggle handle **750.** One end of the toggle handle **750** may be pivotally coupled to the housing **712** of the clamp apparatus **710.** In the embodiment shown in **FIGS 8A-8D****,** the toggle handle **750** attaches to the right (relative to **FIG. 8D****)** end cap **745** of the housing **712.** As shown, the right end cap **745** projects perpendicularly from the bottom face **743** of the housing **712** toward the top of the page. The right end cap **745** may be fixedly coupled to the housing **712** via screws, bolts, welds, etc. or may be molded as a continuous part of the housing **712.**

The right end cap **745** may comprise a number of other features. As shown in **FIGS. 8A-8D****,** the right end cap **745** may comprise a pair of projections **746** which project toward the fixed gripper assembly **703.** The projection **746** may extend parallel to the front wall **741** and back wall **742** of the housing **712.** Extension spring pegs **760** may protrude from each of the pair of projections **746.** In the embodiment depicted in **FIGS. 8A-8D****,** each of the extension spring pegs **760** project substantially perpendicularly from one of the pair of projections **746.** One end of an extension spring **762** is placed around each extension spring peg **760.** The extension springs **762** will be elaborated upon later.

Extending from the top edge of the right end cap **745** toward the fixed gripper assembly **703** may be a guide piece **748.** The guide piece **748** may extend parallel to the plane of the bottom face **743** of the housing **712.** The guide piece **748** may overhang the bottom face **743** of the housing **712.** As shown, the guide piece **748** in **FIGS. 8A-8D****,** may only extend from the medial section of the top edge of the right end cap **745.**

The right end cap **745** may also comprise a pair of U-brackets **747.** In the embodiment shown, the U-brackets **747** are disposed on the right end cap **745** such that the uprights of each U-bracket **747** project in the same direction and plane as the pair of projections **746.** One of the upright sections of one U-bracket **747** may be flush with the front edge of the right end cap **745** and abut the interior face of the front wall when the clamp apparatus **710** is assembled. One of the upright sections of the other U-bracket **747** may be flush with the back edge of the right end cap **745** and abut the interior face of the back wall **742** of the housing **712** when the clamp apparatus **710** is assembled. The other upright of each U-bracket **747** may be offset from the first upright of each U-bracket **747** such that it nearly abuts the extension spring pegs **760.** The bottom span of the U-bracket **747** may be formed by a face of the right end cap **745.** In alternate embodiments, the number, location, and orientation of projections **746,** U-brackets **747,** extension spring pegs **760,** and extension springs **762** may differ.

In the embodiment shown in **FIGS. 8A-8D****,** the toggle handle **750** is pivotally coupled into the U-brackets **747.** As shown, this is accomplished by means of dowel pins **749** which run through the U-brackets **747** and into the coupling spans **752** of the toggle handle **750.** The toggle handle **750 in** the exemplary embodiment may be divided up into a number of sections. As indicated above, the toggle clamp may have one or more coupling spans **752** to which other components of the clamp apparatus **710** may be coupled. Relative to **FIG. 8D****,** the coupling spans **752** are two vertical spans. As shown, the coupling spans **752** are offset from each other. Extending toward the right of the page from the each coupling span **752** at an angle roughly perpendicular to each coupling span **752** may be a horizontal span **753.** The horizontal spans **753** may be joined by a strut **754.** In some embodiments, the strut **754** may complete the toggle handle **750.** In the illustrated embodiment in **FIGS. 8A-8D****,** the toggle handle **750** comprises additional sections. Projecting off the strut **754** vertically toward the top of the page (relative to **FIG. 8D**) are two extension spans **757.** The extension spans **757** may be connected together by a handle grip **758** which a user may grasp when actuating the toggle handle **750.**

At least a portion of the handle grip **758** may be made of the same material as the rest of the toggle handle **750,** may be made of a different material, or may be made of a combination thereof. Possible materials may include, but are not limited to, rubber, polymer, composite, metal, plastic, foam, fabric, etc. Additionally, the handle grip **758** may comprise ergonomic finger groves, nubs, a ribbed texture, a honeycombed texture, etc. to facilitate ease of grasping and gripping.

In addition to the coupling spans **752** coupling the toggle handle **750** to the clamp apparatus **710,** the coupling spans **752** may also comprise a pair of handle extension spring pegs **763.** In the example embodiment shown in **FIGS. 8A-8D****,** one of the pair of handle extension spring pegs **763** projects perpendicularly from each coupling span **752** of the toggle handle **750.** In the example embodiment in **FIGS. 8A-8D****,** the handle extension spring pegs **763** project from the surface of each coupling span **752** which faces the opposite coupling span **752.** The end of the each extension spring **762** not seated on the first pair of extension spring pegs **760** is seated around one of the pair of handle extension spring pegs **763.** In the example embodiment in **FIGS. 8A-8D****,** the extension springs **762** act as over-center springs. When the toggle handle **750** is in the open position, the extension springs **762** bias the toggle handle **750** to stay in the open position. When the toggle handle **750** is in the closed position, the extension springs **762** move to an over-center position and bias the toggle handle **750** to stay in the closed position.

The coupling spans **752** of the toggle handle **750** may additionally couple to linkages **770.** In the example embodiment in **FIG. 8A-8D****,** one end of each linkage 770 is pivotally coupled to the driven member **720.** As shown, one linkage **770** is pivotally coupled between the front upright wall **723** of the driven member **720** and the vertical span of the appendage **725** which extends off the front upright wall **723 of** the driven member **720.** Also as shown in **FIGS. 8A-8D****,** the other linkage **770** is pivotally coupled between the back upright wall **724** of the driven member **720** and the vertical span of the appendage **725** which extends off the back upright wall **724** of the driven member **720.** In the example embodiment in **FIGS. 8A-8D****,** a dowel **771** is used to pivotally couple the linkages **770** to the driven member **720.**

The other end of each linkage **770** pivotally couples to the top of one of the coupling spans **752** of the toggle handle **750.** The linkage **770** and coupling spans **752** may be pivotally coupled by means of a coupling dowel pin **772.** Any other suitable coupling means may also be used.

When the clamp apparatus **710** is actuated, the coupling span **752** of the toggle handle **750** and the linkages 770 collectively may act as an over-center linkage. To actuate the toggle handle **750** a user may grasp the handle grip **758** of toggle handle **750.** The user may then rotate the toggle handle **750** substantially a full 90° counter-clockwise from the orientation of the handle toggle handle **750** shown in **FIG. 8A****.** In some embodiments, the sufficient degree of rotation may be larger or smaller (e.g. 95°). As the toggle handle **750** is rotated, the linkage **770** and coupling span **752** which comprise the over-center linkage move toward the center position. This pushes the driven member **720** and movable gripper assembly **704** as detailed above. Slightly before the toggle handle **750** has been rotated a full 90° counter-clockwise, the linkage **770** and coupling span **752** comprising the over-center linkage reach the center position. When the linkage and coupling span **752** comprising the over-center linkage reach the center position a large force is generated on the moveable gripper assembly 704 by applying only a small force to the toggle handle **750.** When the toggle handle **750** is rotated the full 90° counter-clockwise, the linkage **770** and the coupling span **752** comprising the over-center linkage reach an over-center position which keeps the toggle handle **750** and clamp apparatus **710** in the closed and clamped position and acts as a passive latch. This clamping action makes actuation of the clamp apparatus **710** easy for the user while also providing a sufficiently strong clamping force.

In some embodiments, the toggle handle **750** comprises a toggle handle latch **780** that operatively secures the toggle handle **750** and clamp apparatus **710** in the closed and clamped position. The toggle handle latch **780** may be disposed on the handle grip **758** of the toggle handle **750** such that it fits in a concavity **759** in the handle grip **758.** The toggle handle latch **780** may be pivotally coupled to the handle grip **758** and may be pivotable between an advanced and a retracted position. In some embodiments a pivot pin bearing **781** runs the length of the toggle handle latch **780.** In the embodiment shown in **FIGS. 8A-8D****,** the pivot pin bearing **781** runs along the bottom edge of the toggle handle latch **780.** A pivot pin **782** may pivotally couple the toggle handle latch **780** to the handle grip **758** by running through the pivot pin bearing **781** and into at least part of the handle grip **758.**

In some embodiments, including the embodiment illustrated in **FIGS. 8A-8D****,** the toggle handle latch **780** may be adapted such that a torsion spring **783** may be slid over at least a portion of the pivot pin bearing **781.** The torsion spring **783** may bias the toggle handle latch **780** to the advanced position. When the toggle handle latch **780** is pivoted toward the retracted position, the torsion spring **783** is spring loaded such that the restoring force of the torsion spring **783** causes the toggle handle latch **780** to automatically pivot back to the advanced position. In the advanced position, the toggle handle latch **780** is in its most protruding position. In the retracted position, the toggle handle latch **780** is pushed into the concavity **759** such that it protrudes minimally from the handle grip **758.**

In some embodiments, the toggle handle latch **780** may comprise a stop surface **784** along at a part of at least one face of the toggle handle latch **780.** The stop surface **784** catches on a part of the concavity **759** in the handle grip **758** and ensures the torsion spring **783** cannot eject the toggle handle latch **780** out of the concavity **759.**

The toggle handle latch **780** may also comprise a latch projection **785.** The latch projection **785** in the example embodiment depicted in **FIGS. 8A-8D** runs substantially the full length of the toggle handle latch **780** and projects off the toggle handle latch **780** toward the bottom of the page (relative to **FIG. 8D**). This may differ in alternative embodiments.

In some embodiments, the left face (relative to **FIG. 8D**) of the fixed gripper cradle **711** comprises a ramp catch **786** for the latch projection **785** of the toggle handle latch **780.** The catch **786** in alternative embodiments need not comprise a ramp. The catch **786** may take any other suitable form.

In the example embodiment in **FIGS. 8A-8D****,** as the toggle handle **750** and toggle handle latch **780** are rotated toward the closed position, the latch projection **785** of the toggle handle latch **780** abuts the catch **786** ramp. As the toggle handle **750** continues to rotate toward the closed position, the latch projection **785** of the toggle handle latch **780** rides up the catch **786** ramp. This causes the toggle handle latch **780** to be pivoted into the retracted position, i.e. into the concavity **759** of the handle grip **758.** In turn, this twists the torsion spring **783** and stores mechanical energy in the torsion spring **783.** When the toggle handle **750** is in the fully closed position, the latch projection **785** of the toggle handle latch **780** clears the catch **786** ramp and the restoring force of the torsion spring **783** causes the toggle handle latch **780** to spring back to the advanced position. This locks the clamp apparatus **710** in the closed position as any movement toward the open position is prevented by the latch projection **785** of the toggle handle latch **780** catching on the lip of the catch **786** ramp.

To rotate the toggle handle **750** back toward the open position and/or unclamp the clamp apparatus **710,** a user must manually push in the toggle handle latch **780** to the retracted position. This allows the latch projection **785** of the toggle handle latch **780** to clear the lip of the catch **786** ramp, thus allowing rotation of the toggle handle **750** toward the open position.

In some embodiments, the toggle handle latch **780** may have various contours which provide an ergonomic benefit to the user as a user tries to depress the toggle handle latch **780** to the retracted position when opening the clamp apparatus **710.** In the embodiment shown in **FIGS 8A-8D****,** the toggle handle latch **780** comprises a valley **788** which may better accommodate a user's fingertips as they pivot the toggle handle latch **780** into the retracted position. In other embodiments there may be additional ergonomic contours which supplement or replace the valley **788.**

In some embodiments including a toggle handle **750** or actuator similar to the toggle handle **750,** the toggle handle **750** or toggle handle latch **780** may include an anti-pinch feature (not shown) to preclude a user from pinching a finger when rotating the toggle handle **750** to the closed position. In some embodiments, the anti-pinch feature may be a guard protrusion. In other embodiments, the anti-pinch feature may be an extended gripping portion on the toggle handle **750** which distances a users fingers from the latch projection **785** and the catch **786.**

In some embodiments, the housing **712** of the clamp apparatus **710** may also feature any of a variety of mechanisms **790** (not shown) to attach a load to the clamp apparatus **710.** Such mechanisms may include, but are not limited to, brackets, magnets, straps, suction cups, hooks, screws, bolts, a friction fit, etc. This load could be any number of things, especially a medical device (such as an infusion pump, or peristaltic infusion pump), I.V. bag, etc.

In some embodiments, the clamp apparatus **710** may be adapted such that the fixed gripper assembly and **703** movable gripper assembly **704** may be oriented obliquely to the right and left ends (relative to **FIG. 8D**) of the housing **712.** In embodiments where the gripper assemblies **703** and **704** are oriented obliquely, any load attached to the clamp apparatus **710** by any of the variety of mechanisms **790** detailed above would be at an angle oblique to a clamped object **100** clamped in the clamp apparatus **710.** Such an orientation may be helpful in accommodating the needs of a load attached to the clamp apparatus **710** through any of the variety of mechanisms **790** described in the preceding paragraph.

**FIGS. 8E-8F** show an alternative embodiment of the example clamp apparatus **710** shown in **FIGS. 8A-****8D.** As shown, the alternative embodiment of the clamp apparatus **710** shown in **FIG. 8E** comprises a fixed gripper assembly **703** similar to the fixed gripper assembly **703** shown in **FIGS 8A-8D****.** The fixed gripper assembly **703** in **FIG. 8E** is somewhat simplified and allows the clamp apparatus **710** to have a more open concept which facilitates ease of cleaning. The fixed gripper assembly **703** in **FIG. 8E** does not include a fixed gripper base **717** as it does in **FIGS. 8A-8D****.** The fixed gripper assembly in **FIG. 8E** features two support legs **4000.** Each support leg **4000** may be coupled to the left (relative to **FIG. 8E**) face of the fixed gripper cradle **711.** The support legs **4000** may be coupled to the fixed gripper cradle **711** at an angle which is substantially perpendicular to the left face of the fixed gripper cradle **711.** In some embodiments, including the embodiment shown in **FIG. 8E****,** the support legs **4000** may be formed as a continuous part of the fixed gripper cradle **711b.**

One support leg **4000** may coupled to the fixed gripper cradle **711** near the front edge of the fixed gripper cradle **711.** The second support leg **4000** may be coupled to the fixed gripper cradle **711** near the back edge of the fixed gripper cradle **711.** The support legs **4000** are slightly arched in the example embodiment shown in **FIG. 8E****.** As shown, the width of the support legs **4000** may gradually decrease as the support legs **4000** extend toward the bottom of the page. The bottom of the support legs **4000** may be substantially parallel to the direction of elongation of the housing **712.**

As shown in the example embodiment in **FIGS. 8E****,** the threaded holes **791** which are located in the fixed gripper base **717** in **FIGS. 8A-8D** may be disposed at the bottom of the support legs **4000.** The threaded holes **791** may extend through the bottom of the support legs **4000** in a direction substantially perpendicular to the front and back faces of each support leg **4000.** As shown, four screws **714** may run through the housing **712** of the clamp apparatus **710** and into the corresponding threaded holes **791** in the bottom of the support legs **4000** thereby coupling the fixed gripper assembly **703** to the housing **712.**

As shown, the alternative embodiment of the clamp apparatus **710** shown in **FIGS. 8E-8F** comprises a movable gripper assembly **704** similar to the movable gripper assembly **704** shown in **FIGS 8A-8D****.** As shown, the movable gripper assembly **704** comprises a gripper sled **705.** The gripper sled **705** may be roughly planate and rectangular. The gripper sled **705** in **FIGS. 8E-8F** is roughly planate and rectangular though one end of the rectangular gripper sled **705** is rounded. The gripper sled **705** may comprise a dovetail cutout **4002** as shown in **FIG. 8E****.** The gripper sled **705** may be extruded.

The dovetail cutout **4002** of the gripper sled **705** may be sized to accommodate and slide along a dovetail projection **4004** on the housing **712** of the clamp apparatus **710.** As shown in the example embodiment in **FIG. 8E****,** the dovetail projection **4004** in the housing **712** may run roughly parallel with the front wall **741** and back wall **742** of the housing **712.** The dovetail projection **4004** on the housing **712** may run along the medial portion of the bottom face **743** of the housing **712.**

As shown in **FIG. 8E****,** the housing **712** may include roller tracks **4006.** As shown, the roller tracks **4006** are similar to the grooved tracks **740** shown in **FIGS. 8A-8D****.** The roller tracks of the housing **712** will be further elaborated on later. The housing **712** may also include any number of housing voids **4008.** The housing voids **4008** may be cut into the housing **712** or may be created during manufacture of the housing **712.** The housing voids **4008** help to keep debris and unwanted matter from accumulating in and on the clamp apparatus **710.** The housing voids **4008** may also aid in making the clamp apparatus **710** easier to clean. In some embodiments, the housing **712** may be extruded. In such embodiments, the clamp **710** may be extruded from any suitable material.

The movable gripper assembly **704** may comprise a number of additional components in addition to the gripper sled **705.** Projecting perpendicularly from the top face of the gripper sled **705** on the right (relative to **FIG. 8E****)** of the gripper sled **705** there may be a spring housing **4010.** The spring housing **4010** may project in a direction that is substantially perpendicular to the top face of the gripper sled **705.** The spring housing **4010** may be dimensioned such that the sides of the spring housing **4010** are flush with the edges of the gripper sled **705.** The spring housing **4010** may be coupled to the gripper sled **705** by any of a variety of fastening means.

In some embodiments, the movable gripper cradle **706** may be coupled to the left side (relative to **FIG. 8E****)** of the spring housing **4010.** In such embodiments, the movable gripper cradle **706** may be coupled to the spring housing **4010** by any suitable fastener. In the example embodiment, the movable gripper cradle **706** is made as a continuous part of the spring housing **4010.** As shown, the movable gripper cradle **706** is disposed on the spring housing **4010** such that it is at substantially the same height as the fixed gripper cradle **703.**

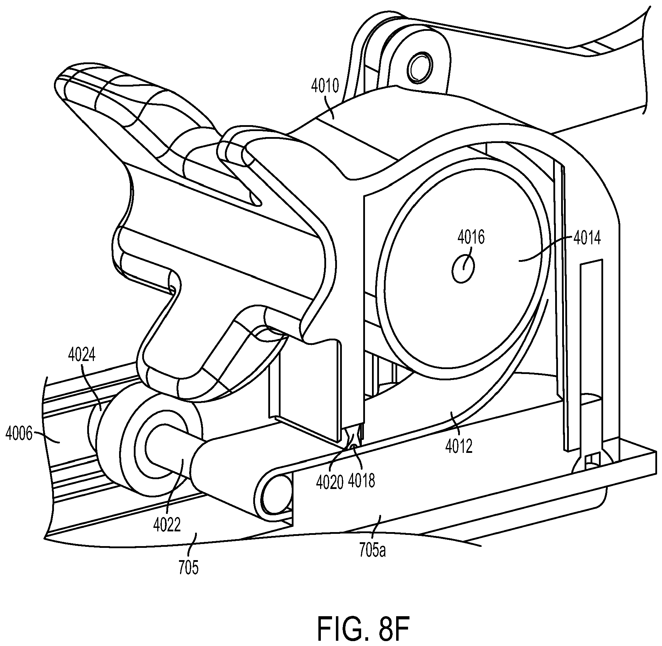

As shown in the cross section of the clamp apparatus **710** in **FIG 8F****,** the spring housing **4010** is substantially hollow. Within the hollow portion of the spring housing **4010** a constant force spring **4012** is housed. In some embodiments, there may be more than one constant force spring **4012** housed in the spring housing **4010.** The constant force spring **4012** in some example embodiments may be a rolled ribbon of spring steel. The constant force spring **4012** may be a laminar spring. In some embodiments, the constant force spring **4012** may be a triple laminar spring. In some embodiments, the constant force spring **4012** may be an approximately 19lb constant force spring **4012.** Use of a constant force spring **4012** provides many benefits over other varieties of bias members as detailed above.

As shown, the constant force spring **4012** may be disposed about a mandrel **4014** which is capable of rotating about the axis of an axle **4016.** In the example embodiment, the mandrel **4014** is a solid spindle. In other embodiments, the mandrel **4014** may not be solid. In some embodiments, the mandrel **4014** may be a hollow cylinder. In some embodiments, the mandrel **4014** may be mostly hollow and comprise a number of supporting spokes. The axle **4016** may span across the hollow section of the spring housing **4010.** The axle **4016** may extend in a direction substantially perpendicular to the front wall **741** and back wall **742** of the housing **712** shown in **FIG. 8E****.**

In the example embodiment in **FIG. 8F****,** the gripper sled **705** features a raised section **705a.** The raised section **705a** of the gripper sled **705** projects off the gripper sled **705** toward the top of the page in manner substantially perpendicular to the rest of the gripper sled **705.** As shown, a small gap **4018** may be left between the top of the raised portion **705a** of the gripper sled **705** and the bottom of the left side of the spring housing **4010.** The constant force spring **4012** may extend out of the spring housing **4010** through the small gap **4018.**

To help keep debris and other matter from entering the spring housing **4010,** spring housing sealing member **4020** may be placed at the bottom of the left side of the spring housing **4010.** As shown in the example embodiment in **FIG. 8F****,** a part of the spring housing sealing member **4020** may be seated in a cavity recessed into the bottom face of the left side of the spring housing **4010.** The spring housing sealing member **4020** may be made of a deformable material. As the constant force spring **4012** is advanced and retracted out of and back into the spring housing **4010** during operation of the clamp apparatus **710,** the spring housing sealing member **4020** blocks any debris or other matter on the constant force spring **4012** from being pulled into the spring housing **4010** as the constant force spring **4012** retracts back into the spring housing **4010.**

One end of the constant force spring **4012** may be located exterior to the spring housing **4010** at all times. The end of the constant force spring **4012** located exterior to the spring housing **4010** may be fixedly coupled to a roller axle **4022.** By pulling the roller axle **4022** toward the left of the page (relative to **FIG. 8F**) the constant force spring **4012** is unwound and spooled out of the spring housing **4010.** If the roller axle **4022** is released, the restoring force of the constant force spring **4012** will cause the roller axle **4022** to be biased back to the position shown in **FIG. 8F****.** The constant force spring **4012** will also retract back into the spring housing **4010.**

A roller **4024** may be seated on each end of the roller axle **4022.** One of the rollers **4024** is visible in **FIG. 8F****.** The rollers **4024** are capable of rotation about the axis of the roller axle **4022.** As shown in **FIGS. 8E-8F****,** the rollers **4024** may ride and roll along the roller tracks **4006** on the front wall **741** and back wall **742** of the housing **712.**

Referring back to **FIG. 8E****,** the linkages **770** extending from the toggle handle **750** may be coupled onto the roller axle **4022.** As such, the roller axle **4022** functions similarly to the driven member **720** in **FIGS. 8A-8D** and may be referred to as an alternative driven member. When the clamp apparatus **710** is actuated from the open position to a clamped position via rotation of the toggle handle **750,** the roller axle **4022** moves toward the fixed gripper assembly **703** and the rollers **4024** on the roller axle **4022** slide along the roller tracks **4006** on the housing **712.** In turn, this displaces the movable gripper assembly **704** toward the fixed gripper **703** assembly. Until the movable gripper **701** contacts a clamped object **100,** the roller axle **4022** and movable gripper assembly **703** move as a unit. When the movable gripper **701** comes into contact with a clamped object **100,** the movable gripper assembly **704** can make no further progress toward the fixed gripper assembly **703** because the clamped object **100** is in the way. The roller axle **4022** continues to move toward the fixed gripper assembly **703.** This causes the constant force spring **4012** to be pulled out of the spring housing **4010.** The restoring force of the constant force spring **4012** causes the movable gripper assembly **704** to exert a more vigorous clamping force on the clamped object **100.**

When the clamp apparatus **710** is moved from a clamped position toward an open position by rotation of the toggle handle **750,** the restoring force of the constant force spring **4012** may automatically spring the clamp apparatus **710** back to the unclamped and open position.

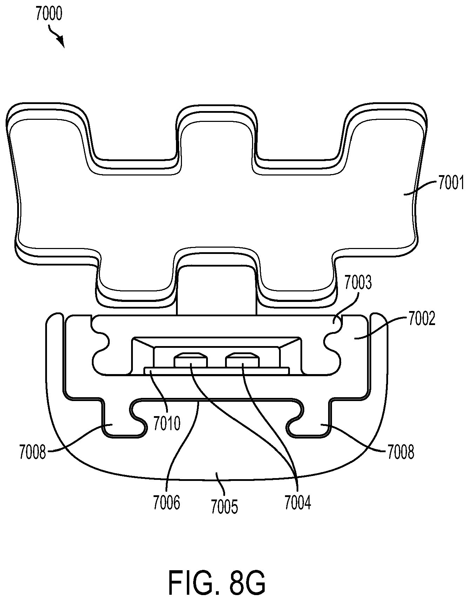

**Fig. 8G** shows an alternate embodiment of a moveable gripper assembly **7000** with a housing **7005** in accordance with an embodiment of the present disclosure. The moveable gripper assembly **7000** may be similar to the moveable gripper assembly shown in **Fig. 8F****.** The moveable gripper assembly **7000** includes a moveable gripper **7001,** a driven member **7002,** and a gripper sled **7003** with a housing **7005.** The driven member **7002** is guided via guide members **7008** along a track **7006.** Note that the contact force spring **7010** (e.g., spring **4012** as shown in **Fig. 8F**) is secured to the driven member **7002** by fasteners **7004.**

### A Rack Apparatus

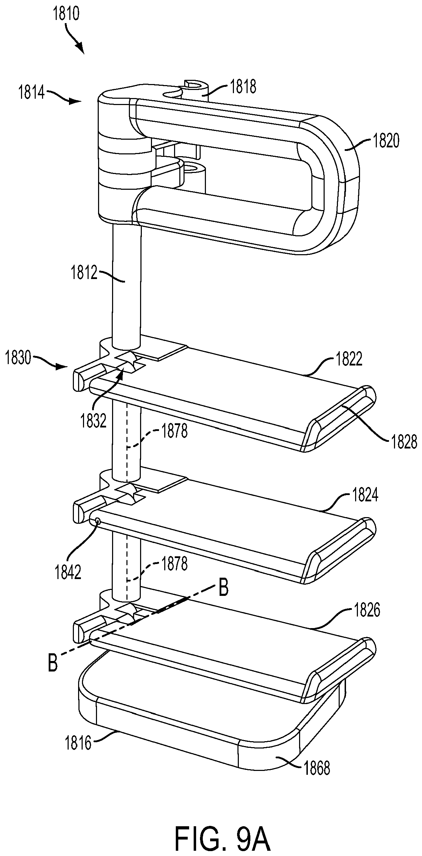

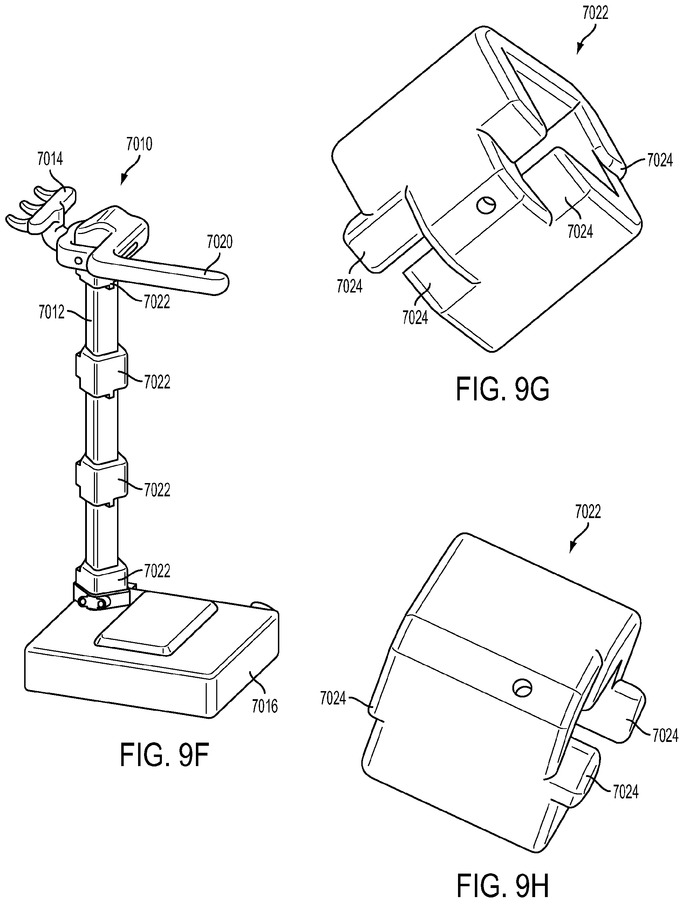

**FIG. 9a** depicts one exemplary embodiment of a rack **1810.** The rack **1810** includes a cylindrically-shaped support pole **1812.** A clamp assembly **1814** may be attached to a first end portion of the support pole **1812.** The clamp assembly **1814** may further include a clamp mechanism **1818** and an elongated, U-shaped handle **1820** that may be oriented perpendicularly to the longitudinal axis of the support pole **1812.** The clamp assembly **1814** and the clamp mechanism **1818** may be configured to removably couple with a support structure such as an IV pole. As should be appreciated by those having ordinary skill in the art, any number of clamp mechanisms may be used to accomplish this objective, including the clamp mechanisms described below and above. The handle **1820** enables the rack **1810** and any received medical devices to be carried as unit from one location to another. In certain embodiments, the handle **1820** may serve as a means to actuate the clamp mechanism **1818.** One such embodiment could include a handle **1820** that shares an axis of rotation with a clamp mechanism **1818,** wherein the clamp mechanism **1818** includes at least one fixed gripper and at least one mobile gripper that may be coupled to the handle **1820.** Actuation of the clamp mechanism **1818** may be achieved by rotating the handle **1820** in a first direction such that the at least one mobile gripper rotates towards the at least one fixed gripper and a support structure therebetween. The at least one mobile gripper and the at least one fixed gripper may be secured in a clamped position by a latch or any other means known in the relevant art when the aforementioned grippers exert a sufficient clamping force on the support structure. Rotating the handle **1820** in a second, opposite direction may rotate the at least one mobile gripper away from the at least one fixed gripper, and the clamp mechanism **1818** may be decoupled from the support structure when the at least one mobile gripper is sufficiently far from the support structure.

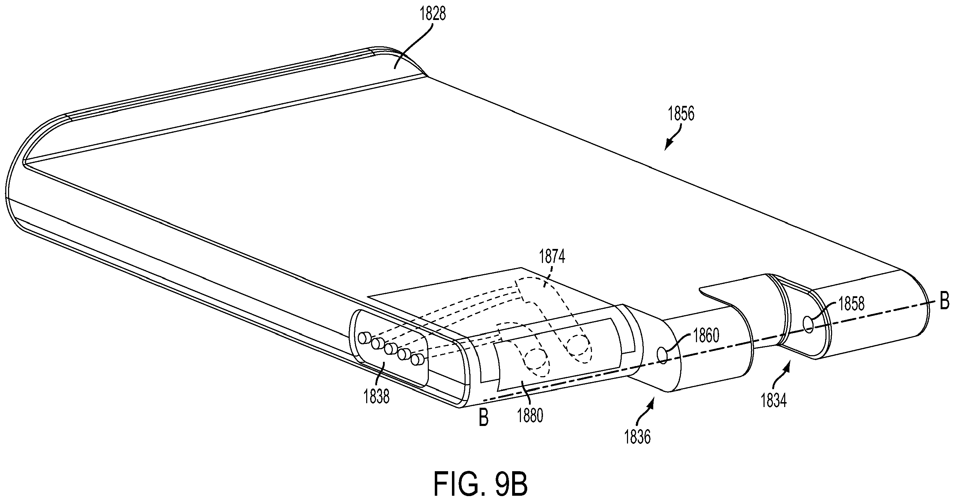

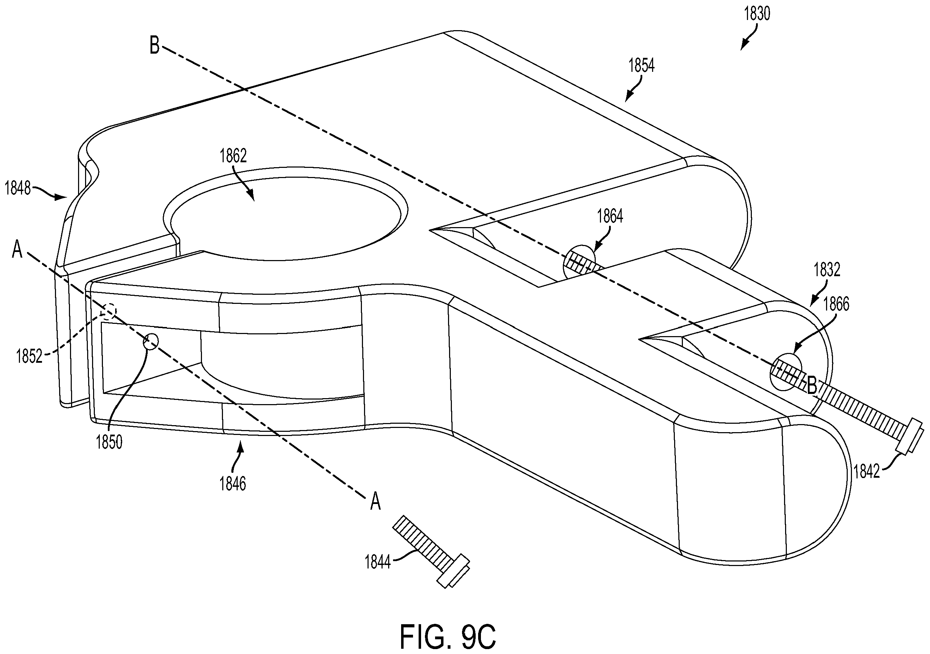

A variety of medical device mounts may be disposed between the first end and a second end of the support pole **1812.** **FIGS. 9a** and **9b** depict an exemplary embodiment where the mounts may be elongated support plates that extend perpendicularly to the support pole **1812.** **FIG. 9a** depicts a rack **1810** having a first support plate **1822,** a second support plate **1824,** and a third support plate **1826.** **FIG. 9b** depicts an embodiment of an individual support plate **1856.** The support plate **1856** may be sized to receive and support a medical device. Examples of medical devices that may be received by the support plate **1856** include syringe pumps, infusion pumps, dialysis machines, pill dispensers, and chemotherapy devices. A first end portion of the support plate **1856** may be coupled to the support pole **1812** using a joint member **1830.** The support plate **1856** may include a first support plate projection **1834** and a second support plate projection **1836** that may interface with the joint member **1830** (see Fig. 9C) to facilitate coupling. To more securely receive and retain a medical device, the support plate **1856** may include a flange **1828** that extends upwardly from a second end portion of the support plate **1856.**

To reduce the need to run power cables from electrical outlets to each individual medical device, each support plate **1856** may include a mount connector **1838** that may be adapted to transmit electrical power to a received medical device. In certain embodiments, the mount connector **1838** may also be adapted to enable signals to be communicated between two or more medical devices and thus provide each medical device with a network connection.

In the embodiment depicted in **FIG. 9a****,** a corresponding number of joint members **1830** couple each of the support plates **1822, 1824, 1826** to the support pole **1812.** Each joint member **1830** may be configured to receive a support plate **1856** such that the joint member **1830** enables the received support plate **1856** to rotate around a longitudinal axis of the support pole **1812.** **FIG. 9c** depicts an exemplary joint member **1830** that permits rotation around a longitudinal axis of the support pole **1812.** The joint member **1830** may include a joint member aperture **1862** that is sized to receive the support pole **1812.** The joint member **1830** may be rotated and re-secured to the support pole **1812** by loosening a threaded screw **1844,** rotating the exemplary joint member **1830** and a received support plate **1856** to the desired position, and retightening the threaded screw **1844.**

As depicted in **FIG. 9c****,** the exemplary joint member **1830** may include a first clamping arm **1846** and a second clamping arm **1848,** each having an inner surface that forms a portion of the joint member aperture **1862.** The first and the second clamping arms **1846, 1848** may further include a first threaded aperture **1850** and a second threaded aperture **1852** respectively. The first threaded aperture **1850** and the second threaded aperture **1852** may be aligned along a line **A-A** and each may be sized to receive the threaded screw **1844.** As will be understood by persons having ordinary skill in the art, rotating the threaded screw **1844** in a first direction, generally clockwise, may pull the first and the second clamping arms **1846, 1848** towards one another and enable the joint member aperture **1862** to exert a predominantly horizontal force against a received support pole **1812** such that the received support pole **1812** may support, against the force of gravity, the weight of the joint member **1830,** the received support plate **1856,** and any received medical devices. Turning the threaded screw **1844** in a second, opposite, and generally counter-clockwise direction may push the first and the second clamping arms **1846, 1848** apart and may reduce the force applied to the support pole **1812** by the joint member aperture **1862** and may enable the joint member **1830** to be rotated about the support pole **1812.**

In addition, the joint member **1830** may be hingably coupled with a received support plate **1856,** and the joint member **1830** may be placed in one of a vertical or a horizontal orientation such that the received support plate **1856** (eg. **1822, 1824, 1826** in **FIG. 9a**) can rotate in a transverse plane or a longitudinal plane of the support pole **1812.** **FIGs. 9b** and **9c** respectively depict an embodiment of the present disclosure wherein a support plate **1856** and a joint member **1830** are configured to be hingably coupled, and wherein the resulting hinged joint may be placed in a substantially horizontal orientation such that the support plate **1856** may rotate in a longitudinal plane of the support pole **1812.** **FIG. 9a** depicts an embodiment wherein the rack **1810** includes three of this type of coupling mechanism. Alternatively, a support plate **1856** or other type of medical device mount may be fixedly and rigidly coupled to the support pole **1812** in different embodiments.

In the embodiment depicted in **FIG. 9b****,** the support plate **1856** may include a first support plate projection **1834** and a second support plate projection **1836** that extend in substantially parallel directions from a first end portion of the support plate **1856.** The first support plate projection **1834** and the second support plate projection **1836** respectively include a first support plate aperture **1858** and a second support plate aperture **1862** that may be aligned along a line **B-B,** and wherein each is sized to receive a pin **1842.**

In the embodiment depicted in **FIG. 9c****,** the joint member **1830** may include a first joint member projection **1832** and a second joint member projection **1856** that extend in substantially parallel directions. The first joint member projection **1832** and the second joint member projection **1856** may respectively include a first joint member aperture **1864** and a second joint member aperture **1866** that may be aligned along a line **B-B,** and wherein each is sized to receive a pin **1842.**

To hingably couple the support plate **1856** to the joint member **1830** as depicted in **FIG. 9a****,** the first and the second support plate projections **1834, 1836** and the first and the second joint member projections **1832, 1856** (referring now also to **FIGS. 9b****-c)** may be respectively sized and disposed on the support plate **1856** and joint member **1830** such that the respective projections **1832, 1834, 1836, 1856** are capable of interleaving. The apertures **1864, 1866** of the joint member **1830** are configured to align with the apertures **1860, 1858** of the support plate **1856** such that all four apertures **1858, 1862, 1864, 1866** will align along the line **B-B** when the four projections **1832, 1834, 1836, 1856** are interleaved. When properly aligned, a pin **1842** may be inserted through and retained in the four apertures **1858, 1862, 1864, 1866** such that the joint member **1830** retains the support plate **1856.** As will be understood by persons having ordinary skill in the art, a number of methods are available to maintain the position of the support plate **1856** about the pin **1842.** In certain embodiments, the friction between the interleaved projections **1832, 1834, 1836, 1856** and/or the friction between the pin **1842** and the four apertures **1858, 1862, 1864, 1866** in which the pin **1842** is disposed may be sufficient to maintain the position of the support plate **1856** about the pin **1842.** Any other structure may secure the joint member **1830** to the support plate **1856** known to one of ordinary skill in the relevant art.

In other embodiments, the position of the support plate **1856** about the pin **1842** may be maintained at one of several predefined positions by a detent pin (not shown) that is capable of engaging one of several detents (not shown) in an inner joint member projection. The detents may be annularly inscribed at several positions about the pin **1842.** In embodiments having such detents, a detent pin aperture may retain the detent pin and be disposed in an outer support plate projection so as to enable the detent pin to selectively engage any one of the detents in the inner joint member projection. Once a healthcare provider engages the detent pin with the appropriate detent, the detent and the detent pin can prevent the support plate **1856** from rotating out of the selected position.

In particular embodiments, like the embodiment depicted in **FIG. 9a****,** the weight of multiple received medical devices may cause the rack **1810** to become unbalanced and begin to rotate about the point where the clamp mechanism **1818** couples with a support structure like an IV pole. To mitigate this type of rotation, a base member **1816** may be employed that exerts a stabilizing force on the support structure. As depicted in **FIG. 9a****,** the base member **1816** may comprise an elongated housing **1868** that is coupled to a second end portion of the support pole **1812** and that extends perpendicularly to the support pole **1812.** The base member **1816** may include a rounded notch **1840** that is configured to abut a substantially cylindrical support structure. The notch **1840** may be disposed on the elongated housing **1868** such that the base member **1816** and the clamp mechanism **1818** position the support pole **1812** in spaced relation to and substantially parallel to an elongated, cylindrical support structure like an IV pole. In other embodiments, the base member may comprise a second clamp assembly like the clamp assembly **1814** that may be coupled to the first end of the support pole **1812.**

An advantage of the exemplary embodiment depicted in **FIG. 9a****,** is that the base member **1816** and the elongated housing **1868** can serve other functions in addition to providing a counterbalancing force to the rack **1810.** For example, the elongated housing **1868** may serve as a bedside surface on which a healthcare provider may temporarily store items that are needed to care for a patient. In another embodiment, the elongated housing **1868** could also be configured to receive a medical device and include the same features as a support plate **1856,** such as a mount connector **1838** that is configured to provide one or both of electrical power and a network connection to a received medical device. In embodiments where the base member **1816** does not include an elongated housing **1868,** the base member **1816** may nevertheless be configured to receive, power, and provide a network connection to an additional medical device.

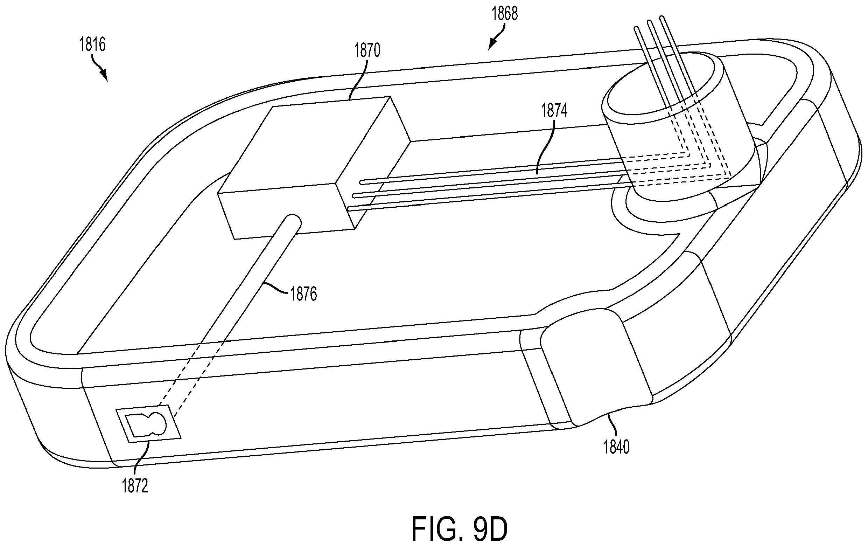

Another advantage of the exemplary embodiment depicted in **FIG. 9a** and the exemplary base member **1816** depicted in **FIG. 9d** is that the elongated housing **1868** may provide space to contain certain elements of a power system. **FIG. 9d** depicts an exemplary power system that includes a power supply **1870,** a power connector **1872,** power transmission cables **1874,** and a main power cable **1876.** As discussed above, embodiments that include a power system may have the advantage of reducing the number of cables that are needed to power the received medical devices. Rather than having to run a separate power cable from an electrical outlet to each medical device, a single power cable may be connected from an electrical outlet to a power connector **1872** that is preferably located on the elongated housing **1868** of the base member **1816.** A main power cable **1876** may then deliver power to a power supply **1870.** The power supply **1870** may be configured to convert balanced or unbalanced AC current to direct current and provide the desired voltage and amperage for any received medical devices. A respective power transmission cable **1874** may be used to transmit electrical power from the power supply **1870** to a respective mount connector **1838** and a received medical device. The power transmission cables **1874** may provide one more DC voltages for use by any received medical devices. In certain embodiments, the respective power transmission cable **1874** may operatively run from a power supply **1870,** up through a hollow support pole **1812,** and may be operatively distributed to the respective mount connector **1838.** Each of the support plates **1856** may include a mount connector **1838** and receive a respective power transmission cable **1874** that enables the mount connector **1838** to supply electrical power to a received medical device. In some embodiments, a common power bus may be positioned within a hollow support pole **1812** that receives power from the power transmission cables **1874;** each mount connector **1838** may be electrically coupled to the power bus.

In addition to supplying power to a received medical device, the exemplary mount connector **1838** depicted in **FIG. 9b** may be configured to provide a network connection to a received medical device. In embodiments that are capable of receiving two or more medical devices, it may be advantageous to enable the received medical devices to communicate with one another. For example, a patient may require a regime of several different drugs that are administered by respective syringe pumps. In other instances, it may be desirable to arrange are relay infusion of the same drug using two or more pumps. Enabling the rack **1810** to transmit signals between network-capable syringe pumps may allow for each syringe pump to know how much of which drugs were delivered by the other syringe pumps in the rack **1810** network. To achieve this objective, exemplary embodiments like the embodiment depicted in **FIG. 9a** may include a central bus **1878** that is operatively coupled to the support pole **1812.** Each of the support plates **1856** may include a support-plate bus **1880** that operatively interfaces with the central bus **1878** and that is coupled to a mount connector **1838.**

In some embodiments, each received medical device may broadcast its data over the central bus **1878.** In other embodiments a turn-based communication scheme may be used by the received medical devices to communicate with each other using the central bus **1878.** In yet additional embodiments, a carrier-sense, multiple-access with optional collision avoidance communication scheme may be used by the medical devices when communicating via the central bus **1878.**

Yet another advantage of the exemplary embodiment of the rack **1810** depicted in **FIG. 9a** and the exemplary base member **1816** depicted in **FIG. 9d** is that the elongated housing **1868** may optionally include provisions, such as casters and the like, for coupling with two or more wheels. In addition to the handle **1820,** wheels may allow the rack **1810** to be more easily moved from one location to another, particularly when transporting multiple received medical devices. As should be understood by persons having ordinary skill in the art, wheels may be coupled to the elongated housing **1868** by any number of well-known means. In addition, two or more wheels may be coupled to a wheel assembly structure that enables the wheels to be coupled to or decoupled from the elongated housing **1868** as a group. In other exemplary embodiments, the support pole **1812** may include provisions for mounting two or more wheels or a wheel assembly.

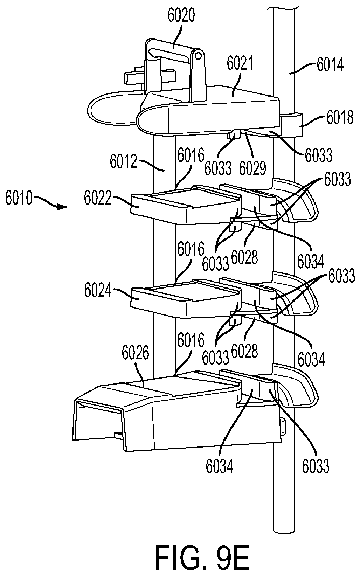

**FIG. 9e** depicts one exemplary embodiment of a rack **6010.** The rack **6010** includes a cylindrically-shaped support pole **6012.** A clamp mechanism **6018** and a U-shaped handle **6020** on a handle plate **6021** may be oriented perpendicularly to the longitudinal axis of the support pole **6012.** The clamp mechanism **6018** may be configured to removably couple with a support structure **6014** such as an IV pole. As should be appreciated by those having ordinary skill in the art, any number of clamp mechanisms **6018** may be used to accomplish this objective, including the clamp mechanisms described below and above. The handle **6020** enables the rack **6010** and any received medical devices to be carried as unit from one location to another. In certain embodiments, the handle **6020** may serve as a means to actuate the clamp mechanism **6018.** One such embodiment could include a handle **6020** that shares an axis of rotation with a clamp mechanism **6018,** wherein the clamp mechanism **6018** includes at least one fixed gripper and at least one mobile gripper that may be coupled to the handle **6020.** Actuation of the clamp mechanism **6018** may be achieved by rotating the handle **6020** in a first direction such that the at least one mobile gripper rotates towards the at least one fixed gripper and a support structure **6014** therebetween. The at least one mobile gripper and the at least one fixed gripper may be secured in a clamped position by a latch or any other means known in the relevant art when the aforementioned grippers exert a sufficient clamping force on the support structure **6014.** Rotating the handle **6020** in a second, opposite direction may rotate the at least one mobile gripper away from the at least one fixed gripper, and the clamp mechanism **6018** may be decoupled from the support structure **6014** when the at least one mobile gripper is sufficiently far from the support structure **6014.**

A variety of medical device mounts may be disposed between the first end and a second end of the support pole **6012.** **FIG. 9e** depicts an exemplary embodiment where the mounts may be elongated support plates that extend perpendicularly to the support pole **6012.** **FIG. 9e** depicts a rack **6010** having a first support plate **6022,** a second support plate **6024,** and a third support plate **6026.** The first support plate **6022,** second support plate **6024,** and third support plate **6026** may be sized to receive and support a medical device such as any of those described above. One end portion of each of the first support plate **6022,** a second support plate **6024,** and a third support plate **6026** may be coupled to the support pole **6012** via a joint member **6016.** The joint member **6016** may be similar to the joint member **1830** described above.

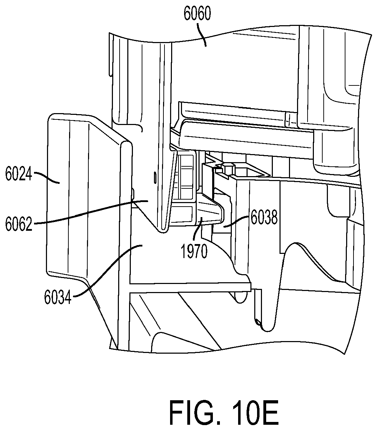

The third support plate **6026** may perform the same function as the base member **1816** and elongate housing **1868** in **FIGS. 9a****-d.** In some embodiments, the third support plate **6026** may also house elements of a power system like the power system described above and may include a mount connector **6038** (best shown in **FIG. 10E**) that is configured to provide one or both of electrical power and a network connection to a received medical device. As described above in relation to **FIGS. 9a****-d** the first support plate **6022,** second support plate **6024,** and third support plate **6026** may each include a mount connector **6038.**

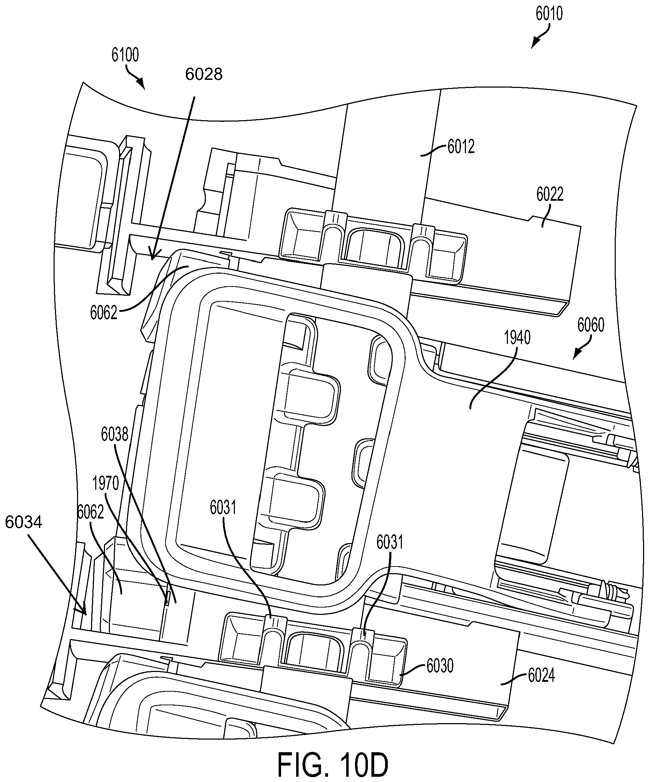

As shown in **FIG. 9e****,** the first support plate **6022,** a second support plate **6024,** and a third support plate **6026** each may include a first guide trough **6034** and a second guide trough **6028.** As shown, the third support plate **6026** only includes a first guide trough **6034.** As shown, the handle plate **6021** also includes a handle plate guide trough **6029.** The guide troughs **6026, 6034, 6029** may also include guide rails **6033.** The guide rails **6033** may be the sides of the guide troughs **6026, 6034, 6029,** or may be projections which project off the first support plate **6022,** second support plate **6024,** and third support plate **6026** or handle plate **6021.** In embodiments where the guide rails **6033** are projections, the guide rails **6033** may define the sides of the guide troughs **6026, 6034, 6029.** As in the exemplary embodiment in **FIG. 9e****,** the ends of the guide rails **6033** may bow out or angle out away from the longitudinal axis of the guide troughs **6026, 6034, 6029.** This may allow a medical device to be easily and sightlessly slid into the guide troughs **6026, 6034, 6029** and docked on the supports plates **6022, 6024, 6026.** In some embodiments, the medical device may include a feature or features such as a flanges **6062** (see **FIG. 10d**) which may be sized to fit within the guide troughs **6026, 6034, 6029.**

**FIG. 9f** depicts one exemplary embodiment of a rack **7010.** The rack **7010** includes a support pole **7012.** A clamp mechanism or assembly (not shown) may be attached to a first end portion of the support pole **7012.** The rack **7010** may include handle **7020** that may be oriented perpendicularly to the longitudinal axis of the support pole **7012.** The clamp mechanism or assembly may be configured to removably couple with a support structure such as an IV pole. As should be appreciated by those having ordinary skill in the art, any number of clamp mechanisms or assemblies may be used to accomplish this objective, including the clamp mechanisms and assemblies described herein. The handle **7020** enables the rack **7010** and any received medical devices to be carried as unit from one location to another. In certain embodiments, the handle **7020** may serve to actuate the clamp mechanism the clamp mechanism or assembly. The example rack **7010** shown in **FIG. 9f** also includes a hanger feature **7014** which may for example be used to hang IV bags, IV lines, etc.

The rack **7010** may include a base member **7016** similar to that described in **FIG. 9d****.** In such embodiments, the base member **7016** may include, for example, certain elements of a power system. The base member **7016** may also include certain components of a communication system. The base member **7016** may include wheels to aid in transporting the rack **7010** and any attached medical devices.

The rack **7010** depicted in **FIG. 9f** may optionally include, in yet additional embodiments, support plates like those embodiments depicted in **FIGS. 9a****-e.** The rack **7010** includes a number of collars **7022** which help to assure that a medical device coupled to the rack **7010** is correctly and securely coupled to the rack **7010.** The collars **7022** may be coupled to the support pole **7012** at suitable locations. In some embodiments, the collars **7022** may be spaced apart from one another at equal intervals. The example embodiment depicted in **FIG. 9f** includes four collars **7022.**

Referring now also to **FIGS. 9g****-h,** the collars **7022** may include a number of alignment features **7024.** **FIG. 9g** depicts a front perspective view of an example collar **7022** and **FIG. 9f** depicts a back perspective view of the same example collar **7022.** The alignment features **7024** may be one of or any combination of protuberances, recesses, steps, cutouts, pegs, posts, or any other suitable feature in various embodiments. The alignment features **7024** may be dimensioned such that any medical device which is to be attached to the rack **7010** can only be attached in a correct orientation. In some embodiments, the alignment features **7024** may not be included on a collar **7022** but rather on the support pole **7012** itself. Such embodiments may not include collars **7022.**

In the example embodiment in **FIGS. 9f****-h,** the alignment features **7024** include a number of protuberances and cutouts. The alignment features **7024** are dimensioned such that a clamp apparatus (for example, the clamp apparatus **710** in **FIGS. 8a****-f**) is prevented from clamping closed on the support pole **7012** in all but a correct orientation. In turn, this causes a medical device which is attached to the clamp apparatus to be correctly oriented on the rack **7010.**

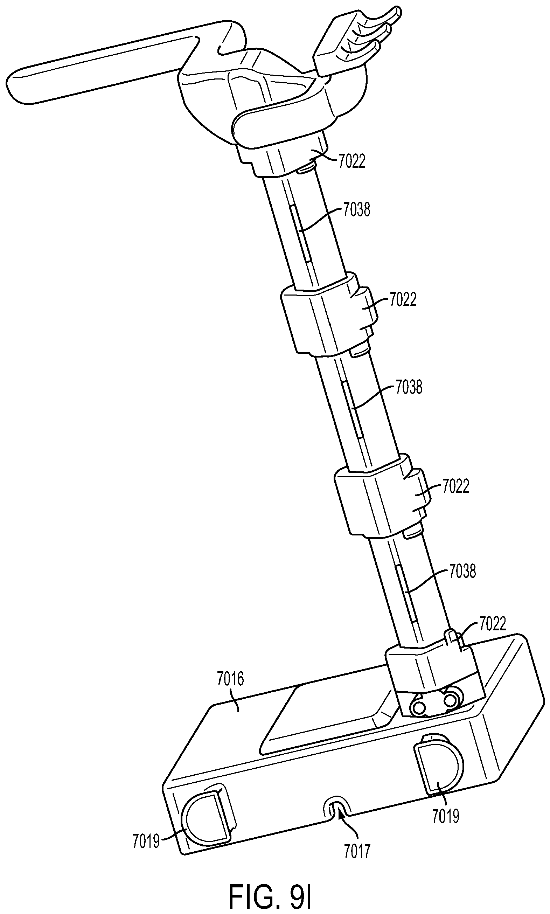

**FIG. 9i** shows a view of the back of the example rack **7010** in **FIG. 9f****.** As shown, the base member **7016** includes an opening **7017** to allow for a power cable, communications cable, etc. to enter the interior of the base member **7016.** The base member **7016** may also includes a number of projections **7019** which may be used to wrap a power cable, communications cable, etc. around when the entire length of the cable is not needed or the cable is not in use. In some embodiments, the rack **7010** may include at least one plug or receptacle which may be configured to receive a power cable, communications cable, etc.

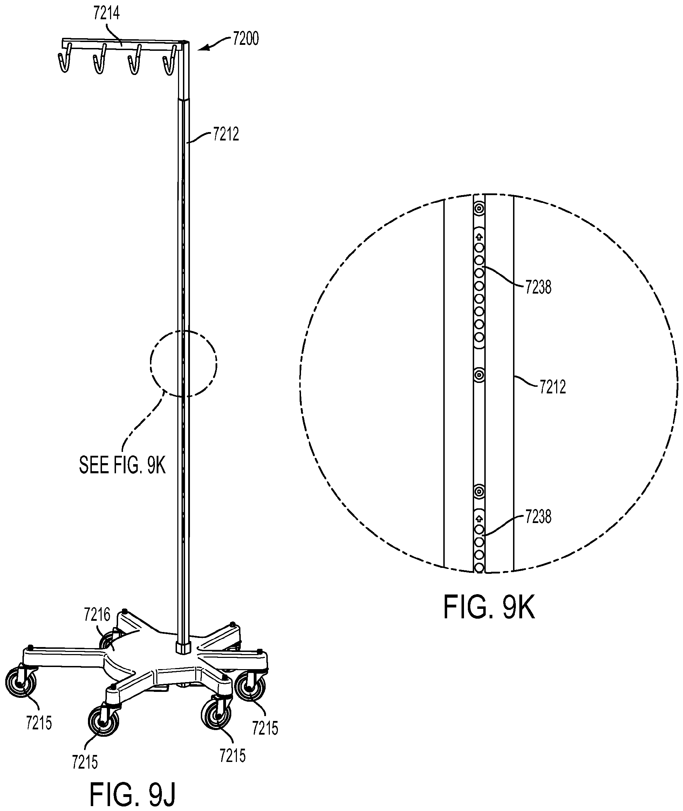

As shown in **FIG. 9i** the rack **7010** may include one or a number of mount connectors **7038.** The mount connectors **7038** may be configured to provide one or both of electrical power and a network connection to a received medical device. In some embodiments, the mount connectors **7038** may be configured to allow a received medical device to communicate over a CANbus and/or over USB. In other embodiments, the mount connectors **7038** may allow for communication using other communication schemes, such as, for example, any of those described above in relation to **FIGS. 9a****-d.** The rack **7010** may include a mount connector **7038** for each attached medical device. In the example embodiment, the rack **7010** includes three mount connectors **7038.** As shown, the mount connectors **7038** are included as a part of the support pole **7012** and are located on the back of the support pole **7012.** In other embodiments, the mount connectors **7038** may be located elsewhere on the rack **7010.** In embodiments which include mount connectors **7038,** the collars **7022** may assure that a medical device can only be received by the support pole **7012** in a manner in which it operatively engages a respective mount connector **7038.** Referring now to **FIG. 9j****,** another example embodiment of a rack **7200** is shown. The rack **7200** may be an IV pole, as shown. The rack **7200** may include a support pole **7212** to which a number of medical devices may be coupled. In some embodiments of the rack **7200,** the rack **7200** may include collars and/or alignment features similar to those described in reference to **FIGS. 9f****-i.** The collars and/or alignment features may help to ensure that medical devices are attached to the rack **7200** in a correct and secure manner. In other embodiments, the rack **7200** may include one or more support plates such as any of those described in reference to **FIGS. 9a****-e.** In some embodiments, the rack **7200** may include a combination of collars alignment features and support plates.

A hanger feature **7214** may also be included on the rack **7200.** In the example embodiment, a hanger feature **7214** is attached to the top end of the support pole **7212.** In alternate embodiments, the hanger feature **7214** may be located elsewhere on the rack **7200.** The hanger feature **7214** may be used to hang IV bags, IV lines, etc.

The bottom of the support pole **7212** of the rack **7200** may couple into a base member **7216** as it does in **FIG.** 9j. The base member **7216** may include a number of wheels or casters **7215** which may allow the rack **7200** and any attached devices to be easily moved around a care facility. The base member **7216** may be similar to that described in **FIG. 9d****.** For example, the base member **7216** may include certain elements of a power system as described in relation to **FIG. 9d****.** In other embodiments, the base member **7216** may also include certain elements of a communication system.

Referring now also to **FIG. 9k****,** the support pole **7212** may be similar to that depicted in **FIGS. 9f****-i.** The support pole **7212** may include a number of mount connectors **7238.** The mount connectors **7238** may be configured to provide one or both of electrical power and a network connection to a received medical device. In some embodiments, the mount connectors **7238** may allow a received medical device to communicate over a CANbus and/or over USB. In other embodiments, the mount connectors **7238** may allow for communication using other communication schemes, such as, for example, any of those described above in relation to **FIGS. 9a****-d.** The rack **7200** may include a mount connector **7238** for each attached medical device. In the example embodiment, the rack **7200** may, for example, include up to nine mount connectors **7238** and be capable of receiving nine medical devices. In other embodiments, the number of mount connectors **7238** and number of medical devices which can be received may differ. In embodiments which include mount connectors **7038,** collars and/or alignment features (such as the collars **7022** and alignment features **7024** shown in **FIG. 9i**) may be included. The collars and/or alignment features may assure that a medical device can only be received by the support pole **7212** in a manner in which it operatively engages a respective mount connector **7238.**

As will be understood by persons having ordinary skill in the art, the racks **1810, 6010, 7010, 7200** and their components can be made from a variety of rigid, engineering materials. Possible materials include aluminum alloys, stainless steel alloys, steel alloys, and engineering polymers. In addition, a variety of coatings may be applied to the racks **1810, 6010, 7010, 7200** and their components. Many of the possible coatings may provide a means of reducing the likelihood of cross-contamination. Cross-contamination may pose a serious health risk to young and old patients and patients with weakened immune systems. Optionally, an antibacterial, an antiviral, or an antimicrobial coating may be applied to the structural components of the racks **1810, 6010, 7010, 7200** to kill or inhibit the growth bacteria, viruses, fungi, and various other microorganisms. Exemplary coatings may include copper, copper particles, silver, silver particles, or other materials that have antibacterial, antiviral, or antimicrobial properties.

### Rack Systems

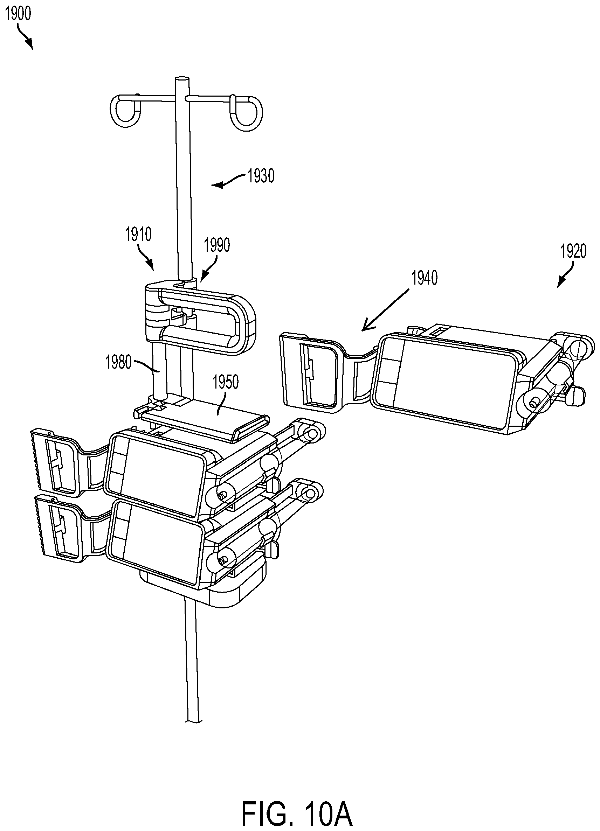

**Fig. 10a** shows an exemplary rack system **1900.** The exemplary embodiment of a rack **1810** depicted in **FIG. 9a** may be one element of a rack system **1900** shown in **Fig. 10a****.** Another element of the rack system **1900** may be a device that includes a mounting mechanism that is configured to couple with the rack, such as a clamp mechanism like any one of those described above. It should be understood that the exemplary embodiment depicted in **FIG. 9a** is but one embodiment of a rack that may be used in a rack system, and alternative embodiments of the rack and mounting mechanism may depart, perhaps substantially, from the exemplary embodiments described herein.

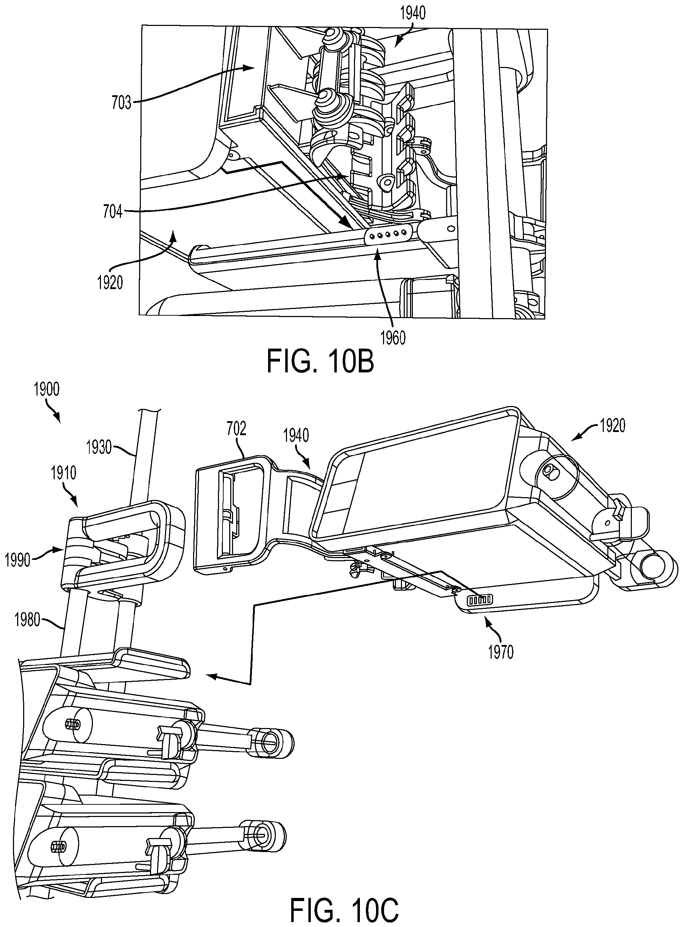

**FIG. 10a** depicts an embodiment of a rack system **1900** comprising a rack **1910** that is substantially the same as the rack **1810** embodiment described above and depicted in **FIGs. 9a****-d,** a medical device **1920** that may be received by a support plate **1950** of the rack **1910,** and a clamp mechanism **1940** that is coupled to a first side of a medical device **1920** and that is adapted to securely couple the medical device **1920** to the rack **1910.** **FIG. 10b** depicts the same embodiment as **FIG. 10a** but from a different perspective. **FIG. 10b** includes a view of the clamp mechanism **1940,** described in detail below, and a mount connector **1960** that is disposed on the support plate **1950.** **FIG. 10c** is yet another perspective of the embodiment depicted in **FIGs. 10a** and **10b** and includes a view of a device connector **1970** that is disposed on the medical device **1920.** The mount connector **1960** and the device connector **1970** are preferably disposed on the support plate **1950** and the medical device **1920** respectively so that they are operatively aligned and capable of coming into contact when the clamp mechanism **1940** couples the medical device **1920** to the rack **1910.**

In a preferred embodiment of the rack system **1900,** the clamp mechanism **1940** may be a mechanism like the embodiment depicted in **FIGs. 8a-8d** or described in relevant portions of the specification above. The clamp mechanism **1940** may latch onto the support pole **1980** depicted in **FIGs. 10a-10c****.**

As should be evident from the description of the above embodiments of a clamp mechanism **1940,** actuating the clamp mechanism **1940** to couple an attached medical device **1920** to a support pole **1980** may have a first phase and a second phase. Refer now to **FIGs. 8a-8d** and **FIGs. 10a-10C****.** In the first phase, user rotation of the handle **702** may move the driven member **710** and the slidably attached mobile gripper **704** towards the fixed gripper **703** until the girth of the support pole **1980** arrests the movement of the mobile gripper **704.** Thus, the first phase ends when the fixed gripper **703** and the mobile gripper **704** contact the support pole **1980.** In the second phase, continued rotation of the handle **702** may continue to drive the driven member **710** towards the fixed gripper **703** and bias the compression spring **730** (or other bias member) because the driven member **710** may continue to move independently of the mobile gripper **704.** Therefore, the second phase enables the user to increase the clamping force and ensure that the medical device **1920** is securely coupled to the rack **1910.**

In some embodiments, the medical device may be a monitoring client (e.g., a tablet computer) to monitor the operation of the other medical devices (e.g., via wireless communications such as WiFi or Bluetooth, for example). The monitoring client may have a serial interface to connect to the mount connector **1960** (see **Fig. 10B**). Additionally, as mentioned above, the monitoring client may couple to a clamp such as any of those described herein. The clamp may then be used to secure the monitoring client to a rack **1910.**

Referring now to **Figs. 10a-10c****,** the rack system **1900** may be best employed where a patient requires treatment with a coordinated regime of drugs, particularly where the drugs are to be administered by syringe pumps. Because syringe pumps are capable of continuously or discretely delivering precise quantities of fluid over a period of time, syringe pumps are well-suited to administering a regime of different drugs at predefined times. Computerized and networked syringe pumps may allow such a regime to be administered automatically. Embodiments of the present disclosure, like the embodiment of a rack system **1900** depicted in **FIGs. 10a****-c,** may enable a healthcare provider to quickly setup a group of networked syringe pumps to administer such a regime of drugs. Additionally, embodiments of the present disclosure, like the embodiment of a rack system **1900** depicted in **FIGS. 10a****-c,** may help to minimize the number of cords and cables which would otherwise be present when a group of pumps is setup.

For example, a healthcare provider may quickly couple the clamp assembly **1990** to a support structure **1930,** such as an IV pole, and connect the rack **1910** to a source of electrical power. If no syringe pumps or other devices are already coupled to the rack **1910,** the healthcare provider may proceed to couple the required syringe pumps to the rack **1910** one at a time. The healthcare provider may couple each syringe pump to the rack **1910** by placing a portion of each syringe pump on one of the support plates **1950** such that the support plate **1950** bares at least a portion of the weight of the syringe pump, allowing the healthcare provider to more easily maneuver the syringe pump into position. Once the support pole **1980** is positioned between the fixed gripper **703** (see **FIGs. 8a-****8d**) and the mobile gripper **702** (see **FIGs. 8a-8d**) and once the mount connector **1960** and the device connector **1970** are in general alignment, the healthcare provider may rotate the handle **702** through the first phase of operation. During the first phase of operation, the device clamp-mechanism **1940** may automatically adjust to the size of the support pole **1980** and the mount connector **1960** and the device connector **1970** may be brought into contact with one another. The healthcare provider may secure the syringe pump to the rack **1910** by continuing to rotate the handle **702** through the second phase of operation, and the healthcare provider may repeat the procedure for as many syringe pumps as may be desired. Thus, the healthcare provider may provide each syringe pump with electrical power and a network connection to other syringe pumps via the mount connector **1960** and device connector **1970** without having to run multiple power and network cables that may complicate the setup procedure and clutter the environment around the patient. Moreover, any one of the syringe pumps may be decoupled from the rack **1910,** or another syringe pump may be coupled to the rack **1910,** without having to detach or attach any additional cables. When treatment is complete, certain syringe pumps may remain coupled to the rack and continue to treat the patient while others may be decoupled, again without having to detach any additional cables, and used to treat a different patient. Alternatively, a healthcare provider could transport the entire rack system **1900** and any syringe pumps coupled thereto by decoupling the rack **1910** from the support structure **1930.** A rack **1910** that includes a handle **1820** and/or wheels may make transporting the rack system **1900** and medical devices **1920** easier in this scenario.

**FIG. 10d** shows a view of another embodiment of a rack system **6100** including the rack **6010** embodiment shown in **FIG. 9e****.** A medical device, which for exemplary purposes is shown as an infusion pump **6060,** is in place on the rack **6010.** A medical device may also, for example, be a monitoring client, PCA, physiological monitor, etc. As shown, the flanges **6062** of the infusion pump **6060** are disposed within the guide troughs **6028, 6034** of the first support plate **6022** and second support plate **6024,** respectively. A clamp mechanism **1940** is included on the infusion pump **6060** and is shown clamped around the support pole **6012.** Additionally, as shown in **FIG. 10d****,** the joint members **6030** of the support plates **6022, 6024, 6026** may include functional protrusions **6031.** In the example embodiment, the functional protrusions **6031** are hooks or hangers. The functional protrusions **6031** may, for example, be used to hang various cabling, lines, or IV bags.

**FIG. 10e** depicts an infusion pump **6060** with a flange **6062** which is in place within a guide trough **6034** of the second support plate **6024.** As shown, the infusion pump **6060** includes a device connector **1970** and the second support plate **6024** includes a mount connector **6038.** The mount connector **6038** may be included in the side wall of the guide trough **6034.** The device connector **1970** and the mount connector **6038** are not in contact with one another in **FIG. 10e****.** Additionally, the flange **6062** of the infusion pump **6060** is relatively loose within the guide trough **6034.** As mentioned above, any or all of the support plates **6022, 6024, 6026** may include mount connectors **6038** which may or may not be similarly disposed.

Referring now back to **FIG. 10d****,** the device connector **1970** and the mount connector **6038** are shown in contact with one another. Additionally, the flanges **6062** of the infusion pump **6060** are well retained within the guide troughs **6034, 6028** of the support plates **6022, 6024.** In order to bring the device connector **1970** and the mount connector **6038** into contact and firmly retain the flanges **6062** within the guide troughs **6034, 6028** it may be needed , in some specific embodiments, to actuate the clamp mechanism **1940** to the clamped position.

In **FIG. 10e****,** the clamp mechanism **1940** (not shown) is not in the clamped position. When the clamp mechanism **1940** is not in the clamped position, the flanges **6062** of the infusion pump **6060** may be easily moved around within the guide troughs **6034, 6028.** This may be helpful in inserting the infusion pump **6060** and in aligning the device connector **1970** and mount connector **6038.** The clamp mechanism **1940** may then be actuated as described above into the clamped position. This action may drive the device connector **1970** and the mount connector **6038** into contact and cause the flanges **6062** to cinch up against a side wall of the guide troughs **6028, 6034** thus retaining the infusion pump **6060** on the rack **6010.**

The healthcare provider may repeat the procedure for as many infusion pumps **6060** or medical devices as may be desired. Thus, the healthcare provider may provide each infusion pump **6060** or medical device with electrical power and a network connection to other infusion pumps **6060** or medical devices via the mount connector **6038** and the device connector **1970** without having to run multiple power and network cables that may complicate the setup procedure and clutter the environment around the patient. Moreover, any one of the infusion pumps **6060** or medical devices may be decoupled from the rack **6010,** or another infusion pump **6060** or medical device may be coupled to the rack **6010,** without having to detach or attach any additional cables. When treatment is complete, certain infusion pumps **6060** or medical devices may remain coupled to the rack **6010** and continue to treat the patient while others may be decoupled, again without having to detach any additional cables, and used to treat a different patient. Alternatively, a healthcare provider could transport the entire rack **6010** and any infusion pump **6060** or medical devices coupled thereto by decoupling the rack **6010** from a support structure **6014** (see **FIG. 9e****).** A rack **6010** that includes a handle **6020** and/or wheels may make transporting the rack **6010** and infusion pumps **6060** or medical devices easier in this scenario.

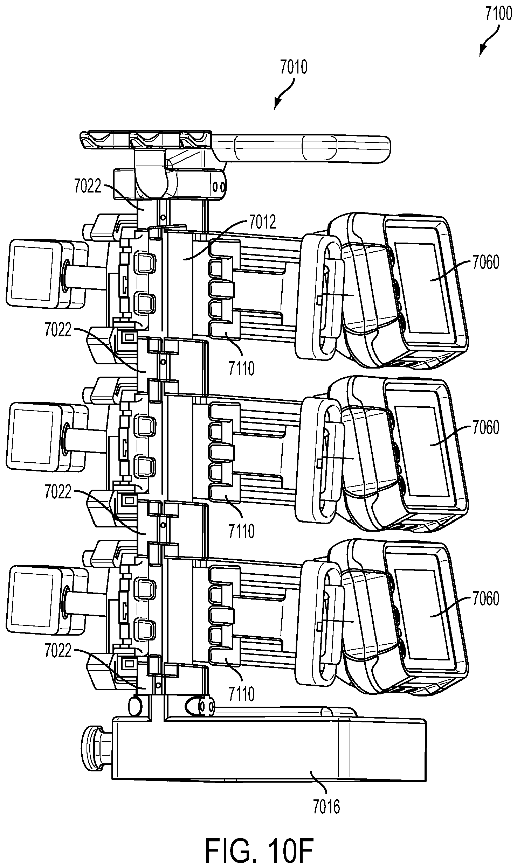

**FIG. 10f** shows yet another embodiment of a rack system **7100** which includes the example rack **7010** shown in **FIGS. 9f****-i.** The rack system **7100** may allow for a number of medical devices to be coupled onto the rack **7010.** The rack system **7100** may also be configured to provide power and/or a network connection to any medical devices coupled to the rack **7010.** Some embodiments of the rack system **7100** may differ, perhaps substantially, from the embodiment shown herein.

A number medical devices, which for exemplary purposes, are shown as an infusion pumps **7060,** are in place on the rack **7010** in **FIG. 10f****.** A medical device may also, for example, be a monitoring client, a PCA, physiological monitor, etc. As shown, a clamp apparatus **7110** is coupled to each of the infusion pumps **7060.** The clamp apparatuses **7110** shown are similar to those in the embodiments depicted above in **FIGS. 8a-8g****.** In other embodiments, the clamp apparatuses **7110** may be any suitable clamp described herein. The clamp apparatuses **7110** are shown in the open position in **FIG. 10f****.** The infusion pumps **7060** may be securely coupled to the rack **7010** by actuating the example clamp apparatuses **7110** to the closed position as described above in reference to **FIGS. 8a-8f****.** The collars **7022** ensure that as the clamp apparatuses **7110** are closed, the infusion pumps **7060** are in the proper orientation on the support pole **7012** of the rack **7010.**

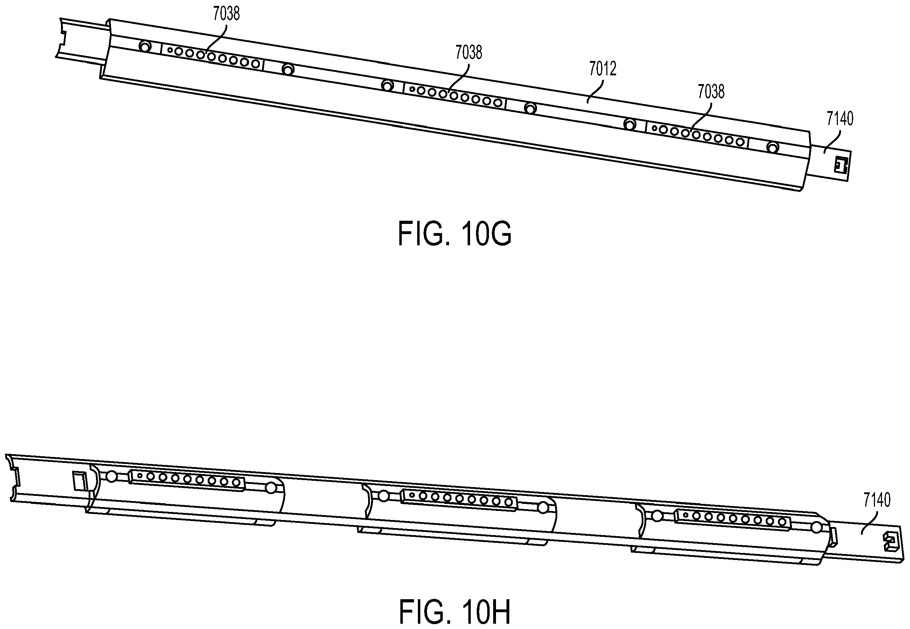

Referring now also to **FIG. 10g****,** a view of the support pole **7012** of the rack system **7100** is shown. The collars **7022** have been removed from the support pole **7012** in **FIG. 10g****.** As depicted also in **FIG. 9i****,** the support pole **7012** includes a number of mount connectors **7038.** At least a part of the mount connectors **7038** project through openings in and are proud of the tube which forms the support pole **7012** in the example embodiment. As mentioned above, the mount connectors **7038** may provide power to received medical devices. As also mentioned above, the mount connectors **7038** may be configured to enable a received medical device to communicate over CANbus and/or over a USB.

Referring now also to **FIG. 10h** the mount connectors **7038** may be included on a mount connector strip **7140.** The mount connector strip **7140** may help to facilitate assembly. When assembled, the mount connectors **7038** may be snap fit onto the mount connector strip **7140.** The mount connector strip **7140** may then be placed into the tube which forms the support pole **7012.** This ensures that the mount connectors **7038** are easily lined up with the openings in the tube of the support pole **7012.** Fasteners may then be used to fixedly couple the mount connectors **7038** on the mount connector strip **7140** to the support pole **7012.**

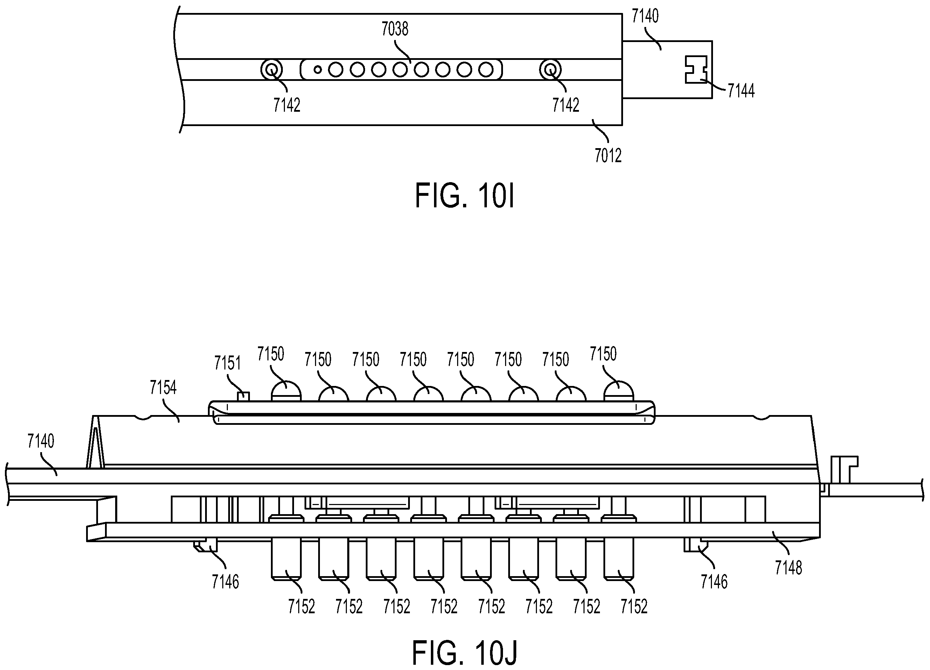

**FIG. 10i** depicts a close up view of a portion of the support pole **7012** for the rack system **7100.** As shown, an example mount connector **7038** is disposed in its assembled location. Holes **7142** are included in the support pole **7012.** Fasteners (not shown) may be inserted into the holes **7142** to fixedly couple the mount connectors **7038** on the mount connector strip **7140** to the support pole **7012.** An end of the mount connector strip **7140** is also shown protruding from the end of the support pole **7012.**

As shown, in some embodiments, the end of the mount connector strip **7140** may include a coupling feature **7144.** The coupling feature **7144** may be configured to receive a coupling feature **7144** on another mount connector strip **7140.** This may be useful in assembly of alternative embodiments of rack systems which are designed to receive a large number of pumps. In such embodiments, multiple mount connector strips **7140** may, for example, be coupled together and fed into a longer tube of a longer support pole **7012.** In embodiments a coupling feature **7144** on a mount connector strip **7140** may couple into the end cap of a support pole **7012.**

**FIG. 10j** depicts a side view of an example mount connector **7038** which has been snap fit into place on an example mount connector strip **7140.** The example mount connector **7038** includes a bottom portion **7148,** a top portion **7154,** connector pins **7150,** and sockets **7152.** Two snap fit features **7146** are visible and are holding the bottom portion **7148** in place on the mount connector strip **7140.** The top portion may also be held in place on the mount connector strip **7140** by snap fit features **7146.** In the embodiment depicted in **FIG. 10j****,** the snap fit features **7146** holding the top portion **7154** in place are not visible.

The connector pins **7150** may be biased to project off the top portion **7154** of the mount connector **7038.** In such embodiments, compression springs (not shown) may provide the biasing force. Also as shown, the two outside connector pins **7150** are prouder of the top portion **7154** than all other connector pins **7150.** These two connector pins **7150** may be connected to ground in order to ensure a ground connection is made before other connections. As shown, the connector pins **7150** may always be engaged in the sockets **7152.** This may be done to ensure that a received medical device will always be provided with electrical power and/or a network connection via the mount connector **7038.** In some embodiments, a different number of connector pins **7150** and sockets **7152** may be included.

The example mount connector **7038** also includes a hall sensor **7151.** The hall sensor **7151** may be tripped by a magnet on a received medical device or a clamp on a medical device, for example. When tripped, the hall sensor **7151** may create a delay of predetermined duration before power is supplied to the received medical device.

In the example embodiment, the bottom portion **7148** of the mount connector **7140** is a PCB. The PCB may be configured and populated such that it may allow a received medical device to communicate over a CANbus and over USB. In such embodiments, and referring now also to **FIG. 10f****,** the base member **7016** of the example rack **7010** may include a USB port (not shown) to allow for connection to a computer. Such a computer may be used, for example, by trained personnel to update, access data or logs from, perform diagnostics on, etc. an attached medical device.

In order to reduce cost, in some embodiments, the PCB making up the bottom portion **7148** may not be entirely populated. For example, some PCBs may not include the components which would enable USB communication. A care facility, such as a hospital, thus may only have a few rack systems **7100** which are CANbus and USB configured. These may be used, for example, as special diagnostic rack systems **7100** when needed while less expensive, non-USB capable rack systems **7100,** may be used to provide everyday patient care.

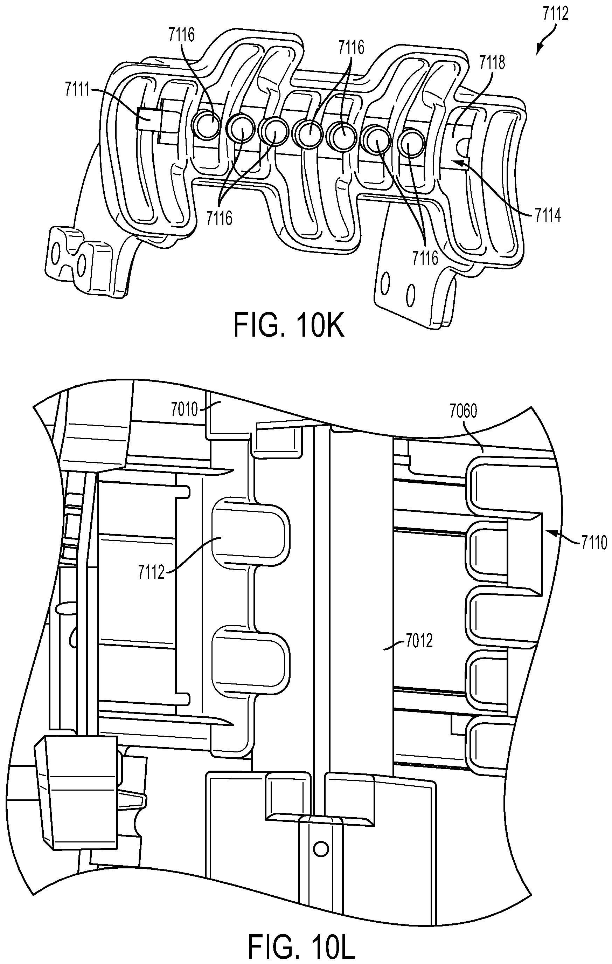

Referring now **FIG. 10k****,** an example embodiment of a gripper **7112** of a clamp apparatus **7110** is shown. The gripper **7112** is similar to the fixed gripper assembly **703** shown in **FIG. 8e****.** The gripper **7112** includes a device connector **7114.** As shown, the device connector **7114** includes a number of connector pins **7116** attached to a PCB **7118.** A high friction, compliant gripper material (not shown) may be attached to the gripping face of the gripper **7112.** In such embodiments, the gripper material may be overmolded onto the gripper **7112.** The gripper material, may include holes through which the connector pins **7116** may interface with a mount connector **7038** on a support pole **7012.** In some embodiments the gripper material may have a thickness such that the connector pins **7116** do not protrude out of the gripper material.

As shown, the gripper **7112** may also include a magnet **7111.** In such embodiments, the magnet may or may not be covered by the overmolded gripper material. The magnet **7111** may trip a hall sensor **7151** on a mount connector **7038** (see **FIG. 10j**) as the gripper **7112** comes into close proximity of the mount connector **7038.** Tripping the hall sensor **7151** may cause a delay of predetermined duration before power is supplied from the mount connector **7038** to the device connector **7114** in the gripper **7112.**

Referring now also to **FIG. 10l****,** a clamp apparatus **7110** attached to an infusion pump **7060** is depicted as it is being attached to the support pole **7012** of a rack **7010.** The gripper **7112** is in contact with the support pole **7012.** The device connector **7114** on the gripper **7112** is in contact with a mount connector **7038** (see, for example, **FIG. 10g****)** on the support pole **7012.** The clamp apparatus **7110** may be actuated to the clamped position to couple the infusion pump **7060** onto the rack **7010** and keep the device connector **7114** in contact with the mount connector **7038.** A connection from the device connector **7114** to the infusion pump **7060** (not shown) may be included to provide power and/or a network connection to the infusion pump **7060.** In some embodiments, a connector on the on infusion pump **7060** may be disposed such that the infusion pump **7060** may be provided power and/or a network connection via another connector on the clamp apparatus **7110** when the clamp apparatus **7110** and infusion pump **7060** are coupled together.

Once an infusion pump **7060** is attached, the healthcare provider may repeat the procedure for as many infusion pumps **7060** or medical devices as may be desired. Thus, the healthcare provider may provide each infusion pump **7060** or medical device with electrical power and a network connection to other infusion pumps **7060** or medical devices via the mount connector **7038** and the device connector **7114** without having to run multiple power and network cables that may complicate the setup procedure and clutter the environment around the patient. Moreover, any one of the infusion pumps **7060** or medical devices may be decoupled from the rack **7010,** or another infusion pump **7060** or medical device may be coupled to the rack **7010,** without having to detach or attach any additional cables. When treatment is complete, certain infusion pumps 7060 or medical devices may remain coupled to the rack **7010** and continue to treat the patient while others may be decoupled, again without having to detach any additional cables, and used to treat a different patient. Alternatively, a healthcare provider could transport the entire rack **7010** and any infusion pump **7060** or medical devices coupled thereto by decoupling the rack **7010** from a support structure such as an IV pole. A rack **7010** that includes a handle **7020** (best shown in **FIG. 9f****)** and/or wheels may make transporting the rack **7010** and infusion pumps **7060** or medical devices easier in this scenario.

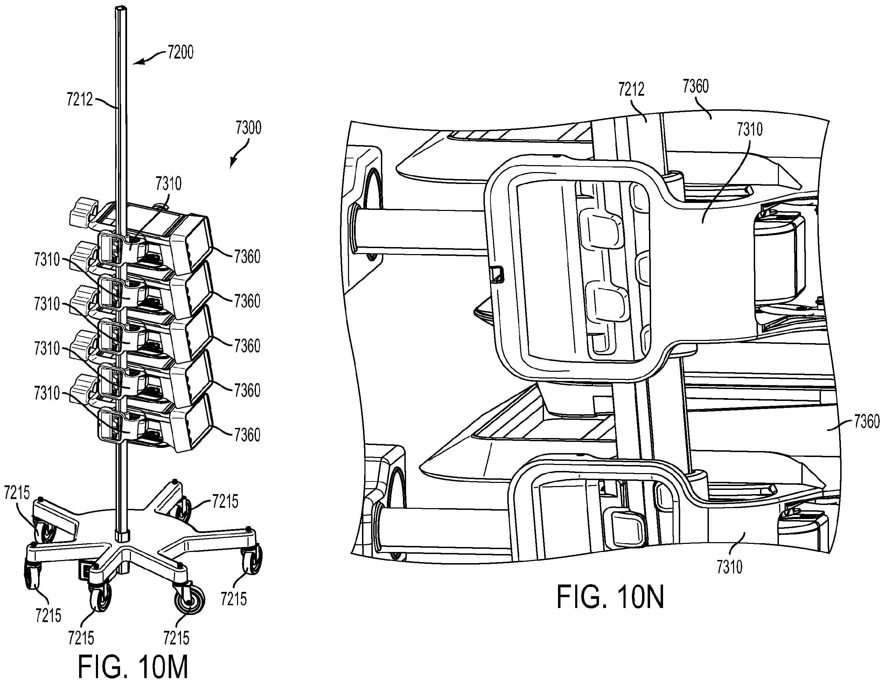

**FIG. 10m** depicts yet another embodiment of an example rack system **7300** including the example rack **7200** shown in **FIGS. 9j****-k.** The rack system **7300** may allow for a number of medical devices to be coupled onto the rack **7200.** The rack system **7300** may also be configured to provide power and/or a network connection to any medical devices coupled to the rack **7200.** Some embodiments of the rack system **7300** may differ from the embodiment shown herein.

As shown, the example rack system **7300** also comprises a number of medical devices **7360** and a number of clamps **7310** which are coupling the medicals devices **7360** to the rack **7200.** In the example embodiment, the medical devices **7360** are depicted as infusion pumps. Other medical devices **7360,** for example, a monitoring client, PCA, physiological monitor, etc. may also be coupled to the rack **7200.** Also as shown, the clamps **7310** are similar to the clamp apparatus **710** embodiments depicted in relation to **FIGS. 8a****-g.** In other embodiments, the clamps **7310** may differ. For example, the clamps **7310** may be, but are not limited to, other embodiments of clamps described herein.

As shown, only five medical devices **7360** are coupled to the rack **7200.** In the example embodiment shown in **FIG. 10m****,** there is space above the five coupled medical devices **7360** for additional medical devices **7360** to be coupled to the support pole **7212** of the rack **7200** if necessary. In some embodiments, a user may couple another rack (eg. any of racks **1810, 1910, 6010, 7010** shown and described in relation to **FIGS. 10a****-l**) to the rack system **7300.**

A clamp **7310** attached to a medical device **7360** may be actuated to a closed and clamped position around the support pole **7212** of the rack **7200** in order to couple a medical device **7360** to the rack **7200.** Referring now also to **FIG. 10n****,** two medical devices **7360** are shown coupled to the support pole **7212.** The clamps **7310** holding the medical devices **7360** in place on the support pole **7212** are shown actuated to the closed position. As mentioned above in relation to **FIG. 9j****,** one or more collars and/or alignment features may be included to help ensure that medical devices **7360** are coupled to the support pole **7212** in a correct and secure manner.

As mentioned in reference to **FIG. 9k****,** the example rack **7200** of the rack system **7300** may include a number of mount connectors **7238** which may provide power and/or a network connection to attached medical devices **7360.** In such embodiments of the rack system **7300,** the mount connectors **7238** may, for example, be similar to any those described above in relation to **FIGS. 10f-1****.** Additionally, the clamps **7310** of the example rack system **7300** may include a device connector which is similar to the device connector **7114** described above in relation to **FIGS. 10k****-l.** When a medical device **7360** is coupled to a support pole **7212** as shown in **FIGS. 10m****-n,** the medical device **7360,** a respective mount connector **7238** and respective device connector may operatively engage and provide power and/or a network connection to the medical device **7360.** In embodiments where the rack system **7300** is configured to allow other racks to couple into the rack system **7300,** the rack system **7300** may provide power and/or a network connection to the other racks in a similar manner.

Once a medical device **7360** is coupled to the rack **7200,** the healthcare provider may repeat the procedure for as many infusion pumps or medical devices **7360** as may be desired. Thus, the healthcare provider may provide each infusion pump or medical device **7360** with electrical power and a network connection to other infusion pumps or medical devices **7360** via the mount connector **7238** and a device connector without having to run multiple power and network cables that may complicate the setup procedure and clutter the environment around the patient. Moreover, any one of the infusion pumps or medical devices **7360** may be decoupled from the rack **7200,** or another infusion pump or medical device **7360** may be coupled to the rack **7200,** without having to detach or attach any additional cables. When treatment is complete, certain infusion pumps or medical devices **7360** may remain coupled to the rack **7200** and continue to treat the patient while others may be decoupled, again without having to detach any additional cables, and used to treat a different patient. Alternatively, a healthcare provider could transport the entire rack **7200** and any infusion pump or medical devices **7360** coupled thereto. A rack **7200** that includes a handle and/or wheels **7215** may make transporting the rack **7200** and infusion pumps or medical devices **7360** easier in this scenario.

### PROTECTIVE MECHANISMS

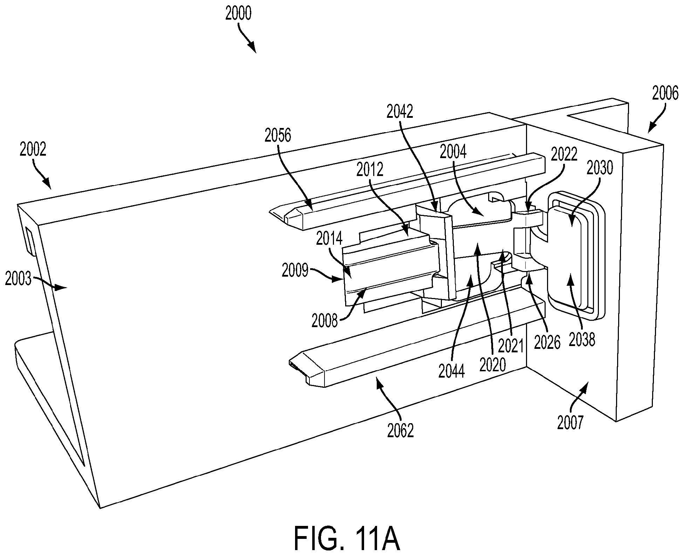

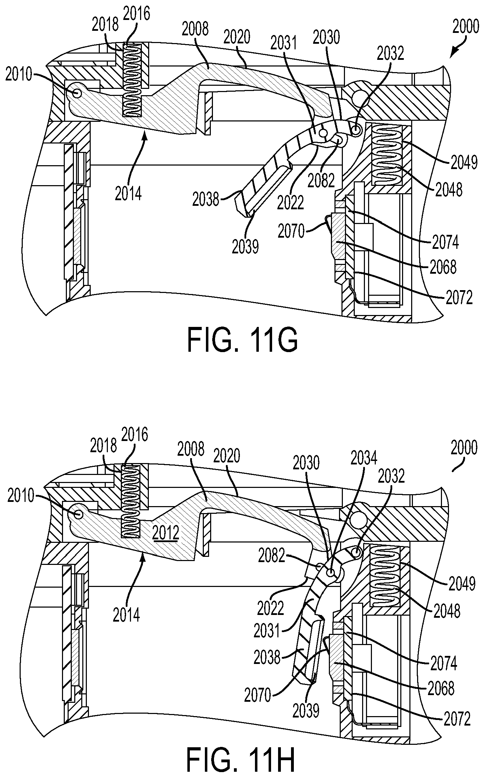

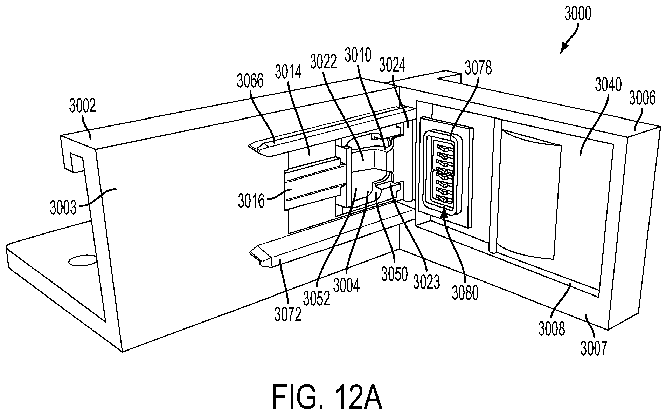

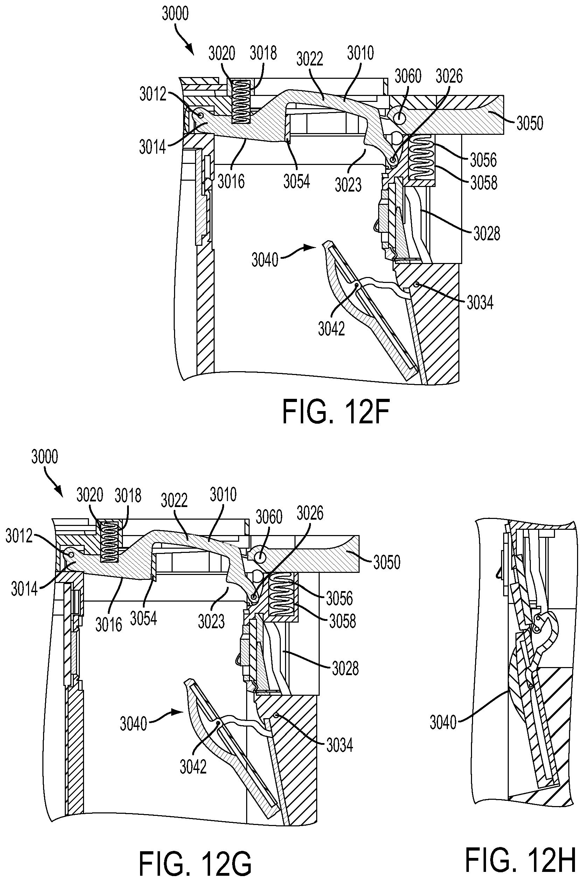

In addition, the medical device mounts of the rack **1810, 1910, 6010, 7010, 7200** may each include a protective mechanism that may protect the mount connector when not in use and during cleaning. For example, the mount connector **2068, 3078** may be covered by a pivotable-cover mechanism **2000** or a clamshell mechanism **3000.** Such protective mechanisms may also be used in other systems, for example, in medical systems beyond the rack embodiments described herein. **FIG. 11a** depicts an embodiment of a pivotable-cover mechanism **2000.** **FIG. 12a** depicts an embodiment of a clamshell mechanism **3000.** In the embodiments depicted in **FIG. 11a** and **12a** of the pivotable-cover and clamshell mechanisms **2000, 3000** respectively, coupling an electronic device, such as syringe pump, to the mechanism may automatically reveal the mount connector **2068, 3078** and allow the mount connector **2068, 3078,** to interface with the connector of the received electronic device.

### Pivotable-Cover Mechanism & Clamshell Mechanism: Common Components

In the embodiments of the present disclosure depicted in **FIGs. 11a** and **12a****,** the pivotable-cover mechanism **2000** and the clamshell mechanism **3000** may utilize similar components to receive an electronic device and initiate steps to reveal the respective mount connector **2068, 3078.** However, the two mechanisms **2000, 3000** are only two of many possible embodiments to complete the process of revealing the mount connector **2068, 3078.** The two example mechanisms **2000, 3000** may respectively include a guide member **2002, 3002,** a first rail projection **2056, 3066** and a second rail projection **2062, 3072.** The first rail projection **2056, 3066** and the second rail projection **2062, 3072** may be disposed on a guide member face **2003, 3003,** and run in parallel to one another along a longitudinal axis of the guide member **2002, 3002** such that the first and the second rail projections **2056, 3066, 2062, 3072** are capable of aligning the connector of a received device with the mount connector of the protective mechanisms **2068, 3078.**

The pivotable-cover mechanism **2000** and the clamshell mechanism **3000** may also each include a backstop member **2006, 3006** having a backstop member face **2007, 3007** to which the mount connector **2068, 3078** may be coupled. The backstop member face **2007, 3007** may be approximately perpendicular to the guide member face **2003, 3003.** The first and the second rail projections **2056, 3066, 2062, 3072** may extend to the backstop member face **2007, 3007** such that the first and the second rail projections **2056, 3066, 2062, 3072** are long enough to support and stabilize a received electronic device. The mount connector **2068, 3078** may be coupled to the backstop member face **2007, 3007** such that the mount connector **2068, 3078** is operatively positioned to interface with the connector of a received electronic device. The protective mechanisms **2000, 3000** may also include a bus **2072, 3082** that is operatively coupled to the respective mount connector **2068, 3078.** In preferred embodiments of the two protective mechanisms **2000, 3000,** the mount connector **2068, 3078** may be coupled to the backstop member face **2007, 3007** such that the mount connector **2068, 3078** is positioned between the first and the second rail projections **2056, 3066, 2062, 3072.**

To provide a mechanism for initiating the process of revealing the mount connector **2068, 3078,** the pivotable-cover mechanism **2000** and the clamshell mechanism **3000** may respectively include an actuation member **2008, 3010** that is pivotally coupled to the guide member **2002, 3002** at a first, stationary pivot **2010, 3012,** wherein the first, stationary pivot **2010, 3012** may be disposed between the backstop member face **2007, 3007** and a first end of the guide member **2002, 3002.** The actuation member **2008, 3010** may include a sloped portion **2012, 3014** that extends from the first, stationary pivot point **2010, 3012** towards the mount connector **2068, 3078.** The sloped portion **2012, 3014** may further include a sloped face **2014, 3016,** wherein the sloped face **2014, 3016** may be configured such that it may slope upwardly and out of the plane of the guide member face **2003, 3003** from the first, stationary pivot **2010, 3012** towards the mount connector **2068, 3078.** Thus, the sloped portion **2012, 3014** may protrude from the plane of the guide member **2002, 3002** when the actuation member **2008, 3010** is in a first position and the mount connector **2068, 3078** is covered. To reveal the mount connector **2068, 3078,** the actuation member **2008, 3010,** the sloped portion **2012, 3014,** and the sloped face **2014, 3016** may pivot about the first, stationary pivot **2010, 3012** in a first direction from the first position to a second position. When the actuation member **2008, 3010** is in the second position and the mount connector **2068, 3078** is uncovered, the sloped face **2014, 3016** may lie substantially in the plane of the guide member **2002, 3002.**

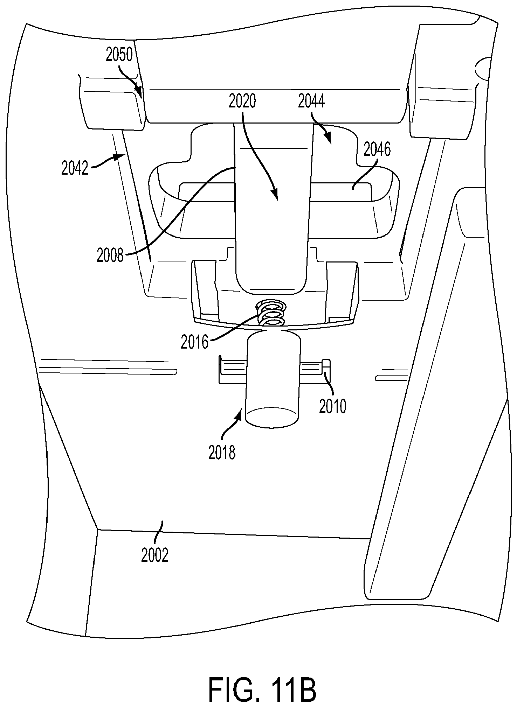

In addition, actuation springs **2016, 3018** may provide a mechanism for biasing the actuation members **2008, 3010** such that the actuation members **2008, 3010** may automatically return to the first position. To house the actuation springs **2016, 3018,** the guide members **2002, 3002** may include an actuation spring pocket **2018, 3020.** In certain embodiments, the actuation springs **2016, 3018** and the actuation spring pockets **2018, 3020** may be disposed on the guide members **2002, 3002** such that the actuation springs **2016, 3018** may be coupled to and exert a biasing force on the sloped portion **2012, 3014** of the actuation member **2008, 3010.** **FIG. 11b** depicts the actuation spring **2016** of an embodiment of the pivotable-cover mechanism **2000** acting on the sloped portion **2012** of the actuation member **2008,** wherein the actuation spring **2016** is disposed in the actuation spring pocket **2018.** The clamshell mechanism **3000** may be similarly configured.

The pivotable-cover mechanism **2000** and the clamshell mechanism **3000** may also respectively include a latch member **2042, 3050** that may be configured to latch onto a received electronic device when the latch member **2042, 3050** is in a latched position. The latch member **2042, 3050** may be disposed within an aperture or a guide member recess **2004, 3004** defined by the guide member **2002, 3002.** In addition, the guide member recess **2004, 3004** and the latch member **2042, 3050** may be adapted to extend below a portion of the backstop member **2006, 3006** such that a first end portion of the latch member **2042, 3050** may be adjacent to the sloped portion **2012, 3014** of the actuation member **2008, 3010,** and a second, opposite end portion of the latch member **2042, 3050** may be disposed within the guide member recess **2004, 3004** on the opposite side of the backstop member **2006, 3006.**

To allow a user to selectively engage or disengage the latch member **2042, 3050,** the latch member **2042, 3050** may be adapted to pivot about a third, stationary pivot **2050, 3060.** To latch onto a received electronic device, the latch member **2042, 3050** may include a latch projection **2046, 3054** disposed on a first end portion of the latch member **2042, 3050** and adapted to protrude from the guide member face **2003, 3003** when the latch member **2042, 3050** is in the latched position. The latch member **2042, 3050** may also include a latch member aperture **2044, 3052** through which the actuation member **2008, 3010** may pass, and thus, the latch member aperture **2044, 3052** may be sized and adapted to allow the actuation member **2008, 3010** to pivot through its full range of motion without interference from the latch member **2042, 3050.**

Like the actuation member **2008, 3010,** the latch member **2042, 3050** may be adapted such that a biasing force automatically returns the latch member **2042, 3050** to the latched position. Thus, a latch member spring **2048, 3056** may be disposed within a latch member spring pocket **2049, 3058** operatively defined by the guide member **2002, 3002** or the backstop member **2006, 3006.** In a preferred embodiment, the backstop member **2006, 3006** defines the latch member spring pocket **2049, 3058** such that the latch member spring **2048, 3056** may exert a downward force on the latch member **2042, 3050** between the third, stationary pivot **2050, 3060** and the second end portion of the latch member **2042, 3050.** To position the latch member projection **2046, 3054** at a desired height above the guide member face **2003, 3003,** the backstop member **2006, 3006** may be operatively sized and disposed on the guide member **2002, 3002** such that the backstop member **2006, 3006** may arrest pivotal movement of the latch member **2042, 3050** at the correct position relative to the guide member face **2003, 3003.** An alternative mechanism may include at least one arrester projection operatively disposed within the guide member recess **2004, 3004** and adapted to do the same. In a preferred embodiment, a first arrester projection **2052, 3062** (not shown) and a second arrester projection **2054, 3064** (not shown) may be disposed on opposite sides of the guide member recess **2004, 3004** between the third, stationary pivot point **2050, 3060** and the second end portion of the latch member **2042, 3050,** wherein the first and second arrester projections **2052, 2054, 3062, 3064** are capable of exerting a normal moment of force to counteract the moment of force that the latch member spring provides **2048, 3056.**

As will be understood by persons having ordinary skill in the art, the pivotable-cover and clamshell mechanisms **2000, 3000** and their components can be made from a variety of rigid, engineering materials. Possible materials include aluminum alloys, stainless steel alloys, steel alloys, and engineering polymers. In addition, a variety of coatings may be applied to the mechanisms **2000, 3000** and their components. Many of the possible coatings provide a means of reducing the likelihood of cross-contamination. Cross-contamination may pose a serious health risks to young and old patients and patients with weakened immune systems. Optionally, one or more of an antibacterial, an antiviral, or an antimicrobial coating may be applied to the structural components of the pivotable-cover and clamshell mechanisms **2000, 3000** to kill or inhibit the growth bacteria, viruses, fungi, and various other microorganisms. Exemplary coatings may include copper, copper particles, silver, silver particles, or other materials that have antibacterial, antiviral, or antimicrobial properties.

### A Pivotable-Cover Mechanism

While both the pivotable-cover mechanism **2000** and the clamshell mechanism **3000** may include the aforementioned elements to receive an electronic device and initiate the steps to reveal the mount connector **2068, 3078,** the pivotable-cover mechanism **2000** and the clamshell mechanism **3000** employ additional, different mechanical linkages to complete the task.

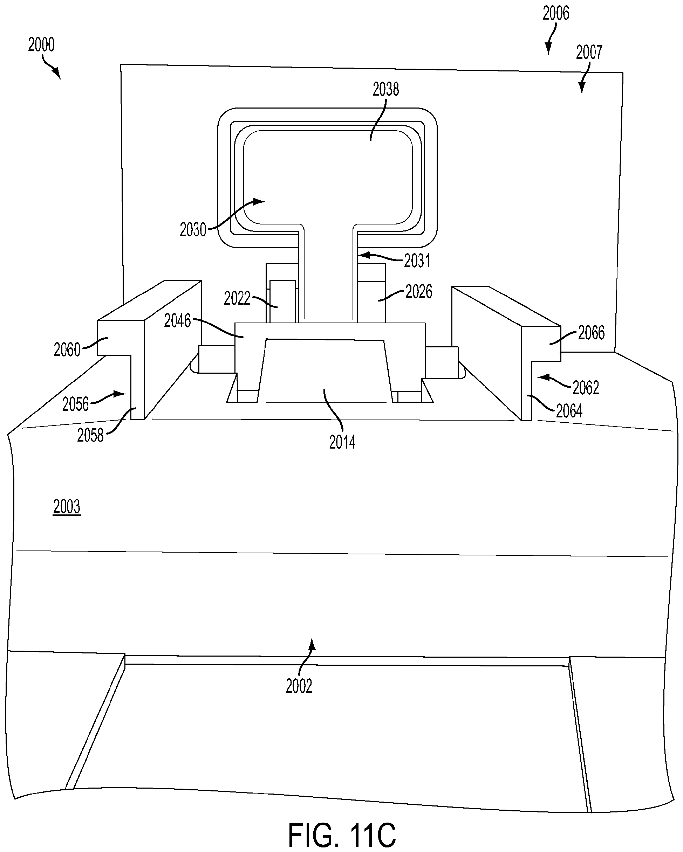

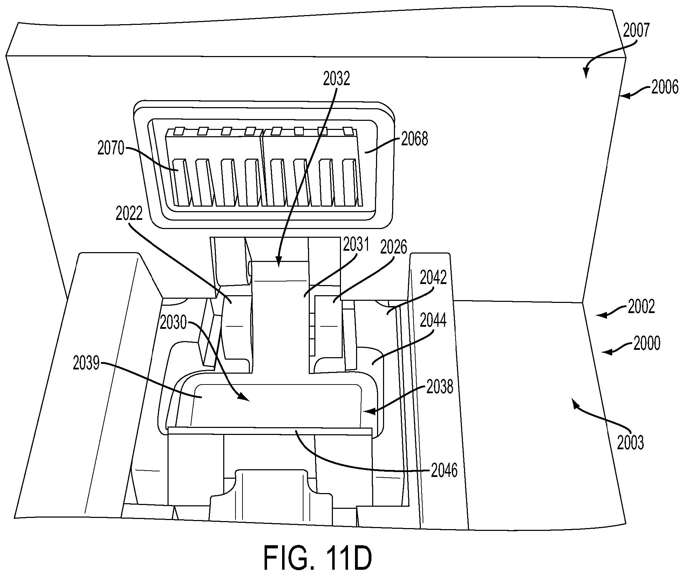

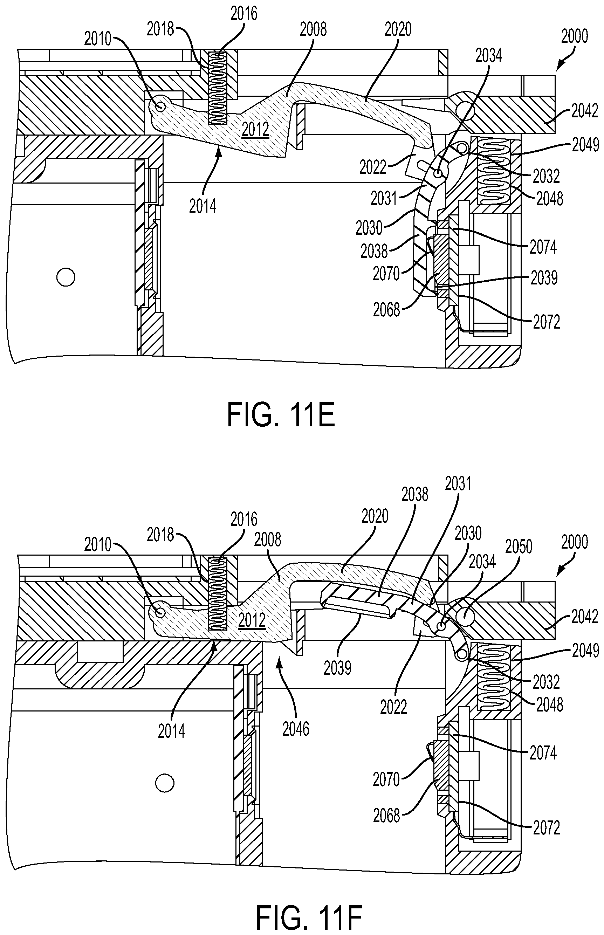

**FIGS. 11a-11i** depict an exemplary embodiment of the pivotable-cover mechanism **2000.** **FIGS. 11a** and **11c** depict an embodiment of the pivotable-cover mechanism **2000** wherein the mount connector **2068** is covered by a protective member **2030.** **FIG. 11e** depicts a cross-section of the pivotable-cover mechanism **2000** wherein the mechanism has received an electronic device, the actuation member **2008** is in the second position, and the mount connector **2068** is uncovered. In addition, **FIG. 11e** depicts a number portions that may comprise the actuation member **2008,** including: the sloped portion **2012,** a bridge portion **2020,** a first channeled projection **2022,** and a second channeled projection **2026.** **FIG. 11d** more clearly shows the first and second channeled projections **2022, 2026** and the gap between them.

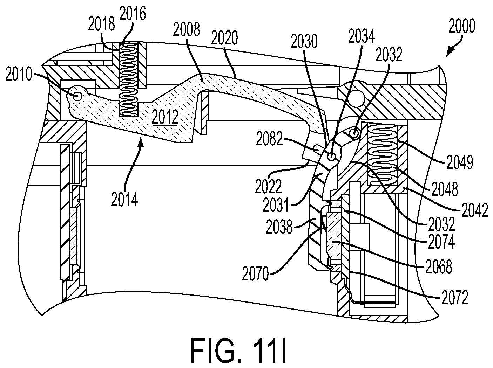

The protective member **2030** of the pivotable-cover mechanism **2000** may be pivotally coupled to either the backstop member **2006** or the guide member **2002** at a second, stationary pivot **2032.** As depicted in **FIG. 11f****,** the protective member **2030** is coupled to the backstop member **2006** in this particular embodiment. The third, stationary pivot **2050** lies on the guide member **2002** immediately above the second, stationary pivot **2032.** Additionally, the protective member **2030** may comprise a cover portion **2038** and a stem portion **2031** that couples the cover portion **2038** to the backstop member **2006.** The cover portion **2038** may be configured to cover the mount connector **2068.** The protective member **2030** may also include first and second actuation projections **2034, 2036** (not shown) wherein the first and second actuation projections **2034, 2036** are adapted to respectively engage the first channel **2024** and the second channel **2028** of the first and second channeled projections **2022, 2026** of the actuation member **2008.** Therefore, the first and second channeled projections **2022, 2026** may be shaped and sized such that the pivotal moment of the actuation member **2008** from the first position to the second position may pivot the protective member **2030** from a protective position, wherein the cover portion **2038** covers the mount connector **2068,** to a non-protective position, wherein the protective member **2030** is disposed within the guide member recess **2004.** Consequently, the bridge portion **2020** of the actuation member **2008** may be curved between the sloped portion **2012** and the first and second channeled projections **2022, 2026** to allow the protective member **2030** to nest within the actuation member **2008** when the protective member **2030** is in the non-protective position. **FIGS. 11e-11i** depict the positions of the actuation member **2008** and the protective member **2030** as they move from their respective positions when the mount connector **2068** is uncovered and in contact with the connector of an electronic device (**FIG. 11e**) to their respective positions when the mount connector **2068** is covered (**FIG. 11i**).

In addition, the pivotable-cover mechanism **2000** may include a latch member **2042** having a latch member aperture **2044** adapted to operatively receive a portion of the protective member **2030** when the protective member **2030** is in the non-protective position. **FIG. 11d** depicts the protective member **2030** in the non-protective position and shows the latch member aperture **2044** receiving the cover portion **2038** of the protective member **2030.** **FIG. 11d** also depicts the stem portion **2031** of the protective member **2030** disposed between the first and second channeled projections **2022, 2026.**

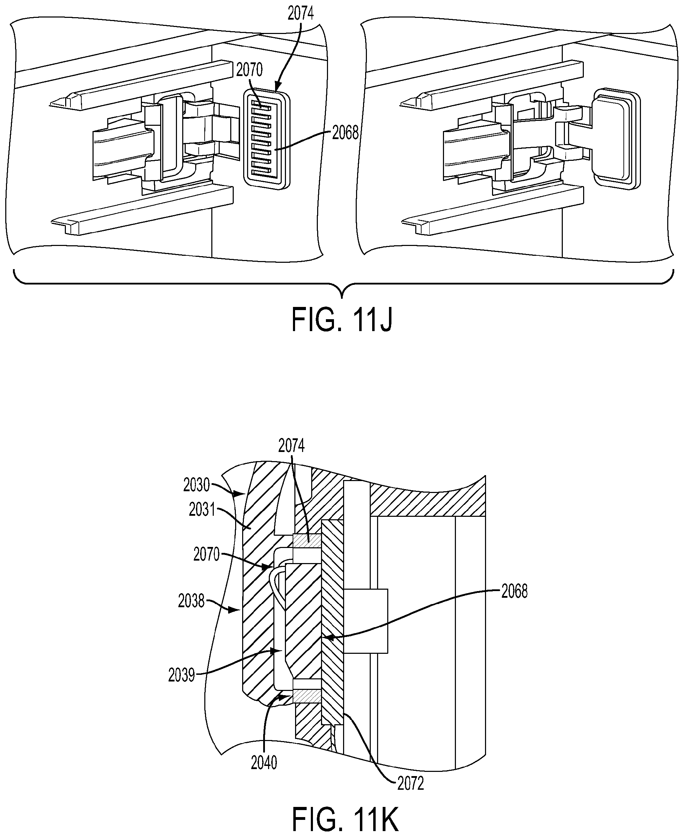

To protect the mount connector **2068** during cleaning and normal maintenance, the pivotable-cover mechanism **2000** may include a compliant gasket **2074** configured to, when the protective member **2030** is in the protective position, mechanically seal the mount connector **2068** within a cover portion recess **2039** defined by a perimeter rib **2040** of the cover portion **2038.** In an exemplary embodiment depicted in **FIG. 11k****,** the compliant gasket **2074** may encompass and abut the mount connector **2068.** In a preferred embodiment, the actuation spring **2016,** acting through the actuation member **2008,** may bias the protective member **2030** such that the protective member **2030** may automatically return to the protective position. Consequently, the spring force of the actuation spring **2016** may enable the perimeter rib **2040** to contact and compress the compliant gasket **2074** when the protective member **2030** is the protective position. Thus, the perimeter rib **2040** may create mechanical seal with the compliant gasket **2074.** **FIG. 11k** depicts an exemplary embodiment of the pivotable-cover mechanism **2000** wherein the perimeter rib **2040** has created a mechanical seal with the compliant gasket **2074.**

As will be appreciated by persons having ordinary skill in the art, the compliant gasket **2074** may be made of any suitably compliant material; such materials may include, but are not limited to, isobutylene, natural rubber, neoprene, styrene butadiene, and silicone. In addition, the compliant gasket material may be chosen so that the compliant gasket **2074** is capable of resisting corrosion from solvents ordinarily used for cleaning device surfaces.

**FIGS. 11j** and **11k** depict an embodiment of the present disclosure, wherein the mount connector **2068** is of a type having multiple spring contacts **2070.** To protect the spring contacts **2070** when the cover portion **2038** covers the mount connector **2068,** the cover portion recess **2039** may include a compressible material, including but not limited to a polyurethane foam, disposed within the cover portion recess **2039** and adapted to receive the spring contacts **2070.**

### A Clamshell Mechanism

Whereas the protective member **2030** of the pivotable-cover mechanism **2000** is capable of pivoting towards the guide member **2002** to expose the mount connector **2068,** the clamshell mechanism **3000** includes a cover member **3040** that is capable of pivotally sliding across the backstop member face **3007** to expose the mount connector **3078.**

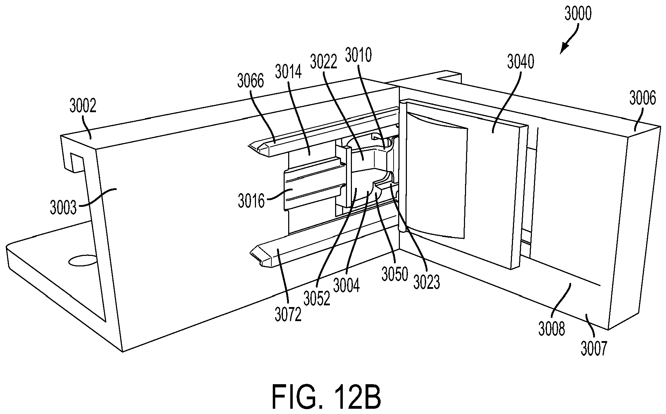

**FIG. 12a** depicts an exemplary embodiment of the clamshell mechanism **3000,** wherein the cover member **3040** is in a non-protective position and the mount connector **3078** is exposed. **FIG. 12b** depicts the same embodiment of the clamshell mechanism **3000,** wherein the cover member **2040** is in a protective position and the mount connector **3078** is covered.

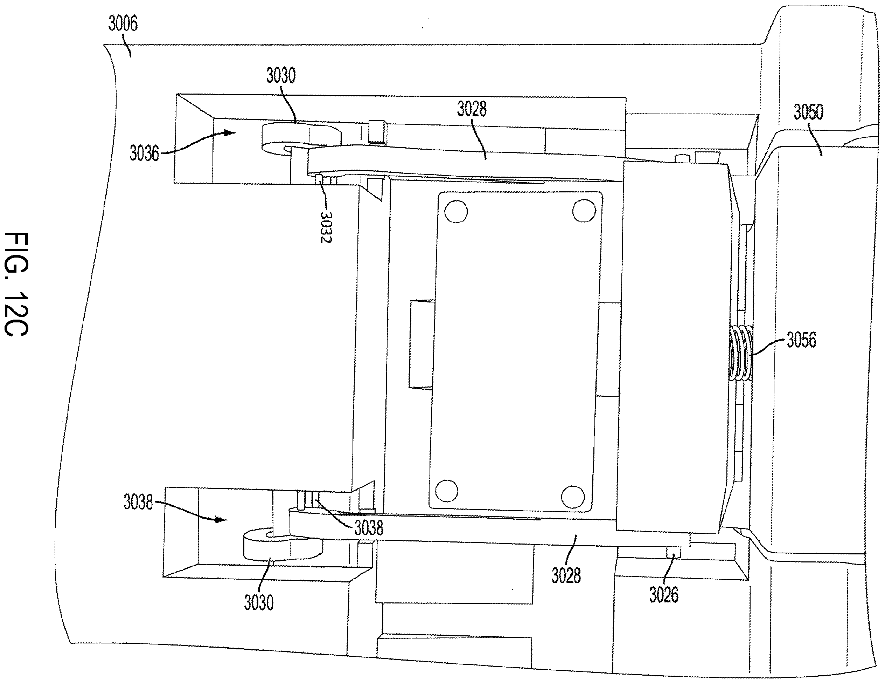

To pivotally slide the cover member **3040** across the backstop member face **3007,** the clamshell mechanism **3000** may include at least one first link-member **3028** and at least one second link-member **3030** to enable movement of the actuation member **3010** to pivotally slide the cover member **3040.** In the embodiment of the clamshell mechanism **3000** depicted in **FIG. 12a****,** the actuation member **3010** includes a sleeve portion **3024** that is coupled to the bridge portion **3022** and that is opposite to the sloped portion **3014.** **FIG. 12c** depicts an embodiment having a pair of first-link members **3028** that are pivotally coupled at respective first end portions to the sleeve portion **3024** of the actuation member **3010** at a first moveable pivot **3026.** In addition, the pair of first link-members **3028** of the embodiment depicted in **FIG. 12c** are pivotally coupled at respective second end portions to respective first end portions of a pair of second link-members **3030** at a second moveable pivot **3032.** As should be understood by persons having ordinary skill in the art, the backstop member **3006** is adapted to and coupled to the guide member **3002** so as to enable the at least one first link-member **3028** to couple with the actuation member **3010** and pass behind the backstop member face **3007.** Moreover, the backstop member **3006** may be adapted to enable the at least one first and second link-members **3028, 3030** to move through their respective ranges of motion without interference from the backstop member **3006.**

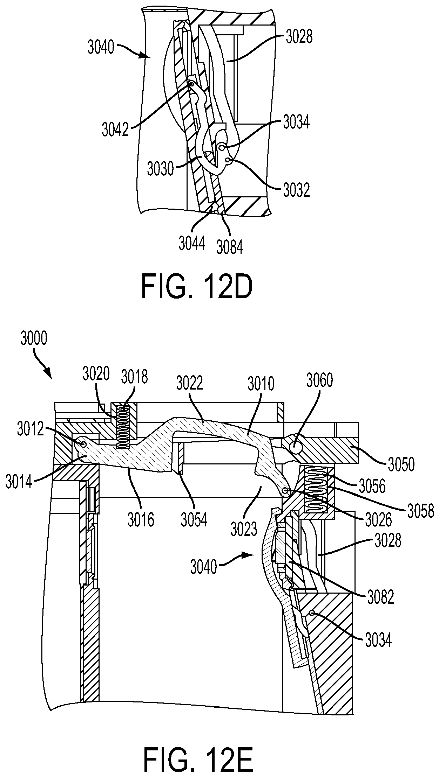

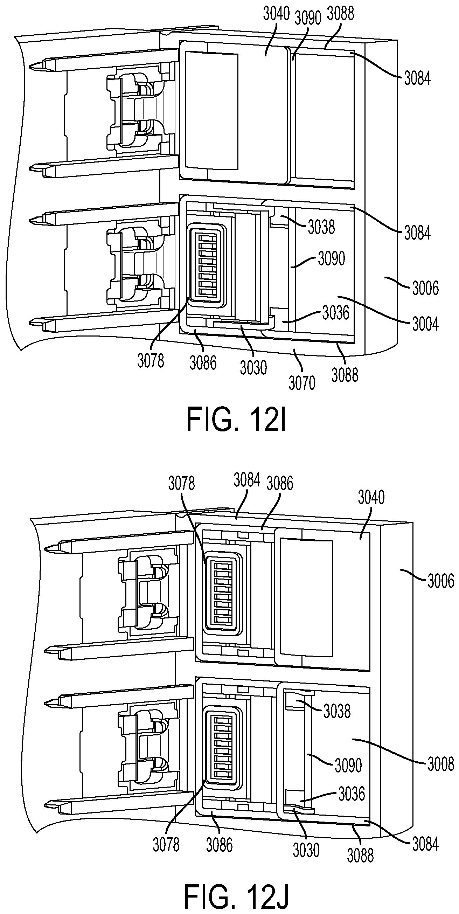

By way of movement of the at least one second link-member **3030,** the cover member **3040** may pivotally slide across the backstop member face **3007** to reveal the mount connector **3078.** In the embodiment of the clamshell mechanism **3000** depicted in **FIGS. 12d-12h****,** the respective first end portions of the pair of second link-members **3030** are coupled to respective second end portions of the pair of first link-members **3028** at the second moveable pivot **3032** and to the backstop member **3006** at a stationary clamshell pivot **3034.** **FIGS. 12d-12h** also depict the pair of second link-members **3030,** wherein respective second end portions of the pair of second link-members **3030** are coupled to the cover member **3040** at a third moveable pivot **3042.** In addition, the backstop member **3006** defines first and second pass-thru apertures **3036, 3038,** depicted in **FIGs. 12i** and **12j****,** through which the pair of second-link members **3030** respectively pass to couple with the cover member **3040.** The first and second pass-thru apertures **3036, 3038** may be respectively shaped and sized so as to enable the pair of second link-members **3030** to move through their respective ranges of motion without interference from the backstop member **3006.**

As the clamshell mechanism **3000** progresses through the stages of uncovering the mount connector **3078** depicted in **FIGS. 12e-12h****,** movement of the actuation member **3010** in a first direction from a first position to a second position may cause the cover member **3040** to move from a protective position, wherein the mount connector **3078** is covered, to a non-protective position, wherein the mount connector **3078** is uncovered. During the uncovering process, pivotal movement of the actuation member **3010** in the first direction may cause the pair of first link-members **3028** to pivot slightly about the first moveable pivot **3026** and to move in a substantially translational direction towards the guide member **3002.** In turn, the substantially translational movement of the pair of first link-members **3026** may cause the second moveable pivot **3032** to transit a plane that is parallel to the guide member face **3003** and that passes through the stationary clamshell pivot **3034.** In doing so, the second moveable pivot **3032** may move from a first position to a second position, wherein the second moveable pivot **3032** is closer to the guide member **3002** in the second position than in the first position. Movement of the second moveable pivot **3032** from the first position to the second position may thereby cause the pair of second link-members **3030** to pivot about the stationary clamshell pivot **3034** and pivotally slide the cover member **3040** from the protective position to the non-protective position. The reverse process, depicted in the progression of figures from **FIG. 12h** to **FIG. 12e****,** may be used to cover the mount connector **3078.**

Like the pivotable-cover mechanism **2000,** an actuation spring **3018** may be used to bias the clamshell mechanism **3000** so that the actuation member **3010** automatically returns to the first position under the force of the actuation spring **3018,** and acting through the at least one first link-member **3028** and the at least one second link-member **3030,** the actuation member **3010** may thereby cause the cover member **3040** to automatically return to the protective position.

To house the cover member **3040** when it is in the non-protective position, the backstop member may define a backstop member recess **3008** that the cover member **3040** may pivotally slide into as it pivotally slides across the backstop member face **3007** and exposes the mount connector **3078.** **FIGS. 12e-12h** depict the progression of the cover member **3040** as it uncovers the mount connector **3078** and slides into the backstop member recess **3008.** As depicted in **FIGS. 12e-12h****,** the deepest portion of the backstop member recess **3008** may be an end portion that is furthest from the guide member **3002,** and the backstop member recess **3008** may slope from the deepest portion towards the backstop member face **3007.** The backstop member recess **3008** may be shaped and sized such that the cover member **3040** lies below the plane of the backstop member face 3007when the cover member **3040** is in the non-protective position. In addition, the backstop member recess **3008** may be shaped and sized such that the cover member **3040** may surround and cover the mount connector **3078** when the cover member is in the protective position.

The clamshell mechanism **3000** may also include a compliant gasket system **3084** designed to protect the mount connector **3078,** wherein the compliant gasket system **3084** includes a first gasket portion **3086,** a second gasket portion **3088,** and a transitional gasket portion **3090.** The second gasket portion **3088** may mirror the first gasket portion **3086** and may be disposed on an opposite side of the transitional gasket portion **3090.** **FIGS. 12i** and **12j** depict an exemplary embodiment of a series of the clamshell mechanisms **3000** with compliant gasket systems **3084.** **FIG. 12i** depicts the cover member **3040** in relation to the compliant gasket system **3084** when the cover member **3040** is in the protective position. In contrast, **FIG. 12j** depicts the cover member **3040** in relation to the compliant gasket system **3084** when the cover member **3040** is in the non-protective position. As depicted in FIG. 12d, the cover member **3040** may include a perimeter rib **3044** that may be shaped and sized such that, when cover member **3040** is in the protective position, the perimeter rib **3044** may compress the first gasket portion **3086** and a portion of the transitional gasket portion **3090.** Likewise, **FIG. 12j** depicts the mechanical seal created by the perimeter rib **3044** (see **FIG. 12d**) the second gasket portion **3088,** and a portion of the transitional gasket portion **3090.** In both of **FIGS. 12i** and **12j****,** the perimeter rib **3044** compresses respective portions of the transitional gasket portion **3090** such that the first and second pass-thru apertures **3036, 3038** are within the mechanical seal created by the perimeter rib **3044** and the compliant gasket system **3084.** The first and second pass-thru apertures **3036, 3038** may be contained with the mechanical seal to protect at least the first and second pairs of link-members **3028, 3030** against the threat of contamination from foreign matter, particularly during cleaning of the clamshell mechanism **3000.**

Like the compliant gasket **2074** of the pivotable-cover mechanism **2000,** the compliant gasket system **3084** of the clamshell mechanism **3000** may be made of any suitably compliant material; such materials may include, but are not limited to, isobutylene, natural rubber, neoprene, styrene butadiene, and silicone. In addition, the compliant gasket material may be chosen so that the compliant gasket system **3084** is capable of resisting corrosion from solvents ordinarily used for cleaning device surfaces.

Additionally, and like the pivotable-cover mechanism **2000,** the mount connector **3078** of the embodiment depicted in **FIGS. 12a-12j** may be of a type having multiple spring contacts **3080.** Moreover, the cover member **3040** may likewise include a compliant material, such as but not limited to a polyurethane foam, that may be shaped and sized to receive and protect the spring contacts **3080** when the cover member **3040** is in the protective position.

### A SYSTEM FOR RECEIVING A DEVICE

The aforementioned pivotable-cover or clamshell mechanisms **2000, 3000** may be an embodiment of a protective mechanism **5002** that is a first element of a system for receiving a device **5000.** A second element of the system for receiving a device **5000** may be a receivable device **5020** that may include a device connector **5022** and a means for being received by the protective mechanism **5002,** such as the pivotable-cover or clamshell mechanisms **2000, 3000.**

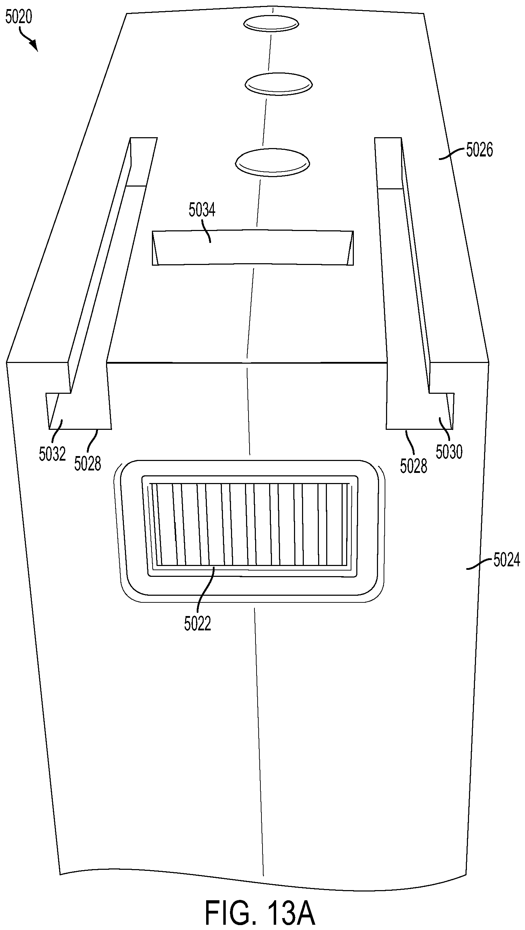

**FIG. 13a** depicts an exemplary embodiment of a receivable device **5020,** wherein the receivable device includes a device connector **5022** that is disposed on a first face **5024** of the receivable device **5020** such that the device connector **5022** is adapted to interface with a mechanism connector **5004** like the respective mount connectors **2068, 3078** of the pivotable-cover and clamshell mechanisms **2000, 3000.**

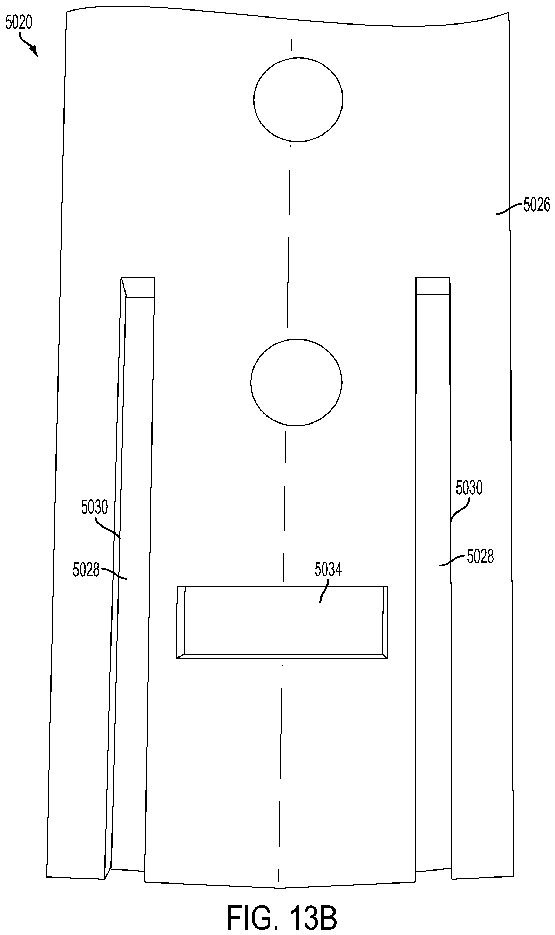

To receive the receivable device **5020,** the protective mechanism **5002** may include at least one rigid member **5008** disposed on a guide member **5006.** The at least one rigid member **5008** may be similar to the respective first and second rail projections **2056, 3066, 2062, 3072** of the pivotable-cover and clamshell mechanisms **2000, 3000** as described herein. The receivable device **5020** may include at least one channel **5028** defined by a second face **5026** of the receivable device **5020** and each of the at least one channel **5028** may be adapted to receive a respective at least one rigid member **5008** of the protective mechanism **5002.** In embodiments of the protective mechanism **5002** that include respective first and second rail projections **2056, 3066, 2062, 3072** like those of the pivotable-cover and clamshell mechanisms **2000, 3000,** the at least one channel **5028** may comprise a first channel **5030** adapted to receive the respective first rail projection **2056, 3066** and a second channel **5032** adapted to receive the respective second rail projection **2062, 3072.** **FIGS. 13a** and **13b** depict an embodiment of the receivable device **5020** that includes the aforementioned first and second channels **5030, 5032** that are adapted to receive the respective first and second rail projections **2056, 3066, 2062, 3072** of the pivotable-cover and clamshell mechanisms **2000, 3000.**

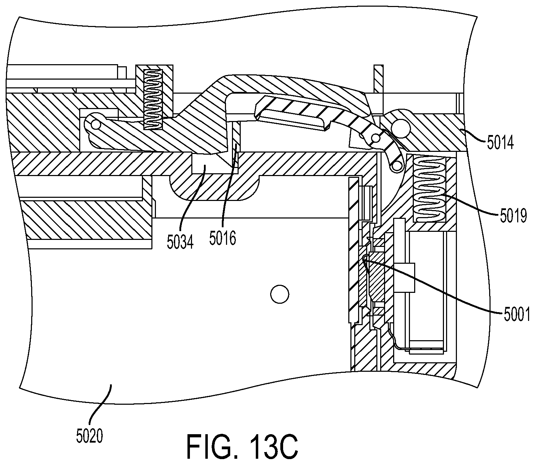

To secure the receivable device **5020** in place after the protective mechanism **5002** receives the receivable device **5020,** the protective mechanism **5002** may include a latch member **5014** having a latch member projection **5016** that engages a latch recess **5034** defined by the second face **5026** of the receivable device **5020.** **FIGS. 13a** and **13b** depict an exemplary embodiment having a latch recess **5034,** and **FIG. 13c** depicts how the latch member projection **5016** may engage the latch recess **5034** to secure a received receivable device **5020.** Additionally, the protective mechanism **5002** may include any of the features discussed above with respect to the pivotable-cover and clamshell mechanisms **2000, 3000;** such features may include, but are not limited to, a latch member spring **2048, 3056** and latch member aperture **2044, 3052,** for example.

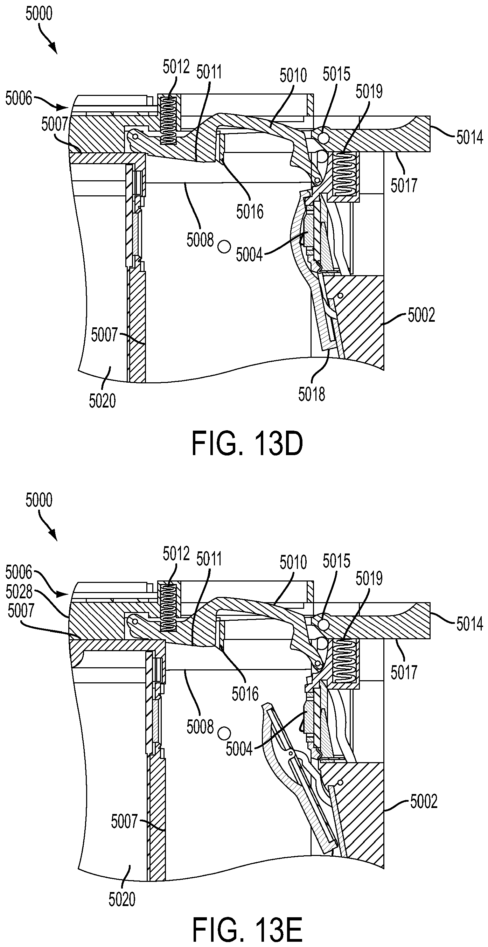

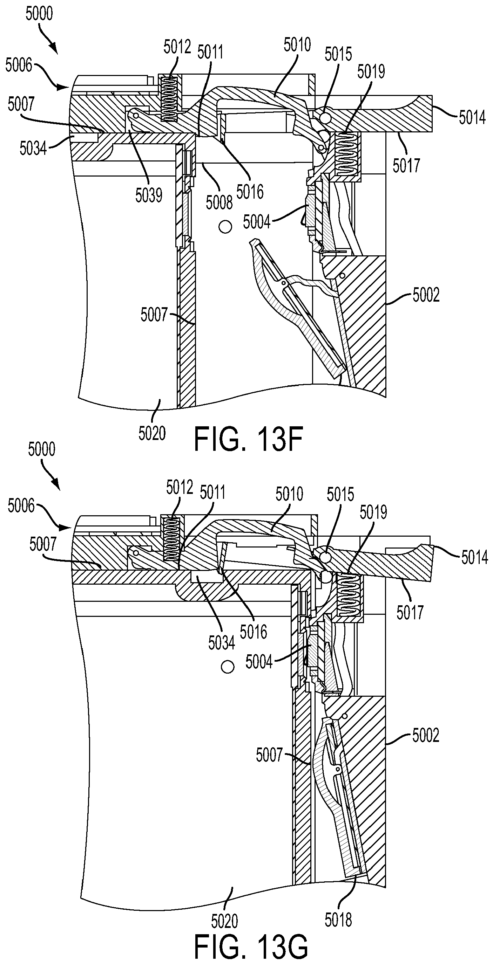

When used in combination, the receivable device **5020** may cause the protective mechanism **5002** to automatically reveal the mechanism connector **5004** as the protective mechanism **5002** receives the receivable device **5020,** thereby allowing the mechanism connector **5004** and the device connector **5022** to interface with each other. For example, the progression of **FIGS. 13d-13g** demonstrates how receiving a receivable device **5020** may cause the clamshell mechanism **3000** to automatically reveal a mechanism connector **5004.** As each of the at least one rigid member **5010** of the a respective protective mechanism slides within a corresponding at least one channel **5028** of the receivable device **5020,** the receivable device **5020** engages the sloped face **5011** of the actuation member **5010** as it slides towards the backstop member face **5007** and mechanism connector **5004 (****FIG. 13d****).** As the receivable device **5020** continues to slide toward the backstop member face **5007,** the receivable device **5020** may begin to pivot the actuation member **5010** in a first direction from a first position to a second position **(****FIGS. 13e** and **13f****).** As described above with respect to the pivotable-cover and clamshell mechanisms **2000, 3000,** or other embodiment of the protective mechanism **5002,** this pivotal movement of the actuation member **5010** may cause the cover member **5018** to reveal the mechanism connector **5004.** The receivable device **5020** may slide toward the backstop member face **5007** until it is in a received position where it contacts the backstop member face **5007** and the device connector **5022** interfaces with the mechanism connector **5004** (**FIG. 13g**).

The progression from **FIG. 13g** to **FIG. 13d** depicts the reverse process wherein decoupling the receivable device **5020** from the protective mechanism **5002** may cause the actuation member **5010** to pivot from the second position to the first position under the force of an actuation spring **5012** and thereby cover the mechanism connector **5004.**

As should be understood by persons having ordinary skill in the art, the at least one protective mechanism **5002** may be designed such that the cover member **5020** is capable of pivoting from the protective position to the non-protective position as the receivable device **5020** slides towards the backstop member face **5007** and the mechanism connector **5004.** The pivotable-cover and clamshell mechanisms **2000, 3000** described above are but two exemplary embodiments wherein the mechanical linkages and the constituent components are shaped and sized so as to pivot or otherwise move through their respective ranges of motion while the receivable device **5020** causes the actuation member **5010** to pivot as the receivable device **5020** coupled or decouples with a protective mechanism **5002.**

When the receivable device **5020** is in the received position, the latch member **5014** of the respective protective mechanism **5002** may pivot to a latched position such that the latch member projection **5016** engages the latch recess **5034** defined by a second face of the receivable device **5020.** In a preferred embodiment of the system for receiving a device **5000,** the latch recess **5034** may be disposed on the first face of the receivable device **5020** such that it is between the first channel **5030** and the second channel **5032.** Like the sloped face **5011,** the latch member projection **5016** may slope away from the plane of the guide member **5006** and towards the backstop member face **5007** such that the receivable device **5020** may cause the latch member projection **5016** and latch member **5014** to pivot out of the way as the receivable device **5020** slides towards the backstop member face **5007.** As described above with respect to the pivotable-cover and clamshell mechanisms **2000, 3000** a latch spring **5019** may be used to automatically restore the latch member **5014** to the latched position when the receivable device **5020** is in the received position.

To decouple the receivable device **5020** from the protective mechanism **5002,** pivoting the latch member **5014** away from the latched position may cause the latch member projection **5016** to disengage from the latch recess **5034** and allow the receivable device **5020** to slide in the opposite direction away from the backstop member face **5007** and the mechanism connector **5004.** Pivoting the latch member **5014** away from the latched position may be achieved by manually pulling on a latch member release tab **5017** that is disposed on an opposite end portion of the latch member **5014** with respect to the latch member projection **5016.** Where the latch member release tab **5017** is on an opposite side of the latch member pivot point **5015** with respect to the latch member projection **5016,** pulling towards the mechanism connector **5004** causes the latch member projection **5016** to disengage from the latch recess **5034.**

Various alternatives and modifications can be devised by those skilled in the art without departing from the disclosure. Accordingly, the present disclosure is intended to embrace all such alternatives, modifications and variances. Additionally, while several embodiments of the present disclosure have been shown in the drawings and/or discussed herein, it is not intended that the disclosure be limited thereto, as it is intended that the disclosure be as broad in scope as the art will allow and that the specification be read likewise. Therefore, the above description should not be construed as limiting, but merely as exemplifications of particular embodiments. And, those skilled in the art will envision other modifications within the scope and spirit of the claims appended hereto. Other elements, steps, methods and techniques that are insubstantially different from those described above and/or in the appended claims are also intended to be within the scope of the disclosure.

The embodiments shown in drawings are presented only to demonstrate certain examples of the disclosure. And, the drawings described are only illustrative and are non-limiting. In the drawings, for illustrative purposes, the size of some of the elements may be exaggerated and not drawn to a particular scale. Additionally, elements shown within the drawings that have the same numbers may be identical elements or may be similar elements, depending on the context.

Where the term "comprising" is used in the present description and claims, it does not exclude other elements or steps. Where an indefinite or definite article is used when referring to a singular noun, e.g. "a" "an" or "the", this includes a plural of that noun unless something otherwise is specifically stated. Hence, the term "comprising" should not be interpreted as being restricted to the items listed thereafter; it does not exclude other elements or steps, and so the scope of the expression "a device comprising items A and B" should not be limited to devices consisting only of components A and B. This expression signifies that, with respect to the present disclosure, the only relevant components of the device are A and B.

Furthermore, the terms "first", "second", "third" and the like, whether used in the description or in the claims, are provided for distinguishing between similar elements and not necessarily for describing a sequential or chronological order. It is to be understood that the terms so used are interchangeable under appropriate circumstances (unless clearly disclosed otherwise) and that the embodiments of the disclosure described herein are capable of operation in other sequences and/or arrangements than are described or illustrated herein.

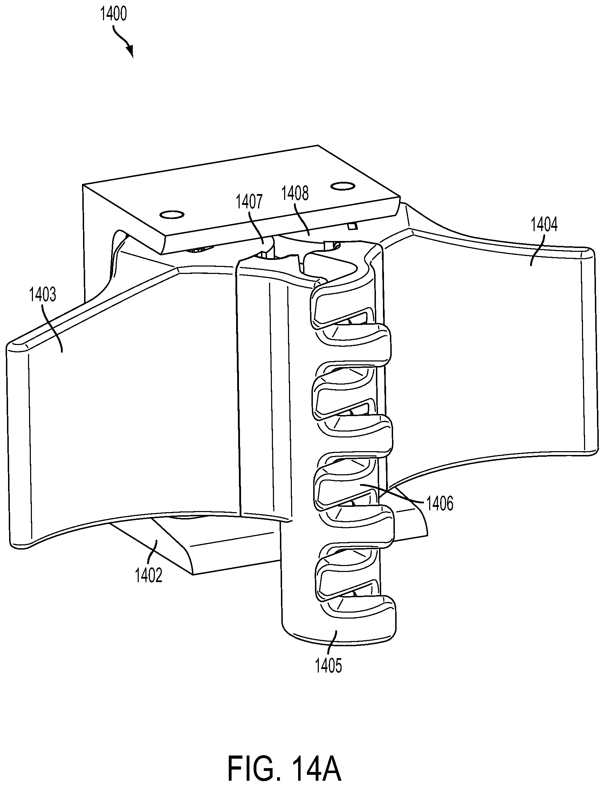

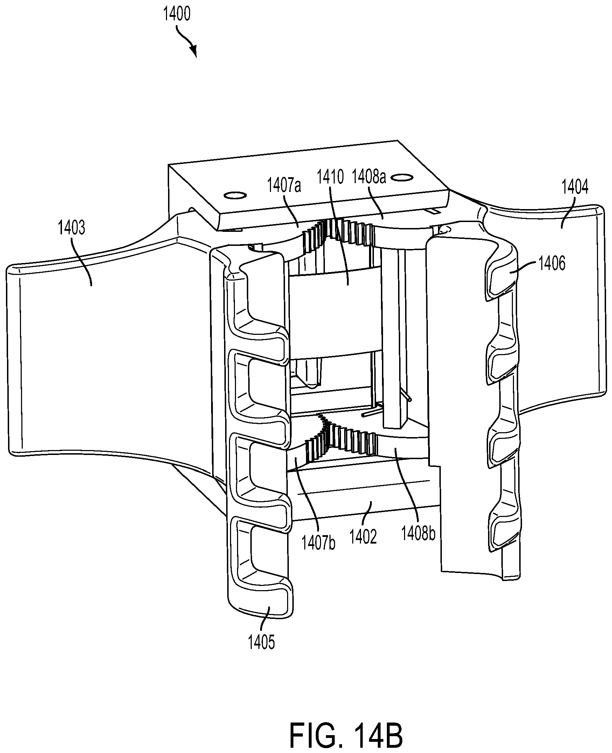

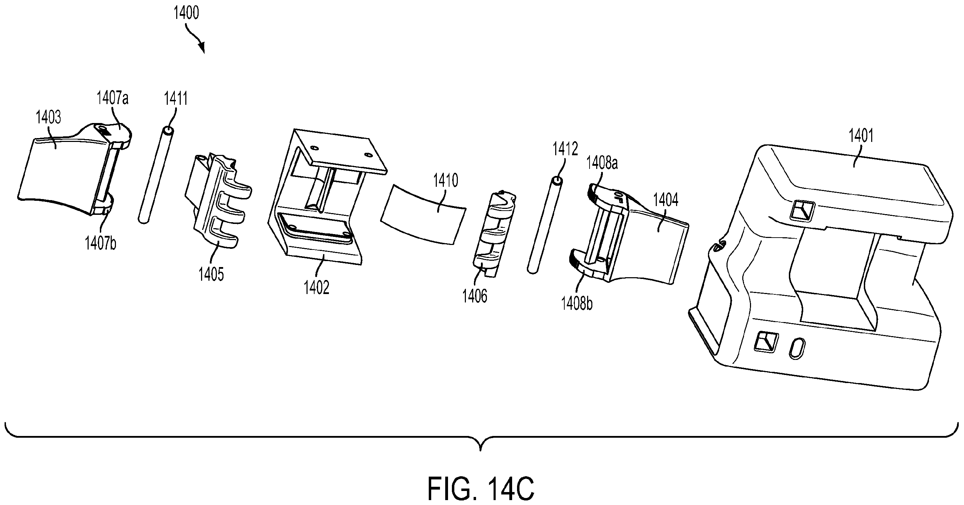

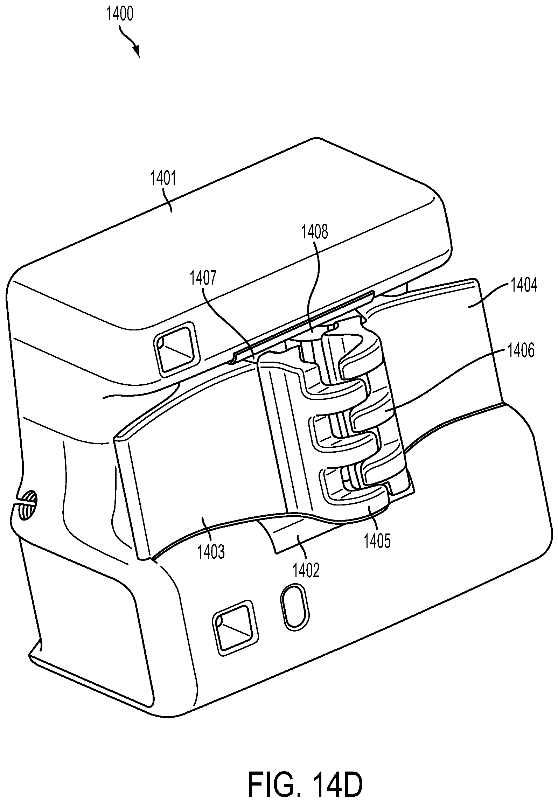

In one example embodiment, as shown in **FIGS. 14A-14E****,** a clamp apparatus **1400** is depicted. The clamp apparatus **1400** comprises a body **1402.** In the shown embodiment, the clamp apparatus **1400** has a first handle **1403** and a second handle **1404.** The first handle **1403** and the second handle **1404** may be operatively coupled to the body **1402.** The clamp apparatus also includes a first movable gripper **1405** and a second movable gripper **1406.** The first movable gripper **1405** and the second movable gripper **1406** are coupled to the first handle **1403** and the second handle **1404,** respectively. In one example embodiment, the body **1402** is positioned intermediately between the handles and the grippers. The first handle **1403** and the second handle **1404** are fixedly coupled to the first movable gripper **1405** and the second movable gripper **1406,** respectively, thereby controlling the movement of the first movable gripper **1405** and the second movable gripper **1406.** The clamp apparatus **1400** also includes a first gear set **1407** and a second gear set **1408** that are operatively coupled to the first handle **1403** and the second handle **1404,** respectively, as well as the first movable gripper **1405** and the second movable gripper **1406,** respectively, and are also rotatably coupled to the body. The first gear set **1407** and the second gear set **1408** are configured to operatively engage one another. In one example embodiment, the first gear set **1407** may include an upper first gear **1407a,** and a lower first gear **1407b** that is fixedly coupled to the upper first gear **1407a,** such that the upper first gear **1407a** and the lower first gear **1407b** move together in unison. Similarly, the second gear set **1408** may include an upper second gear **1408a,** and a lower second gear **1408b** that is fixedly coupled to the upper second gear **1408a,** such that the upper second gear **1408a** and the lower second gear **1408b** move together in unison. The upper first gear **1407a** and the lower first gear **1407b** may be configured to operatively engage the upper second gear **1408a** and the lower second gear **1408b,** respectively.

The clamp apparatus **1400** also includes at least one bias member **1410** operatively engaged with the first handle **1403** and the second handle **1404,** such that the handles are configured for operation by a user so as to overcome the at least one bias member 1410. The at least one bias member **1410** is configured to bias the first handle **1403** and the second handle **1404** toward a first position. The first movable gripper **1405** and the second movable gripper **1406** are engaged with one another, defining a clamped position, when the first handle **1403** and the second handle **1404** are in the first position. The first handle **1403** and the second handle **1404** are configured to thereby move, under actuation, to a second position, whereby the first movable gripper **1405** and the second movable gripper **1406** are disengaged from one another, defining an unclamped position.

In some embodiments, the clamp apparatus further comprises a gripping surface on the first movable gripper **1405** and the second movable gripper **1406,** configured to engage a clamped object. In some embodiments, the grippers are configured to clamp onto a pole. In one example embodiment, the clamp apparatus **1400** is for use with medical devices and medical accessories. In one example embodiment, the clamp apparatus **1400** is configured to couple a medical device **1401** to a support pole. The pole may be an IV pole. The medical device **1401** may be a monitor comprising a tablet computer. In one example embodiment, the clamp apparatus **1400** is configured to couple an infusion pump to a support pole. The infusion pump may be a peristaltic infusion pump. In one example embodiment, the clamp apparatus **1400** is capable of automatically mimicking the girth of a variety of different clamped objects.

In one example embodiment, at least part of at least one of the first movable gripper **1405** and the second movable gripper **1406** may be comprised of a material which will firmly grip, but not deform, a clamped object. In some embodiments, at least a part of at least one of the first movable gripper **1405** and the second movable gripper **1406** may be comprised of polyurethane. In some embodiments, at least part of at least one of the grippers may be comprised of rubber, or may be coated in a rubbery, gripping material. In some embodiments, at least one of the first movable gripper **1405** and the second movable gripper **1406** may be at least partially covered by a removable surface. In some embodiments, at least one of the first movable gripper **1405** and the second movable gripper **1406** may comprise at least one approximately arcuate, semi-circular, or contoured face at least on the gripping surface.

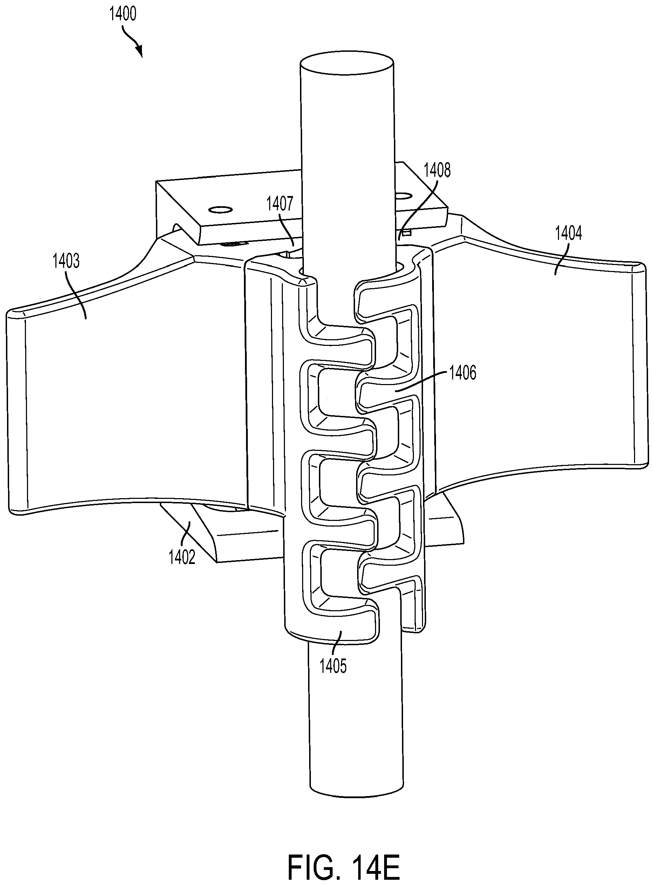

In one example embodiment, at least a part of at least one of the first movable gripper **1405** and the second movable gripper **1406** has fingers. In one example embodiment, the first movable gripper **1405** and the second movable gripper **1406** both have fingers. As shown in **FIG. 14A****,** the fingers of the first movable gripper **1405** and the second movable gripper **1406** are interdigitated when the grippers are engaged with one another, corresponding to the handles being in the first position. As shown in **FIG. 14E****,** the fingers of each gripper are partially interdigitated due to partial engagement of the grippers with one another, corresponding to the handles being in an intermediate position between the first and second positions.

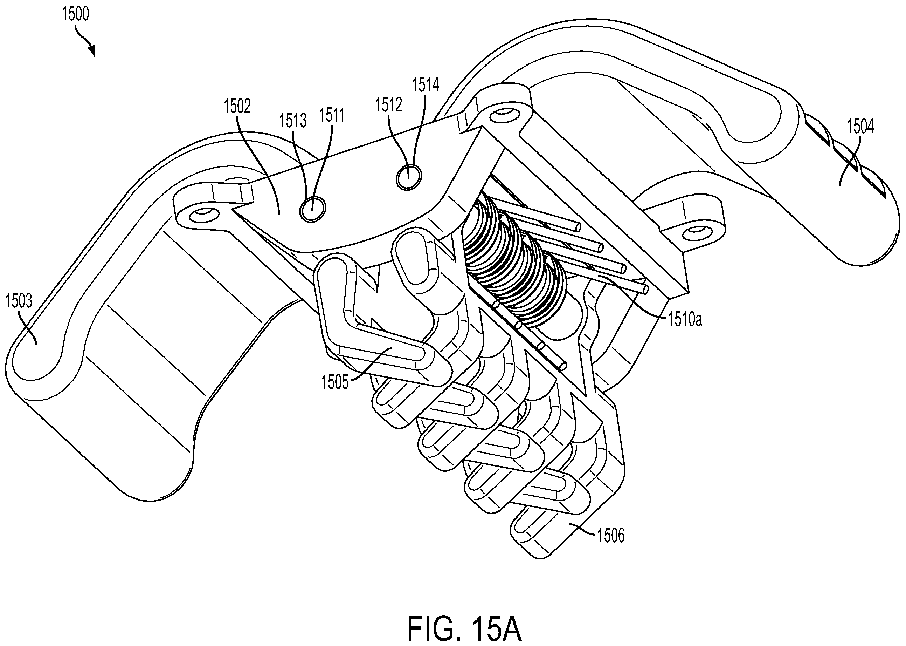

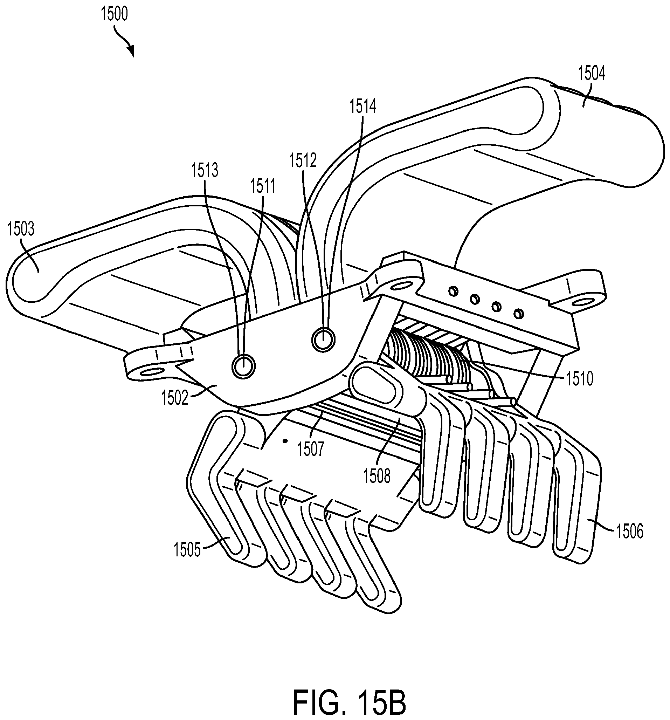

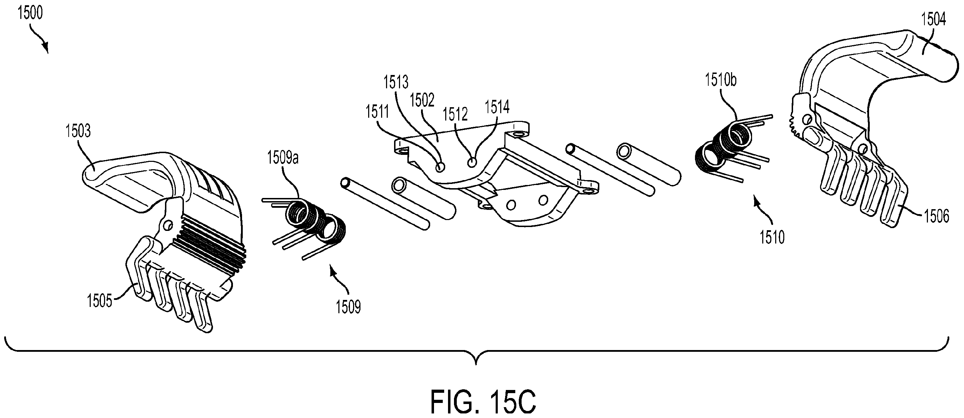

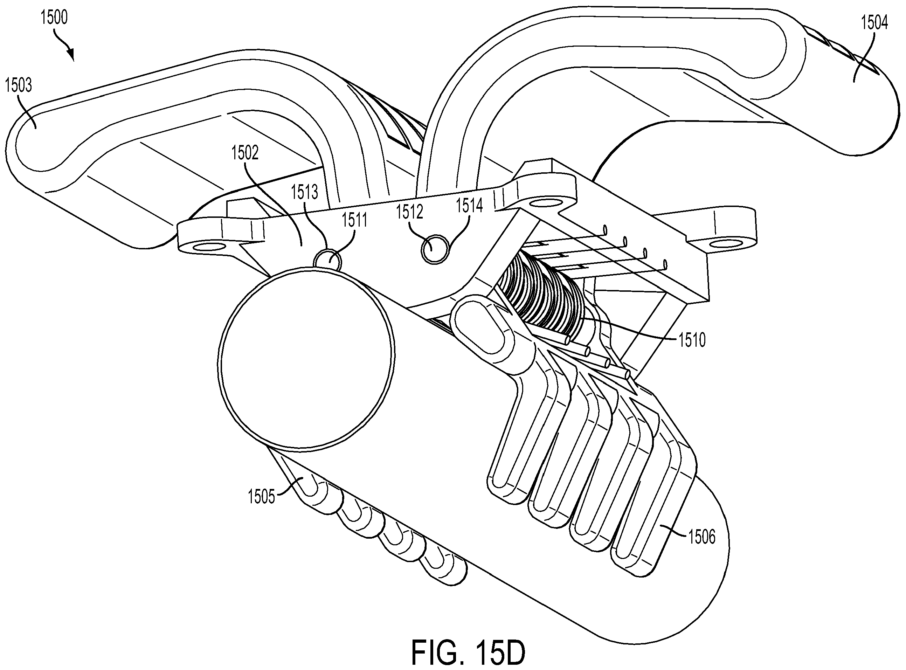

In some embodiments, the at least one bias member **1410** is a spring. Further, the at least one bias member **1410** may be a flat spring. The at least one bias member **1410** may also be a leaf spring. In one example embodiment, the at least one bias member may be at least one array of multiple bias members. Further, the at least one bias member may be an array of multiple flat springs arranged in a layered configuration. In one example embodiment, the at least one bias member **1410** may be made of a flexible, compressible material. In some embodiments, as shown in **FIGS. 15A-15D****,** the at least one bias member **1410** may comprise a first bias member and a second bias member. In one embodiment, the first bias member may be a first bias member array **1509,** including multiple individual bias members **1509a,** and the second bias member may be a second bias member array **1510,** including multiple individual bias members **1510a.** In one example embodiment, the first and second bias members may each include a single bias member. Additionally, the first and second bias members or the individual bias members **1509a** and **1510a** may be springs, or, in one example embodiment, may be torsion springs.

In one example embodiment, the first handle **1503** and the second handle **1504** may be paddles. In one example embodiment, the handles may be concave shaped away from or towards the body **1502,** the handles being actuatable. The handles may be configured to be pulled by a user from a first side, or pushed by the user from a second side, in order to move the grippers from the first position to the second position. In some embodiments, the first handle **1503** and the second handle **1504** may further comprise a palm support. The member adapted as a palm support may be U-shaped. In one example embodiment, the first handle **1503** and a second handle **1504** may provide a pair of pull handles configured for operation by a user so as to actuate the first movable gripper **1505** and the second movable gripper **1506** from the first position to the second position.

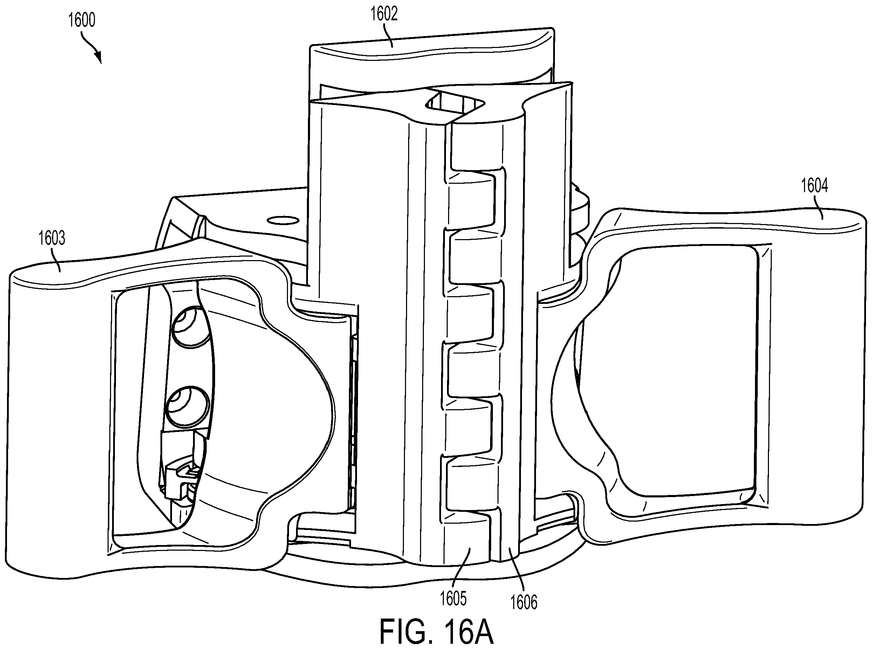

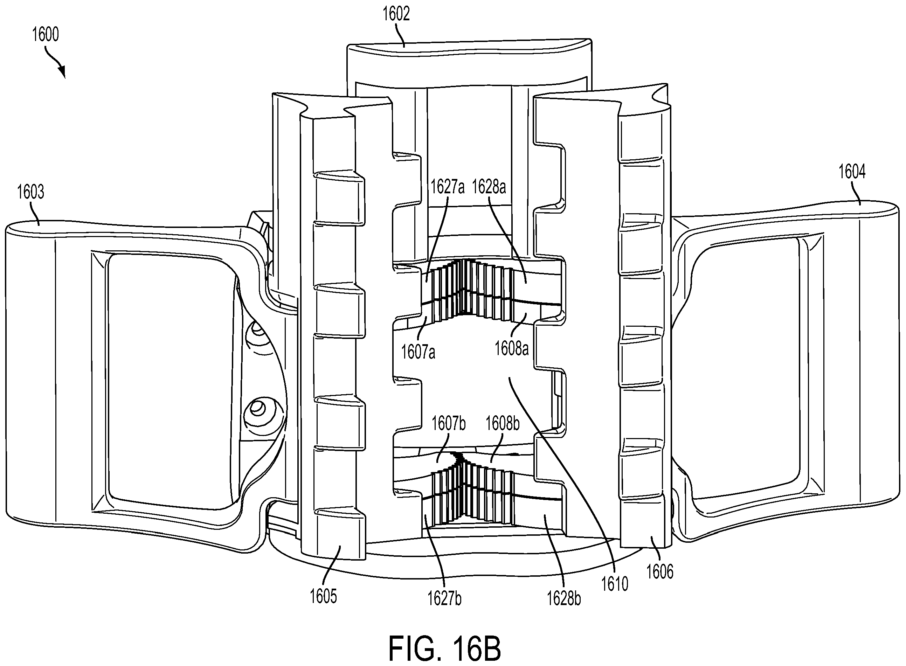

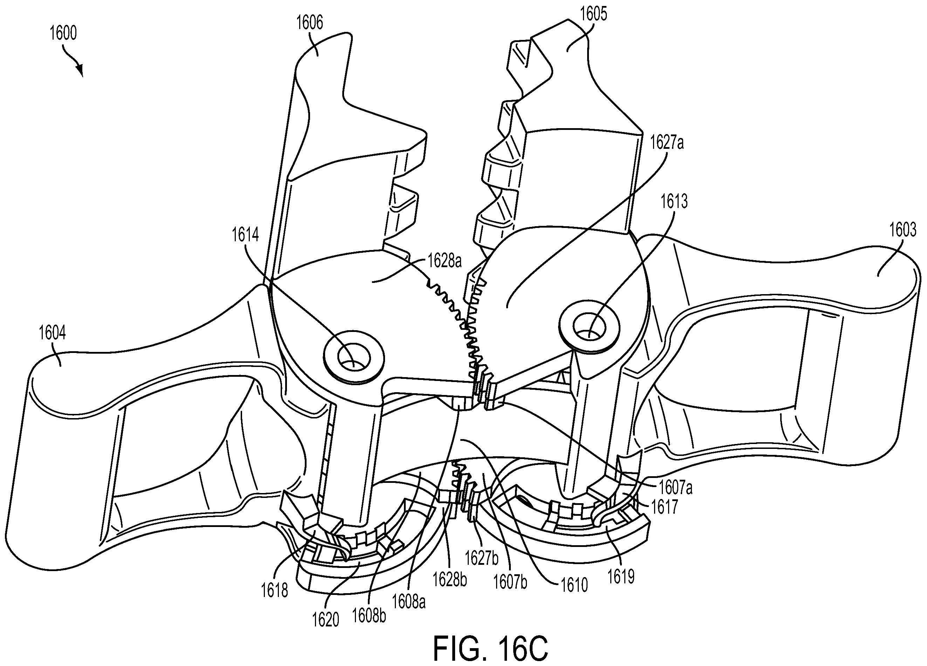

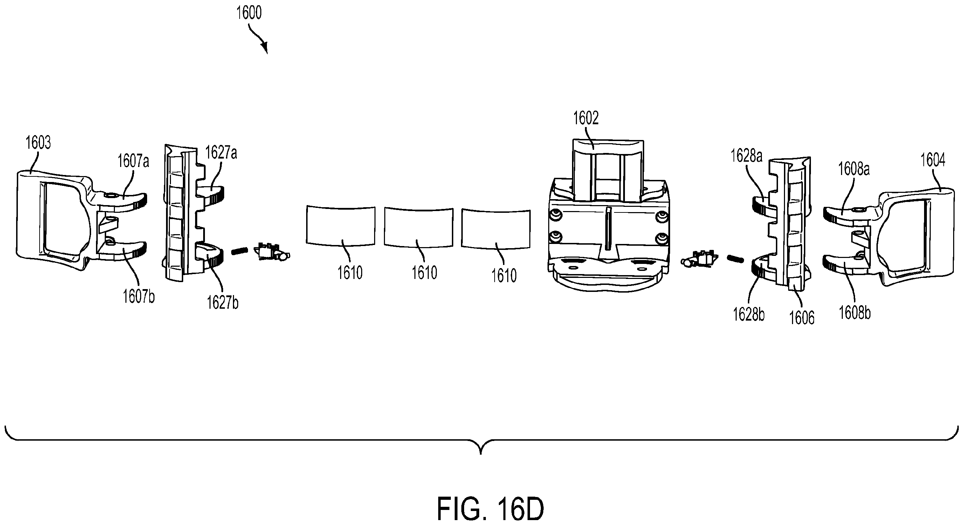

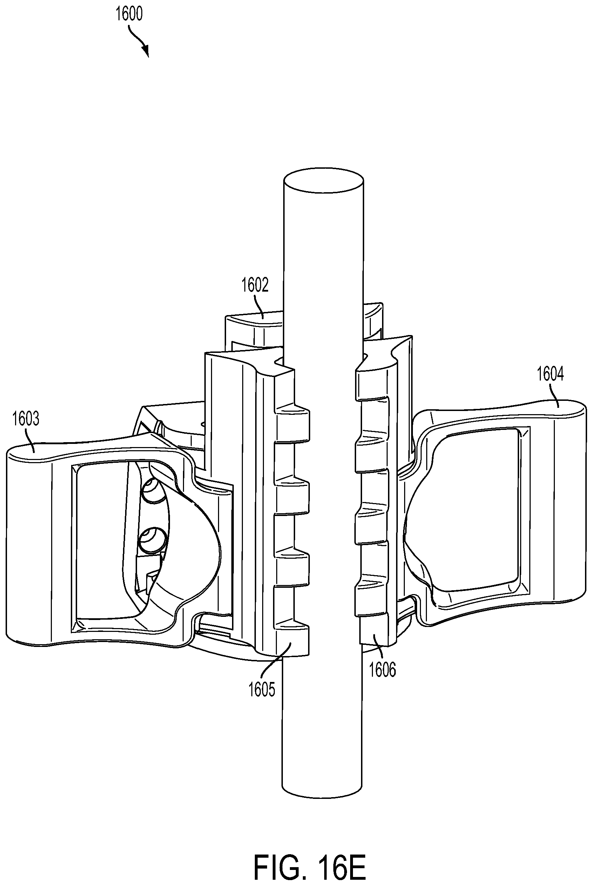

In one example embodiment, as shown in **FIGS. 16A-16E****,** a clamp apparatus **1600** is depicted. The clamp apparatus **1600** comprises a third gear set **1627** and a fourth gear set **1628,** the gear sets operatively coupled to the first handle **1603** and the second handle **1604,** respectively, and rotatably coupled to the body **1602.** In one example embodiment, the third gear set **1627** and fourth gear set **1628** may share an axis of rotation with the first gear set **1607** and the second gear set **1608,** respectively. The third gear set **1627** and the fourth gear set **1628** may be operatively coupled to the first movable gripper **1605** and the second movable gripper **1606,** respectively. The third and fourth gear sets may be configured to operatively engage a locking mechanism in association with the handles. The locking mechanism comprises a first hook **1617,** a first catch **1619,** a second hook **1618,** and a second catch **1620.** In one example embodiment, the third and fourth gear sets may be operatively engaged with one another.

In one example embodiment, the handles and third and fourth gear sets may be configured to permit slight initial rotational movement of the handles in advance of subsequent rotational movement of the grippers, when moving the first and second handles from the first position to the second position. Similarly, the handles and gears may be configured to permit slight additional rotational movement of the handles after the grippers stop their rotational movement, when moving the first and second handles from the second position back to the first position. The initial slight rotational movement of the handles may perform an unlocking function, freeing the grippers to move, while the additional slight rotational movement of the handles after the grippers stop moving may perform a locking function, preventing the grippers from moving.

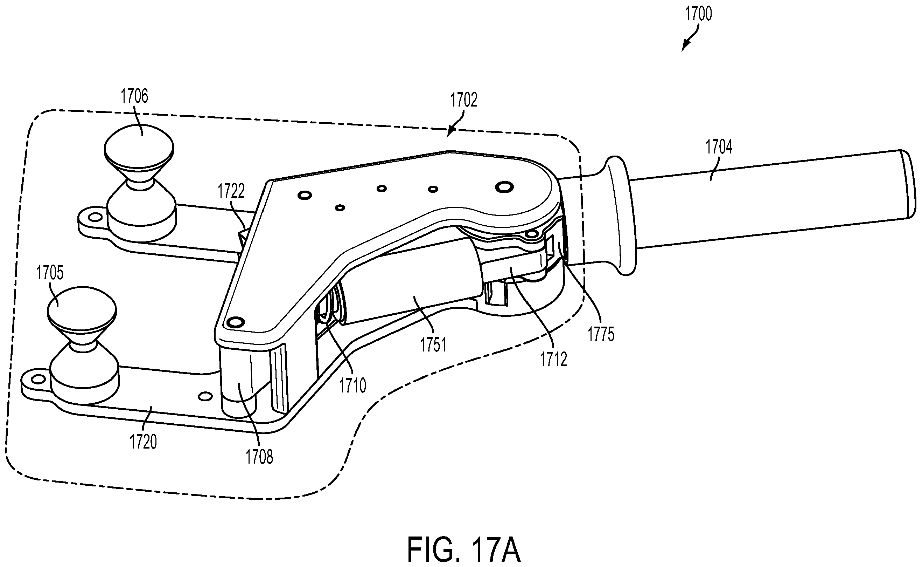

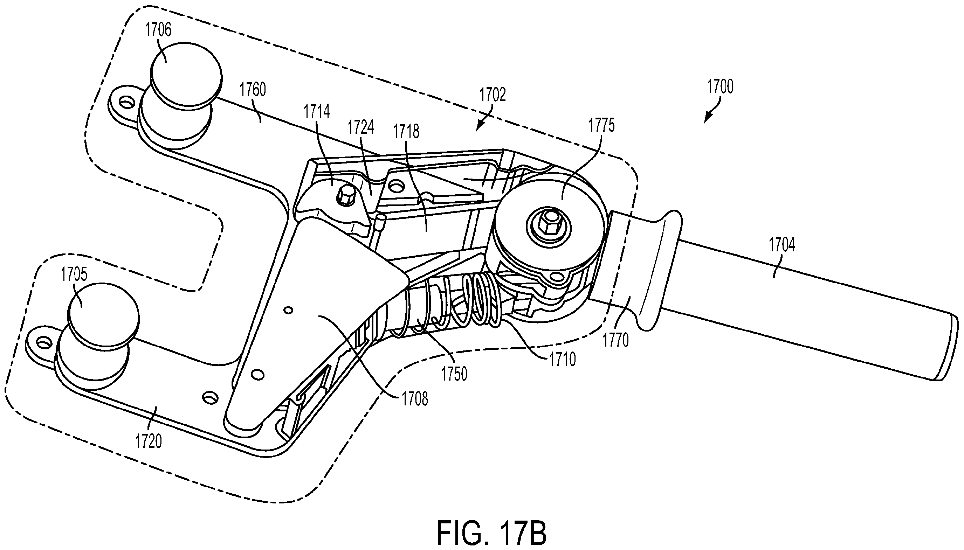

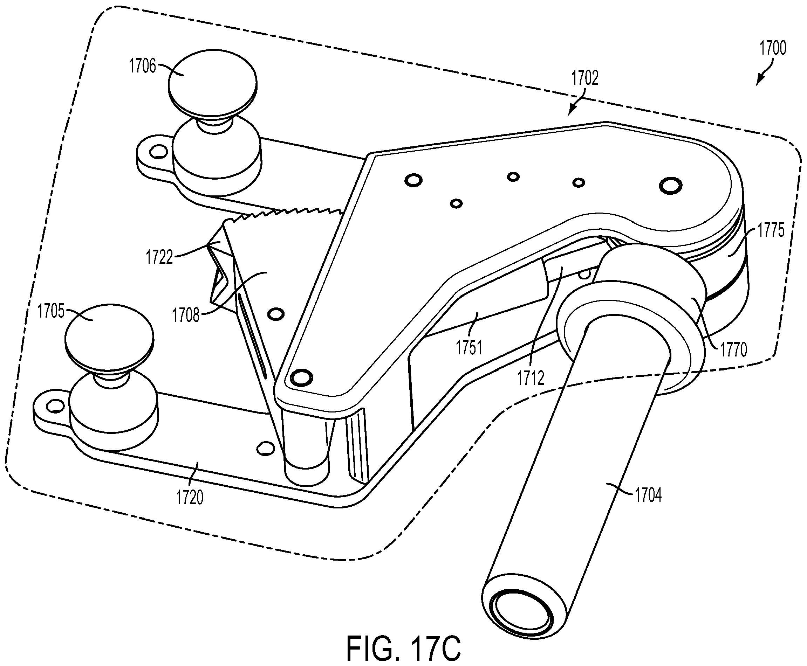

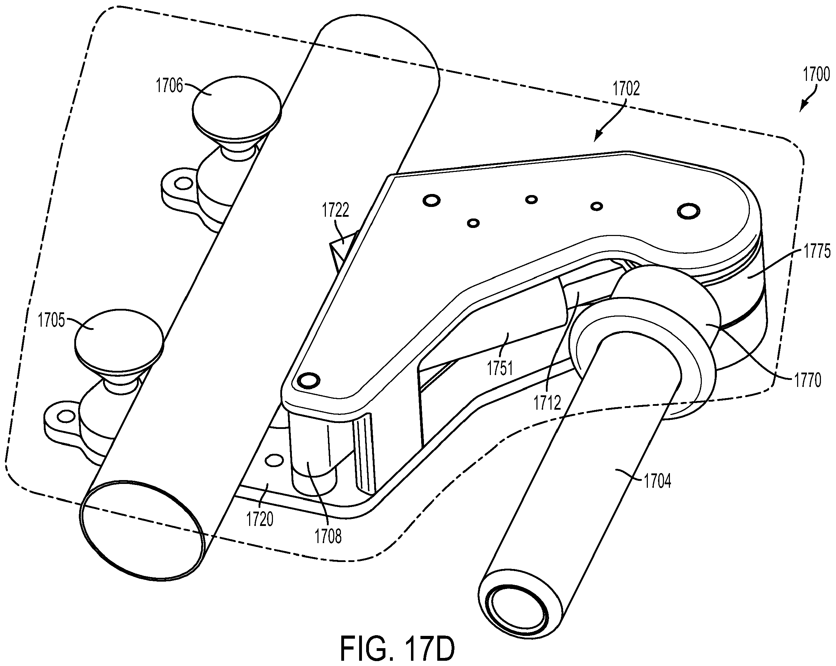

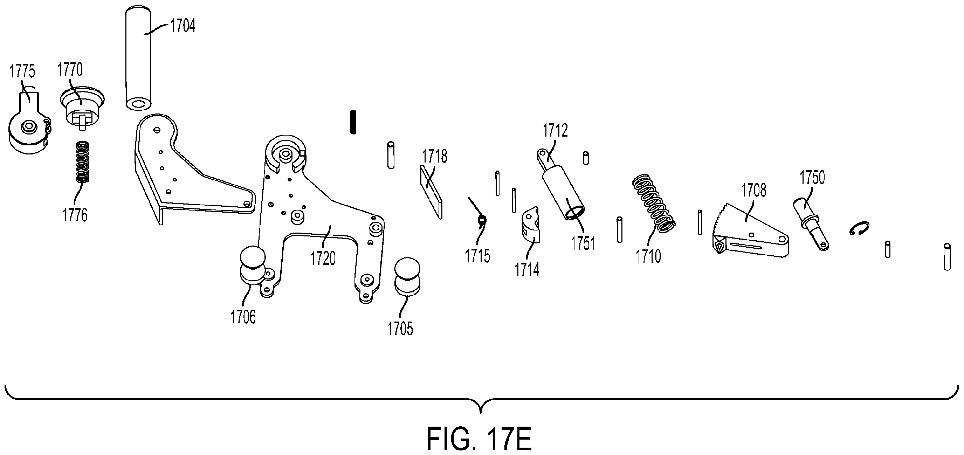

In one example embodiment, as shown in **FIGS. 17A-17E****,** a clamp apparatus **1700** is depicted. The clamp apparatus **1700** comprises a body **1702,** the body **1702** having a first end and a second end. The clamp also includes a lever **1704,** the lever **1704** operatively coupled to the first end of the body **1702.** The clamp apparatus **1700** also includes a movable gripper **1708.** The movable gripper **1708** is coupled to an intermediate portion of the body **1702,** between the first end and second end. The clamp apparatus **1700** includes a first fixed gripper **1706** and a second fixed gripper **1705.** The fixed grippers are positioned at the second end of the body **1702.** The fixed grippers are configured to approximately oppose the movable gripper **1708** such as to secure a pole from opposing sides. The clamp apparatus **1700** also includes a connector member **1712.** The connector member **1712** has a first end operatively coupled to the lever **1704** and a second end operatively coupled to the movable gripper **1708.**

In one example embodiment, the movable gripper **1708** is rotatable about a coupling point of the intermediate portion of the body **1702.** The movable gripper **1708** is approximately wedge-shaped, having a narrow end and a wide end. The narrow end of the movable gripper **1708** is coupled to the body **1702,** and the movable gripper **1708** is rotatable about the narrow end. The wide end of the movable gripper **1708** may have a ridged surface. Further, the ridged surface may extend along the wide end of the wedge-shaped movable gripper **1708.** The wide end of the movable gripper **1708** may have a contoured face **1722** opposing the at least two fixed grippers. In some embodiments, the contoured face **1722** may be a semi-circular or wedge-shaped face. The face **1722** of the wide end of the movable gripper **1708** may be configured to complement the shape of a pole.

In one example embodiment, the grippers further comprise gripping surfaces configured to engage a clamped object. The gripping surfaces may be made of a material which will firmly grip, but not deform, a clamped object. In one example embodiment, the grippers are configured to close onto a pole. In one example embodiment, the grippers are rubber. In another example embodiment, the grippers are coated in a rubbery, gripping material.

In one example embodiment, the body **1702** may comprise a back plate **1720** to which the first fixed gripper **1706** and the second fixed gripper **1705** are fixed.

In one example embodiment, the movable gripper **1708,** the first fixed gripper **1706** and the second fixed gripper **1705** may be configured such that the movable gripper **1708** and the fixed grippers are substantially opposite and are capable of automatically mimicking the girth of a clamped object.

In one example embodiment, the connector member **1712** is configured to rotate the movable gripper 1708 about the narrow end upon actuation of the connector member **1712.**

In one example embodiment, the connector member **1712** includes a bias member **1710.** In one example embodiment, the bias member **1710** is a spring, and in some embodiments the spring is a compression spring. In other embodiments, the bias member **1710** may be a compressible or expandable spring. In some embodiments, the connector member **1712** includes a piston. The piston may be a spring-biased piston. The bias member **1710** is oriented such that movement of the connector member **1712** towards the movable gripper **1708** stores mechanical energy in the bias member **1710.**

In one example embodiment, the connector member **1712** is rotatably connected to the lever **1704** at the first end of the connector member **1712.** The connector member **1712** is coupled to a lever joint **1775,** the lever joint **1775** positioned at, and also operatively coupled to, an end of the lever **1704.** The connector member **1712** may also be rotatably connected to the movable gripper **1708** at the second end of the connector member **1712.** The connector member **1712** is configured to, under actuation of the lever **1704,** extend towards the movable gripper **1708,** thereby rotating the movable gripper **1708** towards the fixed grippers.

In one example embodiment, the clamp apparatus **1700** further comprises a bias member support **1750** coupled to the connector member **1712.** The bias member support **1750** has a portion with a diameter less than a diameter of the bias member **1710.** The portion of the bias member support **1750** is positioned to fit inside the diameter of the bias member **1710.**

In one example embodiment, the clamp apparatus **1700** further comprises a bias member housing **1751** coupled to the connector member **1712.** The bias member housing **1751** is hollow and has a sealed end. The bias member housing **1751** has a diameter greater than the diameter of the bias member **1710.** In one example embodiment, the lever **1704** of the clamp apparatus **1700** is a handle. The lever **1704** is configured to, under actuation, rotate towards the body **1702.** The lever **1704** is configured to move the connector member **1712** toward a first position and thereby move the movable gripper **1708** toward the fixed grippers. The lever **1704** is further configured to move the connector member **1712** toward a second position and thereby move the movable gripper **1708** away from the fixed grippers. In one example embodiment, the lever joint **1775** is a cam, such that when the lever **1704** is moved to the first position, the cam pushes the connector member **1712,** thereby pushing the movable gripper **1708** closer to the fixed grippers. In one example embodiment, the clamp apparatus **1700** is configured to allow the moveable gripper **1708** to stop when abutting against an object while allowing the connector member **1712** to continue to move as the lever **1704** is further actuated.

In one example embodiment, the lever **1704** includes a slideable ring **1770** coaxially aligned with and surrounding the top end of the lever **1704,** the top end being nearest the lever joint **1775.** The slideable ring **1770** is configured to free the lever **1704** from a locked position. The slideable ring **1770** is configured to slide out of a notch in the lever joint **1775,** thereby unlocking the lever **1704** and freeing the lever **1704** to rotate. The slideable ring **1770** includes a ring bias member **1776,** the ring bias member **1776** configured to bias the slideable ring **1770** to a notched position. In one example embodiment, the ring bias member **1776** is a compression spring, while in another embodiment the ring bias member **1776** is an expansion spring.

In one example embodiment, the clamp apparatus **1700** further comprises a locking assembly, the locking assembly configured to interact with the movable gripper **1708.** The locking assembly includes a pawl **1714,** and the pawl **1714** includes a pawl bias member **1715.** In one example embodiment, the pawl bias member **1715** is a spring, and in some embodiments the pawl bias member **1715** is a torsion spring. The pawl **1714** is rotatably coupled to the locking assembly, the pawl **1714** configured to rotate into contact with an upper ridged surface of the movable gripper **1708,** locking the gripper in place.

In one example embodiment, the locking assembly further comprises a slideable member **1718,** and the pawl **1714** positioned in contact with the slideable member **1718.** The slideable member **1718** has a first end in contact with the lever joint **1775.** The slideable member **1718** contacts an outer surface of the lever joint **1775,** the outer surface having a depressed portion. The locking assembly is configured to move the slideable member **1718** into the depressed portion of the lever joint **1775,** allowing the pawl **1714** to rotate into contact with the movable gripper **1708** and thereby locking the movable gripper **1708** in place.

In one example embodiment, the clamp apparatus **1700** is configured for use with medical devices and medical accessories.

In one example embodiment, the body **1702** includes a means of coupling the clamp to a load. In one example embodiment, the load is a medical device. In some embodiments, the medical device is a peristaltic infusion pump or syringe infusion pump.
In one example embodiment, the clamp apparatus **1700** is configured to couple a medical device to a support pole. In one embodiment, the pole may be an IV pole. In one embodiment, the medical device is a monitor comprising a tablet computer.

## Claims

1. A rack apparatus, comprising:
at least one support pole (1812, 6012, 7012) having a first end portion and a second end portion;
at least one of a clamp assembly (1814, 1990), hanger (7014, 7214), or handle (6020, 7020) attached to the first end portion of the support pole;
at least one mount connector (1960, 1838, 2068, 3078, 6038, 7038, 7140, 7238);
at least one alignment feature (7024) operatively coupled to the support pole;
a base member (1816, 7016, 7216) attached to the second end portion of the support pole; and
a clamp;
wherein the support pole is configured to accept the clamp (10, 110, 202, 310, 410, 510, 610, 710, 7110, 1400, 1600, 1700), the clamp comprising:
a housing (12, 112, 204, 580, 612, 712) including first and second tracks (22, 123, 223, 328, 598, 628);
a fixed gripper (322, 401, 403, 501, 713, 1705, 1706) coupled to the housing;
a driven member (710, 720, 7002) configured to slide within the first and second tracks of the housing;
a movable gripper (1405, 1406, 1505, 1506, 1605, 1606, 1708) operatively coupled to the driven member; and
an actuator configured to move the driven member towards a first position to thereby move the movable gripper towards the fixed gripper, the actuator further configured to move the driven member towards a second position to thereby move the movable gripper away from the fixed gripper;
wherein the clamp is configured to couple a medical device (1920, 7360, 1401) to the support pole of the rack apparatus; and
wherein a medical device connector (1970, 5022, 7114) is disposed on the clamp and is configured to operatively engage with at least one of the at least one mount connector.

2. The rack apparatus according to claim 1, wherein the medical device connector (1970, 5022, 7114) is configured to operatively engage with the at least one of the at least one mount connector to receive at least one of a network connection and power for the medical device.

3. The rack apparatus according to claim 2, wherein at least one of the at least one alignment feature is configured to align the medical device connector with the at least one of the at least one mount connector.

4. The rack apparatus according to claim 2, wherein at least one of the at least one alignment feature is included on a collar (7022) on the support pole.

5. The rack apparatus according to claim 2, wherein the rack apparatus further comprises a power system.

6. The rack apparatus according to claim 5, wherein the power system is configured to provide power to at least one medical device via the at least one mount connector.

7. The rack apparatus according to claim 2, wherein the rack apparatus further comprises a communication system configured to allow an attached medical device to communicate with at least one other attached medical device via the at least one mount connector.

8. The rack apparatus according to claim 7, wherein the communication system is configured use at least one of a CANbus protocol and USB protocol.

9. The rack apparatus according to claim 2, wherein the base member comprises:
a power connector (1872);
a power supply (1870);
a main power cable (1876) electrically connecting the power connector and the power supply; and
at least one transmission cable (1874) connecting the power supply and the at least one mount connector.

10. The rack apparatus according to claim 2, wherein the rack apparatus is configured to couple to an IV pole.

11. The rack apparatus according to claim 2, wherein the at least one support pole is an IV pole.

12. The rack apparatus according to claim 2, wherein the base member further comprises at least one wheel (7215).

13. A system comprising a rack apparatus as claimed in claim 1, wherein the medical device connector is disposed on one of the fixed gripper or movable gripper of the clamp.

14. The system according to claim 13, wherein at least one of the at least one alignment feature is configured to align the medical device connector with the at least one of the at least one mount connector.

15. The system according to claim 14, wherein the medical device connector is configured to operatively engage with the at least one of the at least one mount connector to receive at least one of a network connection and power for a medical device.

16. The system according to claim 15, wherein the medical device is a monitoring client comprising a tablet computer.

## Patentansprüche

1. Gestelleinrichtung, die Folgendes umfasst:
wenigsten eine Stützstange (1812, 6012, 7012) mit einem ersten Endteil und einem zweiten Endteil;
eine Klammerbaugruppe (1814, 1990) und/oder einen Aufhänger (7014, 7214) und/oder einen Griff (6020, 7020), die/der an dem ersten Endteil der Stützstange angebracht ist;
wenigstens einen Halterungsverbinder (1960, 1838, 2068, 3078, 6038, 7038, 7140, 7238);
wenigstens ein Ausrichtungsmerkmal (7024), das mit der Stützstange wirkgekoppelt ist;
ein Basiselement (1816, 7016, 7216), das an dem zweiten Endteil der Stützstange angebracht ist; und
eine Klammer;
wobei die Stützstange dazu konfiguriert ist, die Klammer (10, 110, 202, 310, 410, 510, 610, 710, 7110, 1400, 1600, 1700) zu empfangen, wobei die Klammer Folgendes umfasst:
ein Gehäuse (12, 112, 204, 580, 612, 712) einschließlich einer ersten und zweiten Spur (22, 123, 223, 328, 598, 628);
einen festen Greifer (322, 401, 403, 501, 713, 1705, 1706), der mit dem Gehäuse gekoppelt ist;
ein angetriebenes Element (710, 720, 7002), das dazu konfiguriert ist, innerhalb der ersten und zweiten Spur des Gehäuses zu gleiten;
einen beweglichen Greifer (1405, 1406, 1505, 1506, 1605, 1606, 1708), der mit dem angetriebenen Element wirkgekoppelt ist; und
einen Aktor, der dazu konfiguriert ist, das angetriebene Element zu einer ersten Position zu bewegen, wodurch der bewegliche Greifer zu dem festen Greifer bewegt wird, wobei der Aktor ferner dazu konfiguriert ist, das angetriebene Element zu einer zweiten Position zu bewegen, um dadurch den beweglichen Greifer von dem festen Greifer weg zu bewegen;
wobei die Klammer dazu konfiguriert ist, eine medizinische Vorrichtung (1920, 7360, 1401) mit der Stützstange der Gestelleinrichtung zu koppeln; und
wobei ein Verbinder (1970, 5022, 7114) für eine medizinische Vorrichtung auf der Klammer angeordnet ist und dazu konfiguriert ist, operativ in wenigstens einen des wenigstens einen Halterungsverbinders einzugreifen.

2. Gestelleinrichtung nach Anspruch 1, wobei der Verbinder (1970, 5022, 7114) für eine medizinische Vorrichtung dazu konfiguriert ist, operativ in den wenigstens einen des wenigstens einen Halterungsverbinders einzugreifen, um eine Netzwerkverbindung und/oder Leistung für die medizinische Vorrichtung zu erhalten.

3. Gestelleinrichtung nach Anspruch 2, wobei wenigstens eines des wenigstens einen Ausrichtungsmerkmals dazu konfiguriert ist, den Verbinder für eine medizinische Vorrichtung mit dem wenigstens einen des wenigstens einen Halterungsverbinders auszurichten.

4. Gestelleinrichtung nach Anspruch 2, wobei wenigstens eines des wenigstens einen Ausrichtungsmerkmals auf einer Manschette (7022) auf der Stützstange enthalten ist.

5. Gestelleinrichtung nach Anspruch 2, wobei die Gestelleinrichtung ferner ein Leistungssystem umfasst.

6. Gestelleinrichtung nach Anspruch 5, wobei das Leistungssystem dazu konfiguriert ist, Leistung über den wenigstens einen Halterungsverbinder an wenigstens eine medizinische Vorrichtung zu liefern.

7. Gestelleinrichtung nach Anspruch 2, wobei die Gestelleinrichtung ferner ein Kommunikationssystem umfasst, das dazu konfiguriert ist, zu ermöglichen, dass eine angebrachte medizinische Vorrichtung über den wenigstens einen Halterungsverbinder mit wenigstens einer anderen angebrachten medizinischen Vorrichtung kommuniziert.

8. Gestelleinrichtung nach Anspruch 7, wobei das Kommunikationssystem dazu konfiguriert ist, ein CANbus-Protokoll und/oder ein USB-Protokoll zu verwenden.

9. Gestelleinrichtung nach Anspruch 2, wobei das Basiselement Folgendes umfasst:
einen Leistungsverbinder (1872);
eine Leistungsversorgung (1870);
ein Hauptleistungskabel (1876), das den Leistungsverbinder und die Leistungsversorgung elektrisch verbindet; und
wenigstens ein Übertragungskabel (1874), das die Leistungsversorgung und den wenigstens einen Halterungsverbinder verbindet.

10. Gestelleinrichtung nach Anspruch 2, wobei die Gestelleinrichtung dazu konfiguriert ist, mit einem Infusionsständer gekoppelt zu werden.

11. Gestelleinrichtung nach Anspruch 2, wobei die wenigstens eine Stützstange ein Infusionsständer ist.

12. Gestelleinrichtung nach Anspruch 2, wobei das Basiselement ferner wenigstens ein Rad (7215) umfasst.

13. System, das eine Gestelleinrichtung nach Anspruch 1 umfasst, wobei der Verbinder für eine medizinische Vorrichtung auf dem festen Greifer oder dem beweglichen Greifer der Klammer angeordnet ist.

14. System nach Anspruch 13, wobei wenigstens eines des wenigstens einen Ausrichtungsmerkmals dazu konfiguriert ist, den Verbinder für eine medizinische Vorrichtung mit dem wenigstens einen des wenigstens einen Halterungsverbinders auszurichten.

15. System nach Anspruch 14, wobei der Verbinder für eine medizinische Vorrichtung dazu konfiguriert ist, operativ in den wenigstens einen des wenigstens einen Halterungsverbinders einzugreifen, um eine Netzwerkverbindung und/oder Leistung für eine medizinische Vorrichtung zu erhalten.

16. System nach Anspruch 15, wobei die medizinische Vorrichtung ein Überwachungs-Client ist, der einen Tablet-Computer umfasst.

## Revendications

1. Appareil portant, comprenant :
au moins une tige de support (1812, 6012, 7012) ayant une première portion d'extrémité et une deuxième portion d'extrémité ;
au moins l'un parmi un ensemble de pince (1814, 1990), un organe d'accrochage (7014, 7214) ou une poignée (6020, 7020) attaché(e) à la première portion d'extrémité de la tige de support ;
au moins un connecteur de fixation (1960, 1838, 2068, 3078, 6038, 7038, 7140, 7238) ;
au moins un élément d'alignement (7024) accouplé fonctionnellement à la tige de support ;
un organe de base (1816, 7016, 7216) attaché à la deuxième portion d'extrémité de la tige de support ; et
une pince ;
la tige de support étant configurée pour accepter la pince (10, 110, 202, 310, 410, 510, 610, 710, 7110, 1400, 1600, 1700), la pince comprenant :
un boîtier (12, 112, 204, 580, 612, 712) comportant des première et deuxième glissières (22, 123, 223, 328, 598, 628) ;
un organe de préhension fixe (322, 401, 403, 501, 713, 1705, 1706) accouplé au boîtier ;
un organe entraîné (710, 720, 7002) configuré pour coulisser à l'intérieur de la première et de la deuxième glissière du boîtier ;
un organe de préhension mobile (1405, 1406, 1505, 1506, 1605, 1606, 1708) accouplé fonctionnellement à l'organe entraîné ; et
un actionneur configuré pour déplacer l'organe entraîné vers une première position pour ainsi déplacer l'organe de préhension mobile vers l'organe de préhension fixe, l'actionneur étant en outre configuré pour déplacer l'organe entraîné vers une deuxième position pour ainsi déplacer l'organe de préhension mobile à l'écart de l'organe de préhension fixe ;
la pince étant configurée pour accoupler un dispositif médical (1920, 7360, 1401) à la tige de support de l'appareil portant ; et
un connecteur de dispositif médical (1970, 5022, 7114) étant disposé sur la pince et étant configuré pour venir en prise fonctionnellement avec au moins l'un de l'au moins un connecteur de fixation.

2. Appareil portant selon la revendication 1, dans lequel le connecteur de dispositif médical (1970, 5022, 7114) est configuré pour venir en prise fonctionnellement avec l'au moins un de l'au moins un connecteur de fixation pour recevoir au moins l'une d'une connexion de réseau et d'une alimentation électrique pour le dispositif médical.

3. Appareil portant selon la revendication 2, dans lequel au moins un de l'au moins un élément d'alignement est configuré pour aligner le connecteur de dispositif médical à l'au moins un de l'au moins un connecteur de fixation.

4. Appareil portant selon la revendication 2, dans lequel au moins l'un de l'au moins un élément d'alignement est inclus sur un collet (7022) sur la tige de support.

5. Appareil portant selon la revendication 2, l'appareil portant comprenant en outre un système d'alimentation électrique.

6. Appareil portant selon la revendication 5, dans lequel le système d'alimentation électrique est configuré pour alimenter en puissance au moins un dispositif médical par le biais de l'au moins un connecteur de fixation.

7. Appareil portant selon la revendication 2, l'appareil portant comprenant en outre un système de communication configuré pour permettre à un dispositif médical attaché de communiquer avec au moins un autre dispositif médical attaché par le biais de l'au moins un connecteur de fixation.

8. Appareil portant selon la revendication 7, dans lequel le système de communication est configuré pour utiliser au moins l'un d'un protocole CANbus et d'un protocole USB.

9. Appareil portant selon la revendication 2, dans lequel l'organe de base comprend :
un connecteur électrique (1872) ;
une alimentation électrique (1870) ;
un câble d'alimentation principal (1876) assurant la connexion électrique du connecteur électrique et de l'alimentation électrique ; et
au moins un câble de transmission (1874) assurant la connexion de l'alimentation électrique et de l'au moins un connecteur de fixation.

10. Appareil portant selon la revendication 2, l'appareil portant étant configuré pour s'accoupler à une tige à soluté.

11. Appareil portant selon la revendication 2, dans lequel l'au moins une tige de support est une tige à soluté.

12. Appareil portant selon la revendication 2, dans lequel l'organe de base comprend en outre au moins une roue (7215).

13. Système comprenant un appareil portant selon la revendication 1, dans lequel le connecteur de dispositif médical est disposé sur l'un de l'organe de préhension fixe et de l'organe de préhension mobile de la pince.

14. Système selon la revendication 13, dans lequel au moins un de l'au moins un élément d'alignement est configuré pour aligner le connecteur de dispositif médical à l'au moins un de l'au moins un connecteur de fixation.

15. Système selon la revendication 14, dans lequel le connecteur de dispositif médical est configuré pour venir en prise fonctionnellement avec l'au moins un de l'au moins un connecteur de fixation pour recevoir au moins l'une d'une connexion de réseau et d'une alimentation électrique pour un dispositif médical.

16. Système selon la revendication 15, dans lequel le dispositif médical est une supervision client comprenant un ordinateur de type tablette.
